(19) Europäisches Patentamt
European Patent Office
Office européen des brevets

(11) EP 2 032 535 B1

(12) **EUROPÄISCHE PATENTSCHRIFT**

(45) Veröffentlichungstag und Bekanntmachung des Hinweises auf die Patenterteilung:
**01.08.2012 Patentblatt 2012/31**

(21) Anmeldenummer: **07725450.6**

(22) Anmeldetag: **23.05.2007**

(51) Int Cl.:
***C07D 211/66*** (2006.01) ***C07D 213/26*** (2006.01)
***C07D 231/54*** (2006.01) ***C07D 263/52*** (2006.01)
***C07D 295/215*** (2006.01) ***C07D 295/26*** (2006.01)
***C07D 317/72*** (2006.01) ***C07D 471/10*** (2006.01)
***C07D 491/10*** (2006.01) ***C07D 498/10*** (2006.01)
***C07C 237/24*** (2006.01) ***A61K 31/438*** (2006.01)
***A61P 19/00*** (2006.01) ***A61P 35/00*** (2006.01)

(86) Internationale Anmeldenummer:
**PCT/EP2007/004550**

(87) Internationale Veröffentlichungsnummer:
**WO 2007/137738 (06.12.2007 Gazette 2007/49)**

(54) **SPIRO-CYCLISCHE NITRILE ALS PROTEASE-INHIBITOREN**

SPIROCYCLIC NITRILES AS PROTEASE INHIBITORS

NITRILES SPIROCYCLIQUES EN TANT QU'INHIBITEURS DE PROTÉASE

(84) Benannte Vertragsstaaten:
**AT BE BG CH CY CZ DE DK EE ES FI FR GB GR HU IE IS IT LI LT LU LV MC MT NL PL PT RO SE SI SK TR**
Benannte Erstreckungsstaaten:
**AL BA HR MK RS**

(30) Priorität: **01.06.2006 DE 102006025630**

(43) Veröffentlichungstag der Anmeldung:
**11.03.2009 Patentblatt 2009/11**

(73) Patentinhaber: **SANOFI**
**75008 Paris (FR)**

(72) Erfinder:
- **SCHUDOK, Manfred**
**65926 Frankfurt am Main (DE)**
- **WAGNER, Michael**
**65926 Frankfurt am Main (DE)**
- **BAUER, Armin**
**65926 Frankfurt am Main (DE)**
- **KOHLMANN, Anna**
**65926 Frankfurt am Main (DE)**

(74) Vertreter: **Then, Johann et al**
**Sanofi-Aventis**
**Département Brevets**
**174 Avenue de France**
**75013 Paris (FR)**

(56) Entgegenhaltungen:
**WO-A-2004/052921 US-B2- 6 720 319**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft substituierte carbo- und heterocyclische Spiro-Verbindungen der Formel Ia, die Thiol-Proteasen inhibieren, Verfahren zu ihrer Herstellung und Verwendung derselben als Arzneimittel.

Proteolytische Enzyme, bekannt als Proteasen und Peptidasen, sind sehr wichtige Enzyme, die etwa 2 % der Gene im menschlichen Organismus, pathogenen Mikroorganismen und auch anderen Lebensformen ausmachen. Ihre besondere Bedeutung liegt darin, dass sie viele physiologische Prozesse dadurch beeinflussen, in dem sie bei der Aktivierung, Synthese oder Abbau anderer Proteine eine wichtige Rolle spielen. Daraus ergibt sich zwangsläufig eine entscheidende regulatorische Funktion angefangen bei Empfängnis, Geburt, Wachstum, Reifung, Alterung, Erkrankungen bis hin zum Tod.

Die Balance der verschiedenen Prozesse ist von zentraler Bedeutung für das Leben und Überleben der Organismen. Kommt es durch endogene oder exogene Faktoren wie genetische Prädisposition oder Umweltfaktoren zu einer Disbalance proteasekatalysierter Prozesse, können massive Störungen im normalen Entwicklungsprozess, akute bis ernsthaft chronische Gesundheitsstörungen bis hin zu lebensbedrohlichen Erkrankungen auftreten.

Ebenso sind Proteasen essenziell und verantwortlich in Replikations- und Transmissionsprozessen viraler, bakterieller und sonstiger parasitärer Organismen, die etwa für Infektionskrankheiten verantwortlich sind und genauso natürlich für alle weiteren physiologischen und pathophysiologischen Prozesse im Pflanzen- und Tierreich.

Bedingt durch diese allgemeine große Bedeutung für unsere Gesundheit wurde bereits eine Vielzahl von Protease-Inhibitoren entwickelt, die sich auf dem Markt oder in allen Stadien der Entwicklung befinden: Nicht nur als Arzneistoffe, sondern auch als Diagnostika, Vakzine oder Nahrungsergänzungsstoffe.

Man unterscheidet 5 Klassen proteolytischer Enzyme, eingeteilt nach den für die enzymatische Hydrolyse relevanten katalytisch aktiven Resten: Aspartyl-, Serin-, Cystein-, Metallo- and Threonin-Proteasen. Inhibitoren aller dieser Klassen sind Gegenstand umfangreicher Arbeiten in einem weiten Feld zur Bekämpfung verschiedenartiger Erkrankungen. Etliche sehr effektive Protease-Inhibitoren befinden sich auf dem Markt, beispielsweise ACE-Inhibitoren, HIV-1-Protease-Inhibitoren, sowie Thrombin- und Elastase-Inhibitoren, gefolgt von einer großen Anzahl sich in klinischen Phasen befindenden Inhibitoren. Eine Zusammenfassung findet man etwa in Medicinal Chemistry, 2005, Vol. 1, No. 1, S. 71-104.

Cystein- (Thiol-) Proteasen werden in drei Hauptklassen eingeteilt: Papain-ähnliche, ICE-ähnliche (Caspasen) und Picornavirale Proteasen. Vom Mechanismus her erfolgt die Hydrolyse der Amid-Bindung in ähnlicher Weise wie bei der Klasse der Serin-Proteasen, über einen Angriff des Thiolat-Anions am Carbonyl-Kohlenstoff und Bildung eines tetrahedralen Übergangszustandes. Prominenteste Vertreter der Papain-Superfamilie als größter und bedeutendster Gruppe der Thiol-Proteasen sind die Cathepsine, die eine natürliche breite Verteilung in verschiedenen Geweben besitzen und denen eine wichtige Funktion sowohl in normalen physiologischen wie auch pathologischen Prozessen zukommt. Besonders hervorzuheben sind intrazelluläre Protein-Abbau- und -Remodelling-Prozesse. Entsprechend werden Cystein-Cathepsinen in folgenden generellen Formenkreisen Bedeutung zugeschrieben: muskuloskeletären Erkrankungen, besonders Knochen-Abbau-Erkrankungen, entzündlichen Erkrankungen, besonders Arthritiden, Atherosklerotischen Erkrankungen, Emphysemen, Dystrophien, Krebserkrankungen, Krankheiten des Zahn-Halteapparates, infektiösen Erkrankungen (viralen, parasitären und bakteriellen Infektionen), neurodegenerative Erkrankungen, Erkrankungen des Immunsystems, Ischämien, Leukodystrophien, Glomerulonephritis. Der Natur der Proteasen entsprechend werden die pathogenen Eigenschaften insbesondere durch drei übergeordnete Mechanismen ausgeübt: dem Abbau von (Binde-) Gewebe, wodurch vielfältige Symptomatiken und auch weitere Prozesse in Gang gesetzt werden, der Generierung von pathogenen oder bioaktiven Proteinen und Peptiden, die ihrerseits direkt oder in Signalkaskaden ihre Wirkung ausüben, sowie der Antigen-Prozessierung , beispielsweise der Präsentation von antigenen Peptiden an der ZellOberfläche, wodurch dann schließlich eine Immun-Antwort initiiert wird.

Bekannte Vertreter der Cystein-Cathepsine sind besonders Cathepsin B, H, K, L, F, V, W, X, O, C und S (A. J. Barrett; N. D. Rawlings; J. F. Woessner; Hrsg.; Handbook of Proteolytic Enzymes, 2nd. ed., 2004; Publisher: Elsevier, London).

Cathepsin F wurde in Makrophagen erstmals gefunden und ist in der Antigen-Prozession involviert. Bedingt durch das Auftreten in stimulierten Lungen-Makrophagen wurde eine wichtige Funktion in entzündlichen Atemwegserkrankungen postuliert.

Cathepsin L ist in normale lysosomale Proteolyse involviert, aber auch in verschiedene Krankheits-Geschehen wie Melanom-Metastatisierung.

Cathepsin S spielt eine Schlüsselrolle in vielen Prozessen, die im Rahmen der Antigen-Präsentation von Bedeutung sind und findet sich somit verstärkt in Antigen-präsentierenden Zellen. In dieser Hinsicht sind Inhibitoren von Cathepsin S möglicherweise aktive Agenzien in der Prävention, Inhibierung oder Behandlung von Immun- und Autoimmunerkrankungen. Weiterhin wird Cathepsin S auch von etlichen Antigen-präsentierenden Zellen sezerniert und spielt damit eine Rolle in extrazellulären Matrix-Interaktionen, die ebenfalls in vielen pathologischen Prozessen eine entscheidende Bedeutung besitzen. Hervorgehoben werden verschiedene (auto)-immun- und entzündlichen Erkrankungen; besonders die Alzheimer'sche Krankheit, Chorea-Huntington-Krankheit, juvenile Diabetes, Multiple Sklerose, Pemphigus vulgaris, Graves' Krankheit, Myasthenia gravis, systemischer Lupus erythematosus, IBD, Rheumatoide Arthritis und Hashimotos Thyroiditis, MS, ALS, allergische Erkrankungen wie Asthma, allogene Immunantworten, wie Abstoßungsreaktionen bei Organ-Transplantationen oder Gewebsverpflanzungen. Weiterhin wird Cathepsin S in Zusammenhang gebracht mit COPD (wie Emphysem), Bronchiolitis, exzessiver Atemwegs-Elastolyse bei Asthma und Bronchitis, Pneumonitis, aber auch mit cardiovaskulären Erkrankungen wie Plaque-Rupturen und Atheromen sowie Endometriose und chronischem neuropathischem Schmerz. Cathepsin S wird weiterhin mit fibrillären Erkrankungen in Verbindung gebracht und somit können Inhibitoren möglicherweise zur Behandlung systemischer Amyloidosis verwendet werden.

Erhöhte Spiegel von Cathepsin B und entsprechende Verteilungen finden sich in verschiedenen Tumoren - damit wird Cathepsin B eine Rolle bei der Tumor-Invasion und Metastatisierung zugeschrieben. Verstärkte Cathepsin-B-Aktivität wird ebenfalls in Rheumatoider Arthritis, Osteoarthritis, akuter Pankreatitis, entzündlichen Atemwegserkrankungen, Pneumocystisis Carinii und Knochen- oder Gelenkserkrankungen gefunden. Eine signifikante Erhöhung von synovialen Cathepsin B-Spiegeln konnte in Osteoarthritis-Modellen nachgewiesen werden. Einen Überblick über die Cytokinunabhängige Überexprimierung und die Relevanz für Osteoarthritis findet sich bei A. Baici et al., Seminars in Arthritis and Rheumatism, 34, 6, Suppl. 2, 24-28 (2005).

Cathepsin K-Expression ist besonders ausgeprägt (aber nicht ausschließlich) in Osteoclasten (z.B. D. Brömme et al., J. Biol. Chem. 271, 2126-32 (1996)) und repräsentiert dort etwa 98 % der Gesamt-Cystein-Protease Aktivität, lokalisiert hauptsächlich intrazellulär innerhalb der Lysosomen. Eine autosomal rezessive Störung der Cathepsin K-Expression (Fehlen durch Mutation), Pycnodysostosis, ist durch einen osteopetrotischen Phänotyp gekennzeichnet, mit reduzierter Knochenresorption, Verknöcherungsstörungen und massiven Wachstumsstörungen. Ebenso konnte mit Cathepsin K antisense-Nukleotiden sowie Knockout-Mäusen gezeigt werden, dass Cathepsin K verantwortlich für den Osteoclasten-vermittelten Knochen-Abbau ist. Deshalb geht man davon aus, dass eine Inhibierung von Cathepsin K zu reduzierter Knochenresorption führt und somit eine Therapiemöglichkeit für alle Krankheiten darstellen sollte, die durch einen erhöhten Knochenabbau gekennzeichnet sind, besonders also für die Behandlung von Osteoporose Bedeutung besitzen sollte. Bedingt durch eine signifikant erhöhte Aktivität im leicht sauren Bereich zwischen pH 4 und 8 erfolgt enzymatischer Abbau des Collagen-Netzwerkes im Knochen einhergehend mit acidolytischer Zersetzung der mineralischen Matrix. Hier ist besonders humanes Collagen Typ I als Hauptbestandteil der Proteine im Knochen betroffen; dieses ist nachgewiesenermaßen ein sehr gutes Substrat für Cathepsin K. Deshalb werden auch andere Erkrankungen, die mit erhöhter katabolischer Aktivität auf Collagen-Ebene einhergehen, mit Cathepsinen und besonders mit Cathepsin K in Verbindung gebracht. An vorderster Stelle ist hier Osteoarthritis zu nennen, gekennzeichnet durch eine Disbalance von Knorpel-Matrixaufbau und Abbau, verursacht durch katabolisch aktive Enzyme wie beispielsweise Metalloproteinasen, aber auch andere. Deshalb ist es nahe liegend und inzwischen auch nachgewiesen, dass eine Hemmung von Cathepsin K hier ebenfalls günstige Effekte auf das Krankeitsgeschehen haben dürfte (synoviale Fibroblastenvermittelter Collagen-Abbau durch Cathepsin K ist beschrieben in W.-S. Hou et al, Am. J. Pathol. 159, 2167-2177 (2001)). Die Bedeutung der Cathepsine K und S in muskuloskeletären Erkrankungen wie Osteoporose und Osteoarthritis ist im Detail beschrieben von D. Brömme et al., Advanced Drug Delivery Reviews 57, 973-993 (2005).

Bedingt durch die oben beschriebenen detaillierten Kenntnisse über Cystein-Cathepsine in verschiedenen Krankheitsgeschehen gelten diese als sehr viel versprechende Angriffspunkte in der Arzneimittelentwicklung, so dass eine intensive Suche nach spezifischen, gruppen-spezifischen oder auch unspezifischen Inhibitoren eingesetzt hat.

Inhibitoren der Cystein-Proteasen sind seit langem bekannt, beispielsweise die Cystatine als endogene Polypeptid-Inhibitoren. Niedermolekulare Inhibitoren wurden 1981 erstmals aus Aspergillus isoliert. Es handelt sich hierbei um potente irreversible Inhibitoren mit geringer Toxizität, aber auch mangelnder Spezifität, da neben den Cathepsinen B, K, L, S und H auch Calpaine breit gehemmt werden. Seitdem wurde eine Vielzahl von Inhibitoren mit unterschiedlichen Spezifitäten oder Mechanismen gefunden - daher wurden sowohl irreversibel kovalent bindende als auch reversibel kovalent bindende oder reversibel nicht kovalent bindende Inhibitoren gefunden oder synthetisiert. Jüngere Entwicklungen wurden zusammenfassend im Detail beschrieben (W. Kim, K. Kang, Expert Opin. Ther. Patents 13, 3, 419-32 (2002); U. B. Grabowska, Curr. Opin. Drug Disc Dev. 8, 5, 619-30 (2005); R. L. Thurmond et al., Curr. Opin. Invest. Drugs 6, 5, 473-482 (2005)).

Von besonderem Interesse sind reversibel kovalent bindende Inhibitoren. Aus dieser Gruppe konnte besonders die Klasse der Nitrile als sehr viel versprechend identifiziert werden. Diese werden im Detail beschrieben, beispielsweise in den Anmeldungen WO99/24460, WO2000/55125, WO2004/052921 sowie auch in WO2005/040142.

In dem Bestreben, wirksame Verbindungen zur Behandlung von Krankheiten zu finden, die durch Cystein-Cathepsine direkt oder indirekt verursacht werden, wurde nun gefunden, dass die erfindungsgemäßen Spiro-Verbindungen, Spiro-cyclische Nitrile, starke Inhibitoren der Cystein-Cathepsine, besonders von Cathepsin K und/oder S, sind, während andere Cystein-Proteasen wie Calpain deutlich schwächer oder überhaupt nicht gehemmt werden. Ferner weisen die erfindungsgemäßen Verbindungen eine verbesserte Bioverfügbarkeit auf, was sich auch schon in vitro in entsprechenden Caco-Permeabilitätstests zeigt.

Die Erfindung betrifft daher eine Verbindung und/oder alle stereoisomeren Formen der Verbindung der Formel Ia und/oder Gemische dieser Formen in jedem Verhältnis, und/oder ein physiologisch verträgliches Salz der Verbindung der Formel Ia und/oder Solvate oder Hydrate der Verbindung der Formel Ia wobei der Rest

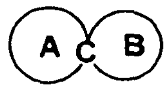

für eine Spiroverbindung steht, worin die Teilringe

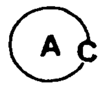

und

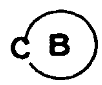

jeweils gleich oder verschieden sind und unabhängig voneinander für

a) ein gesättigtes oder teilweise gesättigtes -($C_3$-$C_{11}$)-Cycloalkyl stehen, worin Cycloalkyl unverbrückt, verbrückt oder annelliert ist und unsubstituiert oder unabhängig voneinander je nach Ringgröße ein-, zwei-, drei-, vier oder fünffach durch R4 substituiert ist, oder
b) einen gesättigten oder teilweise gesättigten drei- bis elfgliedrigen Heterocyclus stehen, die je nach Ringgröße ein, zwei, drei oder vier gleiche oder verschiedene Heteroatome aus der Reihe Sauerstoff, Stickstoff oder Schwefel enthalten können und worin der Heterocyclus unverbrückt, verbrückt oder annelliert ist und unsubstituiert oder unabhängig voneinander je nach Ringgröße ein-, zwei-, drei-, vier oder fünffach durch R4 substituiert ist, wobei

der Formel Ia,

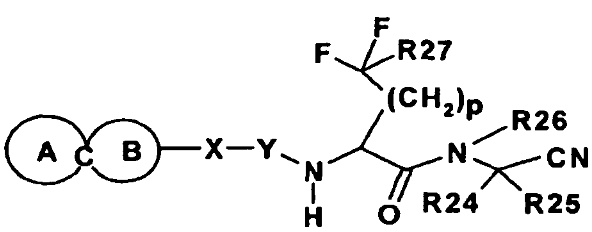

(Ia)

,

R4 für-$NO_2$, -CN, =O, =S, -OH, -$CF_3$, -$SF_5$, -($C_0$-$C_3$)-Alkylen-S-R10, -Si-$(CH_3)_3$, -O-$CF_3$, -S-$CF_3$, -($C_0$-$C_5$)-Alkylen-O-C(O)-R21, -($C_0$-$C_5$)-Alkylen-C(O)-O-R10, -($C_0$-$C_3$)-Alkylen-O-R10, -($C_0$-$C_3$)-Alkylen-N(R21)-R22, -($C_0$-$C_3$)-Alkylen-N(R10)-S($O_2$)-R10, -($C_0$-$C_5$)-Alkylen-($C_3$-$C_8$)-Cycloalkyl-R23, -($C_0$-$C_5$)-Alkylen-($C_1$-$C_3$)-Fluoralkyl, -($C_0$-$C_5$)-Alkylen-N(R10)-C(O)-R21, -($C_0$-$C_3$)-Alkylen-C(O)-N(R21)-R22, -($C_0$-$C_4$)-Alkyl, wobei Alkyl unsubstituiert oder ein-, zwei- oder dreifach unabhängig voneinander durch R9 substituiert ist, -($C_0$-$C_4$)-Alkylen-Aryl, wobei Aryl ausgewählt ist aus der Gruppe Phenyl, Indanyl, Indenyl, Naphthyl, wobei Aryl unsubstituiert oder ein-, zwei- oder dreifach unabhängig voneinander durch R8 substituiert ist, oder- ($C_0$-$C_4$)-Alkylen-Het, wobei Het ausgewählt ist aus der Gruppe Azetidinyl, Benzimidazolinyl, Benzimidazolyl, Benzofuranyl, Benzothiophenyl, Benzoxazolyl, Benzthiazolyl, Benzisoxazolyl, Benzisothiazolyl, Chinolinyl, Dioxolyl, Dioxanyl, Furanyl, Imidazolidinyl, Imidazolinyl, Imidazolyl, Indolinyl, Indolyl, 3H-Indolyl, Isoindolinyl, Isoindolyl, Isochinolinyl, Isothiazolidinyl, 2-Isothiazolinyl, Isothiazolyl, Isoxazolyl, Isoxazolidinyl, 2-Isoxazolinyl, Morpholinyl, Octahydroisochinolinyl, Oxazolyl, Oxazolidinyl, Pyrimidinyl, Piperazinyl, Piperidinyl, Pyranyl, Pyrazinyl, Pyrazolinyl, Pyrazolyl, Pyridazinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, Thiazolyl, Thienyl, Thienopyridinyl, Thiomorpholinyl, Thiophenyl, ist und dieser Het-Rest unsubstituiert oder unabhängig voneinander ein-, zwei- oder dreifach durch R8 substituiert ist, steht,

| | |
|---|---|
| R8 | für Halogen, Carbamimidoyl, $-NO_2$, =O, $-CF_3$, $-SF_5$, -C(O)-O-R10, -CN, $-C(O)-NH_2$, -OH, $-NH_2$, $-O-CF_3$, -C(O)-N(R10)-R20, -N(R10)-R20, $-(C_3-C_8)$-Cycloalkyl, $-O-(C_1-C_8)$-Alkyl, $-O-(C_0-C_4)$-Alkylen-$(C_3-C_6)$-Cycloalkyl, $(C_1-C_8)$-Alkyl, $-(C_0-C_4)$-Alkylen-$(C_3-C_8)$-Cycloalkyl, wobei die genannten Alkylreste jeweils unsubstituiert oder ein-, zwei- oder dreifach unabhängig voneinander durch Halogen, $NH_2$, -OH, $-O-CH_3$, $-SO_2-CH_3$ oder-$SO_2-CF_3$ substituiert sind, steht, |
| R9 | für Halogen, $-NO_2$, -CN, =O, -OH, $-CF_3$, -C(O)-O-R10, -C(O)-N(R21)-R22, -N(R21)-R22, $-(C_3-C_8)$-Cycloalkyl, $-(C_0-C_3)$-Alkylen-O-R10, $-Si-(CH_3)_3$, $-N(R^{10})-S(O)_u$-R10, wobei u die ganze Zahl 1 oder 2 bedeutet, -S-R10, $-SO_r$-R10, wobei r die ganze Zahl 1 oder 2 bedeutet, $-S(O)_v$-N(R10)-R20, wobei v die ganze Zahl 1 oder 2 bedeutet, -C(O)-R10, $-(C_1-C_8)$-Alkyl, $-(C_1-C_8)$-Alkoxy, Phenyl, Phenyloxy-, $-(C_1-C_3)$-Fluoralkyl, -O-R19, -NH-C(O)-NH-R10, $-(C_0-C_4)$-Alkyl-C(O)-O-C(R11, R19)-O-C(O)-R12, -NH-C(O)-NH-R21, -N(R21)-C(O)-R22, $-(C_0-C_4)$-Alkyl-C(O)-O-C(R11, R19)-O-C(O)-O-R12, -NH-C(O)-O-R10, $-O-CF_3$ oder Het, wobei Het wie oben definiert ist und unsubstituiert oder ein-, zwei- oder dreifach unabhängig voneinander durch R8 substituiert ist, steht, |
| R10 und R20 | sind gleich oder verschieden und stehen unabhängig voneinander für Wasserstoffatom, $-(C_1-C_6)$-Alkyl, $-(C_0-C_4)$-Alkyl-OH, $-(C_1-C_3)$-Fluoralkyl, $-(C_0-C_4)$-Alkyl-O-$(C_1-C_4)$-Alkyl, $-(C_0-C_5)$-Alkyl-$(C_3-C_8)$-Cycloalkyl, $-(C_0-C_2)$-Alkylen-Aryl, wobei Aryl wie oben definiert ist und unsubstituiert oder ein-, zwei- oder dreifach unabhängig voneinander durch $-(C_1-C_6)$-Alkyl, $-O-(C_1-C_6)$-Alkyl, Halogen oder $-(C_3-C_8)$-Cycloalkyl substituiert ist, oder $-(C_0-C_2)$-Alkylen-Het, wobei Het wie oben definiert ist und unsubstituiert oder ein-, zwei- oder dreifach unabhängig voneinander durch $-(C_1-C_6)$-Alkyl, $-O-(C_1-C_6)$-Alkyl, Halogen oder $-(C_3-C_8)$-Cycloalkyl substituiert ist, |
| R11 und R19 | gleich oder verschieden sind und unabhängig voneinander für Wasserstoffatom oder $-(C_1-C_6)$-Alkyl stehen, |
| R12 | für $-(C_1-C_6)$-Alkyl, $-(C_1-C_6)$-Alkyl-OH, $-(C_1-C_6)$-Alkyl-O-$(C_1-C_6)$-Alkyl, $-(C_3-C_8)$-Cycloalkyl, $-(C_1-C_6)$-Alkyl-O-$(C_1-C_8)$-Alkyl-$(C_3-C_8)$-Cycloalkyl, $-(C_1-C_6)$-Alkyl-$(C_3-C_8)$-Cycloalkyl, wobei der Cycloalkylrest unsubstituiert oder ein-, zwei- oder dreifach unabhängig voneinander durch -OH, $-O-(C_1-C_4)$-Alkyl oder R10 substituiert ist, steht, |
| R21 und R22 | sind gleich oder verschieden und stehen unabhängig voneinander für Wasserstoffatom, $-(C_1-C_6)$-Alkyl, wobei Alkyl unsubstituiert oder ein-, zwei-oder dreifach unabhängig voneinander durch R8 substituiert ist, $-(C_0-C_6)$-Alkylen-$(C_3-C_8)$-Cycloalkyl, $-SO_t$-R10, wobei t die ganze Zahl 1 oder 2 bedeutet, $-(C_1-C_3)$-Fluoralkyl, -O-R12, $-(C_0-C_6)$-Alkylen-Aryl, wobei Aryl wie oben definiert ist und Alkylen und Aryl unsubstituiert oder ein-, zwei- oder dreifach unabhängig voneinander durch R8 substituiert sind oder $-(C_0-C_6)$-Alkylen-Het, wobei Het wie oben definiert ist und Alkylen und Het unsubstituiert oder ein-, zwei- oder dreifach unabhängig voneinander durch R8 substituiert sind, |
| R21 und R22 | zusammen mit dem Stickstoffatom an das sie gebunden sind einen vier- bis achtgliedrigen monocyclischen heterocyclischen Ring bilden, der neben dem Stickstoffatom zusätzlich noch je nach Ringgröße ein oder zwei gleiche oder verschiedene Heteroatome aus der Reihe Sauerstoff, Stickstoff oder Schwefel enthalten kann und worin der Heterocyclus unsubstituiert oder ein-, zwei- oder dreifach unabhängig voneinander durch R8 substituiert ist, |
| R23 | für Wasserstoffatom, -OH oder $-O-(C_1-C_4)$-Alkyl steht, |
| X | für eine kovalente Bindung, -N(R7)- oder -O- steht, wobei |
| R7 | für Wasserstoffatom, $-(C_0-C_4)$-Alkylen-$(C_3-C_6)$-Cycloalkyl oder $-(C_1-C_4)$-Alkyl steht, |
| Y | für -C(O)-, -C(S)- oder $-S(O_2)$- steht, |
| p | für die ganze Zahl 1 oder 2 steht, |
| R27 | für Wasserstoffatom, $-(C_1-C_6)$-Alkyl, $(C_0-C_4)$-Alkylen-$(C_3-C_6)$-Cycloalkyl, Halogen, $-(C_0-C_4)$-Alkylen-Het, wobei Het wie oben definiert ist und unsubstituiert oder durch Halogen, $-(C_1-C_6)$-Alkyl, $-O-(C_1-C_3)$-Fluoralkyl oder $-O-(C_1-C_6)$-Alkyl substitutiert ist, oder-$(C_0-C_2)$-Alkylen-Phenyl steht, wobei Phenyl unsubstituiert oder durch Halogen, $-(C_1-C_6)$-Alkyl, $-O-(C_1-C_3)$-Fluoralkyl oder $-O-(C_1-C_6)$-Alkyl substitutiert ist, |
| R26 | für Wasserstoffatom, $-(C_1-C_4)$-Alkyl oder $-(C_0-C_4)$-Alkylen-$(C_3-C_6)$-Cycloalkyl steht, |
| R24 | und R25 sind gleich oder verschieden und stehen unabhängig voneinander für Wasserstoffatom, $-(C_1-C_6)$-Alkyl, $-(C_0-C_4)$-Alkylen-$(C_3-C_6)$-Cycloalkyl, $-(C_1-C_3)$-Fluoralkyl, $-(C_0-C_4)$-Alkylen-Aryl, wobei Aryl wie oben definiert ist und unsubstituiert oder ein-, zwei- oder dreifach unabhängig voneinander durch R8 substituiert ist, oder $-(C_0-C_4)$-Alkylen-Het, wobei Het wie oben definiert ist und unsubstituiert oder ein-, zwei- oder dreifach unabhängig voneinander durch R8 substituiert ist, |
| R24 | und R25 bilden zusammen mit dem Kohlenstoffatom an das sie gebunden sind einen drei- bis sechsgliedrigen Cycloalkylring, der unsubstituiert oder ein-, zwei- oder dreifach unabhängig voneinander durch R10 oder Fluor substituiert ist, |

R24 und R25 bilden zusammen mit dem Kohlenstoffatom an das sie gebunden sind einen drei- bis sechsgliedrigen Hetero-Cycloalkylrest, der unsubstituiert oder ein-, zwei- oder dreifach unabhängig voneinander durch R10 oder Fluor substituiert ist.

3) Ein weiterer Gegenstand der Erfindung ist die Verbindung der Formel Ia, wobei die Teilringe

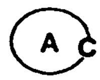

und

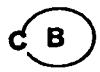

jeweils ausgewählt wurden aus Cyclopropan, Cyclobutan, Cyclopentan, Cyclohexan, Cycloheptan, Cyclooctan, Bicyclo[4.2.0]octan, Octahydro-inden, Decalin, Decahydro-benzocyclohepten, Dodecahydro-heptalen, Bicyclo [3.1.1]heptan, Bicyclo[2.2.1]heptan, Bicyclo[3.3.0]octan, Bicyclo[2.2.2]octan, Spiro[2.5]octan, Spiro[3.4]octan, Azepan, Azepin, Azetidin, Aziridin, Azirin, Azocan, Benzimidazolin, 2,3-Dihydro-benzo[b]thiophen, 1,3-Dihydro-benzo [c]thiophen, 2,3-Dihydro-benzofuran, 2,3-Dihydro-benzooxazol, 2,3-Dihydro-benzothiazol, 1,3-Dihydro-isobenzofuran, 4,5-Dihydro-isothiazol, 2,3-Dihydro-isoxazol, 2,5-Dihydro-isoxazol, 4,5-Dihydro-isoxazol, 5,6-Dihydro-4H-[1,2]oxazin, Benzo[1,3]dioxol, 1,4-Diazepan, 1,2-Diazepin, 1,3-Diazepin, 1,4-Diazepin, Diaziridin, Diazirin, 1,4-Diazocan, Dioxan, 1,3-Dioxan, Dioxazin, [1,3]Dioxepan, 1,4-Diozocan, Dioxol, Dioxolan, 1,3-Dioxolan, 1,3-Dioxolen, [1,3]Dithian, [1,3]Dithiolan, Hexahydro-pyridazin, Hexahydro-pyrimidin, Imidazolin, Imidazolidin, Indan, Indolin, Isoindolin, Isothiazolidin, Isothiazolin, Isoxazolin, Isoxazolidin, 2-Isoxazolin, Morpholin, [1,3,4]Oxadiazinan, [1,3,5] Oxadiazinan, [1,2,3]Oxadiazolidin, [1,3,4]Oxadiazolidin, 1,2-Oxa-thiepan, 1,2-Oxathiolan, [1,3]Oxathiolan, 1,4-Oxazepan, 1,2-Oxazin, 1,3-Oxazin, 1,4-Oxazin, Oxazinan, 1,3-Oxazinan, Oxazocane, Oxaziridin, Oxazolidin, Oxepan, Oxetan, Oxiran, Oxocane, Piperazin, Piperidin, Pyran, Pyrazolin, Pyrazolidin, Pyrrolidin, Pyrrolidinon, Pyrrolin, Tetrahydrochinolin, Tetrahydrofuran, Tetrahydroisochinolin, 1,2,3,4-Tetrahydro-naphthalen, Tetrahydropyran, Tetrahydropyridin, 1,2,3,4-Tetrahydro-pyrimidin, 1,2,5,6-Tetrahydro-pyrimidin, Tetrahydro-thiophen, Tetrazin, Thiadiazin, [1,2,6]Thiadiazinan, [1,3,4]Thiadiazolidin, 1,2-Thiazin, 1,3-Thiazin, 1,4-Thiazin, [1,2]Thiazinan, [1,3]Thiazinan, Thiazolidin, Thiazolin, Thiepan, Thietan, Thiomorpholin, Thiopyran, 1,2,3-Triazin, 1,2,4-Triazin, 1,3,5-Triazin, [1,2,4] Triazinan oder [1,2,4]Triazolidin, und worin die beiden

2) Ein weiterer Gegenstand der Erfindung ist die Verbindung der Forme Ia wobei der Teilring

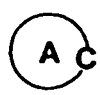

aus der folgenden Gruppe ausgewählt wurde

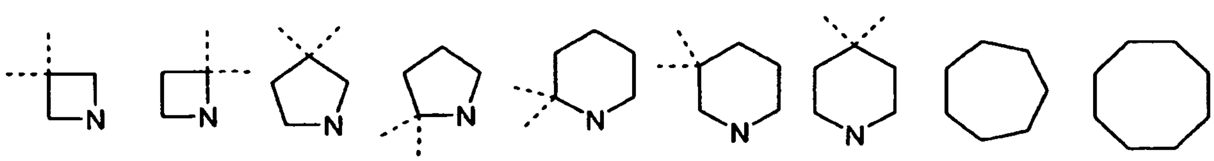

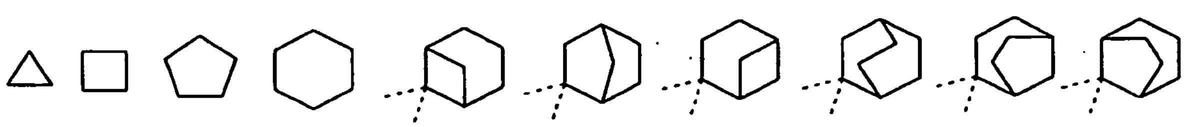

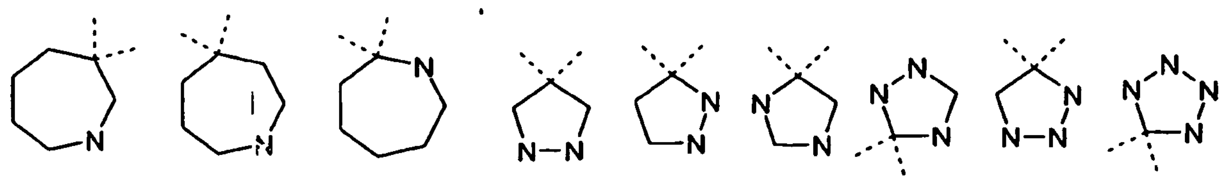

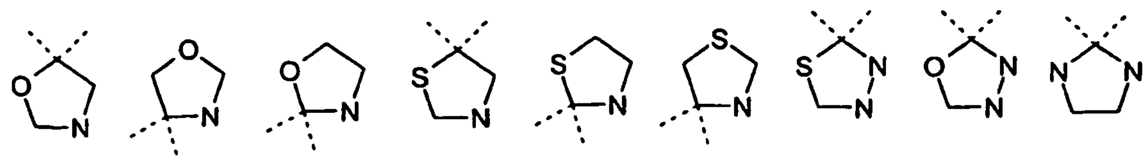

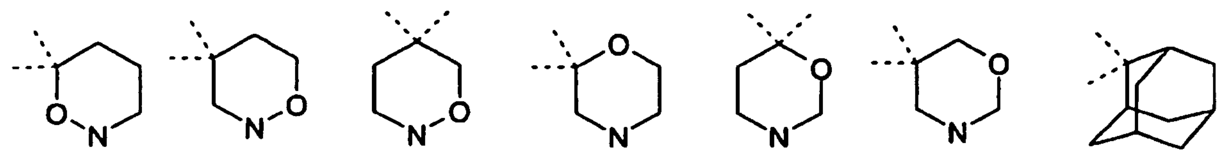

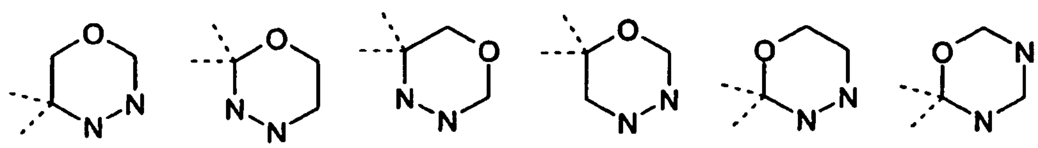

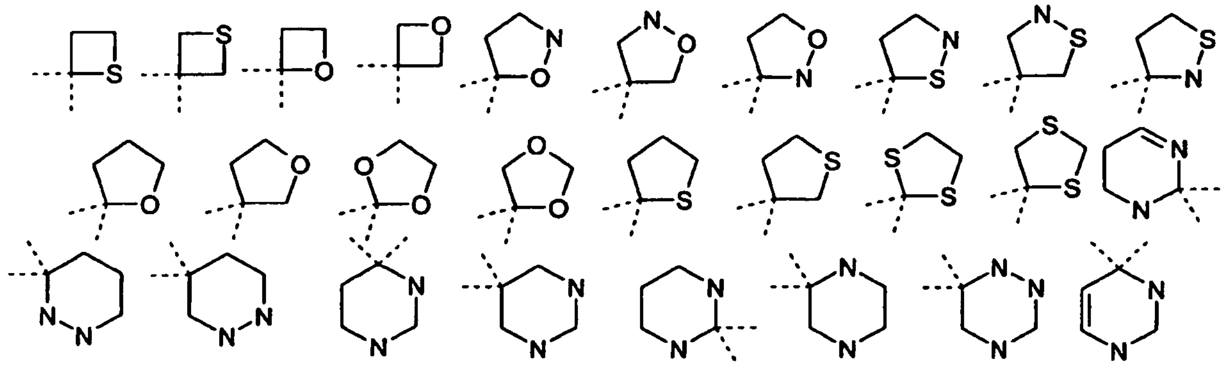

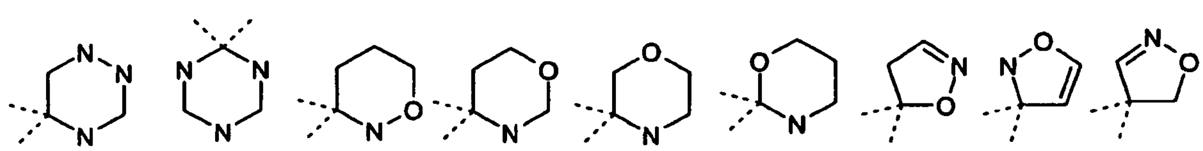

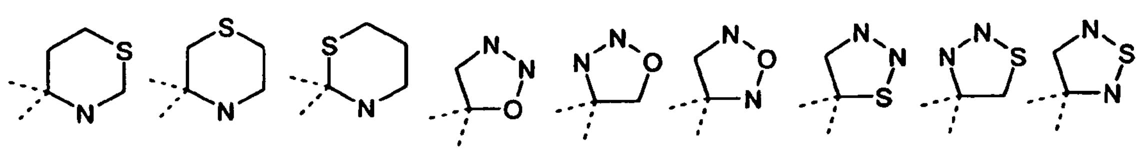

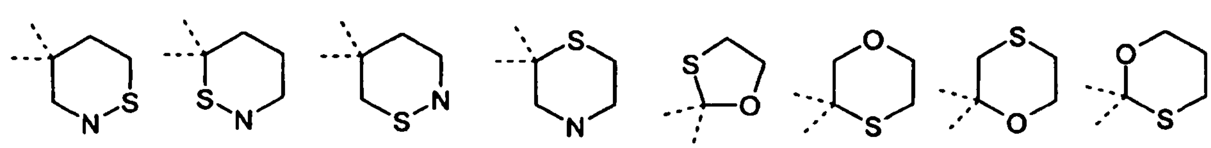

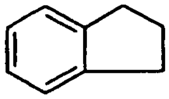 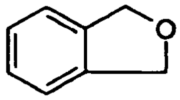 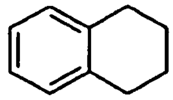

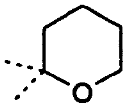 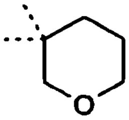 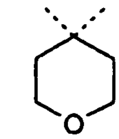 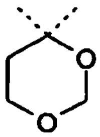 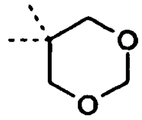 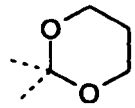 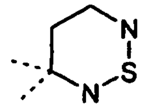

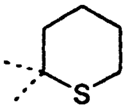 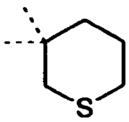 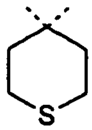 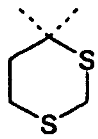 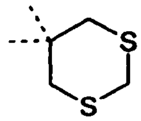 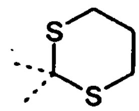 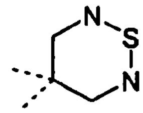

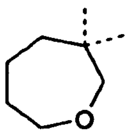 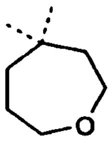 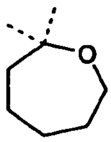 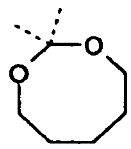 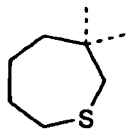 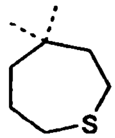 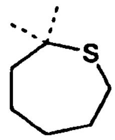 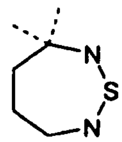

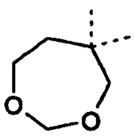 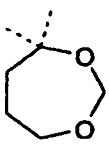 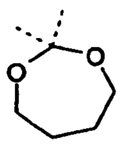 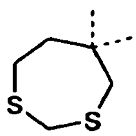 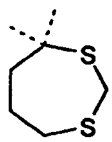 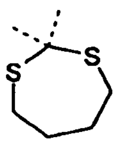 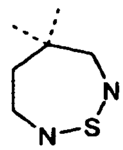

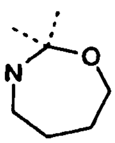 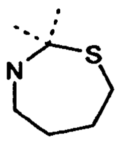 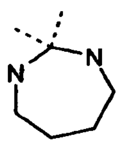 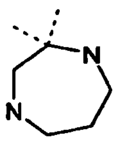 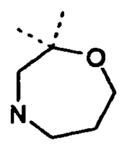 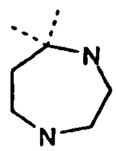 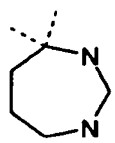

wobei die gestrichelten Linien den jeweiligen Anknüpfungspunkt zum zweiten Teilring darstellen, Einfachbindungen in den aufgeführten Strukturen zum Teil durch Doppelbindungen ersetzt sein können oder weitere Ringsysteme ankondensiert sein können, und
worin der Teilring

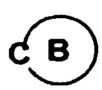

aus der folgenden Gruppe ausgewählt wurde

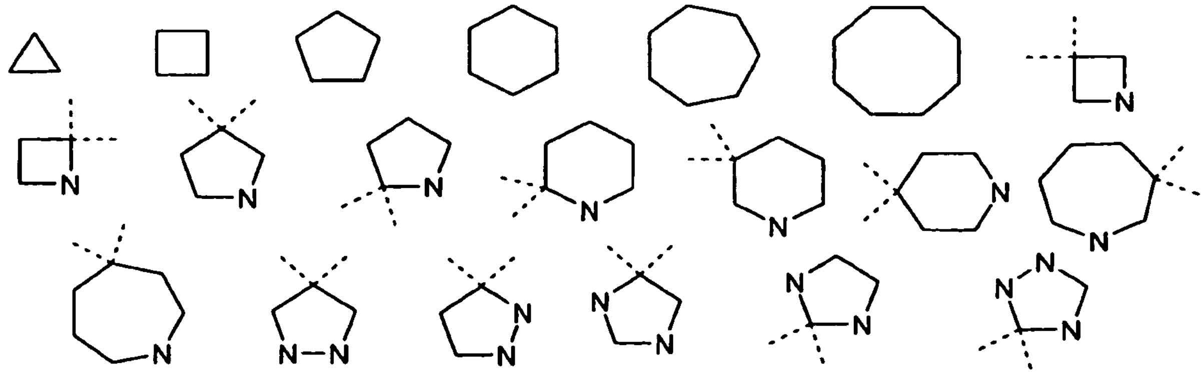

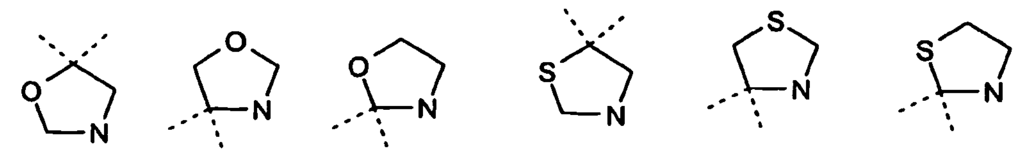

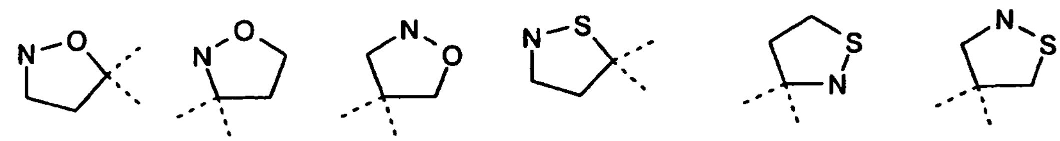

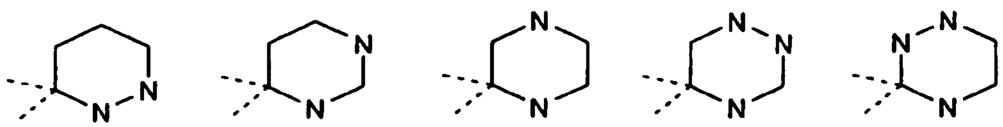

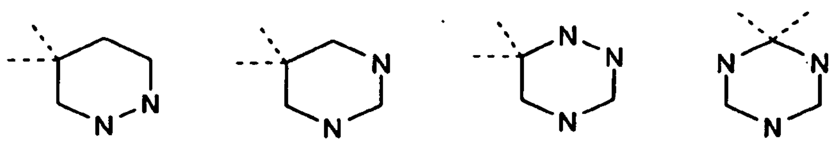

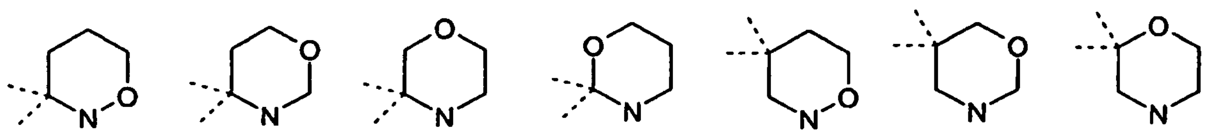

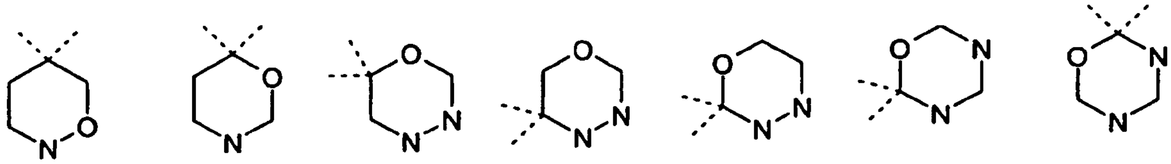

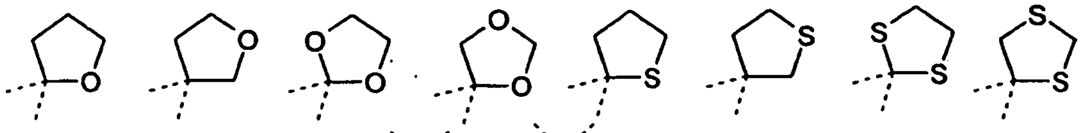

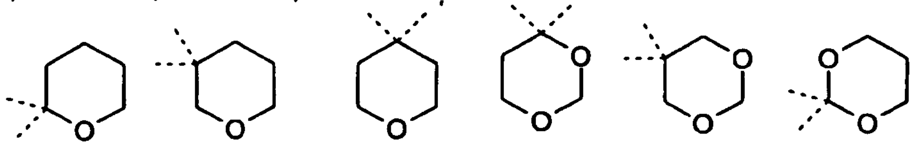

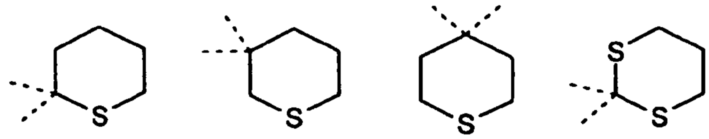

wobei die gestrichelten Linien den jeweiligen Anknüpfungspunkt zum zweiten Teilring darstellen, Einfachbindungen in den aufgeführten Strukturen zum Teil durch Doppelbindungen ersetzt sein können und die beiden Teilringe A und B unsubstituiert oder unabhängig voneinander einfach bis vierfach durch R4 substituiert sind, und die Reste X, Y, R27, p, R26, R24, R25 und R4 die oben genannte Bedeutung aufweisen.
Teilringe jeweils unsubstituiert oder unabhängig voneinander je nach Ringgröße ein-, zwei-, drei-, vier oder fünffach durch R4 substituiert sind,
und die Reste X, Y, R27, p, R26, R24, R25 und R4 wie unter 4) definiert sind.

4) Ein weiterer Gegenstand der Erfindung ist die Verbindung der Formel Ia, wobei

die Teilringe

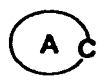

und

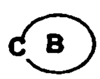

unabhängig voneinander jeweils ausgewählt werden aus der Gruppe Azetidin, Cyclopropyl, Cyclobutyl, Cyclopentyl, Cyclohexyl, 1,3-Dihydro-isobenzofuran, 2,3-Dihydro-isoxazol, 2,5-Dihydro-isoxazol, 4,5-Dihydro-isoxazol, 1,3-Dioxan, Dioxolan, 1,3-Dioxolan, Imidazolidin, Indan, Morpholin, 1,3-Oxazinan, Oxazolidin, Piperazin, Piperidin, Pyrrolidin, Tetrahydrofuran, und 1,2,3,4-Tetrahydronaphthalen, und worin die beiden Teilringe unsubstituiert oder unabhängig voneinander je nach Ringgröße ein-, zwei- oder dreifach durch R4 substituiert sind,

R4 für=O, =S, -($C_0$-$C_3$)-Alkylen-C(O)-O-R10, -($C_0$-$C_3$)-Alkylen-N(R21)-R22, -($C_0$-$C_3$)-Alkylen-NH-C(O)-R21, -($C_0$-$C_4$)-Alkylen-($C_3$-$C_6$)-Cycloalkyl-R23, -($C_0$-$C_3$)-Alkylen-O-R10, -($C_0$-$C_4$)-Alkylen-Phenyl, wobei Phenyl unsubstituiert oder ein-, zwei- oder dreifach unabhängig voneinander durch R8 substituiert ist, oder -($C_0$-$C_4$)-Alkyl, wobei Alkyl unsubstituiert oder ein-, zwei- oder dreifach unabhängig voneinander durch R9 substituiert ist, steht,

R8 für Fluor, Chlor, Brom, -O-($C_1$-$C_3$)-Fluoralkyl oder -O-($C_1$-$C_4$)-Alkyl steht,

R9 für Halogen, $-NO_2$, -CN, =O, -OH, $-CF_3$, -C(O)-O-R10, -C(O)-N(R21)-R22, -N(R21)-R22, -($C_3$-$C_8$)-Cycloalkyl, -($C_0$-$C_3$)-Alkylen-O-R10, -Si-$(CH_3)_3$, -N(R10)-S$(O)_u$-R10, wobei u die ganze Zahl 1 oder 2 bedeutet, -S-R10, $-SO_r$-R10, wobei r die ganze Zahl 1 oder 2 bedeutet, -S$(O)_v$,-N(R10)-R20, wobei v die ganze Zahl 1 oder 2 bedeutet, -C(O)-R10, -($C_1$-$C_8$)-Alkyl, -($C_1$-$C_8$)-Alkoxy, Phenyl, Pheny-

loxy-, -($C_1$-$C_3$)-Fluoralkyl, -O-R19, -NH-C(O)-NH-R10, -($C_0$-$C_4$)-Alkyl-C(O)-O-C(R11,R19)-O-C(O)-R12, -NH-C(O)-NH-R21, -N(R21)-C(O)-R22, -($C_0$-$C_4$)-Alkyl-C(O)-O-C(R11, R19)-O-C(O)-O-R12, -NH-C(O)-O-R10 oder -O-$CF_3$, steht,

R10 und R20 gleich oder verschieden sich und unabhängig voneinander für Wasserstoffatom oder -($C_1$-$C_6$)-Alkyl stehen,

R11 und R19 sind gleich oder verschieden und stehen unabhängig voneinander für Wasserstoffatom oder -($C_1$-$C_6$)-Alkyl,

R12 für -($C_1$-$C_6$)-Alkyl, -($C_1$-$C_6$)-Alkyl-OH, -($C_1$-$C_6$)-Alkyl-O-($C_1$-$C_6$)-Alkyl, -($C_3$-$C_8$)-Cycloalkyl, -($C_1$-$C_6$)-Alkyl-O-($C_1$-$C_8$)-Alkyl-($C_3$-$C_8$)-Cycloalkyl, -($C_1$-$C_6$)-Alkyl-($C_3$-$C_8$)-Cycloalkyl, wobei der Cycloalkylrest unsubstituiert oder ein-, zwei- oder dreifach unabhängig voneinander durch -OH, -O-($C_1$-$C_4$)-Alkyl oder R10 substituiert ist, steht,

R21 und R22 sind gleich oder verschieden und stehen unabhängig voneinander für Wasserstoffatom, -($C_1$-$C_6$)-Alkyl, -($C_0$-$C_6$)-Alkylen-($C_3$-$C_8$)-Cycloalkyl, -O-R12, -$SO_t$-R10, wobei t die ganze Zahl 1 oder 2 bedeutet, oder -($C_1$-$C_3$)-Fluoralkyl,

R23 für Wasserstoffatom, -OH oder -O-($C_1$-$C_4$)-Alkyl steht,

X für eine kovalente Bindung oder -N(R7)- steht, wobei

R7 für Wasserstoffatom oder -($C_1$-$C_4$)-Alkyl steht,

Y für -C(O)- oder -S($O_2$)- steht,

p für die ganze Zahl 1 oder 2 steht,

R26 für Wasserstoffatom steht,

R27 für Wasserstoffatom, -($C_1$-$C_6$)-Alkyl, ($C_0$-$C_4$)-Alkylen-($C_3$-$C_6$)-Cycloalkyl, -($C_0$-$C_2$)-Alkylen-Phenyl, wobei Phenyl unsubstituiert oder durch Halogen, -($C_1$-$C_6$)-Alkyl, -O-($C_1$-$C_3$)-Fluoralkyl oder -O-($C_1$-$C_6$)-Alkyl substitutiert ist, oder -($C_0$-$C_2$)-Alkylen-Pyridyl steht,

R24 und R25 sind gleich oder verschieden und stehen unabhängig voneinander für Wasserstoffatom, -($C_1$-$C_4$)-Alkyl oder -($C_0$-$C_4$)-Alkylen-($C_3$-$C_6$)-Cycloalkyl,

R24 und R25 bilden zusammen mit dem Kohlenstoffatom an das sie gebunden sind einen Cycloalkylring ausgewählt aus der Gruppe Cyclopropyl, Cyclobutyl, Cyclopentyl oder Cyclohexyl, der unsubstituiert oder ein-, zwei- oder dreifach unabhängig voneinander durch R10 oder Fluor substituiert ist,

R24 und R25 bilden zusammen mit dem Kohlenstoffatom an das sie gebunden sind einen drei- bis sechsgliedrigen Hetero-Cycloalkylrest, ausgewählt aus der Gruppe Aziridin, Azetidin, Diazetidin, Diaziridin, Hexohydropyridazin, Hexohydropyrimidin, Imidazolidin, Morpholin, Oxadiazinan, Oxadiazolidin, Oxathianan, Oxathiolan, Oxazetidin, Oxazolidin, Oxetan, Oxirane, Piperazin, Piperidin, Pyrazolidin, Pyrrolidin, Tetrahydrofuran, Tetrahydropyran, Tetrahydrothiophen, Tetrahydrothiopyran, Tetrazinan, Thiadiazolidin, Thiazetidin, Thiaziridin, Thiazolidine, Thietan, Thiiran, Thiomorpholin, Triazetidin, Triazinan oder Triazolidin, der unsubstituiert oder ein-, zwei- oder dreifach unabhängig voneinander durch R10 oder Fluor substituiert ist.

5) Ein weiterer Gegenstand der Erfindung ist die Verbindung der Formel Ia, wobei der Teilring

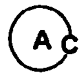

ausgewählt wird aus der Gruppe Azetidin, Cyclopropyl, Cyclobutyl, Cyclopentyl, Cyclohexyl, 1,3-Dihydro-isobenzofuran, 1,3-Dioxan, 1,3-Dioxolan, Imidazolidin, Indan, Morpholin, 1,3-Oxazinan, Piperazin, Piperidin, Pyrrolidin, Tetrahydrofuran, und 1,2,3,4-Tetrahydro-naphthalen,
der Teilring

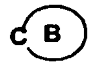

ausgewählt wird aus der Gruppe Azetidin, Cyclopropyl, Cyclopentyl, Cyclohexyl, Morpholin, Oxazolidin, Piperidin und Pyrrolidin, und worin die beiden Teilringe unsubstituiert oder unabhängig voneinander je nach Ringgröße ein-, zwei-, oder dreifach durch R4 substituiert sind, wobei

R4 für -($C_1$-$C_4$)-Alkyl, -O-($C_1$-$C_4$)-Alkyl, =O, -($C_0$-$C_4$)-Alkylen-($C_3$-$C_6$)-Cycloalkyl oder -($C_0$-$C_4$)-Alkylen-Phenyl, wobei Phenyl unsubstituiert oder durch F, Cl, Br oder -O-($C_1$-$C_4$)-Alkyl substituiert ist, steht,

X für eine kovalente Bindung oder -NH- steht,

Y für -C(O)- oder -S($O_2$)- steht,

p für die ganze Zahl 1 steht,

R27 für Wasserstoffatom, -($C_1$-$C_6$)-Alkyl, 4-F-Benzyl oder Benzyl steht,

R26 für Wasserstoffatom steht,

R24 und R25 sind gleich oder verschieden und stehen unabhängig voneinander für Wasserstoffatom, Methyl oder Ethyl,

R24 und R25 bilden zusammen mit dem Kohlenstoffatom, an das sie gebunden sind, einen Cyclopropyl oder Cyclobutyl-Rest, oder

R24 und R25 bilden zusammen mit dem Kohlenstoffatom an das sie gebunden sind einen Piperidin-Ring, der unsubstituiert oder durch -($C_1$-$C_4$)-Alkyl substitutiert ist.

6) Ein weiterer Gegenstand der Erfindung sind Verbindungen der Formeln Ia aus der Reihe
3-Azaspiro[5.5]undecan-3-carbonsäure [(S)-1-(1-cyanocyclopropylcarbamoyl)-3,3-difluorbutyl]-amid,
8-Azaspiro[4.5]decan-8-carbonsäure [(S)-1-(1-cyanocyclopropylcarbamoyl)-3,3-difluor-butyl]-amid,
1,4-Dioxa-8-azaspiro[4.5]decan-8-carbonsäure [(S)-1-(1-cyanocyclopropylcarbamoyl)-3,3-difluorbutyl]-amid,
2-Azaspiro[5.5]undecan-2-carbonsäure [(S)-1-(1-cyanocyclopropylcarbamoyl)-3,3-difluor-butyl]-amid,
8-Azaspiro[4.5]decan-8-carbonsäure [(S)-1-(1-cyanocyclopropylcarbamoyl)-3,3-difluorohexyl]-amid,
3-Azaspiro[5.5]undecan-3-carbonsäure [(S)-1-(1-cyanocyclopropylcarbamoyl)-3,3-difluorhexyl]-amid,
2-(4-Methoxyphenyl)-1-oxo-2,8-diazaspiro[4.5]decan-8-carbonsäure [(S)-1-(1-cyano-cyclopropylcarbamoyl)-3,3-difluorbutyl]-amid,
4-Oxo-1-phenyl-1,3,8-triazaspiro[4.5]decan-8-carbonsäure [(S)-1-(1-cyano-cyclopropylcarbamoyl)-3,3-difluorbutyl]-amid,
1,5-Dioxa-9-azaspiro[5.5]undecan-9-carbonsäure [(S)-1-(1-cyano-cyclopropylcarbamoyl)-3,3-difluorbutyl]-amid,
1-Oxo-2,8-diazaspiro[4.5]decan-8-carbonsäure [(S)-1-(1-cyanocyclopropylcarbamoyl)-3,3-difluorbutyl]-amid,
2-Methyl-1-oxo-2,8-diazaspiro[4.5]decan-8-carbonsäure [(S)-1-(1-cyano-cyclopropylcarbamoyl)-3,3-difluorbutyl]-amid,
3,3-Dimethyl-1-oxa-5,9-diazaspiro[5.5]undecan-9-carbonsäure [(S)-1-(1-cyano-cyclopropylcarbamoyl)-3,3-difluorbutyl]-amid,
8-Azaspiro[4.5]decan-8-carbonsäure [(S)-1-(1-cyanocyclopropylcarbamoyl)-3,3-difluor-4-phenylbutyl]-amid,

2,4-Dioxo-1,3,8-triazaspiro[4.5]decan-8-carbonsäure [(S)-1-(1-cyano-cyclopropylcarbamoyl)-3,3-difluorbutyl]-amid,
2-Azaspiro[4.4]nonan-2-carbonsäure [(S)-1-(1-cyanocyclopropylcarbamoyl)-3,3-difluorbutyl]-amid,
2-Benzyl-1-oxo-2,8-diazaspiro[4.5]decan-8-carbonsäure [(S)-1-(1-cyano-cyclopropylcarbamoyl)-3,3-difluorbutyl]-amid,
2-(4-Fluorphenyl)-1-oxo-2,8-diazaspiro[4.5]decan-8-carbonsäure [(S)-1-(1-cyano-cyclopropylcarbamoyl)-3,3-difluorbutyl]-amid,
3-Phenyl-1,5-dioxa-9-azaspiro[5.5]undecan-9-carbonsäure [(S)-1-(1-cyano-cyclopropylcarbamoyl)-3,3-difluorbutyl]-amid,
9-Butyl-3,9-diazaspiro[5.5]undecan-3-carbonsäure [(S)-1-(1-cyano -cyclopropylcarbamoyl)-3,3-difluorbutyl]-amid,
9-Cyclopropyl-3,9-diazaspiro[5.5]undecan-3-carbonsäure-[(S)-1-(1-cyano-cyclopropylcarbamoyl)-3,3-difluorbutyl]-amid,
Spiro[2.3]hexan-1-carbonsäure [(S)-1-(1-cyano-cyclopropylcarbamoyl)-3,3-difluor-butyl]-amid,
2,2-Dimethyl-1-oxa-8-aza-spiro[4.5]decan-8-carbonsäure [(S)-1-(1-cyano-cyclopropylcarbamoyl)-3,3-difluor-butyl]-amid,
2-Aza-spiro[4.5]decan-2-carbonsäure [(S)-1-(1-cyano-cyclopropylcarbamoyl)-3,3-difluor-butyl]-amid,

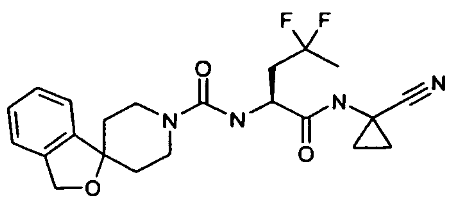

,

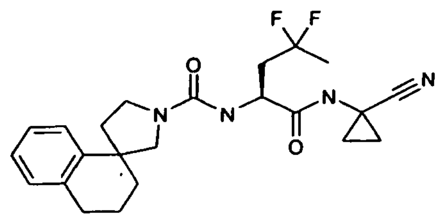

1-Oxa-4-aza-spiro[4.5]decan-4-carbonsäure [(S)-1-(1-cyano-cyclopropyl-carbamoyl)-3,3-difluor-butyl]-amid,
(S)-2-[3-(1,4-Dioxa-spiro[4.5]dec-8-yl)-ureido]-4,4-difluor-pentansäure (1-cyano-cyclopropyl)-amid,

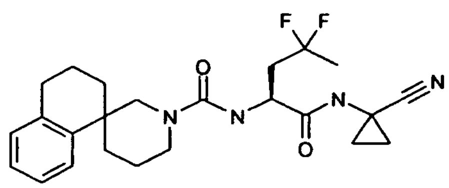

,

7-Cyclopropyl-2,7-diaza-spiro[3.5]nonan-2-carbonsäure [(S)-1-(1-cyano-cyclopropylcarbamoyl)-3,3-difluor-butyl]-amid,
2-Cyclopropyl-2,7-diaza-spiro[3.5]nonane-7-carbonsäure [(S)-1-(1-cyano-cyclopropylcarbamoyl)-3, 3-difluor-butyl]-amid,
2-Propyl-2,7-diaza-spiro[3.5]nonan-7-carbonsäure [(S)-1-(1-cyano-cyclopropylcarbamoyl)-3,3-difluor-butyl]-amid,
(S)-2-(8-Aza-spiro[4.5]decan-8-sulfonylamino)-4,4-difluor-pentansäure (1-cyano-cyclopropyl)-amid,
4-Cyclopropyl-1-oxa-4,9-diaza-spiro[5.5]undecan-9-carbonsäure [(S)-1-(1-cyano-cyclopropylcarbamoyl)-3,3-difluor-butyl]-amid,
9-Cyclopropyl-1-oxa-4,9-diaza-spiro[5.5]undecan-4-carbonsäure [(S)-1-(1-cyano-cyclopropylcarbamoyl)-3,3-difluor-butyl]-amid,
2-Cyclopropylmethyl-3-oxo-2,8-diaza-spiro[4.5]decan-8-carbonsäure[(S)-1-(1-cyano-cyclopropylcarbamoyl)-3,3-difluor-butyl]-amid,

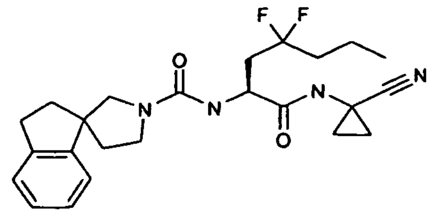

,

2-(4-Methoxy-phenyl)-1-oxo-2,8-diaza-spiro[4.5]decan-8-carbonsäure [(S)-1-(1-cyano-cyclopropylcarbamoyl)-3,3-difluor-hexyl]-amid,
2-(4-Methoxy-phenyl)-2,8-diaza-spiro[4.5]decan-8-carbonsäure [(S)-1-(1-cyano-cyclopropylcarbamoyl)-3,3-difluor-hexyl]-amid,
2-Cyclopropyl-2,7-diaza-spiro[3.5]nonan-7-carbonsäure [(S)-1-(1-cyano-cyclo-propylcarbamoyl)-3,3-difluor-hexyl]-amid,
2-Cyclopropyl-2,8-diaza-spiro[4.5]decan-8-carbonsäure [(S)-1-(1-cyano-cyclopropylcarbamoyl)-3,3-difluor-hexyl]-amid,
9-Cyclopropyl-3,9-diaza-spiro[5.5]undecan-3-carbonsäure [(S)-1-(1-cyano-cyclo-propylcarbamoyl)-3,3-difluor-hexyl]-amid,
7-Cyclopropyl-2,7-diaza-spiro[3.5]nonan-2-carbonsäure [(S)-1-(1-cyano-cyclo-propylcarbamoyl)-3,3-difluor-hexyl]-amid,
7-Propyl-2,7-diaza-spiro[3.5]nonan-2-carbonsäure [(S)-1-(1-cyano-cyclopropyl-carbamoyl)-3,3-difluor-hexyl]-amid,
9-Cyclopropyl-3,9-diaza-spiro[5.5]undecan-3-carbonsäure [(S)-1-(1-cyano-cyclo-propylcarbamoyl)-3,3-difluor-4-phenyl-butyl]-amid,
2-Cyclopropyl-2,7-diaza-spiro[3.5]nonan-7-carbonsäure [(S)-1-(1-cyano-cyclopropyl-carbamoyl)-3,3-difluor-4-phenyl-butyl]-amid,
2-Propyl-2,7-diaza-spiro[3.5]nonan-7-carbonsäure [(S)-1-(1-cyano-cyclopropyl-carbamoyl)-3,3-difluor-4-phenyl-butyl]-amid,
7-Cyclopropyl-2,7-diaza-spiro[3.5]nonan-2-carbonsäure [(S)-1-(1-cyano-cyclopropyl-carbamoyl)-3,3-difluor-4-phenyl-butyl]-amid,
6-Aza-spiro[2.5]octan-6-carbonsäure [(S)-1-(1-cyano-cyclopropylcarbamoyl)-3,3-difluor-butyl]-amid,
6-Aza-spiro[2.5]octan-6-carbonsäure [(S)-1-(cyanomethyl-carbamoyl)-3,3-difluor-butyl]-amid,
6-Aza-spiro[2.5]octan-6-carbonsäure [(S)-1-(1-cyano-cyclopropylcarbamoyl)-3,3-difluor-hexyl]-amid,
6-Aza-spiro[2.5]octan-6-carbonsäure [(S)-1-(4-cyano-1-methyl-piperidin-4-ylcarbamoyl)-3,3-difluor-butyl]-amid,
6-Aza-spiro[2.5]octan-6-carbonsäure [(S)-1-(4-cyano-1-methyl-piperidin-4-ylcarbamoyl)-3, 3-difluor-hexyl]-amid,
6-Aza-spiro[2.5]octan-6-carbonsäure [(S)-1-(1-cyano-cyclopropylcarbamoyl)-3,3-difluor-pentyl]-amid und
6-Aza-spiro[2.5]octan-6-carbonsäure [(S)-1-(1-cyano-cyclopropylcarbamoyl)-3,3-difluor-4-phenyl-butyl]-amid.

Unter den Begriffen "$(C_1-C_3)$-Alkyl", "$(C_1-C_4)$-Alkyl" oder "$(C_1-C_{10})$-Alkyl" werden Kohlenwasserstoffreste verstanden, deren Kohlenstoffkette geradkettig oder verzweigt ist und 1 bis 3, 1 bis 4 oder 1 bis 10 Kohlenstoffatome enthält, beispielsweise Methyl, Ethyl, Propyl, Iso-Propyl, Butyl, Iso-Butyl, tertiär-Butyl, Pentyl, Iso-Pentyl, Neopentyl, Hexyl, 2,3-Dimethylbutyl, Neohexyl, Heptyl, Octanyl, Nonanyl oder Decanyl.

Unter den Begriffen "-$(C_0-C_3)$-Alkylen", "-$(C_0-C_4)$-Alkylen" oder "-$(C_0-C_5)$-Alkylen" werden Kohlenwasserstoffreste verstanden, deren Kohlenstoffkette geradkettig oder verzweigt ist und 1 bis 3, 1 bis 4 oder 1 bis 5 Kohlenstoffatome enthält, beispielsweise Methylen, Ethylen, Propylen, Iso-Propylen, Iso-Butylen, Butylen, tertiär-Butylen, iso-Pentylen oder Neopentylen. "-$C_0$-Alkylen" ist eine kovalente Bindung.

Unter den Begriffen "$(C_1-C_8)$-Alkoxy" werden Reste wie -O-$(C_1-C_8)$-Alkyl verstanden, wobei -$(C_1-C_8)$-Alkyl mit einem Kohlenstoffatom an ein Sauerstoffatom gebunden ist. Unter dem Rest "-C(O)-" wird ein Keto- oder Aldehydrest verstanden.

Unter dem Rest "Carbamimidoyl" wird ein -$C(NH_2)$=NH Rest verstanden.

Unter dem Begriff "-$(C_3-C_8)$-Cycloalkyl" werden Reste verstanden, die sich von 3- bis 8-gliedrige Monocyclen wie den Monocyclen Cyclopropan, Cyclobutan, Cyclopentan, Cyclohexan, Cycloheptan oder Cyclooctan herleiten.

Unter dem Begriff "Aryl" werden aromatische Kohlenstoffreste verstanden mit 6 bis 14 Kohlenstoffatomen im Ring. Arylreste sind beispielsweise Phenyl, Indanyl, Indenyl, Naphthyl, 1,2,3,4-Tetrahydronaphthyl, Biphenylyl, 2-Biphenylyl, 3-Biphenylyl, 4-Biphenylyl, Anthryl oder Fluorenyl.

Unter dem Begriff "ein gesättigtes oder teilweise gesättigtes -$(C_3-C_{11})$-Cycloalkyl, worin Cycloalkyl unverbrückt, verbrückt oder annelliert ist " werden Reste verstanden wie beispielsweise Verbindungen, die sich von 3- bis 11-gliedrigen

Mono-, Bicyclen, überbrückten Cyclen oder Spirocyclen herleiten : z.B von den Monocyclen wie Cyclopropan, Cyclobutan, Cyclopentan, Cyclohexan, Cycloheptan oder Cyclooctan, z.B. von den Bicyclen wie Bicycloheptan, Bicyclo[4.2.0]octan, Octahydro-inden, Decalin, Decahydro-benzocyclohepten oder Dodecahydro-heptalen, z.B. von den überbrückten Cyclen wie Bicyclo[3.1.1]heptan, Bicyclo[2.2.1]heptan, Bicyclo[3.3.0]octan oder Bicyclo[2.2.2]octan, oder z.B. von den Spirocyclen wie Spiro[2.5]octan und Spiro[3.4]octan.

Unter dem Begriff "einen gesättigten oder teilweise gesättigten drei- bis elfgliedrigen Heterocyclus stehen, die je nach Ringgröße ein, zwei, drei oder vier gleiche oder verschiedene Heteroatome aus der Reihe Sauerstoff, Stickstoff oder Schwefel enthalten können und worin der Heterocyclus unverbrückt, verbrückt oder annelliert ist"
werden Ringsysteme verstanden mit 3 bis 11 Ringatomen, die neben den Kohlenstoffatomen je nach Ringgröße ein, zwei, drei oder vier gleiche oder verschiedene Heteroatome aus der Reihe Sauerstoff, Stickstoff oder Schwefel enthalten. Beispiele für diese Ringsysteme sind Ringsysteme wie Azepan, Azepin, Azetidin, Aziridin, Azirin, Azocan, Benzimidazolin, 2,3-Dihydro-benzo[b]thiophen, 1,3-Dihydro-benzo[c]thiophen, 2,3-Dihydro-benzofuran, 2,3-Dihydro-benzooxazol, 2,3-Dihydro-benzothiazol, 1,3-Dihydro-isobenzofuran, 2,3-Dihydro-isoxazol, 2,5-Dihydro-isoxazol, 4,5-Dihydro-isoxazol, Benzo[1,3]dioxol, 1,4-Diazepan, 1,2-Diazepin, 1,3-Diazepin, 1,4-Diazepin, Diaziridin, Diazirin, 1,4-Diazocan, Dioxan, 1,3-Dioxan, Dioxazin, 1,4-Diozocan, Dioxol, Dioxolan, 1,3-Dioxolan, 1,3-Dioxolen, Imidazolin, Imidazolidin, Indan, Indolin, Isoindolin, Isothiazolidin, Isothiazolin, Isoxazolin, Isoxazolidin, 2-Isoxazolin, Morpholin, 1,2-Oxa-thiepan, 1,2-Oxathiolan, 1,4-Oxazepan, 1,2-Oxazin, 1,3-Oxazin, 1,4-Oxazin, Oxazinan, 1,3-Oxazinan, Oxazocane, Oxaziridin, Oxazolidin, Oxetan, Oxiran, Oxocane, Piperazin, Piperidin, Pyran, Pyrazolin, Pyrazolidin, Pyrrolidin, Pyrrolidinon, Pyrrolin, Tetrahydrochinolin, Tetrahydrofuran, Tetrahydroisochinolin, 1,2,3,4-Tetrahydro-naphthalen, Tetrahydropyran, Tetrahydropyridin, Tetrazin, Thiadiazin, 1,2-Thiazin, 1,3-Thiazin, 1,4-Thiazin, Thiazolidin, Thiazolin, Thietan, Thiomorpholin, Thiopyran, 1,2,3-Triazin, 1,2,4-Triazin oder 1,3,5-Triazin.
Spiroverbindungen sind Verbindungen von zwei oder drei Ringen, worin jeweils ein Ringatom zwei Ringen gemeinsam angehört. Bevorzugt sind Spiroverbindungen, die aus zwei Ringen bestehen, worin jeweils ein Ringatom zwei Ringen gemeinsam angehört. Dieses Ringatom ist entweder ein Kohlenstoff-Atom oder ein StickstoffAtom, bevorzugt ein Kohlenstoff-Atom. Die Spiroverknüpfung der beiden Teilringe kann über alle denkbaren Positionen erfolgen. Bevorzugte Spiroverbindungen in allen möglichen stereoisomeren Formen sind:

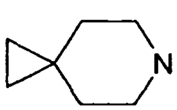 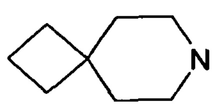 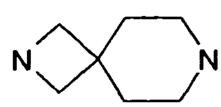 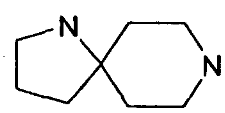

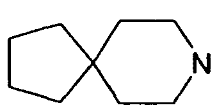 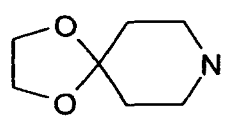 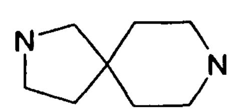 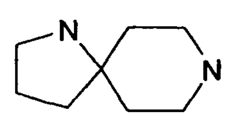

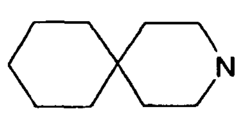 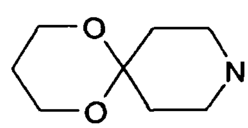 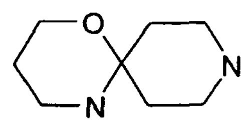 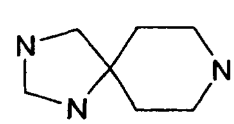

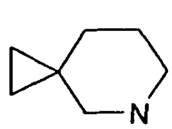 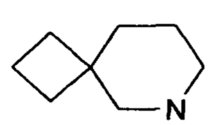 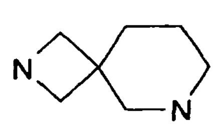 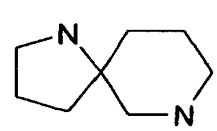

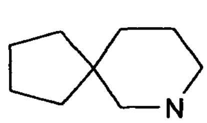 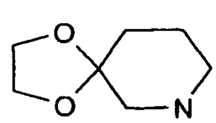 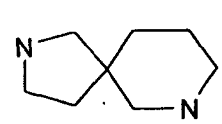 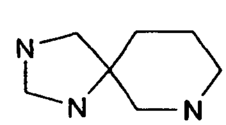

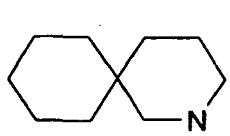 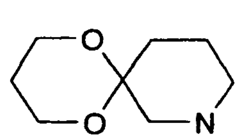 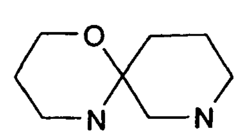 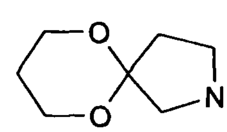

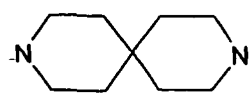
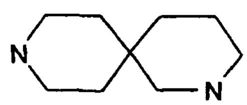
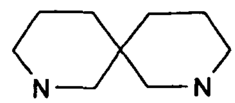
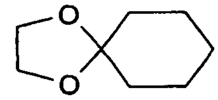
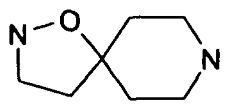
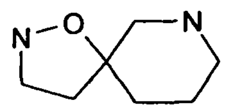
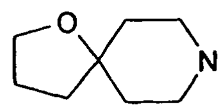
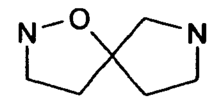
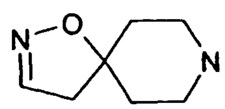
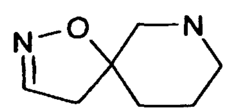
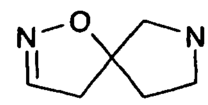
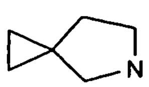
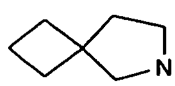
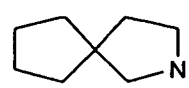
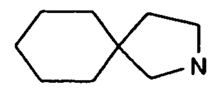
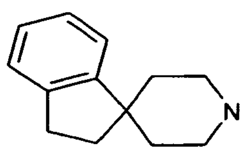
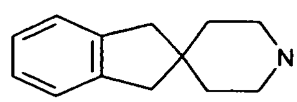
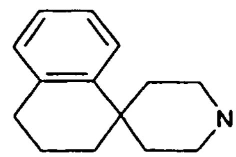
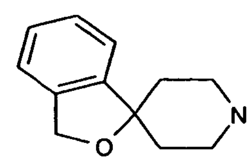
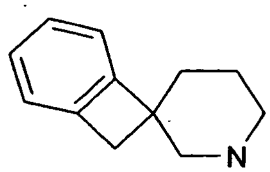
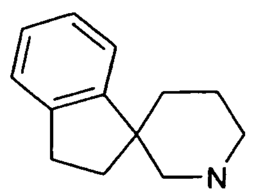
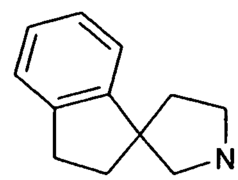
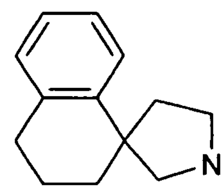
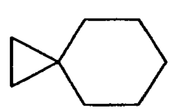
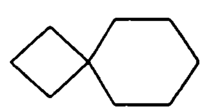
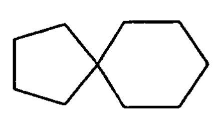
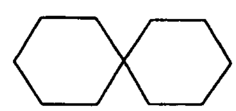
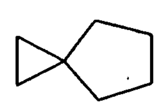
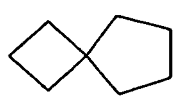
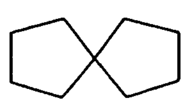
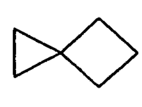
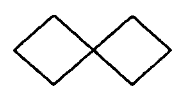
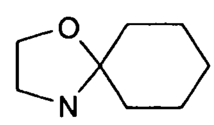
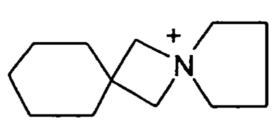
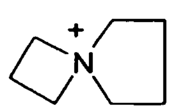
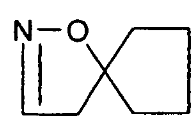
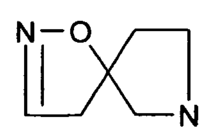
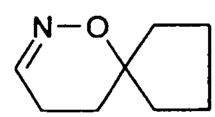

Unter dem Begriff "Halogen" wird Fluor, Chlor, Brom oder Jod verstanden, bevorzugt sind Fluor, Chlor oder Brom, insbesondere Fluor.

Unter dem Begriff "Het-Ring" oder "Het" werden Ringsysteme verstanden mit 3 bis 15 Kohlenstoffatomen, die in ein, zwei oder drei miteinander verbundenen Ringsystemen vorliegen und die je nach Ringgröße ein, zwei, drei oder vier gleiche oder verschiedene Heteroatome aus der Reihe Sauerstoff, Stickstoff oder Schwefel enthalten. Beispiele für diese Ringsysteme sind die Reste Acridinyl, Azepanyl, Azepinyl, Azetidinyl, Aziridinyl, Benzimidazolinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Homomorpholinyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Oxothiolanyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridinyl, Pyridyl; Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienoimidazolyl, Thienooxazolyl, Thienopyridin, Thienothiazolyl, Thiomorpholinyl, Thiophenyl, Triazinyl, 1,2,3-Triazolyl, 1,2,4-Triazolyl, 1,2,5-Triazolyl, 1,3,4-Triazolyl und Xanthenyl.

Unter dem Begriff "-($C_1$-$C_3$)-Fluoralkyl" wird ein partiell oder vollständig fluorierter Alkylrest verstanden, der sich beispielsweise von folgenden Resten ableitet $-CF_3$, $-CHF_2$, $-CH_2F$, $-CHF-CF_3$, $-CHF-CHF_2$, $-CHF-CH_2F$, $-CH_2-CF_3$, $-CH_2-CHF_2$, $-CH_2-CH_2F$, $-CF_2-CF_3$, $-CF_2-CHF_2$, $-CF_2-CH_2F$, $-CH_2-CHF-CF_3$, $-CH_2-CHF-CHF_2$, $-CH_2-CHF-CH_2F$, $-CH_2-CH_2-CF_3$, $-CH_2-CH_2-CHF_2$, $-CH_2-CH_2-CH_2F$, $-CH_2-CF_2-CF_3$, $-CH_2-CF_2-CHF_2$, $-CH_2-CF_2-CH_2F$, $-CHF-CHF-CF_3$, $-CHF-CHF-CHF_2$, $-CHF-CHF-CH_2F$, $-CHF-CH_2-CF_3$, $-CHF-CH_2-CHF_2$, $-CHF-CH_2-CH_2F$, $-CHF-CF_2-CF_3$, $-CHF-CF_2-CHF_2$, $-CHF-CF_2-CH_2F$, $-CF_2-CHF-CF_3$, $-CF_2-CHF-CHF_2$, $.-CF_2-CHF-CH_2F$, $-CF_2-CH_2-CF_3$, $-CF_2-CH_2-CHF_2$, $-CF_2-CH_2-CH_2F$, $-CF_2-CF_2-CF_3$, $-CF_2-CF_2-CHF_2$ oder $-CF_2-CF_2-CH_2F$.

Unter den Begriffen, "R19 und R11", "R13 und R14" oder "R24 und R25 bilden zusammen mit dem Kohlenstoffatom an das sie gebunden sind einen drei- bis sechsgliedrigen Cycloalkylring" werden Cycloalkylreste wie Cyclopropyl, Cyclobutyl, Cyclopentyl oder Cyclohexyl verstanden.

Unter den Begriffen "R24 und R25 bilden zusammen mit dem Kohlenstoffatom an das sie gebunden sind einen drei- bis sechsgliedrigen Hetero-Cycloalkylrest" werden Reste wie Aziridin, Azetidin, Diazetidin, Diaziridin, Hexohydropyridazin, Hexohydropyrimidin, Imidazolidin, Morpholin, Oxadiazinan, Oxadiazolidin, Oxathianan, Oxathiolan, Oxazetidin, Oxazolidin, Oxetan, Oxirane, Piperazin, Piperidin, Pyrazolidin, Pyrrolidin, Tetrahydrofuran, Tetrahydropyran, Tetrahydrothiophen, Tetrahydrothiopyran, Tetrazinan, Thiadiazolidin, Thiazetidin, Thiaziridin, Thiazolidine, Thietan, Thiiran, Thiomorpholin, Triazetidin, Triazinan oder Triazolidin verstanden.

Unter dem Begriff "R21 und R22 zusammen mit dem Stickstoffatom, an das sie gebunden sind, einen vier- bis achtgliedrigen monocyclischen Heterocyclischen Ring bilden, der neben dem Stickstoffatom zusätzlich noch je nach Ringgröße ein oder zwei gleiche oder verschiedene Heteroatome aus der Reihe Sauerstoff, Stickstoff oder Schwefel enthalten kann" werden Reste wie Azepan, Azepin, Azetidin, Dioxazol, Dioxazin, 1,4-Diazepan, 1,2-Diazepin, 1,3-Diazepin, 1,4-Diazepin, Imidazol, Imidazolin, Imidazolidin, Isothiazol, Isothiazolidin, Isothiazolin, Isoxazol, Isoxazolin, Isoxazolidin, 2-Isoxazolin, Morpholin, [1,4]Oxazepane, Oxazol, Piperazin, Piperidin, Pyrazin, Pyrazol, Pyrazolin, Pyrazolidin, Pyridazin, Pyridin, Pyrimidin, Pyrrol, Pyrrolidin, Pyrrolidinon, Pyrrolin, Tetrahydropyridin, Tetrazin, Tetrazol, Thiazol, Thiadiazol, Thiazolidin, Thiazolin, Thiomorpholin, 1,2,3-Triazin, 1,2,4-Triazin, 1,3,5-Triazin, 1,2,3-Triazol oder 1,2,4-Triazol verstanden.

Unter dem Begriff "zwei benachbarte R4 bilden zusammen mit den Ringatomen, an die sie gebunden sind, einen vier- bis achtgliedrigen Heterocyclus oder Phenyl, der zusammen mit dem Teilring , an den der Heterocyclus oder das Phenyl anneliert ist, ein bicyclisches System bildet", werden Verbindungen verstanden, die aus zwei miteinander verbundenen Ringsystemen bestehen, worin ein Ring den Teilring

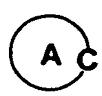

oder

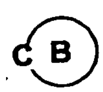

darstellt und der andere Ring einen teilweise gesättigten oder aromatisches Ringsystem bildet, das je nach Ringgröße ein, zwei oder drei gleiche oder verschiedene Heteroatome aus der Reihe Sauerstoff, Stickstoff oder Schwefel enthält. Beispiele für diese Ringsysteme sind Reste wie Benzimidazol, Benzisothiazol, Benzisoxazol, Benzo[1,3]dioxol, Benzofuranyl, Benzothiazol, Benzisoxazol, Benzothiofuran, Benzothiophen, Benzo[1,3]oxathiol, Benzoxazol, Benzthiazol, Benztriazolyl, Chinazolin, Chinazolon, Chinolin, 4H-Chinolizin, Chinoxalin, Chroman, Chromen, Cinnolin, 2,3-Dihydro-benzo[1,4]dioxin, 2,3-Dihydro-benzofuranyl, 1,3-Dihydro-isobenzofuran, 3,4-Dihydro-2H-benzo[1,4]oxazin, 2,3-Dihydro-benzooxazol, 2,3-Dihydro-benzothiazol, 1,3-Dihydro-benzo[c]thiophen, 2,3-Dihydro-benzo[b]thiophen, Indazol, Indol, Indolin, Isobenzofuran, Isochinolin, Isochroman, Isoindazol, Isoindol, Isoindolin, 7-Oxa-bicyclo[4.2.0]octa-1,3,5-trien, Phthalazin, 2,3,4,5-Tetrahydro-1H-benzo[b]azepin, 6,7,8,9-Tetrahydro-5-oxa-9-aza-benzocyclohepten, 3,4,5,6-Tetrahydro-2H-benzo[b][1,4]oxazozin, Tetrahydrochinolin, 1,2,3,4-Tetrahydro-chinoxalin oder Tetrahydroisochinolin.

Unter dem Begriff "=O" wird ein Oxo-Rest wie in Carbonyl- (-C(O)-) oder Sulfonyl- oder Sulfoxid ($S(O)_2$ oder S(O)) verstanden.

Unter der Teilstruktur

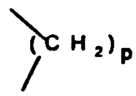

in der Verbindung der Formel Ia wird für den Fall p ist 1 eine Methylen-Rest und für den Fall p ist 2 ein Ethylen-Rest verstanden.

Die erfindungsgemäßen Verbindungen können nach wohlbekannten Verfahren oder nach hier beschriebenen Verfahren hergestellt werden.

Die Erfindung betrifft ferner ein Verfahren zur Herstellung der Verbindungen der Formeln Ia und/oder einer stereoisomeren Form der Verbindung der Formeln Ia und/oder eines physiologisch verträglichen Salzes der Verbindung der Formeln Ia und/oder eines Solvats oder Hydrats der Verbindung der Formeln Ia, dadurch gekennzeichnet, dass man

a) eine Verbindung der Formel II,

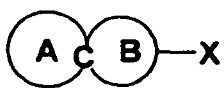 (II)

wobei A und B wie in der Verbindung der Formel Ia definiert sind, mit einer Verbindung der Formel IIIa oder IIIb oder IIIc,

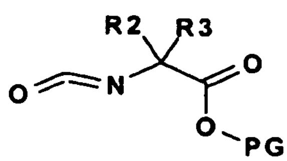 (IIIa)

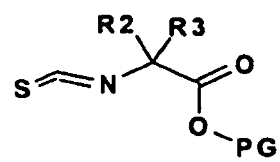 (IIIb)

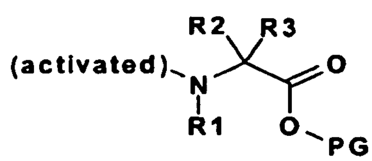 (IIIc)

wobei X, R1, R2 und R3 die Bedeutung wie in der Verbindung der Formel Ia haben, PG eine Ester-Schutzgruppe bedeutet, und "activated" bedeutet, dass das Amin in einer aktivierten Form vorliegt, beispielsweise als Chlorcarbonyl-Verbindung, zu einer Verbindung der Formel IVa oder IVb umsetzt

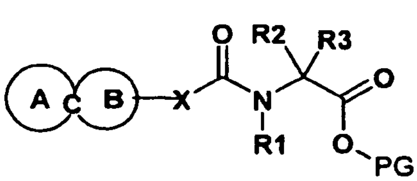 (IVa)

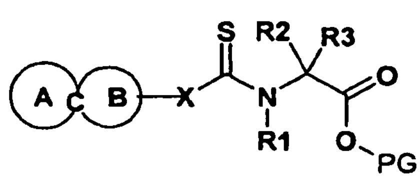 (IVb)

und die erhaltenen Verbindungen der Formel IVa oder IVb nach Umwandlung des Esters in die Carbonsäure mit Z zur Verbindung der Formel Ia umsetzt, oder

b) eine Verbindung der Formel Va oder Vb, wobei A, B, X und Y die Bedeutung wie in der Verbindung der Formel

Ia haben,

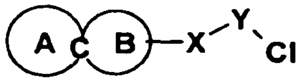 (Va) 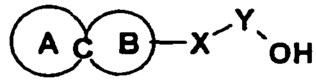 (Vb)

mit einer Verbindung der Formel VI, wobei R1, R2 und R3 die Bedeutung wie in der Verbindung der Formel Ia haben und PG eine Ester-Schutzgruppe darstellt,

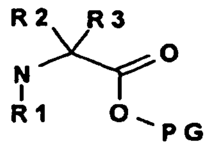 (VI)

zu einer Verbindung der Formel IVa oder IVb umsetzt und die erhaltene Verbindung der Formel IVa oder IVb nach Umwandlung der Ester-Schutzgruppe in die Carbonsäure mit Z zur Verbindung der Formel Ia umsetzt, oder

c) eine Verbindung der Formel VIIa oder VIIb, wobei A, B und X die Bedeutung wie in der Verbindung der Formel Ia haben,

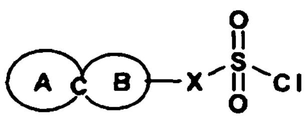 (VIIa) 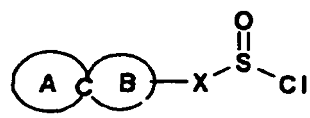 (VIIb)

mit einer Verbindung der Formel VI,

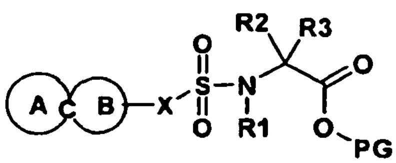 (VIIIa) 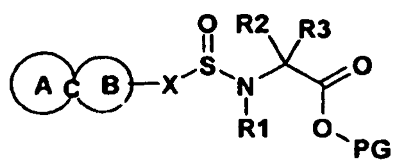 (VIIIb)

zu einer Verbindung der Formel VIIIa oder VIIIb umsetzt und die erhaltene Verbindung der Formel VIIIa oder VIIIb nach Umwandlung des Esters in die entsprechende Carbonsäure mit Z zur Verbindung der Formeln Ia umsetzt, oder

d) eine Verbindung der Formel IX

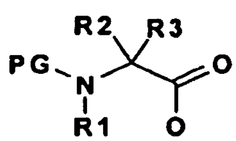 (IX)

mit einem Amin Z, wobei Z die Bedeutung wie in der Verbindung der Formel Ia hat, zu einer Verbindung der Formel X umsetzt,

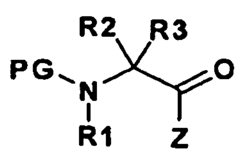 (X)

und die so erhaltene Verbindung X anschließend im Sinne einer Schutzgruppenabspaltung zu einer Verbindung der Formel XI umsetzt,

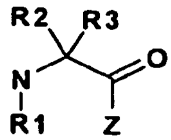

(XI)

und diese Verbindung XI anschließend mit einer Verbindung Va oder Vb, wie unter b) aufgeführt, zur erfindungsgemäßen Verbindung der Formeln Ia la umsetzt, oder

e) eine nach den Verfahren a), b), c) oder d) hergestellte Verbindung der Formeln Ia, oder eine geeignete Vorstufe der Formeln Ia, die aufgrund ihrer chemischen Struktur in enantiomeren oder diastereomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren oder Diastereomeren auftrennt, oder

f) die nach den Verfahren a), b), c) oder d) hergestellte Verbindung der Formeln Ia entweder in freier Form isoliert oder aus physiologisch unverträglichen Salzen freisetzt oder im Falle des Vorliegens von sauren oder basischen Gruppen in physiologisch verträgliche Salze umwandelt, oder

g) die nach den Verfahren a), b), c) oder d) hergestellte Verbindung der Formeln Ia, oder eine geeignete Vorstufe der Formeln Ia, die aufgrund ihrer chemischen Struktur dazu befähigt ist, ein N-Oxid zu bilden, in dieses zu überführen oder im Falle des Vorliegens eines N-Oxids, dieses in das freie Amin oder das Salz eines Amins umwandelt.

Die Synthese der erfindungsgemäßen Produkte kann ferner auf der Basis von drei Ausgangsprodukten ausgehen, wobei auch jede Variation der Komponenten möglich ist, die zu den erfindungsgemäßen Strukturen führt. Der Einfachheit halber werden die Synthesemöglichkeiten durch diese drei Komponenten beschrieben; dies soll jedoch keine Einschränkung auf weitere Möglichkeiten zur Synthese darstellen.

Beispielsweise kann die Komponente A ein Spiro-Amin sein:

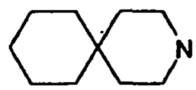

Beispielsweise kann die Komponente B ein Aminosäure-Derivat sein:

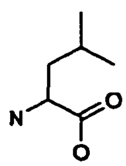

und

beispielsweise kann die Komponente C ein Amino-Nitril sein:

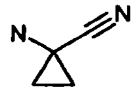

Ein bevorzugter Weg ist es, von diesen drei Ausgangsprodukten aus durch Wahl geeigneter bestimmter Derivate dieser Komponenten, beispielsweise geschützter oder modifizierter Vorstufen oder Vorstufen mit definierter Stereochemie, die erfindungsgemäßen Verbindungen durch geeignete Verknüpfungsreaktionen eventuell nach Aktivierung mit reaktiven Reagenzien, wie bekannten Peptid-Kupplungsreagenzien oder Reagenzien, die zu aktivierten Harnstoff-Vorstufen führen, herzustellen. Methoden zur Peptidkupplung sind etwa bei Bodanszky (M. Bodanszky, Principles of Peptide Synthesis, 2nd ed, Springer, Berlin, 1993) beschrieben. Schutzgruppen, deren Einführung, Abspaltung und Stabilität sind beispielsweise bei Greene (T. W. Greene, P. G. M. Wuts, Protective Groups in Organic Synthesis, 3rd ed, Wiley, New York, 1999) beschrieben: Die Herstellung von Harnstoffen ist beispielsweise bei G. Sartori; R. Maggi, Acyclic and cyclic ureas. Science of Synthesis (2005), 18, 665-758, im Detail beschrieben.

Dabei können sowohl viele verschiedene, dem Fachmann bekannte, geeignete, wie auch von ihrer Chemie

her verschiedenartige Aktivierungs- und Kupplungsreagenzien zum Einsatz kommen. Nur beispielsweise genannt werden Carbodiimide, Uronium-Salze oder auch Chlorameisensäureester zur Carbonsäure-Aktivierung sowie Phosgen, Carbonyldiimidazol oder auch Chlorameisensäureester zur Herstellung aktivierter Harnstoff-Vorstufen, sowie Chlorsulfonsäure oder Schwefeltrioxid zur Herstellung aktivierter Sulfonylharnstoff-Vorstufen.

Ebenso kann die Reihenfolge der Verknüpfung variiert werden, oder aber auch die Einzelkomponente oder der Zweierbaustein (der durch Verknüpfung zweier benachbarter Einzelkomponenten erhalten wurde), an der die Aktivierung vorgenommen wird, oder es können verschiedene Schutzgruppen eingesetzt werden, gefolgt von der Zugabe der noch fehlenden Komponente oder der noch fehlenden Komponenten. Weiterhin ist es möglich, die Schutzgruppen zu verändern, beispielsweise Schutzgruppenabspaltungen nach erfolgter Verknüpfung oder am Ende der Synthese zur Herstellung der Verbindung der Formeln Ia.

Ferner kann die Herstellung der Verbindung der Formeln Ia über den Weg 1 a erfolgen:

Weg 1a

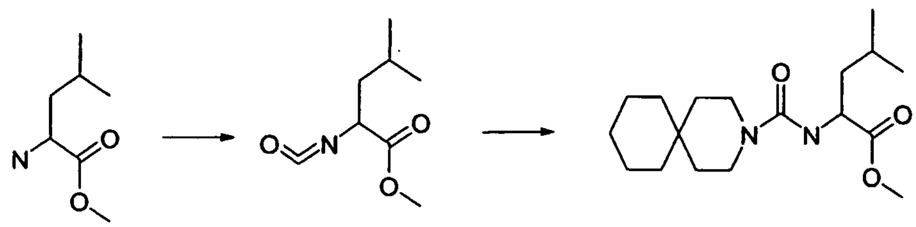

Als Komponente B - Aminosäurederivat - wird ein geeigneter Ester eingesetzt, beispielsweise ein Methylester, aber es sind auch viele andere Ester-Schutzgruppen möglich.

Dieses Derivat wird am Stickstoff zu einer Harnstoff-Vorstufe aktiviert, beispielsweise durch Umsetzung mit Triphosgen, Diphosgen oder Phosgen selbst zu einem Isocyanat, so dass durch nachfolgende Umsetzung mit dem Spiroamin A eine Harnstoffbildung erfolgt. Es sind aber auch viele weitere Arten der Aktivierung möglich, beispielsweise erhält man durch Umsetzung mit Carbonyldiimidazol das aktivierte Imidazolid, mit Chlorameisensäure-4-nitrophenylester das entsprechende Carbamat und mit diversen ähnlichen, aktivierenden Carbonat-Reagentien wie Bissuccinimidylcarbonat oder Bis-(4-nitrophenyl)-carbonat entsprechende Derivate. Die analogen schwefelhaltigen Derivate (Thioharnstoff-Analoge) lassen sich entsprechend durch die Verwendung von Thiophosgen erhalten.

Allerdings muss man berücksichtigen, dass die Stärke der Aktivierung durch die aufgeführten Reagenzien unterschiedlich ist, das heißt, die nachfolgende Umsetzung mit dem sekundären Spiro-Amin kann unterschiedlich schnell verlaufen oder auch sehr unterschiedliche Ausbeuten oder Nebenprodukte liefern.

Weiterhin könnte es notwendig sein, durch Silylierung des Amins mit beispielsweise Trimethylsilylchlorid oder Bis-trimethylsiliylacetamid oder Bis-Trifluormethyltrimethylsilylacetamid eine Voraktivierung zu erreichen, damit sich das eigentliche aktivierte Harnstoff-Vorprodukt leichter bilden kann. Darüber hinaus könnte es in manchen Fällen von Vorteil sein, von einer freien Aminosäure auszugehen und diese mit einem der genannten oder auch anderen geeigneten Silylierungsreagentien sowohl am Stickstoff wie auch an der Carbonsäure zu silylieren und anschließend das Isocyanat darzustellen.

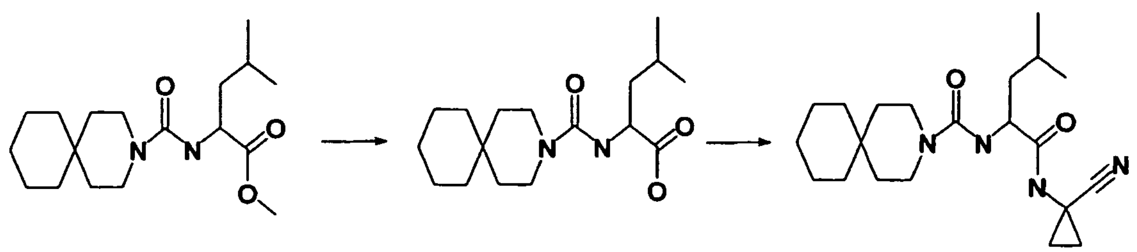

Als nächstes erfolgt die Spaltung des Esters unter Bedingungen, die möglichst keine Nebenreaktionen an dem Molekül hervorrufen, im Falle des Methylesters bevorzugt unter basischen Bedingungen, beispielsweise mit NaOH oder LiOH als Base, wobei es sinnvoll ist, Basenüberschüsse oder lange Reaktionszeiten zu vermeiden. Im Falle einer Silyl-Schutzgruppe kann es aber auch schon ausreichen, durch wässrige Behandlung, eventuell unter Zusatz von wenig Mineralsäure, eine Esterspaltung zu erreichen.

Bevor die Amidbindung mit der 3. Komponente, dem Aminonitril, geknüpft werden kann, erfolgt in der Regel nach der basischen Spaltung eine Freisetzung der Carbonsäure aus dem Salz mit einer Mineralsäure.

Anschließend kann mit einer Vielzahl der dem Fachmann bekannten Methoden die Peptidkupplung erfolgen, wie oben bereits erwähnt, gegebenenfalls unter Zusatz weiterer Hilfsreagenzien zur Erhöhung der Kupplungseffizienz oder der Racemisierungsunterdrückung.

Weg 1b:

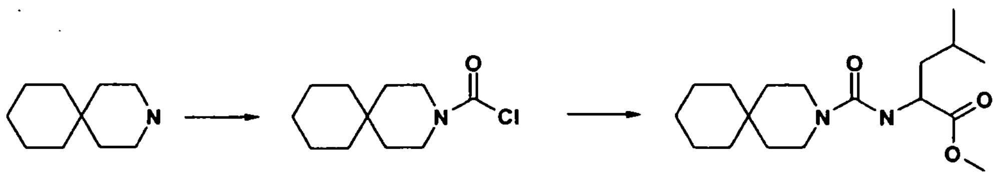

Es werden die gleichen Komponenten wie unter Weg 1a beschrieben eingesetzt. Im Unterschied zu Weg 1 a erfolgt jedoch die Aktivierung zur Harnstoff-Vorstufe nicht an der Komponente B, dem Aminosäure-Derivat, sondern an der Komponente A, dem Spiro-Amin. Da es sich jedoch um ein sekundäres Amin handelt, sind Reaktivitäten und Ausbeuten oftmals verändert gegenüber Weg 1a.

Beispielsweise ist es aber möglich, das Spiroamin als Chlorcarbonyl-Derivat durch Umsetzung mit Phosgen, Triphosgen oder Diphosgen zu aktivieren, und dieses anschließend mit dem oben erwähnten Aminosäure-Ester umzusetzen. Der oben erwähnte Aminosäure-Ester kann dabei auch in der aktivierten silylierten Form eingesetzt werden.

Die weitere Umsetzung, also Freisetzung der Carbonsäure und Kupplung mit der Aminkomponente C erfolgt dann wie unter 1 a beschrieben.

Weg 2a

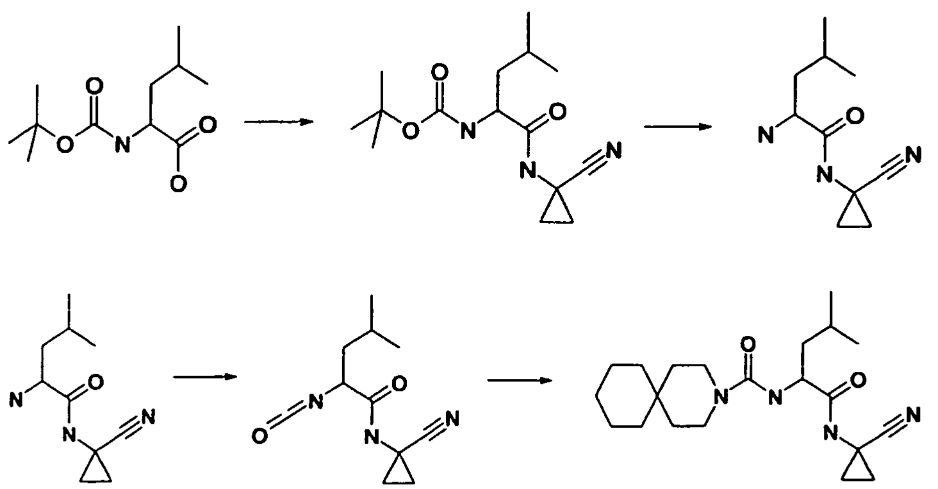

Ausgangsprodukt ist ein geeignetes N-terminal geschütztes Aminosäure-Derivat B. Dieses wird zunächst mit dem Aminonitril C gekuppelt; hierbei können wieder die bekannten Methoden der Peptidknüpfung eingesetzt werden. Anschließend wird die N-terminale Schutzgruppe abgespalten und es erfolgt Umsetzung zur aktivierten Harnstoff-Vorstufe, wie unter 1 a beschrieben. Hier ist es unter Umständen besonders von Bedeutung, die Schutzgruppen einzusetzen, deren Abspaltungsbedingungen mit den Funktionalitäten am Gesamtmolekül B-C kompatibel sind. Dieses aktivierte Derivat, wie beispielsweise das Isocyanat, wird anschließend mit dem Spiroamin zur erfindungsgemäßen Verbindung I umgesetzt.

Weg 2b

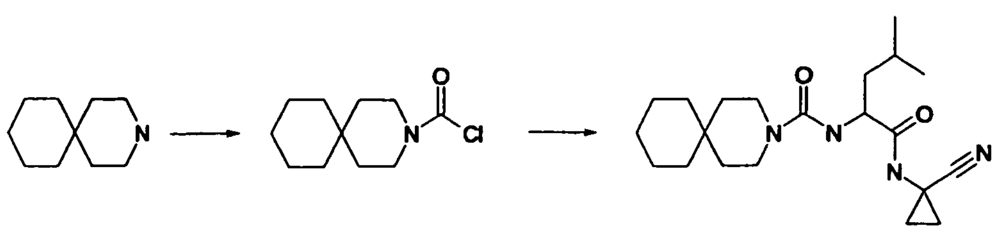

Alternativ ist es auch hier möglich, analog zu dem unter 1 b beschriebenen Weg zunächst das Spiro-Amin A zu aktivieren, um dann die Umsetzung mit der Komponente B-C zum Harnstoff durchzuführen.

Die Herstellung von Chlorsulfonylaminen oder erfindungsgemäßen Sulfonylharnstoffen erfolgt nach folgendem Schema:

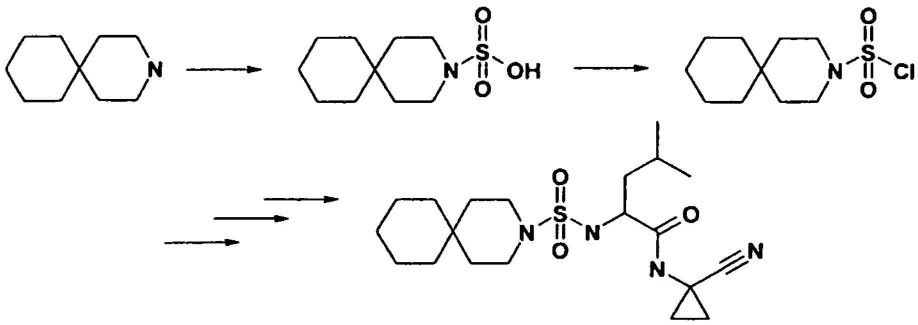

Die Synthese von Sulfonyl-Harnstoffen ist breit beschrieben. Eine häufig verwendete Methode geht von einer Aktivierung der Amine zu Sulfonylchloriden durch Umsetzung mit bevorzugt Chlorsulfonsäure und nachfolgender Chlorierung der erhaltenen Sulfonsäure mit beispielsweise Phosphorpentachlorid oder Phosphoroxychlorid aus, gefolgt von der Reaktion mit der zweiten Amino-Komponente und einer geeigneten Base. Ebenso kann auch $SO_3$ in der ersten Stufe der Synthese eingesetzt werden. Weiterhin ist es auch möglich, die gewünschte Umsetzung mit Sulfurylchlorid zu erreichen.

Bevorzugt wird dabei die Aktivierung an dem sekundären Spiro-Amin-Baustein vorgenommen, gefolgt von der Umsetzung beispielsweise des Aminosäure-Amins, geschützt als Ester. Anschließend erfolgt die Spaltung des Esters und Umsetzung mit dem Aminonitril-Baustein.

Alternativ ist es auch möglich, den aktivierten Spiro-Baustein mit dem Aminosäureamidonitril, hergestellt aus der N-terminal geschützten Aminosäure durch Kupplung mit dem Aminonitril-Baustein gefolgt von der Schutzgruppenabspaltung, umzusetzen.

Die Herstellung von bis-Amiden ausgehend von Aminosäure-Bausteinen erfolgt nach folgendem Schema:

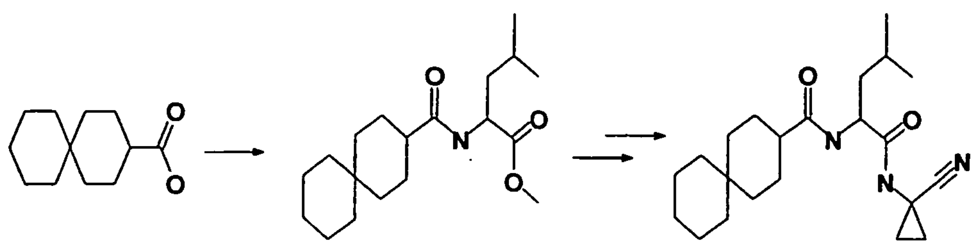

Liegt der Spiro-Baustein nicht als sekundäres Amin, sondern als Carbonsäure vor, erfolgt Anknüpfung an die Aminosäure über eine Amidbindung. Diese kann nun in der Synthese entweder zuerst erfolgen, in diesem Fall wird ein Aminosäureester eingesetzt, wie oben beschrieben in dem analogen Fall 1 b gefolgt von Esterspaltung und Amidkupplung mit dem Aminonitril-Baustein, oder, wenn ein Aminosäureamidonitril-Baustein eingesetzt wird, als zweite Kupplungsstufe in der Synthese, jeweils begleitet von entsprechenden Schutzgruppenmanipulationen, wie oben beschrieben in dem Fall 2b.

Optisch aktive Kohlenstoff-Atome in den erfindungsgemäßen Verbindungen der Formeln I und Ia können unabhängig voneinander in der R- oder S-Konfiguration vorliegen. Die Verbindungen der Formeln I und Ia können in Form der reiner Enantiomeren oder reiner Diastereomeren oder in der Form von Gemischen in beliebigen Anteilen der Enantiomeren und/oder Diastereomeren vorliegen, beispielsweise in Form ihrer Racemate oder enantiomeren Diastereomerenpaare. Die vorliegende Erfindung betrifft somit reine Enantiomere und Gemische der Enantiomere genauso wie reine Diasteromere und Gemische der Diasteromere. Ebenso umfasst die Erfindung Gemische von zwei oder von mehr als zwei Stereoisomeren der Formeln I und Ia und ebenfalls alle möglichen Mischungs-Verhältnisse dieser Stereoisomeren in den Gemischen. Für den Fall, dass die Verbindungen der Formeln I und Ia in Form von E- oder Z-Isomeren, oder cis- bzw. trans-Isomeren, oder als "Spiran" vorliegen, betrifft die Erfindung jeweils sowohl die reinen E- bzw. reinen Z-Isomeren, wie auch die reinen cis- oder reinen trans-Isomere, ebenso ganz analog die entsprechenden Spiro-Isomere, wie auch E/Z- bzw. cis/trans-Gemische in jedem Verhältnis. Ebenso umfasst die Erfindung alle tautomeren Formen der erfindungsgemäßen Verbindungen der Formeln I und Ia.

Die Verbindung der ormeln Ia wird, sofern sie als Gemisch von Diastereomeren oder Enantiomeren auftritt oder bei der gewählten Synthese als deren Gemische anfällt, in die reinen Stereoisomeren getrennt, entweder durch Chromatographie an einem gegebenenfalls chiralen Trägermaterial, oder, sofern die racemische Verbindung der Formeln Ia zur Salzbildung befähigt ist, durch fraktionierte Kristallisation der mit einer optisch aktiven Base oder Säure als Hilfsstoff gebildeten diastereomeren Salze. Als chirale Stationärphasen für die dünnschicht- oder säulenchromatographische Trennung von Enantiomeren eignen sich zum Beispiel modifizierte Kieselgelträger (sogenannte Pirkle-Phasen) sowie hochmolekulare Kohlenhydrate wie Triacetylcellulose. Für analytische Zwecke sind nach entsprechender, dem Fachmann bekannter Derivatisierung, auch gaschromatographische Methoden an chiralen Stationärphasen anwendbar. Zur Enantiomerentrennung der racemischen Carbonsäuren werden mit einer optisch aktiven, in der Regel kommerziell erhältlichen Basen wie (-)-Nicotin, (+)- und (-)-Phenyl-ethylamin, Chininbasen, L-Lysin oder L-und D-Arginin die unterschiedlich löslichen diastereomeren Salze gebildet, die schwerer lösliche Komponente als Feststoff isoliert, das leichter lösliche Diastereomer aus der Mutterlauge abgeschieden und aus den so gewonnenen diastereomeren Salzen die reinen Enantiomeren gewonnen. Auf prinzipiell gleiche Weise kann man die racemischen Verbindungen der Formeln I und Ia, die eine basische Gruppe wie z. B. eine Aminogruppe enthalten, mit optisch aktiven Säuren, wie z. B. (+)-Campher-10-sulfonsäure, D- und L- Weinsäure, D-und L- Milchsäure sowie (+) und (-)-Mandelsäure in die reinen Enantiomeren überführen. Auch kann man chirale Verbindungen, die Alkohol- oder Amin-funktionen enthalten, mit entsprechend aktivierten oder gegebenenfalls N-geschützten enantiomerenreinen Aminosäuren in die entsprechenden Ester oder Amide, oder umgekehrt chirale Carbonsäuren mit carboxygeschützten enantiomerenreinen Aminosäuren in die Amide oder mit enantiomerenreinen Hydroxycarbonsäuren wie Milchsäure, in die entsprechenden chiralen Ester überführen. Sodann kann die Chiralität des in enantiomerenreiner Form eingebrachten Aminosäure- oder Alkoholrestes zur Trennung der Isomeren genutzt werden, indem man eine Trennung der nunmehr vorliegenden Diastereomeren durch Kristallisation oder Chromatographie an geeigneten Stationärphasen vornimmt und danach den mitgeführten chiralen Molekülteil mittels geeigneter Methoden wieder abspaltet.

Weiterhin ergibt sich bei einigen der erfindungsgemäßen Verbindungen die Möglichkeit, zur Herstellung der Gerüststrukturen diastereo- oder enantiomerenrreine Ausgangsprodukte einzusetzen. Dadurch können auch andere oder vereinfachte Verfahren zur Aufreinigung der Endprodukte eingesetzt werden. Diese Ausgangsprodukte wurden zuvor nach literatur-bekannten Verfahren enantiomeren-oder diastereomerenrein hergestellt. Hierzu können beispielsweise auch enzymatische Verfahren eingesetzt werden. Entweder können solche enzymatischen Verfahren zum Einsatz kommen, die in einem Syntheseschritt enantio- oder diastereoselektiv verlaufen, d. h. selektiv eine Verbindung liefern. Oder aber, im Sinne einer kinetischen enzymatischen Synthese oder Spaltung derart, dass beispielsweise ein bereits vorliegendes Enantiomer oder Diastereomer stark bevorzugt in der enzymatischen Reaktion umgesetzt wird, beispielsweise im Sinne einer selektiven Acylierung bzw. Veresterung oder Acylspaltung bzw. Esterspaltung. Erfolgreich eingesetzt werden beispielsweise Acylierungsreaktionen mit Lipasen oder Acylase-Spaltungen von N-Acetylverbindungen, bzw. Protease-vermittelte Veresterungen in organischen Lösemitteln oder Esterspaltungen, aber auch viele andere Möglichkeiten sind denkbar.

Werden Aminosäure-Derivate eingesetzt, sind diese häufig bereits in enantiomerenreiner Form kommerziell erhältlich. Im Fall von nicht-proteinogenen Aminosäuren können diese jedoch oft aber auch aus enantiomeren- oder diastereomerenreinen natürlichen Vorstufen hergestellt werden, beispielsweise aus proteinogenen Aminosäuren oder auch anderen natürlichen chiralen Ausgangsprodukten des "chiral pool". Oder aber, diese Vorstufen werden durch eine der genannten Trennmethoden oder durch Anwendung von verschiedenartigen enzymatischen Verfahren in optisch reiner Form erhalten und in der Synthese entsprechend eingesetzt.

Saure oder basische Produkte der Verbindung der Formeln Ia können in Form ihrer Salze oder in freier Form vorliegen. Bevorzugt sind pharmakologisch verträgliche Salze, beispielsweise Alkali- oder Erdalkalimetallsalze bzw. Hydrochloride, Hydrobromide, Sulfate, Hemisulfate, alle möglichen Phosphate sowie Salze der Aminosäuren, natürlicher Basen oder Carbonsäuren. Die vorliegende Erfindung beinhaltet außerdem alle Solvate der Verbindungen der allgemeinen Formeln Ia, wie stöchiometrische oder nicht-stöchiometrische Hydrate oder Alkohol-Addukte.

Die Herstellung physiologisch verträglicher Salze aus zur Salzbildung befähigten Verbindungen der Formeln Ia, einschließlich deren stereoisomeren Formen erfolgt in an sich bekannter Weise. Die sauren Verbindungen der Formeln la bilden mit basischen Reagenzien wie Hydroxiden, Carbonaten, Hydrogencarbonaten, Alkoholaten sowie Ammoniak oder organischen Basen, beispielsweise Methylamin, Dimethylamin, Ethylamin, Trimethyl- oder Triethylamin, Ethanolamin, Diethanolamin oder Triethanolamin, Trometamol oder auch basischen Aminosäuren, etwa Lysin, Ornithin oder Arginin, stabile Alkali-, Erdalkali- oder gegebenenfalls substituierte Ammoniumsalze. Sofern die Verbindungen der Formeln Ia basische Gruppen aufweisen, lassen sich mit starken Säuren auch stabile Säureadditionssalze herstellen. Hierfür kommen sowohl anorganische als auch organische Säuren wie Chlorwasserstoff-, Bromwasserstoff-, Schwefel-, Hemischwefel-, Phosphor-, Salpeter-, Methansulfon-, Benzolsulfon-, p-Toluolsulfon-, 4-Brombenzol-sulfon-, Cyclohexylamidosulfon-, Trifluormethylsulfon-, 2-Hydroxyethansulfon-, Ameisen-, Essig-, Oxal-, Wein-, Bernstein-, Glycerolphosphor-, Milch-, Äpfel-, Adipin-, Citronen-, Fumar-, Malein-, Malon-, Benzoe-, Sorbin-, Glucon-, Glucuron- Palmitin-, Stearin-, oder Trifluoressigsäure in Frage. Hydrate der Verbindungen der Formeln Ia lassen sich beispielsweise durch

(Um-)Kristallisation aus einem organisch-wässrigem Lösemittelgemisch herstellen, beispielsweise durch Verwendung solcher organischer Lösemittel wie Dioxan, Tetrahydrofuran, Ethanol oder Methanol.

Umgekehrt ist es auch möglich, freie Säure- oder freie Basenformen der Verbindungen der Formeln Ia aus den entsprechenden Salzen herzustellen. Beispielsweise kann eine Verbindung der Formeln Ia aus ihrer Säure-Salz-Form durch Behandlung mit geeigneten Basen (wie Ammoniak-Lösung, NatriumhydroxidLösung) freigesetzt werden. In manchen Spezialfällen kann auch eine Behandlung mit Oxiranen eine Freisetzung bewirken, beispielsweise können Hydrochloride durch Behandlung mit Methyloxiran freigesetzt werden, besonders im Falle von AminosäureDerivaten. Verbindungen der Formeln Ia, die in Form ihrer Base-Salz-Form vorliegen, können durch Behandlung mit geeigneten Säuren (Zitronensäure, Salzsäure oder Schwefelsäure) in die freie Säure-Form überführt werden.

Stickstoff-Verbindungen der Formeln Ia können auch in Form ihrer N-Oxide vorliegen. Diese lassen sich durch verschiedene Verfahren herstellen, die dem Fachmann bekannt sind. Beispielsweise kann eine nicht-oxidierte Form einer Verbindung der Formeln Ia durch Behandlung mit einem geeigneten Oxidationsmittel (Trifluorperessig-säure, Permaleinsäure, Perbenzoesäure, Peressigsäure, Meta-Chlorperbenzoesäure) in geeigneten inerten organischen Solventen zu den korrespondierenden N-Oxiden oxidiert werden. Alternativ können die N-Oxide der Verbindungen der Formeln Ia auch dadurch hergestellt werden, dass Ausgangs- oder Zwischenprodukte in Form ihrer N-Oxide eingesetzt werden oder als solche hergestellt werden.

Verbindungen der Formeln Ia in ihrer nicht-oxidierten Form können auch aus den N-Oxiden der Verbindungen der Formeln Ia durch Behandlung mit reduzierenden Reagenzien (wie Schwefel, Schwefeldioxid, Triphenylphosphin, verschiedenen Borhydriden, Phosphortrichlorid oder -bromid und ähnlichen) in geeigneten inerten organischen Lösemitteln hergestellt werden.

Verbindungen der Formeln Ia die gleichzeitig eine basische und eine saure Gruppe tragen, beispielsweise eine Amino- oder Guanidino- und eine CarboxylGruppe, können ebenfalls in Form ihrer Zwitterionen (Betaine) vorliegen, die ebenfalls im Umfang der vorliegenden Erfindung enthalten sind.

Ebenso umfasst die Erfindung alle Salze der Verbindungen der Formeln Ia, die wegen ihrer geringen physiologischen Tolerierbarkeit nicht direkt in pharmazeutischen Wirkstoffen eingesetzt werden können, aber beispielsweise als Intermediate auf dem Weg zur Herstellung der erfindungsgemäßen Verbindungen eingesetzt werden, oder auch als Ausgangsprodukte zur Synthese der physiologisch tolerierbaren Salze.

Die Erfindung betrifft auch Arzneimittel, gekennzeichnet durch einen wirksamen Gehalt an mindestens einer Verbindung der Formeln Ia und/oder eines physiologisch verträglichen Salzes der Verbindung der Formeln Ia und/oder eine gegebenenfalls stereoisomere Form der Verbindung der Formeln Ia, zusammen mit einem pharmazeutisch geeigneten und physiologisch verträglichen Trägerstoff, Zusatzstoff und/oder anderen Wirk- und Hilfsstoffen.

Aufgrund der pharmakologischen Eigenschaften eignen sich die erfindungsgemäßen Verbindungen zur Prophylaxe, Sekundär-Prevention und Therapie all solcher Erkrankungen, die durch eine Hemmung der Cystein-Proteasen, besonders der Cathepsine, behandelbar sind. Sie eignen sich sowohl für eine akute Behandlung als auch für eine Langzeittherapie. Cathepsin-Inhibitoren können eingesetzt werden bei abnormal erhöhtem Knochenabbau, Allergien, der Alzheimer'schen Krankheit, Amyloidosis, ARDS, Arterieller Thrombose, Asthma, Atheromen, Atherosklerose, autoimmun-Erkrankungen, bakteriellen Infektionen, Bronchiolitis, Cerebraler Hämorrhagie, Cerebrovaskulärer Ischämie, Chorea-Huntington, chronischen Entzündungen, CIPD (chronischer entzündlicher demyelinisierende Polyradiculoneuropathie), Creutzfeldt-Jakob-Krankheit, der Crohn'schen Krankheit, Diabetes (besonders der juvenilen Form), Emphysem, Encephalomyelitis, Endometriose, entzündlichen Atemwegserkrankungen, entzündlicher Pankreatitis, Epilepsie, Erkrankungen, die durch verstärkte Angiogenese gekennzeichnet sind, exzessiver Atemwegs-Elastolyse, Gewebsverpflanzungen, Gingivitis, Glomerulonephritis, glucocorticoid induzierter Osteoporose, Graves' Krankheit, Guillain-Barre-Syndrom, Hashimotos Thyroiditis, Hepatitis, HIV-Infektion, Huntington-Krankheit, Hypercalcämie, IBD, Immunschwäche, Interstitieller Cystitis, Knochenbruch, Knochenverlust, Krebserkrankungen, Lupus Erythematosus, Malaria, metachromer Leukodystrophie, metastatisierender Osteogenese, Metastatisierung, Multipler Sklerose, multiplem Myelom, Muskel-Dystrophie, Myasthenia gravis, Neurodegenerativen Erkrankungen, neuropathischem Schmerz (besonders chronisch; aber auch diabetische Neuropathie, postherpetische Neuralgie, trigeminale Neuralgie, schmerzhafte diabetische Polyneuropathie, Schmerz nach Schlaganfall, Postamputationsschmerz, myelopathischer oder radiculopathischer Schmerz, atypischer Gesichtsschmerz und Causalgie-ähnliche Syndrome), Organabstoßung bei Transplantationen, Osteoarthritis, Osteogenesis imperfecta, Osteoporose, Paget's Krankheit, Pankreatitis, Parkinson-Krankheit, Pemphigus vulgaris, Periodontitis, Plaque-Ruptur, Pneumocystisis Carinii, Pneumonitis, Psoriasis, Restenose, Rheumatoider Arthritis, Skleroderma, systemischem Lupus erythematosus, Traumata (Hirn-, Rückenmarks-), Tumorzell-Invasion, viralen Infektionen, Zahnverlust sowie bevorzugt, aber nicht ausschließlich, bei folgenden Krebsarten: Brustkrebs, Darmkrebs, Eierstockkrebs, Gebärmutterhalskrebs, Hautkrebs, Hirntumor, Kaposi-Sarkom, Leukämie (B- und T-Zell-), Lungenkrebs, Lymphknotenkrebs, Pankreas-Krebs, Prostata-Krebs und Sarkomen.

Da viele Verbindungen dieser Erfindung besonders Inhibitoren der Cystein-Cathepsine B, K und S sind, können bevorzugt Erkrankungen behandelt werden, bei denen die genannten Cathepsine zur Pathologie oder/und Symptomatik beitragen. Dies betrifft besonders: Schmerz, insbesondere neuropathischer Schmerz, Osteoarthritis, Osteoporose und

verschiedene Krebsarten. Dies betrifft ebenso verschiedene (Auto)-Immunkrankheiten, besonders aus dem rheumatoiden Formenkreis, die oben ebenfalls bereits aufgeführt sind, und Erkrankungen, die durch eine exzessive Elastolyse gekennzeichnet sind, besonders der COPD-Formenkreis und verwandte, oben aufgeführte Erkrankungen, sowie kardiovaskuläre Erkrankungen, die durch Gefäßveränderungen gekennzeichnet sind, wie Atherosklerose.

Die Applikation der erfindungsgemäßen Arzneimittel kann extravaskulär erfolgen, beispielsweise intramuskulär, subkutan, intraokular, intraartikulär, intrasynovial, peroral, oral (bukkal, perlingual, sublingual), rektal, vaginal, (trans-)dermal, pulmonal (inhalativ) oder nasal oder intravaskulär, beispielsweise intravenös, intraarteriell, oder intrakardial, jeweils als Injektion oder Infusion. Bevorzugt ist dabei die orale Applikationsform.

Die Erfindung betrifft auch ein Verfahren zur Herstellung eines Arzneimittels, das dadurch gekennzeichnet, dass man mindestens eine Verbindung der Formeln Ia mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuss- oder Sesamöl, Polyethylenglykol und Lösungsmittel wie etwa steriles Wasser und ein- oder mehrwertige Alkohole wie Glycerin, genannt.

Weiterhin ist es auch möglich, besonders bei der Herstellung von Suspensionen, Verbindungen mit ganz bestimmten, speziell hergestellten Oberflächeneigenschaften einzusetzen. Dazu zählen beispielsweise Trocken- und Naßmahlung, Mikronisierung, Sprütrocknung, Herstellung von Nanokristallen und ähnliche Prozesse, bei denen durch Veränderung der Oberflächeneigenschaften beispielsweise Löslichkeiten oder insbesondere Lösungskinetiken verbessert werden können, wodurch beispielsweise eine verbesserte Aufnahme der entsprechenden Verbindung in den Organismus erreicht werden kann.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formeln I und la enthält. Bei festen Dosierungseinheiten wie Tabletten, Kapseln, Dragees oder Suppositorien, kann diese Dosis bis zu etwa 5000 mg, bevorzugt jedoch etwa 50 bis 1000 mg und bei Injektionslösungen in Ampullenform bis zu etwa 500 mg, vorzugsweise aber etwa 20 bis 200 mg, betragen.

Für die Behandlung eines erwachsenen, etwa 70 kg schweren Patienten sind je nach Wirksamkeit der Verbindung gemäß Formeln Ia, Tagesdosen von etwa 2 mg bis 5000 mg Wirkstoff, bevorzugt etwa 10 mg bis 1000 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Inhibitoren der vorstehenden Art können sowohl als Monotherapie als auch in Kombination oder gemeinsam mit anderen Arzneistoffen verabreicht werden.

Endprodukte werden in der Regel durch massenspektrometrische Methoden (FAB-, ESI-MS) und $^1$H-NMR (in der Regel, wenn nicht anders angegeben, 500 MHz, in DMSO-D6) bestimmt, angegeben sind jeweils der Hauptpeak oder die beiden Hauptpeaks. Temperaturangaben in Grad Celsius. Verwendete Abkürzungen sind entweder erläutert oder entsprechen den üblichen Konventionen.

Ausgangsprodukte oder Synthesezwischenprodukte sind entweder käuflich erhältlich oder werden wie zitiert oder beschrieben hergestellt.

1-Oxa-8-aza-spiro[4.5]decan, 2-Oxa-8-aza-spiro[4.5]decan, 2-Oxa-7-aza-spiro[4.5]decan, 1-Oxa-7-aza-spiro [4.5]decan, 2-Oxa-7-aza-spiro[3.5]nonan sowie analoge oder Alkyl-substituierte oder -disubstituierte Derivate werden wie in WO01/87838 beschrieben, hergestellt.

1,4-Dioxa-8-aza-spiro[4.5]decan, 1-Oxa-4,8-diaza-spiro[4.5]decan, 1,5-Dioxa-9-aza-spiro[5.5]undecan, 1-Oxa-5,9-diaza-spiro[5.5]undecan bzw. ähnliche und substituierte Derivate werden wie in EP 0621267 hergestellt. Ebenso erfolgreich kann auch die bekannte Methode durch Erhitzen der Keto-Vorstufe (z. B. das geschützte Piperidon) mit Diolen und katalytischer Menge p-Toluolsulfonsäure am Wasserabscheider angewendet werden.

3-Aza-spiro[5.5]undecan ist beispielsweise auch aus 3-Aza-spiro[5.5]undecan-2,4-dion zugänglich, gleiches gilt für ähnliche carbocyclische Spiroverbindungen. Die Reduktion kann mit $LiAlH_4$ erfolgen.

2-Methyl-2,8-diaza-spiro[4.5]decan-1-on, in 2-Position anderweitig substituierte Derivate, analoge Harnstoffe und Amine sind in J. Med. Chem. 47 (8), 2037-61 (2004) beschrieben oder können aus den hier beschriebenen Produkten durch weitere Umsetzungen, beispielsweise Reduktion, gewonnen werden. Ebenso sind in dieser Publikation substituierte Spiro-Hydantoine und Lactame mit invertierter Amidbindung (also 2,8-Diaza-spiro[4.5]decan-3-on und Derivate) sowie Imide beschrieben.

Viele verschiedene spirocyclische Indane, Indene, Tetralone und Tetraline sind kommerziell erhältlich.

Die Herstellung von 6-Azaspiro[2.5]octan sowie viele weitere Spiroverbindungen sind in Bull. Soc Chim. France 10, 2572-81 (1964) beschrieben.

Verwendete Abkürzungen:

| | |
|---|---|
| Bis(2-methoxyethyl)aminosulfur trifluorid | BAST |
| tert-Butyl | tBu |
| 2,2'-Bis(diphenylphoshino-1,1'-binaphthyl | Binap |
| Bis-(oxo-3-oxazolidinyl)-phosphorylchlorid | BOP-Cl |
| Dibenzylidenaceton | dba |
| Dichlormethan | DCM |
| Dicyclohexyl-carbodiimid | DCC |
| Diethylphosphorylcyanid | DEPC |
| Diisopropylethylamin | DIPEA |
| 4-Dimethylaminopyridin | DMAP |
| N, N-Dimethylformamid | DMF |
| Dimethylsulfoxid | DMSO |
| 1,1'-Bis(diphenylphosphino)ferrocen | DPPF |
| 1-(3-Dimethylaminopropyl)-3-ethylcarbodiimid -Hydrochlorid | EDCI |
| Äquivalente | eq. |
| gesättigt | ges. |

(fortgesetzt)

| | |
|---|---|
| O-(7-Azabenzotriazol-1-yl)-N,N,N',N'-tetramethyluronium-hexafluorophosphat | HATU |
| 7-Aza-1-Hydroxybenzotriazol | HOAt |
| Lithium diisopropylamin | LDA |
| Methanol | MeOH |
| Methylmagnesiumchlorid-Lösung | MeMgCl-Lsg. |
| Methyl-tert.butylether | MTBE |
| N-Bromsuccinimid | NBS |
| N-Chlorsuccinimid | NCS |
| N-Iodsuccinimid | NIS |
| N-Ethylmorpholin | NEM |
| Raumtemperatur 20 ˚C to 25 ˚C | RT |
| Singulett breit | $S_b$ |
| Tetrahydrofuran | THF |
| Trifluoressigsäure | TFA |
| O-((Ethoxycarbonyl)cyanomethyleneamino)-N,N,N',N'-tetramethyluronium tetrafluoroborat | TOTU |

Beispiele

Die im folgenden ols "Beispiele" bezeichnelen Verbindungen sind nur dann Aus führungsbeispiele, wenn sie unter die Definition der Formel (Ia) gemäss Anspruch 1 fallen.

Die nachfolgenden Beispiele wurden analog zu den vorher genannten allgemeinen oder speziellen Vorschriften hergestellt.

Beispiel 1: (S)-2-Benzyloxycarbonylamino-3-chlorocarbonyl-propionsäuremethylester

Z-Asp-OMe (175 g, 622 mmol) wurde in THF gelöst (1750 ml) und mit 2 ml DMF versetzt. 86,9 g (684 mmol) Oxalylchlorid wurden in 200 ml THF gelöst (leicht exotherm), auf Raumtemperatur (RT) zurückgekühlt und über 1 Stunde (h) zur Lösung der Aminosäure zugetropft. Nach einer weiteren Stunde bei RT wurde zunächst für 10 min mit Stickstoff begast, die Lösung am Rotationverdampfer bei maximal 30 ˚C eingeengt und mehrfach mit Toluol koevaporiert. Der resultierende schmutzigweiße Feststoff wurde im Hausvakuum (etwa 1,5 mBar) bis zu konstantem Gewicht getrocknet und ohne weitere Reinigung weiter umgesetzt. Ausbeute: 186,6 g (quantitativ).

Beispiel 2: (S)-2-Benzyloxycarbonylamino-4-oxopentansäuremethylester

Kupfer-(1)-bromid (60,9 g, 425 mmol, 1,2 Äquivalente (eq.)) wurde in eine 3 Liter, 4-Hals-Flasche (mit Tropftrichter, Innenthermometer, Argon-einlass, Gummi-Septa, im Kühlbad) gebracht und mit 250 ml THF versetzt. In einem separaten Kolben wurden bei 10 ˚C Lithiumbromid (75 g, 864 mmol, 2,4 eq.) vorgelegt und unter Argon mit 470 ml THF versetzt. Nach erfolgtem Lösen und Rückkühlung auf RT wurde diese Lösung zur Suspension des Kupferbromids überführt. Die erhaltene farblose bis leicht grünliche Lösung wurde auf -60 ˚C gekühlt und Methylmagnesiumchlorid (141 ml einer 3,0 M Lösung in THF, 423 mmol) wurde innerhalb von 15 min zugegeben. Es bildete sich ein dicker, gelblicher Niederschlag und die Temperatur stieg auf RT an. Nach erneuter Kühlung auf -60 ˚C wurde die Lösung aus Beispiel 1 (hergestellt aus 106 g (S)-2-Benzyloxycarbonylamino-3-chlorocarbonyl-propionsäuremethylester, 353 mmol, und 360 ml THF) über etwa 30 min zugegeben und die Temperatur unter -25 ˚C gehalten. Nach Beendigung der Zugabe wurde die Mischung 1 h lang nachgerührt, und bei -15 ˚C wurde festes Ammoniumchlorid (30 g) zugegeben. Nach weiteren 2 h wurde abfiltriert, das Filtrat mit 400 ml Heptan verdünnt und 200 ml gesättigte Ammoniumchlorid-Lösung zugegeben. Nachdem 1 h bei RT gerührt wurde, wurde die organische Phase abgetrennt, mehrfach nachgewaschen und die wässrigen Phasen wurden mit Essigsäureethylester rückextrahiert. Die vereinigten organischen Phasen wurden über Natriumsulfat getrocknet, unter vermindertem Druck am Rotationsverdampfer eingeengt und an 1 kg Kieselgel mit 1:3 bis 1:1 Essigester/Heptan chromatographiert. Nach Vereinigung der Produktfraktionen und Trocknen unter vermindertem Druck erhielt man 83 g (84 % Ausbeute) eines weißen Feststoffes. $^1$H-NMR (300 MHz, $CDCl_3$): 7,4 (m, 5H); 5,78 (m, 1H); 5,18 (s, 2H); 4,6 (s, 1 H); 3,75 ("s", 3H); 3,3-2,9 (2dd, 2H); 2,2 (s, 3H)

Beispiel 3: (S)-2-Benzyloxycarbonylamino-4-oxo-heptansäuremethylester

Die Herstellung erfolgte analog zu Beispiel 2, mit dem Unterschied, dass nun N-Propylmagnesiumchlorid (2 M in Diethylether) eingesetzt wurde und auf eine chromatographische Reinigung verzichtet wurde. Stattdessen wurde das erhaltene dunkle Rohprodukt in 1:1 DCM/Heptan aufgenommen und über Celite filtriert. Nach Behandlung mit Aktivkohle und Abfiltrieren erhielt man das gewünschte Produkt in 93 %iger Ausbeute.
$^1$H-NMR (300 MHz, $CDCl_3$): 7,4 (m, 5H); 5,78 (m, 1H); 5,17 (s, 2H); 4,6 (s, 1 H); 3,73 ("s", 3H); 3,3-2,9 (2dd, 2H); 2,4 (m, 2H); 1,6 (m, 2H, überlagert); 0,9 (m, 3H)

Beispiel 4: (S)-2-Benzyloxycarbonylamino-4,4-difluorpentansäuremethylester

BAST (Deoxofluor, 60 g, 271 mmol, 2,95 eq.) wurde in einem 1 Liter-Kolben aus inertem Kunststoff vorgelegt und mit dem Keton aus Beispiel 2, gelöst/suspendiert in 40 ml Dichlormethan, versetzt. Nach einer Reaktionszeit von 1 Tag sowie erneut nach 12 h wurden jeweils 25 ml (113 mmol) BAST zugegeben und bei RT weitergerührt. Nach weiteren 12 h wurde Dichlormethan zugegeben und die erhaltene Lösung schnell in eine eiskalte gesättigte Natriumhydrogencarbonat-Lösung getropft, wobei die Temperatur unter 30 ˚C gehalten wurde. Die organische Phase wurde abgetrennt, die wässrige Phase mehrfach mit Dichlormethan extrahiert und die vereinigten organischen Phasen mit Wasser, 1 N HCl und gesättigter NaCl-Lösung gewaschen, über Natriumsulfat getrocknet und unter vermindertem Druck eingedampft. Das braune Öl wurde anschließend an Kieselgel chromatographiert (3:1 Heptan/MTBE bis 2:1).
Produktfraktionen wurden vereinigt und unter vermindertem Druck eingedampft. Ausbeute: 22,3 g, 56 %. $^1$H-NMR (300 MHz, $CDCl_3$): 7,4 (m, 5H); 5,45 (m, 1H); 5,18 (s, 2H); 4,6 (s, 1 H); 3,78 ("s", 3H); 2,4 (m, 2H); 1,7 (m, 3H)

Beispiel 5: (S)-2-Benzyloxycarbonylamino-4,4-difluorheptansäuremethylester

Die Herstellung erfolgte analog zu Beispiel 4.
$^1$H-NMR (300 MHz, $CDCl_3$): 7,4 (m, 5H); 5,46 (m, 1 H); 5,17 (s, 2H); 4,6 (m, 1 H); 3,77 ("s", 3H); 2,4 (m, 2H); 1,8 (m, 2H, überlagert); 1,5 (m, 2H); 0,95 (m, 3H)
Alle weiteren Umsetzungen in der Reihe der 4,4-Difluorheptansäure-Derivate zu den erfindungsgemäßen Endprodukten wurden ganz analog zu den entsprechenden Pentansäure-Derivaten durchgeführt.

Beispiel 6: (S)-2-Amino-4,4-difluorpentansäuremethylester Hydrobromid

10 g der Verbindung aus Beispiel 4 wurden bei RT mit 35 ml 33.%iger HBr in Eisessig versetzt und für 40 min gerührt. Anschließend wurden 400 ml kalter Diethylether zugegeben und die Reaktionsmischung für 3 h bei 4 ˚C aufbewahrt. Das ausgefallene Produkt wurde über eine Glasfritte abgesaugt, gut mit kaltem Ether nachgewaschen und unter vermindertem Druck von Lösemittelresten befreit. Es kann direkt in der nachfolgenden Reaktion eingesetzt werden. Ausbeute: 6,67 g (82 %) $^1$H-NMR: 8.5 ($s_b$, 3H); 4.3 (t, 1H); 3.76 (s, 3H); 2.53 (m, 2H); 1.7 (t, 3H).

Beispiel 7: (S)-4,4-Difluor-2-isocyanatopentansäuremethylester

Die Verbindung aus Beispiel 6 (6,76 g, 28,8 mmol) wurde vorgelegt und in 240 ml Dichlormethan und 9,46 ml (117 mmol, 4,05 eq.) Pyridin gelöst, im Eisbad auf 0 ˚C heruntergekühlt für 15 min und mit 19,86 ml einer 20 %igen Phosgen-Lösung in Toluol (37,54 mmol, 1,3 eq.) innerhalb von 20 - 30 Sekunden versetzt. Für 2 h wurde bei 0 ˚C nachgerührt, anschließend das Reaktionsgemisch mit kalter halbmolarer HCl und Eis extrahiert. Die Wasserphasen wurden mit Dichlormethan reextrahiert, die vereinigten organischen Phasen mit Eis und gesättigter NaCl-Lösung gewaschen, anschließend über $MgSO_4$ getrocknet und vom Trockenmittel abfiltriert. Nach dem Eindampfen unter vermindertem Druck verblieben 5,21 g braunes Öl (entsprach einer Ausbeute von
94 %), das direkt weiter umgesetzt wurde.

Beispiel 8: (S)-2-[(3-Aza-spiro[5.5]undecan-3-carbonyl)-amino]-4,4-difluoropentansäure

252 mg (1,3 mmol) des Isocyanats aus Beispiel 7 wurden bei 0 ˚C in 5 ml THF gelöst und mit 109 mg (3,25 mmol, 2,5 eq.) Natriumhydrogencarbonat und 209 mg (1,37 mmol, 1,05 eq.) 3-Aza-spiro[5.5]undecan versetzt. Man rührte über Nacht, trennte von ausgefallenen Salzen ab und behandelte die Reaktionsmischung direkt mit 2,5 ml 1 M LiOH-Lösung (2,5 mmol, 1,9 eq.). Die Reaktion wurde per HPLC-MS kontrolliert. War der Massen-Peak des Eduktes vollständig verschwunden, wurde mit verdünnter HCl vorsichtig angesäuert und das Produkt durch Extraktion mit Essigester, Trocknen der organischen Phase über Natriumsulfat und Eindampfen unter vermindertem Druck isoliert. Ausbeute: 440 mg (quantitativ). Das Rohprodukt wurde direkt in der folgenden Amidkupplung eingesetzt.

Beispiel 9: 3-Aza-spiro[5.5]undecan-3-carbonsäure [(S)-1-(1-cyano-cyclopropyl-carbamoyl)-3,3-difluorbutyl]-amid

Das Rohprodukt aus Beispiel 8, (S)-2-[(3-Aza-spiro[5.5]undecan-3-carbonyl)-amino]-4,4-difluoropentansäure), wurde in 6 ml THF/DMF 2:1 gelöst und mit 161,8 mg (1,365 mmol, 1,05 eq.) 1-Amino-1-cyclopropylnitril-Hydrochlorid und 176,9 mg (1,3 mmol, 1 eq.) 1-Hydroxy-7-azabenzotriazol versetzt, auf 0 °C gekühlt und mit 250 mg (1,3 mmol, 1 eq.) EDCI sowie mit 0,496 ml (3,9 mmol, 3 eq.) N-Ethylmorpholin versetzt. Anschließend wurde für 2 h bei 0 °C bis RT gerührt, THF wurde unter vermindertem Druck abdestilliert, Essigester zugegeben und mit stark verdünnter HCl, ges. $NaHCO_3$-Lösung und ges. NaCl-Lösung ausgeschüttelt. Nach dem Einengen der organischen Phase wurde direkt eine präparative HPLC-Trennung (Merck Hibar Purospher RP18, 250x25, Standard-Gradient Acetonitril-Wasser-TFA) durchgeführt. Produkt-Fraktionen wurden vereinigt und gefriergetrocknet. Ausbeute: 222 mg, 43 % d. Theorie. $^1$H-NMR: 8,82 (s, 1 H); 6,6 (s, 1 H); 4,31 (m, 1 H); 3,28 (ungefähr, 4H); 2,29 (m, 2H); 1,7-1,1 (mm, ungefähr 20H); MS (ESI$^+$): 397,17

Beispiel 10: 8-Aza-spiro[4.5]decan-8-carbonylchlorid

1,57 ml Phosgen (20 %ige Lösung in Toluol, 2,96 mmol) wurden in 5 ml Dichlorethan vorgelegt und auf - 20 °C abgekühlt, anschließend wurde eine Mischung aus 8-Aza-spiro-[4,5]-decan (750 mg, 2,96 mmol) und Triethylamin (1,28 ml, 9,2 mmol, 3,1 eq.) langsam zugegeben. Nach 30 min wurde auf RT kommen lassen, nach 1 h war laut LC-MS noch etwas Ausgangsprodukt enthalten, aber auch drei neue Peaks.

Anschließend wurde auf 2 N wässrige HCl gegeben, die wässrige Phase wurde mit Dichlormethan extrahiert, die organische Phase wurde mit gesättigter $NaHCO_3$-Lösung gewaschen und über Natriumsulfat getrocknet; anschließend wurde unter vermindertem Druck einrotiert. Rohausbeute: 592 mg

Das Material wurde ohne weitere Aufreinigung direkt weiter umgesetzt. Allgemeine Arbeitsvorschrift: siehe Beispiel 19

Beispiel 11: (S)-4-Carboxymethyl-5-oxo-oxazolidin-3-carbonsäurebenzylester

Die Verbindung ist käuflich erhältlich oder kann nach Literaturvorschriften durch Refluxieren von Z-Asp-OH mit Paraformaldehyd am Wasserabscheider in Benzol hergestellt werden.

Beispiel 12: (S)-4-Chlorocarbonylmethyl-5-oxo-oxazolidin-3-carbonsäurebenzylester

Die Verbindung aus Beispiel 11 wurde analog wie in Beispiel 1 beschrieben zu (S)-4-Chlorocarbonylmethyl-5-oxo-oxazolidin-3-carbonsäurebenzylester umgesetzt. Das so erhaltene Produkt wurde ohne weitere Reinigung in der anschließenden Reaktion eingesetzt.

Beispiel 13: (S)-5-Oxo-4-(2-oxopropyl)-oxazolidin-3-carbonsäurebenzylester

CuBr (24,54 g, 168 mmol, 1,2 eq.) und LiBr (29,18 g, 336 mmol, 2,4 eq.) wurden im ausgeheizten Kolben unter Argon vorgelegt, anschließend in 600 ml absolutem THF gelöst und für 20 min bei RT gerührt. Man erhielt eine klare gelb-orange Lösung. Dann wurde auf -78°C gekühlt und tropfenweise MeMgCl-Lösung (55,46 ml, 168 mmol, 1,2 eq.) dazugegeben. Dies führte zu einer gelben, schwer zu rührenden Suspension, so dass weitere 75 ml THF zugetropft wurden; diese wurde anschließend für 15 min bei -60 °C gerührt. Das in etwa 150 ml THF gelöste Carbonsäurechlorid aus Beispiel 12 (41,67 g, 140 mmol) wurde bei - 60°C vorgerührt und dann langsam zugetropft. Die so entstandene Reaktionsmischung wurde 1 h bei dieser Temperatur gerührt. Die Aufarbeitung erfolgte durch Zugabe von etwa 100 ml gesättigter $NH_4Cl$-Lösung, es wurde für 10 min bei -60 °C kräftig gerührt, dann wurde 200 ml Heptan und 60 ml Wasser dazugegeben und weitere 15 min bei RT gerührt. Die Phasen wurden getrennt, die wässrige Phase mit 50 ml 1 M HCl versetzt (grüne Lösung) und zweimal mit etwa 100 ml Essigester extrahiert. Die vereinigten organischen Phasen wurden mit 2 M HCl-Lösung, gesättigter $NaHCO_3$- und gesättigter NaCl-Lösung gewaschen, über Natriumsulfat getrocknet und unter vermindertem Druck eingedampft. Anschließend wurde über eine Kieselgel Flash-Chromatographie gereinigt (Heptan/Ethylacetat 5:1-2:1); Produktfraktionen wurden vereinigt und vermindertem Druck von Lösemittelresten befreit. Ausbeute: 21,6 g, 56 % der Theorie
$^1$H-NMR (250 MHz, 390 K, DMSO-d6): 7,35 (s, 5H); 5,43; 5,22 (2 d, 2H); 5,13 (d, 2H); 4,4 (dd, 1 H); 3,26, 3,04 (je 2 dd, 2H); 2,07 (s, 3H).

Beispiel 14: (S)-4-(2,2-Diftuorpropyl)-5-oxo-oxazolidin-3-carbonsäurebenzylester

15,52 g der Verbindung aus Beispiel 13 (56 mmol) wurden in 5 ml Dichlormethan suspendiert und unter Rühren mit 25 g (20,8 ml, 2,02 eq.) BAST versetzt. Für 14 Tage wurde unter Argon gerührt. Zwischendurch zeigten LCMS-Spektren, dass die Umsetzung nach 3 und 9 Tagen noch nicht vollständig war. Zur Aufarbeitung wurde die Reaktionslösung in eine gekühlte Natriumhydrogencarbonat-Lösung getropft (starke Gasentwicklung) und für etwa 30 min nachgerührt (keine Gasentwicklung), die wässrige Lösung wurde dann zweimal mit DCM ausgeschüttelt. Die wässrige Phase wurde mit verdünnter HCl-Lösung auf pH 3 eingestellt und erneut zweimal mit DCM ausgeschüttelt. Die organischen Phasen wurden vereinigt und mit gesättigter NaCl-Lösung gewaschen, über Natriumsulfat getrocknet und unter vermindertem Druck eingedampft. Der Rückstand wurde über Kieselgel mit DCM/Methanol (0-6% Methanol) chromatographiert, Produktfraktionen vereinigt und unter vermindertem Druck von Lösemittelresten befreit. Ausbeute: 8 g, entspricht 48 % d. Theorie.

Beispiel 15: (S)-2-Benzyloxycarbonylamino-4,4-difluorpentansäure

Die Verbindung aus Beispiel 14 (4,9 g, 16,37 mmol) wurde in 30 ml Aceton gelöst, auf 0 °C gekühlt und dann wurde 1 N Natronlauge (32,74 ml, 32,74 mmol, 2 eq.) zugegeben. Für etwa 2,5 h wurde bei RT gerührt, Reaktionskontrolle per LCMS zeigte vollständige Umsetzung an. Anschließend erfolgte Zugabe von 20 ml 1 N HCl, Aceton wurde unter vermindertem Druck abdestilliert und die Lösung auf pH 3 bis 4 gestellt. Es wurde mit Essigester zweimal ausgeschüttelt. Die organische Phase wurde mit gesättigter NaCl-Lösung gewaschen, über Natriumsulfat getrocknet und unter vermindertem Druck bis zur Trockne eingedampft. Das Rohprodukt wurde über Kieselgel mit DCM/Methanol-Gradient (0-6% Methanol) chromatographiert. Ausbeute: 4,7 g (quantitativ).

Beispiel 16: (S)-2-tert-Butoxycarbonylamino-4,4-difluorpentansäure

Die Verbindung aus Beispiel 15 (2,99 g, 10,44 mmol) wurde in 40 ml Methanol gelöst und 580 mg 10 % Pd/C wurden zugegeben. Bei 2 bar wurde für 3 h hydriert. Es wurde nur eine verschwindend geringe Z- Abspaltung festgestellt. Es erfolgte erneute Zugabe von 300 mg Katalysator und Hydrierung für weitere 2 h - geringer Umsatz. Nochmals wurde dieselbe Menge Katalysator zugegeben und über Nacht hydriert, erst jetzt war die Umsetzung vollständig. Es wurde vom Katalysator abfiltriert und eingedampft. Der Rückstand wurde in Dioxan/Wasser (40 ml) angelöst und Natriumcarbonat (700 mg, 0,6 eq), 10,4 ml 1 N NaOH-Lsg. (10,4 mmol, 1 eq.) und 2,6 g Di-tert-butyldicarbonat (11,94 mmol, 1,14 eq.) zugegeben. Nach etwa 2 h war die Umsetzung vollendet. Die Lösung wurde mit Ether extrahiert und die Etherphase wurde verworfen. Anschließend wurde die wässrige Phase mit 1 N HCl auf pH 3 angesäuert und zweimal mit Essigester extrahiert. Die organische Phase wurde mit einer gesättigten NaCl-Lösung nachgewaschen, über Natriumsulfat getrocknet, vom Trockenmittel abfiltriert und unter vermindertem Druck eingedampft. Ausbeute: 950 mg, 36 % der Theorie.
$^1$H-NMR: 12,8 (s, 1H); 7,25 (d, 1H); 4,11 (t, 1 H); 2,3 (m, br., 2H); 1,6 (t, 3H); 1,37 (s, 9H).

Beispiel 17: [(S)-1-(1-Cyano-cyclopropylcarbamoyl)-3,3-difluorbutyl]-carbaminsäure tert-butyl ester

1,35 g (5,33 mmol) des Produkts aus Beispiel 16, 810 mg (6,83 mmol, 1,3 eq.) 1-Amino-1-cyclopropylnitril-Hydrochlorid und 943 mg (6,93 mmol, 1,3 eq.) HOAt wurden in 18 ml Dichlormethan gelöst oder suspendiert, auf 0 °C gekühlt und dann mit 1,33 g (6,93 mmol, 1,3 eq.) EDCI sowie 1,76 ml (1,596 g, 13,86 mmol, 2,6 eq.) NEM versetzt. Es wurde für 16 h bei 0 °C bis RT gerührt. Die Reaktionsmischung wurde dann mit 40 ml DCM verdünnt und gegen 1 N HCl-Lösung, gesättigte Natriumhydrogencarbonat-Lösung und gesättigte NaCl-Lösung ausgeschüttelt. Die organische Phase wurde über Natriumsulfat getrocknet und unter vermindertem Druck eingedampft. Das Produkt war für weitere Umsetzungen hinreichend rein.
Ausbeute: 1,5 g, 89 % der Theorie.

Beispiel 18: (S)-2-Amino-4,4-difluorpentansäure-(1-cyanocyclopropyl)-amid

Zur Abspaltung der Boc-Schutzgruppe wurde die Verbindung aus Beispiel 17 (580 mg, 1,83 mmol) mit 10 ml TFA/DCM 1:1 versetzt und für 30 min bei RT gerührt. Anschließend wurde unter vermindertem Druck eingedampft, mit Dichlormethan und Toluol geschleppt und Lösemittelreste im Hochvakuum entfernt. Das Produkt lag als Trifluoracetat vor. Ausbeute: 340 mg.

Beispiel 19: Allgemeine Herstellungsvorschrift: Umsetzung eines N-Carbonylchlorids mit C-terminal geschützten Aminosäure-Bausteinen

1 mmol der C-terminal geschützten oder als Amid vorliegenden freien Aminosäure (beispielsweise das Produkt aus Beispiel 18, nachdem es durch basische Behandlung freigesetzt worden war) wurde in 10 ml THF gelöst und auf 0 °C gekühlt. In zwei Portionen erfolgte dann langsame Zugabe des Säurechlorides aus Beispiel 10 (1 mmol in 5 ml kaltem THF gelöst) über 60 min. Anschließend wurde über Nacht weitergerührt. Das Reaktionsgemisch wurde in 50 ml

Dichlormethan gegeben, mit Wasser und gesättigter $NaHCO_3$-Lösung gewaschen, über Natriumsulfat getrocknet und einrotiert. Das erhaltene ölige Rohprodukt wurde anschließend jeweils direkt durch HPLC (RP-18, Acetonitril-Wasser) aufgereinigt und Produktfraktionen gefriergetrocknet. Die Ausbeuten betrugen bei diesem Verfahren der Harnstoffbildung etwa 10 bis 50 % der Theorie.

Beispiel 20: (S)-5-Oxo-4-(2-oxo-3-phenylpropyl)-oxazolidin-3-carbonsäurebenzylester

(S)-5-Oxo-4-(2-oxo-3-phenylpropyl)-oxazolidin-3-carbonsäurebenzylester wurde analog zu Beispiel 13 ausgehend von (S)-4-Chlorocarbonylmethyl-5-oxo-oxazolidin-3-carbonsäurebenzylester (5,00 g, 16,8 mmol) und 15 ml einer 20%igen Benzylmagnesiumchlorid-Lösung (20,2 mmol, 1,2 eq.) in THF hergestellt. Das Produkt wurde als gelbes Öl erhalten. Ausbeute: 5,0 g , 84 % d. Theorie.

Beispiel 21: (S)-4-(2,2-Difluor-3-phenylpropyl)-5-oxo-oxazolidin-3-carbonsäurebenzylester

(S)-4-(2,2-Difluor-3-phenylpropyl)-5-oxo-oxazolidin-3-carbonsäurebenzylester wurde analog zu Beispiel 14 ausgehend von (S)-5-Oxo-4-(2-oxo-3-phenylpropyl)-oxazolidin-3-carbonsäurebenzylester (5,00 g, 14,2 mmol) und BAST (12,5 g , 56,60 mmol, 4 eq.) hergestellt. Nach Chromatographie an Kieselgel (Dichlormethan / Methanol) wurde reiner (S)-4-(2,2-Difluor-3-phenylpropyl)-5-oxo-oxazolidin-3-carbonsäurebenzylester in Form eines gelben Öls erhalten. Ausbeute: 1,2 g , 23 % d. Theorie

Beispiel 22: (S)-2-Benzyloxycarbonylamino-4,4-difluor-5-phenylpentansäure

(S)-2-Benzyloxycarbonylamino-4,4-difluor-5-phenylpentansäure wurde analog zu Beispiel 15 ausgehend von (S)-4-(2,2-Difluor-3-phenylpropyl)-5-oxo-oxazolidin-3-carbonsäurebenzylester (1,2 g, 3,20 mmol) hergestellt. Das Produkt wurde in Form eines gelben Öls erhalten, das direkt für die nachfolgenden Umsetzungen eingesetzt wurde. Ausbeute: 1,02 g , 88 % d. Theorie

Beispiel 23: (S)-2-Benzyloxycarbonylamino-4,4-difluor-5-phenyl-pentansäuremethylester

Zu einer Lösung von (S)-2-Benzyloxycarbonylamino-4,4-difluor-5-phenyl-pentansäure (1,02 g, 2,81 mmol) in 40 ml Methanol wurde Trimethylsilylchlorid (0,61 g, 5,61 mmol, 2 eq.) getropft. Nach beendeter Zugabe wurde 3 h bei RT gerührt. Das Lösungsmittel wurde unter vermindertem Druck entfernt und der Rückstand roh in die folgende Reaktion eingesetzt. Ausbeute: 1,0 g , 94 % der Theorie.

Beispiel 24: (S)-2-Amino-4,4-difluor-5-phenylpentansäuremethylester

In einem Dreihalskolben wurde (S)-2-Benzyloxycarbonylamino-4,4-difluor-5-phenylpentansäuremethylester (1,0 g , 2,65 mmol) in 25 ml Methanol gelöst. Nach mehrmaligem Evakuieren und Begasen mit Argon wurden 350 mg Pd/C (10 %) zugegeben. Nach erneutem Ent- und Begasen mit Argon wurde die ArgonAtmosphäre durch Wasserstoff ersetzt (Ballon mit $H_2$-Gas). Es wurde 3 h bei RT gerührt. Wegen unvollständiger Umsetzung wurden weitere 350 mg Katalysator zugesetzt und weitere 5 h bei RT hydriert. Nach vollständiger Umsetzung wurde die Reaktionsmischung filtriert. Es wurde mit 20 ml Methanol gewaschen und das Filtrat unter verminderten Druck eingedampft. Es wurde ein wachsartiger Rückstand erhalten, der neben dem gewünschten Produkt auch geringere Mengen des monodefluorierten und didefluorierten Produktes enthielt. Dieser Rückstand wurde ohne weitere Aufreinigung für die weiteren Reaktionen eingesetzt. Ausbeute: 530 mg, 82 % d. Theorie.

Beispiel 25: (S)-4,4-Difluor-2-isocyanato-5-phenylpentansäuremethylester

Unter Argon wurde (S)-2-Amino-4,4-difluor-5-phenylpentansäuremethylester (400 mg, 1,64 mmol) in 20 ml Dichlormethan gelöst. Bei RT wurde Pyridin (520 mg, 4 eq.) zugetropft und die resultierende Lösung 15 Minuten gerührt. Danach wurde auf 0˚C gekühlt und mit 20 %iger Phosgen-Lösung in Toluol (2,16 ml, 4,1 mmol, 2,5 eq.) versetzt. Es wurde 90 Minuten bei RT gerührt und danach das Lösungsmittel unter verminderten Druck entfernt. Es wurde noch zweimal mit 10 ml Toluol codestilliert. Das so erhaltene Rohprodukt wurde ohne weitere Reinigung für die folgenden Reaktionen eingesetzt. Ausbeute: 425 mg , 96 % d. Theorie

Beispiel 26: (S)-2-[(8-Aza-spiro[4.5]decan-8-carbonyl)-amino]-4,4-difluor-5-phenylpentansäuremethyl ester

(S)-4,4-Difluor-2-isocyanato-5-phenylpentansäuremethylester (425 mg, 1,58 mmol) wurden in 25 ml Dichlormethan gelöst. Zu dieser Lösung wurde 8-Aza-spiro[4.5]decan (220 mg, 1,58 mmol, 1 eq.) und DIPEA (269 µl, 204 mg, 1,58 mmol, 1 eq.) gegeben und über Nacht bei RT gerührt. Das Lösungsmittel wurde unter verminderten Druck entfernt und der Rückstand durch präparative HPLC (Gradient: Acetonitril/Wasser und Zusatz von 0,05 % TFA) gereinigt. Die Produkt enthaltenden Fraktionen wurden vereinigt und unter verminderten Druck vom Lösungsmittel befreit.
Ausbeute: 245 mg , 38 % d. Theorie.

Beispiel 27: (S)-2-[(8-Aza-spiro[4.5]decan-8-carbonyl)-amino]-4,4-difluor-5-phenyl-pentansäure

(S)-2-[(8-Aza-spiro[4.5]decan-8-carbonyl)-amino]-4,4-difluor-5-phenylpentansäure-methyl ester (240 mg, 0,59 mmol) wurde in einer Mischung aus 15 ml THF und 5 ml Methanol gelöst. Eine Lösung von 42 mg LiOH (1,76 mmol, 3 eq.) in 5 ml Wasser wurde zugesetzt und 3 h bei RT gerührt. Nach beendeter Reaktion wurde die Reaktionsmischung durch Zugabe einer 2 M HCl-Lösung auf pH=3 angesäuert. Das organische Lösungsmittel wurde unter verminderten Druck entfernt und die verbliebene wässrige Phase mit Ethylacetat extrahiert. Anschließend wurde die organische Phase noch dreimal mit Wasser und einmal mit gesättigter NaCl-Lösung gewaschen, über $MgSO_4$ getrocknet und unter verminderten Druck vom Lösungsmittel befreit. Das Produkt wurde in Form eines gelben Feststoffes erhalten, der ohne weitere Aufreinigung in der folgenden Reaktion eingesetzt wurde.
Ausbeute: 192 mg, 82 % d. Theorie.

Beispiel 28: 8-Aza-spiro[4.5]decan-8-carbonsäure [(S)-1-(1-cyano-cyclopropylcarbamoyl)-3,3-difluor-4-phenylbutyl]-amid

Zu einer Lösung der als Rohprodukt erhaltenen (S)-2-[(8-Aza-spiro[4.5]decan-8-carbonyl)-amino]-4,4-difluor-5-phenyl-pentansäure (192 mg, 0,49 mmol) in 10 ml DMF wurden nacheinander DIPEA (331 µl, 252 mg, 1,95 mmol, 4 eq.), HATU (185 mg, 0,49 mmol, 1 eq.) und 1-Amino-cyclopropancarbonsäurenitril-Hydrochlorid (58 mg, 0,49 mmol, 1 eq.) gegeben. Die Reaktionsmischung wurde über Nacht bei RT gerührt und am nächsten Tag unter vermindertem Druck eingeengt. Der so erhaltene Rückstand wurde durch präparative HPLC (Gradient: Acetonitril/Wasser und Zusatz von 0,05 % TFA) gereinigt. Die Titelverbindung wurde in Form eines farblosen, kristallinen Materials erhalten. Ausbeute: 34 mg, 15 % d. Theorie.
$^1$H-NMR: 8,83 (s, 1H); 7,48-7,15 (m, 5H); 6,62 (s, 1 H); 4,39 (m, 1 H); 3,30-3,15 (m, 6H); 2,39 (m, 1 H); 2,22 (m, 1 H); 1,65-1,05 (mm, ungefähr 16H); MS (ESI$^+$): 459,2

Beispiel 29: 9-Cyclopropyl-3,9-diaza-spiro[5.5]undecan-3-carbonsäure-tert-butylester

3,9-Diaza-spiro[5.5]undecan-3-carbonsäure-tert-butylester (250 mg, 0,98 mmol) wurden in 10 ml absoluten Methanol gelöst. Zu dieser Lösung wurden 3 g Molsieb (3 Å, welches vorher im Hochvakuum getrocknet wurde) zugegeben. Unter Argon wurden dann nacheinander Eisessig (0,55 ml, 10 eq.), [(1-Ethoxycyclopropyl)oxy]-trimethylsilan (0,69 ml, 3,5 eq.) und 4,4 ml einer 1 M $NaCNBH_3$-Lösung in THF (4,4 mmol, 4,5 eq.) zugegeben. Nach 20 Minuten Rühren bei RT wurde auf 60°C erwärmt und bei dieser Temperatur für etwa 15 h gerührt. Die Reaktionsmischung wurde filtriert und das Filtrat wurde unter verminderten Druck eingeengt. Der Rückstand wurde in Dichlormethan aufgenommen, nacheinander mit 1 M NaOH-Lösung und NaCl-Lösung gewaschen und über $MgSO_4$ getrocknet. Nach Entfernen des Lösungsmittels unter verminderten Druck wurde das Produkt als farbloses Öl erhalten. Ausbeute: 318 mg (quant.)

Beispiel 30: 3-Cyclopropyl-3,9-diaza-spiro[5.5]undecan

Der als Rohprodukt erhaltene 9-Cyclopropyl-3,9-diaza-spiro[5.5]undecan-3-carbonsäure-tert-butylester (318 mg, 0,98 mmol) wurde in 6 ml Dichlormethan gelöst und unter Eisbadkühlung mit 1 ml einer 4 M HCl-Lösung in Dioxan (4 mmol, 4 eq.) versetzt. Es wurde etwa 16 h bei RT gerührt. Das Lösungsmittel wurde unter verminderten Druck verdampft. Der Rückstand wurde in 20 ml Wasser aufgenommen und lyophilisiert. So wurde 3-Cyclopropyl-3,9-diaza-spiro[5.5]undecan-hydrochlorid als Rohprodukt in Form eines farblosen, amorphen Materials erhalten, welches sauber genug für weitere Umsetzungen war. Ausbeute: 252 mg , quantitativ Das so erhaltene Piperidin-Derivat wurde wie in den oben beschriebenen Beispielen zu den Endprodukten umgesetzt, welche in der Tabelle 1 a und 1 b aufgeführt sind.

Beispiel 31: 9-Butyl-3,9-diaza-spiro[5.5]undecan-3-carbonsäure-tert-butylester

3,9-Diaza-spiro[5.5]undecan-3-carbonsäure-tert-butylester (150 mg, 0,59 mmol) wurde in 10 ml absoluten Dichlormethan gelöst und mit Butyraldehyd (52 µl, 1 eq.) versetzt. Unter Eisbadkühlung wurden 17 µl Eisessig (0,5 eq.) und Natriumtriacetoxyborhydrid (137 mg, 1,1 eq.) zugegeben. Es wurde 16 h bei RT gerührt. Wegen unvollständiger Umsetzung wurden erneut Butyraldehyd (52 µl, 1 eq.), Eisessig (17 µl, 0,5 eq.) und Natriumtriacetoxyborhydrid (137

mg, 1,1 eq.) zugesetzt. Nach weiteren 4 h Rühren bei RT wurde wenig Wasser zugegeben und die Reaktionsmischung mit gesättigter $NH_4Cl$-Lösung gewaschen. Die organische Phase wurde über $MgSO_4$ getrocknet und unter verminderten Druck vom Lösungsmittel befreit. Das Produkt wurde als farbloses Öl erhalten. Ausbeute: 211 mg (quant.)

Beispiel 32: 3-Butyl-3,9-diaza-spiro[5.5]undecan

Der als Rohprodukt erhaltene 9-Butyl-3,9-diaza-spiro[5.5]undecan-3-carbonsäure-tert-butylester (183 mg, 0,59 mmol) wurde in 4 ml Dichlormethan gelöst und unter Eisbadkühlung mit 0,6 ml einer 4 M HCl-Lösung in Dioxan (2,4 mmol, 4 eq.) versetzt. Es wurde etwa 16 h bei RT gerührt. Das Lösungsmittel wurde unter verminderten Druck entfernt. Der Rückstand wurde in 20 ml Wasser aufgenommen und lyophilisiert. So wurde 3-Butyl-3,9-diaza-spiro[5.5]undecan-hydrochlorid als Rohprodukt in Form eines farblosen, amorphen Materials erhalten, welches sauber genug für weitere Umsetzungen war. Ausbeute: 176 mg, quantitativ

Das so erhaltene Piperidin-Derivat wurde wie in den oben beschriebenen Beispielen zu den Endprodukten umgesetzt, welche in der Tabelle 1a und 1b aufgeführt sind.

Beispiel 33: 1,4-Dioxaspiro[4.5]decan-8-carbonsäure

3,0 g (14 mmol) der Ethylester-Vorstufe, 1,4-Dioxaspiro[4.5]decan-8-carbonsäure-ethylester, wurden in 5 ml Methanol gelöst und bei RT langsam mit 28 ml (2 eq.) 1 M LiOH-Lösung versetzt. Man rührte über Nacht; HPLC-MS zeigte vollständige Reaktion an. Methanol wurde unter verminderten Druck abdestilliert und der Rückstand vorsichtig mit 1 ml HCl so angesäuert, dass kein Säureüberschuss vorhanden war. Es wurde mit Essigester extrahiert, die Essigester-Phase wurde über Natriumsulfat getrocknet und anschließend unter verminderten Druck eingedampft. Ausbeute: 2,25 g, 86 % d. Theorie.

Beispiel 34: [(S)-1-(1-Cyanocyclopropylcarbamoyl)-3-methylbutyl]-carbaminsäure tert-butyl ester

5 g Boc-(S)-Leu-OH (21,6 mmol), 3,3 g 1-Amino-cyclopropancarbonitril-Hydrochlorid (28,1 mmol, 1,3 eq.) und 3,8 g HOBt (28,1 mmol, 1,3 eq.) wurden in 60 ml Dichlormethan suspendiert und bei 0 ˚C nacheinander mit 5,4 g EDCI (28,1 mmol, 1,3 eq.) und 7,15 ml NEM (6,5 g, 56,2 mmol, 2,6 eq.) versetzt. Man rührte bei 0 ˚C bis RT über Nacht, schüttelte sauer (1 M HCl-Lösung), basisch (gesättigte $NaHCO_3$-Lösung) und neutral (gesättigte NaCl-Lösung) aus, trocknete über Natriumsulfat und destillierte das Lösemittel unter vermindertem Druck ab. Anschließend wurde an Kieselgel in Essigester/Heptan chromatographiert. Ausbeute: 3,62 g, 57 % der Theorie.
$^1$H-NMR: 8,82 (s, 1 H); 6,92 (d, 1 H); 3,86 (m, 1 H); 1,55 (m, 1 H); 1,47 (m, 2H); 1,36 (s, 9H); 1,09 (m, 2H); 0,86 (dd, 6H); MS ($ESI^+$): 296,3.

Beispiel 35: 1,4-Dioxaspiro[4.5]decan-8-carbonsäure-(S)-1-(1-cyanocyclopropyl-carbamoyl)-3-methylbutyl]-amid

Das Produkt aus Beispiel 34, [(S)-1-(1-Cyanocyclopropylcarbamoyl)-3-methylbutyl]-carbaminsäure tert-butyl ester, (1,1 g, 3,72 mmol) wurde mit 20 ml TFA/Dichlormethan 1:1 versetzt und für 30 min bei RT gerührt. Anschließend wurde unter vermindertem Druck eingedampft, erneut in Dichlormethan und Toluol aufgenommen und Lösemittel- und TFA-Reste unter vermindertem Druck entfernt. Das erhaltene N-terminal entschützte Produkt wurde direkt weiter umgesetzt. 283 mg (1,5 mmol) davon wurden mit 136 mg HOAt (1 mmol, 0,67 eq.), 195 mg 1,4-Dioxaspiro[4.5]decan-8-carbonsäure aus Beispiel 33 (1 mmol, 0,67 eq.), 268 mg EDCI (1,4 mmol, 0,93 eq.) und 0,32 ml NEM (2,5 mmol) bei 0 ˚C in 2,5 ml THF und 0,25 ml DMF versetzt und über Nacht bei 0 ˚C bis RT gerührt. Anschließend wurde das Lösemittel unter vermindertem Druck abdestilliert, der Rückstand in Essigester aufgenommen und mit gesättigter Natriumhydrogencarbonat- und gesättigter NaCl-Lösung ausgeschüttelt. Nach dem Trocknen über Natriumsulfat und Abdampfen des Lösemittels erfolgte die Aufreinigung über RP-HPLC (Gradient: Acetonitril/Wasser und Zusatz von 0,05 % TFA). Die Produktfraktionen wurden vereinigt und gefriergetrocknet. Ausbeute: 25 mg (ohne Mischfraktionen), entspricht 7 % der Theorie). $^1$H-NMR: 8,81 (s, 1H); 7,84 (d, 1 H); 4,20 (m, 1H); 3,83 (s, 4H); 2,22 (m, 1H); 1,75-0,8 (3m, ungefähr 13 H; 1,05 (m, 2H); 0,82 (dd, 6H); MS ($ESI^+$): 364,2.

Die Herstellung der in Tabelle 1a aufgelisteten Beispiele erfolgte in analoger Weise wie vorher beschrieben. Tabelle 1a zeigt die Beispiele mit zugehöriger Charakterisierung:

Tabelle 1a:

| Beispiel | Molmasse (Stammverbindung) | Struktur | MS (ESI+) | 1H-NMR |
|---|---|---|---|---|
| 36 | 382,46 | 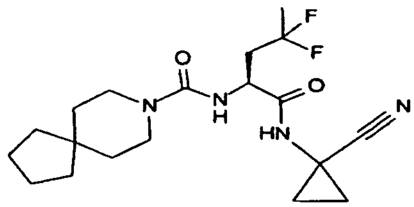 | 383,1 | 8,82 (s, 1 H); 6,62 (d, 1 H); 4,28 (m, 1 H); 3,29 (m, 4H); 2,26 (m, 2H); 1,65-1,05 (mm, 19 H) |
| 37 | 386,4 | 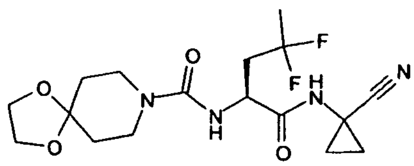 | 387,1 | 8,83 (s, 1); 6,80 (d, 1 H); 4,30 (m, 1 H); 3,6 (m, 1 H); 3,4 (m, approx, 4 H); 2,25 (m, approx, 4H); 1,7-1,4 (mm, approx, 11 H); 1,1 (m, 2H) |
| 38 | 396,48 | 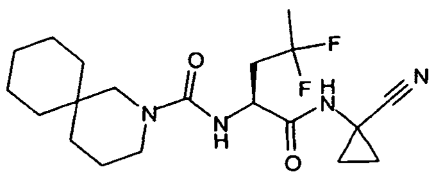 | 397,1 | 8,81 (s, 1 H); 6,6 (d, 1 H); 4,3 (m, 1 H); 3,3 (m, 2H); 3,15 (dd, 2H); 2,29 (m, 2H); 1,6-1,0 (mm, approx, 20H) |
| 39 | 410,51 | 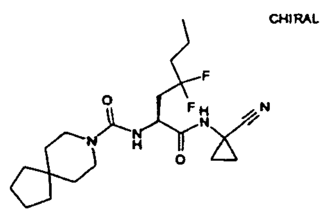 | 411,5 | 8,82 (s, 1 H); 6,62 (d, 1 H); 4,28 (m, 1 H); 3,29 (m, 4H); 2,26 (m, 2H); 1,82 (m, 2H); 1,65-1,05 (mm, approx, 18H); 0,88 (t, 3H) |
| 40 | 424,54 | 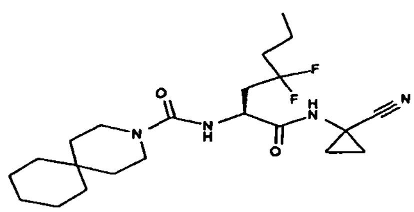 | 425,6 | 8,82 (s, 1 H); 6,62 (d, 1 H); 4,28 (m, 1 H); 3,29 (m, 4H); 2,26 (m, 2H); 1,60-1,05 (mm, approx, 20H); 0,88 (t, 3H) |
| 41 | 503,55 | 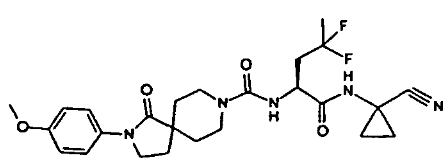 | 504,2 | 9 (s, 1 H); 7,58 (d, 2H); 6,93 (d, 2H); 6,82 (d, 1 H); 4,31 8m, 1 H); 3,92 (m, 2H); 3,77 (m, 2H); 3,76 (s, 3H); 2,92 (m, 2H); 2,30 (m, 2H); 2,07 (m, 2H); 1,69-1,4 (2m, 9H); 1,1 (m, 2H) |

(fortgesetzt)

| Beispiel | Molmasse (Stammverbindung) | Struktur | MS (ESI+) | 1H-NMR |
|---|---|---|---|---|
| 42 | 474,51 | 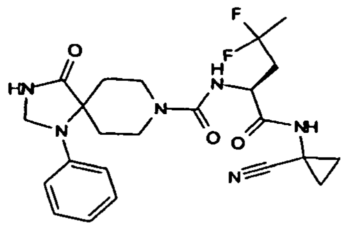 | 475,3 | 8,94 (s, 1 H); 8,88 (s, 1 H); 7,4 (t, 2H); 6,87 (d, 1 H); 6,7 (m, 3H); 4,6 (s, 2H); 4,39 (m, 1 H); 3,96 (m, 2H); 2,6-2,2 (m, br, approx, 6H); 1,6 (m, br, approx, 7H); 1,11 (m, 2H) |
| 43 | 397,43 | 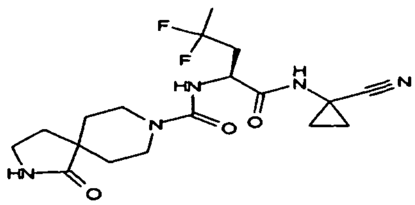 | 398,4 | 8,88 (s, 1H); 7,57 (s, 1 H); 6,72 (d, 1 H); 4,28 (m, 1H); 3,85 (m, 2H); 3,2 (t, 2H); 2,88 (m, 2H); 2,3 (m, 2H); 1,92 (m, 2H); 1,65-1,4 (m, br, 7H); 1,29 (m, 2H); 1,1 (m, 2H) |
| 44 | 411,46 | 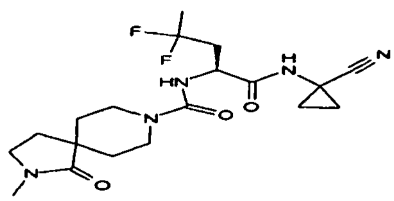 | 412,5 | 8,88 (s, 1H); 6,72 (d, 1 H); 4,28 (m, 1 H); 3,85 (m, 2H); 3,28 (t, 2H); 2,88 (m, 2H); 2,7 (s, 3H); 2,3 (m, 2H); 1,92 (m, 2H); 1,65-1,4 (m, br, 7H); 1,29 (m, 2H); 1,1 (m, 2H) |
| 45 | 427,5 | 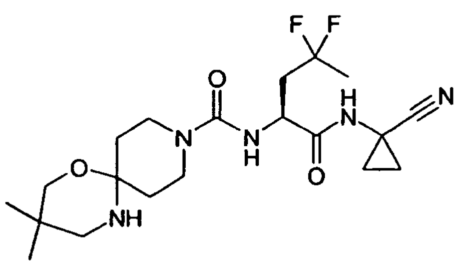 | 429,5 | 8,85 (s, 1 H); 6,75 (d, 1 H); 4,29 (m, 1 H); 3,6 (s, 1 H, overl, water); 3,46 (s, 4H); 3,3 (m, 4H); 2,3 (m, 2H); 1,73-1,42 (3m, 9H); 1,1 (m, 2H); 0,88 (s, 6H) |
| 46 | 430,5 | 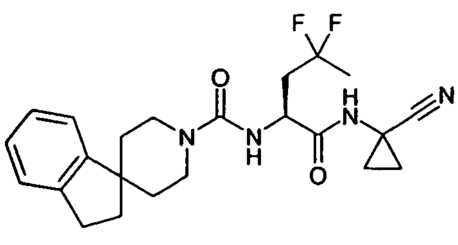 | 431,2 | 8,89 (s, 1 H); 7,15 (m, 4H); 6,74 (d, 1 H); 4,34 (m, 1 H); 3,98 (dd, 2H); 2,9 (m, 4H); 2,3 (m, 2H); 2,0 (m, 2H); 1,65 (m, 5H); 1,45 (m, 4H); 1,1 (m, 2H) |

(fortgesetzt)

| Beispiel | Molmasse (Stammverbindung) | Struktur | MS (ESI+) | 1H-NMR |
|---|---|---|---|---|
| 47 | 412,4 | 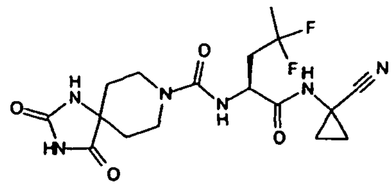 | 413,4 | 8,88 (s, 1 H); 8,51 (s, 1H); 6,8 (d, 1 H); 4,3 (m, 1 H); 3,1 (m, 2H); 2,8-2,1 (m, br, approx, 6H); 1,6 (m, br, approx, 5H); 1,24; 1,11 (2m, 4H) |
| 48 | 368,43 | 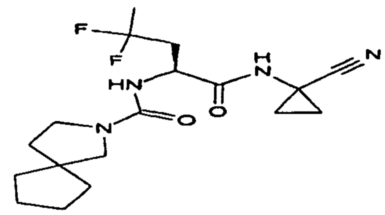 | 369,3 | 8,88 (s, 1 H); 6,24 (d, 1 H); 4,3 (m, 1 H); 3,3 (m, 2H); 3,1 (dd, 2H); 2,3 (m, 1 H); 1,75-0,9 (mm, 15H); 1,1 (m, 2H) |
| 49 | 487,55 | 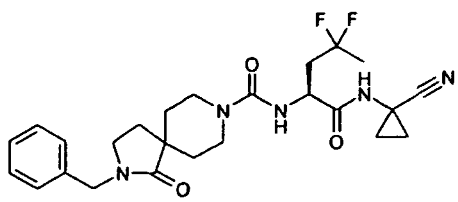 | 488,5 | 8,88 (s, 1 H); 7,4-7,15 (3m, 5H); 6,74 (d, 1 H); 4,39 (s, 2H); 4,3 (m, 1 H); 3,9 (dd, 2H); 3,18 (m, 2H); 2,9 (dt, 2H); 2,3 (m, 2H); 1,7-1,3 (3m, 6H); 1,1 (m, 1 H) |
| 50 | 491,52 | 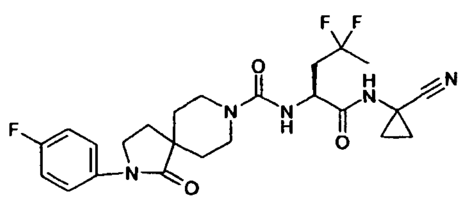 | 492,5 | 8,89 (s, 1 H); 7,68 (m, 2H); 7,20 (m, 2H); 6,78 (d, 1 H); 4,31 8m, 1H); 3,92 (m, 2H); 3,77 (m, 2H); 2,92 (m, 2H); 2,30 (m, 2H); 2,07 (m, 2H); 1,7-1,4 (2m, 9H); 1,1 (m, 2H) |
| 51 | 400,43 | 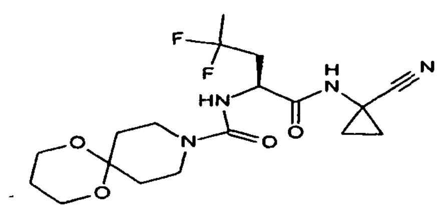 | 401,3 | 8,82 (s, 1 H); 6,72 (d, 1 H); 4,28 (m, 1 H); 3,83 (m, 4H); 3,3 (m, approx, 4H, water overlap); 2,28 (m, 2H); 1,75-1,4 (3m, approx, 11 H); 1,1 (m, 2H) |

(fortgesetzt)

| Beispiel | Molmasse (Stammverbindung) | Struktur | MS (ESI+) | $^1$H-NMR |
|---|---|---|---|---|
| 52 | 476,53 | 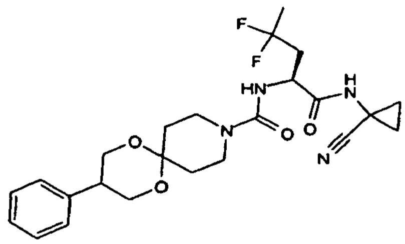 | 477,4 | 8,86 (s, 1 H); 7,31 (s, 5H); 6,8 (d, 1 H); 4,30 (m, 1 H); 4,0 (m, 2H); 3,9 (m, 2H); 3,4 (m, approx, 5H, overlap water); 3,05 (m, 1 H); 2,28 (m, 2H); 2,0 (m, 2H); 1,7-1,3 (2m, approx, 7H); 1,1 (m, 2H) |
| 53 | 372,51 | 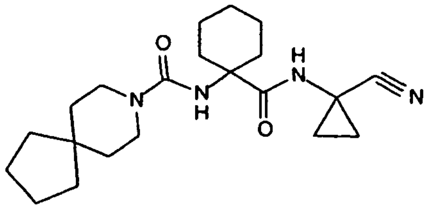 | 373,2 | 8,26 (s, 1 H); 5,83 (s, 1 H); 3,26 (m, approx, 5H); 1,9-1,0 (mm, approx, 25 H) |
| 54 | 386,54 | 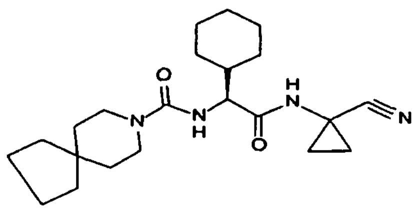 | 387,2 | 8,83 (s, 1 H); 6,20 (m, 1 H); 3,9, 3,8 (2t, 1 H); 3,28 (m, 4H); 1,75-0,8 (mm, approx, 27H) |
| 55 | 453,29 | 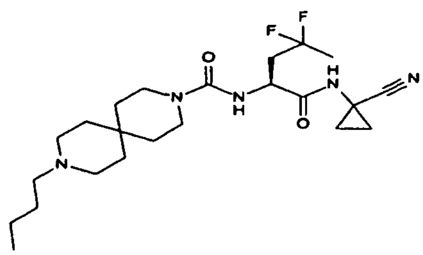 | 454,3 | 8,86 (s, 1 H); 6,68 (d, 1 H); 4,29 (m, 1 H); 3,3 (m, 6H); 3,0 (m, 4H); 2,28 (m, 2H); 1,81 (m, 2H); 1,65-1,4 (m, 11 H); 1,3 (m, 4H); 1,1 (m, 2H); 0,9 (t, 3H) |
| 56 | 437,26 | 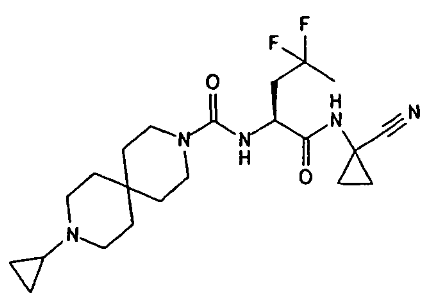 | 438,2 | 8,89 (s, 1 H); 6,7 (d, 1 H); 4,28 (m, 1 H); 3,32 (m, approx, 8H, water overlap); 2,89 (m, 1 H); 2,26 (m, 2H); 1,83 ("d", 2H); 1,66-1,37 (m, 2m, 9H); 1,28 (m, 2H); 1,1 (m, 2H); 0,85 (2m, 4H) |

(fortgesetzt)

| Beispiel | Molmasse (Stammverbindung) | Struktur | MS (ESI+) | 1H-NMR |
|---|---|---|---|---|
| 57 | 303,41 | 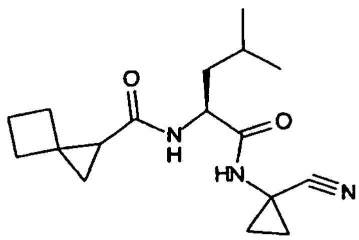 | 302,4 (ES-) | 8,89 (s, 1 H); 8,1 (d, 1 H); 4,18 (m, 1 H); 2,0 (m, br, approx 5H); 1,66-1,32 (m mm, approx, 6H); 1,07 (m, 2H); 0,94-0,74 (m, dd, approx, 10H) |

Weitere spiro-cyclische Amine, die im folgenden zur Herstellung von weiteren erfindungsgemäßen Beispielen verwendet wurden, wurden wie folgt dargestellt: Die spirocyclischen Amine 7-Cyclopropyl-2,7-diaza-spiro[3.5]-nonan, 7-Propyl-2,7-diaza-spiro[3.5]nonan, 2-Cyclopropyl-2,8-diaza-spiro[4.5]decan, 2-Cyclopropyl-2,7-diaza-spiro[3.5]nonan, 2-Propyl-2,7-diaza-spiro[3.5]nonan, 9-Cyclopropyl-1-oxa-4,9-diaza-spiro[5.5]undecan und 4-Cyclopropyl-1-oxa-4,9-diaza-spiro[5.5]undecan wurden entsprechend den oben beschriebenen Beispielen 29-32 ausgehend von den tert.-butyloxycarbonyl-geschützten Vorstufen, die kommerziell erhältlich sind, hergestellt. 2-Cyclopropylmethyl-2,8-diaza-spiro[4.5]decan-3-on wurde durch Alkylierung von 3-Oxo-2,8-diaza-spiro[4.5]decan-8-carbonsäure-tert.-butyl ester (kommerziell erhältlich) mit Cyclopropylmethylbromid und anschließender Abspaltung der tert.-Butyloxycarbonyl-Schutzgruppe mit TFA erhalten. 6-Aza-spiro[2.5]octan kann gemäß der Literatur (Bull. Soc Chim. France 10, 2572-81 (1964) hergestellt werden oder aber wie im folgenden beschrieben durch Cyclopropanierung von 4-Methylen-piperidin-1-carbonsäure-tert.-butylester und anschließender Abspaltung der tert.-Butyloxycarbonyl-Schutzgruppe.

Beispiel 58: 6-Aza-spiro[2.5]octan

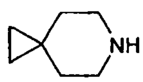

In einem ausgeheizten trockenen Kolben wurde unter Schutzgas (Argon) eine Mischung aus 9,90 g Zink-Staub (151 mmol) und 1,50 g CuCl (15,15 mmol) in 20 ml abs. Diethylether ca. 100 min bei 50˚C (Badtemperatur) gerührt. Es wurde auf 15˚C (Badtemperatur) gekühlt. Nacheinander wurden 6,1 ml Dijodmethan (75,0 mmol), 7,8 ml abs. Dimethoxyethan und letztlich 4,27 g 4-Methylen-piperidin-1-carbonsäure-tert.-butyl- ester (21,7 mmol) zügig zugetropft. Die Reaktionsmischung wurde langsam auf 50˚C (Badtemperatur) erwärmt und für ca. 20 h bei dieser Temperatur gerührt. Nach dieser Zeit wurde wegen noch nicht vollständigen Umsatzes unter Kühlung nochmal 20 ml Diethylether und 6,1 ml Dijodmethan hinzugegeben. Die Reaktionsmischung wurde weitere 8 h auf 50 ˚C (Badtemperatur) erwärmt. Nach Abkühlen wurde mit THF verdünnt und über Celite filtriert. Das Filtrat wurde mit 4,3 g p-Toluolsulfonsäure und wenigen Tropfen Wasser versetzt und anschließend unter verminderten Druck eingeengt. Das so erhaltene Rohprodukt wurde in 100 ml THF gelöst und mit 7,09 g Boc-Anhydrid (1,5 eq.) und 5,5 ml DIPEA (1,5 eq.) versetzt. Es wurde 48 h bei RT gerührt und anschließend unter verminderten Druck eingeengt. Der so erhaltene Rückstand wurde mit ca. 150 ml Diethylether verrührt. Es wurde abgesaugt und der Filterrückstand gut mit Diethylether gewaschen. Das so erhaltene Filtrat wurde eingeengt. Es blieb ein Öl zurück, welches in 200 ml Dichlormethan aufgenommen wurde und zweimal mit ges. $NaHCO_3$-Lsg. und einmal mit verd. HCl-Lsg. (pH = 5) gewaschen wurde. Die organische Phase wurde über $MgSO_4$ getrocknet und anschließend unter vermindertem Druck eingeengt. Auf diese Weise wurden 2,8 g 6-Aza-spiro[2.5]octan-6-carbonsäure-tert.-butylester als helles Öl erhalten. Dieses Öl wurde in 5 ml Dichlormethan gelöst und mit einer Mischung aus 10 ml TFA und 0,5 ml Wasser versetzt. Nach 48 h Rühren bei RT wurde die Reaktionslösung unter vermindertem Druck eingeengt und noch dreimal mit Toluol kodestilliert. Auf diese Weise wurde 6-Aza-spiro[2.5]octan als TFA-Salz in Form eines braunen Öls erhalten, welches sauber genug war für weitere Umsetzungen.
Ausbeute: 1,50 g (31 % d. Theorie) MS (ESI+): 112 .

Das so erhaltene 6-Aza-spiro[2.5]octan wurde zu den erfindungsgemäßen Verbindungen umgesetzt, wie in den Beispielen 61-63 und 68-70 beschrieben wurde:

Beispiel 59: (S)-2-Amino-4,4-difluor-pentansäure-methylester-Hydrochlorid

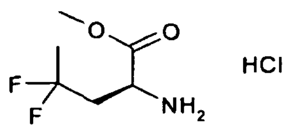

Bei ca. 5 °C (Eisbadkühlung) wurden unter Argon 8,2 ml Thionylchlorid (112,6 mmol; 1,5 eq.) zu 100 ml Methanol (p.a) getropft. Man ließ innerhalb von 30 min auf RT kommen und rührte für weitere 30 min. Zu dieser Reaktionsmischung wurden 19,0 g (S)-2-tert.-Butoxycarbonylamino-4,4-difluor-pentansäure (75,0 mmol) portionsweise zugesetzt. Die Reaktionsmischung wurde 2 h bei RT gerührt. Danach wurde die Reaktionsmischung auf 35 °C (Innentemperatur) erwärmt und noch 3 h bei dieser Temperatur gerührt. Anschließend wurde unter verminderten Druck eingeengt, wodurch (S)-2-Amino-4,4-difluor-pentansäure-methylester-Hydrochlorid als leicht bräunlicher, kristalliner Feststoff erhalten wurde, welcher rein genug für weitere Umsetzungen war. Ausbeute: 13,5 g (89 % d. Theorie)

Beispiel 60: (S)-4,4-Difluor-2-isocyanato-pentansäure-methylester

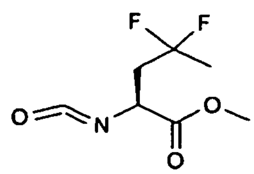

Unter Argon wurden 2,0 g (S)-2-Amino-4,4-difluor-pentansäure-methylester-Hydrochlorid (9,8 mmol) in 80 ml Dichlormethan gelöst, mit 3,2 ml Pyridin (4 eq.) versetzt und auf ca. 5 °C gekühlt (Eisbad). Nach 10 min wurden 6,7 ml (1,3 eq.) einer 20%igen Phosgen-Lsg. in Toluol langsam zugetropft. Die entstandene Suspension wurde 3 h unter Eisbadkühlung gerührt und dann unter vermindertem Druck eingeengt. Der so erhaltene Rückstand wurde in Toluol aufgenommen und filtriert. Nach Entfernen des Lösungsmittels im Vakuum wurde (S)-4,4-Difluor-2-isocyanato-pentansäure-methylester als braunes Öl erhalten, welches ohne weitere Reinigung in der anschließenden Reaktion eingesetzt wurde. Ausbeute: 1,1 g (58 % d. Theorie)

Beispiel 61: (S)-2-[(6-Aza-spiro[2.5]octan-6-carbonyl)-amino]-4,4-difluor-pentansäure-methylester

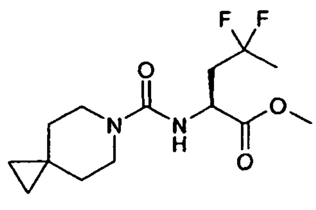

Zu einer Lösung von 0,65 g (S)-4,4-Difluor-2-isocyanato-pentansäuremethylester (3,36 mmol) in 4 ml Dichlorethan wurde unter Argon eine Lösung von 0,75 g 6-Aza-spiro[2.5]octan -Trifluoracetat (3,30 mmol, 1 eq.) und 0,75 ml DIPEA (1,3 eq.) in 6 ml Dichlorethan gegeben. Die Reaktionsmischung wurde bei RT über Nacht gerührt. Es wurde mit 10 ml Dichlormethan verdünnt und mit gesättigter $NaHCO_3$-Lsg. gewaschen. Die organische Phase wurde über $MgSO_4$ getrocknet und anschließend unter vermindertem Druck eingeengt. Der so erhaltene Rückstand wurde durch präparative HPLC (Gradient: Acetonitril/Wasser und Zusatz von 0,05 % TFA) gereinigt. Die Produkt enthaltenden Fraktionen wurden vereinigt und gefriergetrocknet. Ausbeute: 200 mg , 20 % d. Theorie MS ($ESI^+$): 305 .

Beispiel 62: (S)-2-[(6-Aza-spiro[2.5]octan-6-carbonyl)-amino]-4,4-difluor-pentansäure

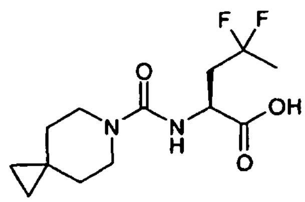

365,0 mg (S)-2-[(6-Aza-spiro[2.5]octan-6-carbonyl)-amino]-4,4-difluoro-pentansäure-methylester (1,2 mmol) wurden in einer Mischung aus 10,8 ml THF und 3,6 ml Methanol gelöst. Zu dieser Lösung wurden 3,6 ml einer wässrigen 1 M LiOH-Lsg. (3 eq.) gegeben. Es wurde eine Stunde bei 45 ˚C (Badtemperatur) gerührt und anschließend 3,6 ml 1 M HCl-Lsg. hinzugesetzt. Die Mischung wurde unter verminderten Druck eingeengt und noch zweimal mit DMF kodestilliert. Die so erhaltene (S)-2-[(6-Aza-spiro[2.5]octan-6-carbonyl)-amino]-4,4-difluor-pentansäure war rein genug für die nachfolgende Umsetzung. Ausbeute: 400 mg (quant.)

Beispiel 63: (6-Aza-spiro[2.5]octan-6-carbonsäure [(S)-1-(1-cyano-cyclopropyl-carbamoyl)-3,3-difluor-butyl]-amid

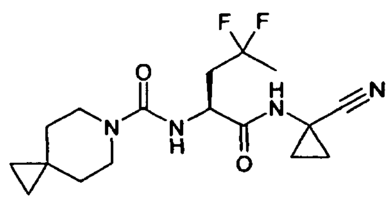

Zu einer Lösung von 348,0 mg (S)-2-[(6-Aza-spiro[2.5]octan-6-carbonyl)-amino]-4,4-difluor-pentansäure (1,2 mmol) in 5 ml DMF wurden nacheinander 142,3 mg 1-Amino-cyclopropancarbonitril-Hydrochlorid (1,2 mmol, 1,0 eq.), 163,3 mg HOAt (1,0 eq.), 0,61 ml DIPEA (3 eq.) und 456 mg HATU (1,0 eq.) gegeben. Die Reaktionsmischung wurde 24 h bei RT gerührt und anschließend unter vermindertem Druck eingeengt. Der Rückstand wurde in 10 ml Dichlormethan aufgenommen, mit wenig ges. $NaHCO_3$-Lsg. und dann wenig verdünnter HCl-Lsg. (pH=5) gewaschen und über $MgSO_4$ getrocknet. Der so erhaltene Rückstand wurde durch präparative HPLC (Gradient: Acetonitril/Wasser und Zusatz von 0,05 % TFA) gereinigt. Die Produkt enthaltenden Fraktionen wurden vereinigt und gefriergetrocknet. Auf diese Weise wurde (6-Aza-spiro[2.5]octan-6-carbonsäure [(S)-1-(1-cyano-cyclopropyl-carbamoyl)-3,3-difluor-butyl]-amid als farbloser, amorpher Feststoff erhalten.
Ausbeute: 200 mg , 47 % d. Theorie. MS (ESI$^+$): 355,1 .
$^1$H-NMR: 8,85 (s, 1 H); 6,68 (d, 1 H); 4,30 (m, 1 H); 3,30-3,40 (m, 4H); 2,30 (m, 2H); 1,60 (t, 3H); 1,50 (m, 2H); 1,25 (m, 4H); 1,10 (m, 2H); 0,30 (s, 4H) .

Beispiel 64: (S)-2-Benzyloxycarbonylamino-4-oxo-5-phenyl-pentansäure-methylester

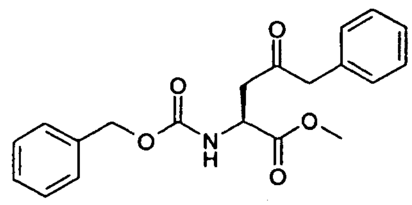

In einem ausgeheizten, mit Argon gespülten Kolben wurden 3,7 g Lithiumbromid (2,4 eq.) und 4,38 g (1,2 eq.) CuBr-$Me_2S$ in 55 ml abs. THF gelöst und 20 min bei RT gerührt. Die entstandene gelbe Lösung wurde auf -70˚C gekühlt

und mit 16,2 ml (1,2 eq.) Benzylmagnesiumchlorid-Lsg. (20%ig in THF) versetzt. Nach weiteren 20 min bei -70 °C wurde eine Lösung von 5,32 g (S)-2-Benzyl-oxycarbonylamino-3-chlorocarbonyl-propionsäure-methylester (Beispiel 1) (17,75 mmol) in 15 ml THF zugesetzt. Nach 2 h wurde langsam auf -35°C erwärmt und 50 ml einer ges. $NH_4Cl$-Lsg. zugesetzt. Die Reaktionsmischung wurde mit 400 ml Dichlormethan verdünnt und mit 100 ml einer 2 M HCl-Lsg. gewaschen. Die wässrige Phase wurde noch zweimal mit 50 ml Dichlormethan gewaschen. Die vereinten organischen Phasen wurden nacheinander mit 2 M HCl-Lsg., ges. $NaHCO_3$-Lsg. und ges. NaCl-Lsg. gewaschen, über $MgSO_4$ getrocknet und unter vermindertem Druck eingeengt. (S)-2-Benzyloxycarbonylamino-4-oxo-5-phenyl-pentansäure-methylester wurde als gelbes Öl erhalten, welches ohne weitere Reinigung in der nächsten Stufe eingesetzt wurde. Ausbeute: 6,39 g (quant.)

Beispiel 65: (S)-2-Benzyloxycarbonylamino-4,4-difluor-5-phenyl-pentansäure-methylester

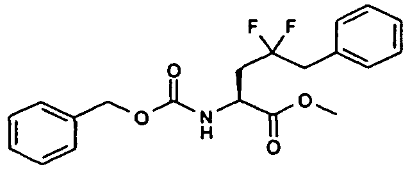

In einem Teflongefäß wurden 6,3 g (S)-2-Benzyloxycarbonylamino-4-oxo-5-phenyl-pentansäure-methylester (17,7 mmol) in 10 ml Dichlormethan gelöst. Unter Argon wurden 5 g BAST (1,28 eq.) zugesetzt. Die Reaktionsmischung wurde 20 h bei 40 °C (Badtemperatur) gerührt. Es wurden erneut 5 g BAST (1,28 eq.) zugesetzt und weitere 20 h bei 40 °C gerührt. Die Reaktionsmischung wurde in 500 ml einer eisgekühlten $NaHCO_3$-Lsg getropft und mit 200 ml Dichlormethan verdünnt. Die Phasen wurden getrennt und die wässrige Phase zweimal mit 100 ml Dichlormethan gewaschen. Die vereinigten organischen Phasen wurden mit einer 1 M HCl-Lsg. und mit ges. NaCl-Lsg. gewaschen, über $MgSO_4$ getrocknet und unter verminderten Druck eingeengt. Der Rückstand (dunkelbraunes Öl) wurde durch Flash-Chromatographie an Kieselgel mit einer Heptan/Ethylacetat-Mischung gereinigt. (S)-2-Benzyloxycarbonylamino-4,4-difluor-5-phenyl-pentansäure-methylester wurde als gelber wachsartiger Stoff erhalten. Ausbeute: 3,03 g (45 % d. Theorie) .

Beispiel 66: (S)-2-Amino-4,4-difluor-5-phenyl-pentansäure-methylester

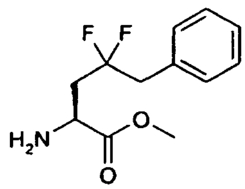

3,0 g (S)-2-Benzyloxycarbonylamino-4,4-difluor-5-phenyl-pentansäure-methylester (8,0 mmol) wurden unter Eisbadkühlung mit 8,0 ml (5 eq.) einer 30%igen HBr-Lsg. in Eisessig versetzt und 3 h gerührt. 100 ml Diethylether wurden zugesetzt und der entstandene Niederschlag abgesaugt. Das so erhaltene Rohprodukt wurde durch präparative HPLC (Gradient: Acetonitril/Wasser und Zusatz von 0,05 % TFA) gereinigt. Der nach Gefriertrocknung erhaltene amorphe Feststoff wurde in Dichlormethan aufgenommen und mit ges. $NaHCO_3$-Lsg. gewaschen. Die organische Phase wurde über $MgSO_4$ getrocknet und unter verminderten Druck eingeengt. So wurde (S)-2-Amino-4,4-difluor-5-phenyl-pentansäure-methylester als gelbes Öl erhalten.
Ausbeute: 817 mg (42 % d. Theorie)

Beispiel 67: (S)-4,4-Difluor-2-isocyanato-5-phenyl-pentansäure-methylester

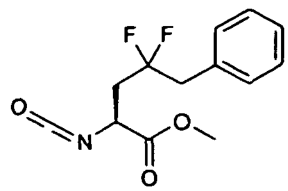

817 mg (S)-2-Amino-4,4-difluor-5-phenyl-pentansäure-methylester (3,36 mmol) wurden in 20 ml Dichlormethan gelöst, mit 1,08 ml Pyridin (4 eq.) versetzt und auf ca. 5 °C gekühlt (Eisbad). Nach 10 min wurden 2,3 ml (1,3 eq.) einer 20%igen Phosgen-Lsg. in Toluol zugetropft. Die entstandene Suspension wurde 4 h unter Eisbadkühlung gerührt und dann unter vermindertem Druck eingeengt. Der so erhaltene Rückstand wurde in Toluol aufgenommen und filtriert. Nach Entfernen des Lösungsmittels unter vermindertem Druck wurde (S)-4,4-Difluor-2-isocyanato-5-phenyl-pentansäure-methylester als orangefarbenes Öl erhalten, welches ohne weitere Reinigung in der anschließenden Reaktion eingesetzt wurde. Ausbeute: 943 mg (quant.)

Beispiel 68: (S)-2-[(6-Aza-spiro[2.5]octan-6-carbonyl)-amino]-4,4-difluor-5-phenyl-pentansäure-methylester

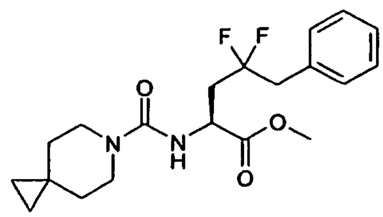

Die Herstellung von (S)-2-[(6-Aza-spiro[2.5]octan-6-carbonyl)-amino]-4,4-difluor-5-phenyl-pentansäure-methylester erfolgte analog zu Beispiel 61 ausgehend von 904 mg (S)-4,4-Difluor-2-isocyanato-5-phenyl-pentansäure-methylester (3,36 mmol) und 907 mg 6-Aza-spiro[2.5]octan -Trifluoracetat (1,2 eq.). Chromatographische Trennung durch präparative HPLC (Gradient: Acetonitrit/Wasser und Zusatz von 0,05 % TFA) ergab (S)-2-[(6-Aza-spiro[2.5]octan-6-carbonyl)-amino]-4,4-difluor-5-phenyl-pentansäure-methylester als gelbes Öl. Ausbeute: 234 mg (18 % d. Theorie)

Beispiel 69: (S)-2-[(6-Aza-spiro[2.5]octan-6-carbonyl)-amino]-4,4-difluor-5-phenyl-pentansäure

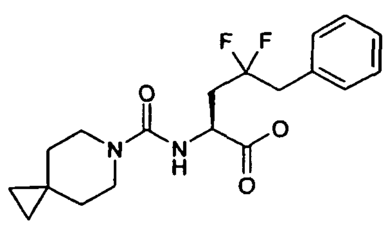

Die Herstellung von (S)-2-[(6-Aza-spiro[2.5]octan-6-carbonyl)-amino]-4,4-difluor-5-phenyl-pentansäure erfolgte analog zu Beispiel 62 ausgehend von 234 mg (S)-2-[(6-Aza-spiro[2.5]octan-6-carbonyl)-amino]-4,4-difluor-5-phenyl-pentansäure-methylester (0,62 mmol). Chromatographische Trennung durch präparative HPLC (Gradient: Acetonitril/Wasser und Zusatz von 0,05 % TFA) ergab (S)-2-[(6-Aza-spiro[2.5]octan-6-carbonyl)-amino]-4,4-difluor-5-phenyl-pentansäure als farblosen, amorphen Feststoff.
Ausbeute: 41 mg (18 % d. Theorie)

Beispiel 70: 6-Aza-spiro[2.5]octan-6-carbonsäure [(S)-1-(1-cyano-cyclopropyl-carbamoyl)-3,3-difluor-4-phenyl-butyl]-amid

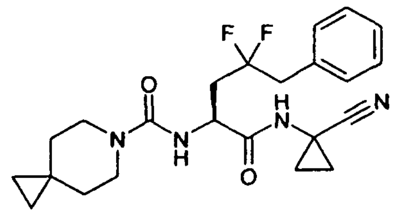

Zu einer Lösung von 41,0 mg (S)-2-[(6-Aza-spiro[2.5]octan-6-carbonyl)-amino]-4,4-difluor-5-phenyl-pentansäure (0,11 mmol) in 5 ml DMF wurden nacheinander 13,3 mg 1-Amino-cyclopropancarbonitril-Hydrochlorid (1,0 eq.), 15,2 mg HOAt (1,0 eq.), 57 μl DIPEA (3 eq.) und 42,5 mg HATU (1,0 eq.) gegeben. Die Reaktionsmischung wurde 4 h bei RT gerührt und anschließend unter verminderten Druck eingeengt. Der so erhaltene Rückstand wurde durch präparative HPLC (Gradient: Acetonitrif/Wasser und Zusatz von 0,05 % TFA) gereinigt. Die Produkt enthaltenden Fraktionen wurden vereinigt und gefriergetrocknet. Auf diese Weise wurde 6-Aza-spiro[2.5]octan-6-carbonsäure [(S)-1-(1-cyano-cyclopropyl-carbamoyl)-3,3-difluor-4-phenyl-butyl]-amid als farbloser, amorpher Feststoff erhalten. Ausbeute: 30 mg , 62 % d. Theorie.
$^1$H-NMR: 8,85 (s, 1 H); 7,22-7,40 (m, 5H); 6,68 (d, 1 H); 4,40 (m, 1 H); 3,20-3,40 (m, 6H); 2,15-2,40 (m, 2H); 1,48 (d, 2H); 1,25 (t, 4H);1,10 (d, 2H); 0,30 (s, 4H);
MS (ESI$^+$): 431,3.

Beispiel 71: 8-Aza-spiro[4.5]decan-8-sulfonylchlorid

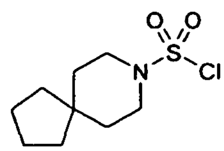

1,0 g 8-Aza-spiro[4.5]decan (7,2 mmol) wurden in 25 ml Dichlormethan gelöst. Nach Zugabe von 1,5 ml Triethylamin (1.5 eq.) wurde auf 0˚C gekühlt. Bei dieser Temperatur wurde eine Lösung von 0,48 ml Chorsulfonsäure (7,2 mmol, 1 eq.) in 5 ml Dichlormethan und anschließend 1,9 ml Pyridin (3,2 eq.) zugegeben. Es wurde 48 h bei RT gerührt. Die Reaktionsmischung wurde mit einer 1 M HCl-Lsg. gewaschen und die wässrige Phase abgetrennt. Daraufhin wurde die organische Phase mit einer $Na_2CO_3$-Lsg. geschüttelt. Die so erhaltene wässr. Phase wurde anschließend noch dreimal mit wenig Diethylether gewaschen. Die wässrige Phase wurde unter verminderten Druck eingeengt und noch dreimal mit je 20 ml Toluol kodestilliert. Der Rückstand wurde in Wasser aufgenommen und lyophilisiert. Der so erhaltene farblose Rückstand wurde dreimal mit je 10 ml Ethanol behandelt. Die vereinigten alkoholischen Phasen wurden unter verminderten Druck eingeengt und noch 2 mal mit Toluol kodestelliert. Der so erhaltene Rückstand (1,58 g 8-Aza-spiro[4.5]decan-8-sulfonsäure-Natriumsalz) wurde in 30 ml Toluol suspendiert und unter Argon mit 1,65 g Phosphorpentachlorid (1,1 eq.) versetzt. Die so erhaltene Reaktionsmischung wurde 18 h bei 100˚C gerührt. Nach Abkühlen wurde die Reaktionsmischung von ungelösten Teilen abfiltriert und das Filtrat unter verminderten Druck eingeengt. Auf diese Weise wurde 8-Aza-spiro[4.5]decan-8-sulfonylchlorid als farbloses Öl erhalten, welches ohne weitere Reinigung in der nächsten Reaktion eingesetzt wurde.
Ausbeute: 520 mg (30 % d. Theorie).

Beispiel 72: (S)-2-(8-Aza-spiro[4.5]decan-8-sulfonylamino)-4,4-difluor-pentansäure-methylester

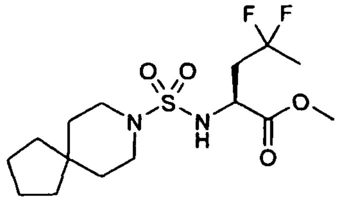

In einem Reaktionsgefäß wurden 100 mg (S)-2-Amino-4,4-difluor-pentansäure-methylester-hydrochlorid (0,49 mmol) mit 320 mg N,O-Bis(trimethylsilyl)acetamid (3,2 eq.) in 3 ml Acetonitril versetzt. Die Reaktionsmischung wurde für 30 min in der Mikrowelle bei 100˚C behandelt. Dann wurden 128 mg 8-Aza-spiro[4.5]decan-8-sulfonylchlorid (1,1 eq.), gelöst in 2,5 ml Acetonitril, hinzugesetzt und die entstandene Reaktionsmischung für weitere 2,5 h in der Mikrowelle bei 100˚C behandelt. Die Reaktionsmischung wurde unter vermindertem Druck eingeengt und der erhaltene Rückstand durch präparative HPLC (Gradient: Acetonitril/Wasser und Zusatz von 0,05 % TFA) gereinigt. (S)-2-(8-Aza-spiro[4.5]decan-8-sulfonylamino)-4,4-difluor-pentansäure-methylester wurde als farbloses, amorphes Material erhalten. Ausbeute: 75 mg, (41 % d. Theorie) MS ($ESI^+$): 369 .

Beispiel 73: (S)-2-(8-Aza-spiro[4.5]decan-8-sulfonylamino)-4,4-difluor-pentansäure

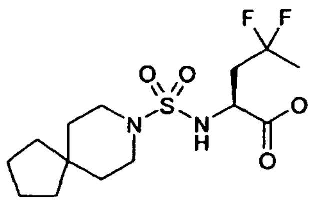

Die Herstellung von (S)-2-(8-Aza-spiro[4.5]decan-8-sulfonylamino)-4,4-difluor-pentansäure erfolgte analog zu Beispiel 62 ausgehend von 50 mg (S)-2-(8-Aza-spiro[4.5]decan-8-sulfonylamino)-4,4-difluor-pentansäure-methylester (0,14 mmol). Ausbeute: 30 mg (62 % d. Theorie)

Beispiel 74: (S)-2-(8-Aza-spiro[4.5]decan-8-sulfonylamino)-4,4-difluor-pentansäure (1-cyano-cyclopropyl)-amid

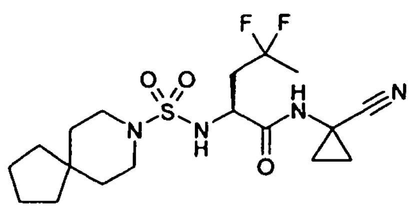

Zu einer Lösung von 30,0 mg (S)-2-(8-Aza-spiro[4.5]decan-8-sulfonylamino)-4,4-difluor-pentansäure (0,08 mmol) in 2 ml THF und 1 ml Dichlormethan wurden nacheinander 12,0 mg 1-Amino-cyclopropancarbonitril-Hydrochlorid (1,2 eq.), 13,8 mg HOAt (1,2 eq.), 33 µl N-Ethylmorpholin (3 eq.) und 19,5 mg EDCI (1,2 eq.) gegeben. Die Reaktionsmischung wurde 4 h bei RT gerührt und anschließend unter vermindertem Druck eingeengt. Der so erhaltene Rückstand wurde durch präparative HPLC (Gradient: Acetonitril/Wasser und Zusatz von 0,05 % TFA) gereinigt. Die Produkt enthaltenden Fraktionen wurden vereinigt und gefriergetrocknet. Auf diese Weise wurde (S)-2-(8-Aza-spiro[4.5]decan-8-sulfonylamino)-4,4-difluor-pentansäure (1-cyano-cyclopropyl)-amid als farbloses, amorphes Material erhalten.
Ausbeute: 20 mg , 56 % d. Theorie.
$^1$H-NMR: 9,1 (s, 1H); 7,85 (d, 1 H); 3,85 (m, 1H); 3,00 (m, 4H); 2,15 (m, 2H); 1,65 (t, 3H); 1,55 (m, 4H), 1,50 (m, 2H); 1,40 (t, 4H); 1,37 (m, 4H); 1,10 (m, 2H) ;
MS ($ESI^+$): 419,1

Analog zu den zuvor ausführlich beschriebenen Beispielen (Beispiele 1-35 und 58-74) wurden folgende weitere Verbindungen hergestellt.

Diese weiteren Verbindungen sind in Tabelle 1b) mit zugehöriger Charakterisierung dargestellt:

Tabelle 1b :

| Beispiel | Molmasse ( Stammverbindung) | Struktur | MS (ESI+) | 1H-NMR |
|---|---|---|---|---|
| 75 | 325,36 | 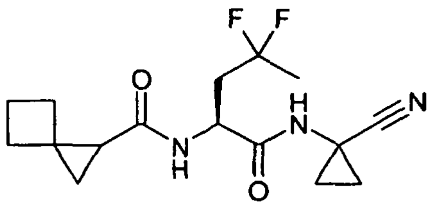 | 326,2 | 9,03 (s, 1 H); 8,35 (d, 1 H); 4,49 (m, 1 H); 1,85-2,40 (m, 9H); 1,62 (t, 3H); 1,50 (d, 2H); 1,10 (d, 2H); 0,75-0,95 (m, 2H) |
| 76 | 412,48 | 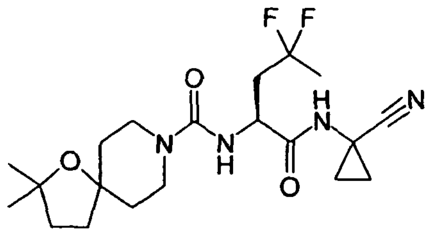 | 413,1 | 8,85 (s, 1 H); 6,70 (d, 1 H); 4,30 (m, 1 H); 3,20-3,40 (m, 4H); 2,25 (m, 2H); 1,80 (m, 4H); 1,62 (t, 3H); 1,45 (m, 6H); 1,20 (s, 6H); 1,10 (m, 2H) |
| 77 | 382,46 | 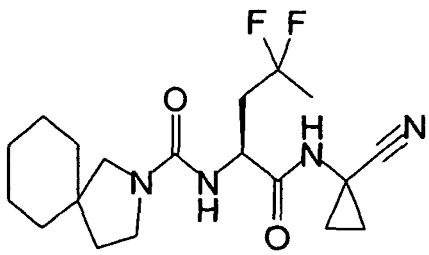 | 383,1 | 8,87 (s, 1H); 6,28 (d, 1 H); 4,30 (m, 1H); 3,22-3,38 (m, 2H); 3,08 (m, 2H); 2,20-2,38 (m, 2H); 1,55-1,68 (m, 5H); 1,30-1,52 (m, 12 H); 1,10 (d, 2H) |
| 78 | 432,47 | 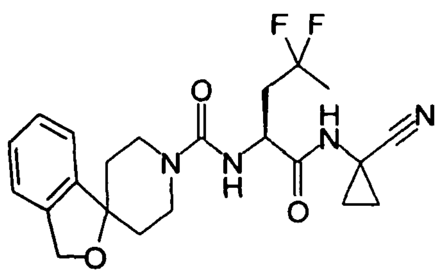 | 433,3 | 8,90 (s, 1 H); 7,29 (m, 3H); 7,20 (m, 1 H); 6,80 (d, 1 H); 5,00 (s, 2H); 4,35 (m, 1 H); 4,00 (m, 2H); 3,05 (m, 2H); 2,30 (m, 2H); 1,80 (m, 2H); 1,65 (t, 3H); 1,58 (m, 2H); 1,50 (d, 2H); 1,10 (d, 2H) |
| 79 | 430,50 | 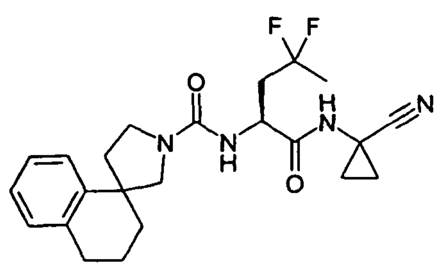 | 431,1 | 8,90 (s, 1H); 7,31 (m, 1H); 7,05-7,20 (m, 3H); 6,40 (d, 1 H); 4,38 (m, 1 H); 3,30-3,45 (m, 4H); 2,80 (t, 2H); 2,15-2,40 (m, 4H); 1,90 (m, 1 H); 1,60-1,80 (m, 6H); 1,50 (d, 2H); 1,10 (d, 2H) |

(fortgesetzt)

| Beispiel | Molmasse ( Stammverbindung) | Struktur | MS ($ESI^+$) | $^1$H-NMR |
|---|---|---|---|---|
| 80 | 384,43 | 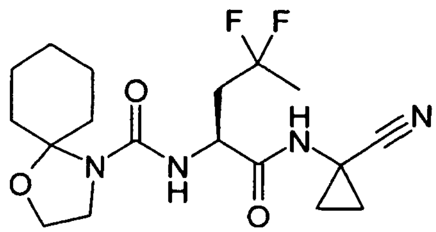 | 385,3 | 8,85 (s, 1H); 6,35 (d, 1H); 4,30 (m, 1H); 3,90 (t, 2H); 3,50 (q, 2H); 3,30-3,40 (m, 2H); 2,20-2,40 (m, 4H); 1,38-1,65 (m, 11H); 1,10(d, 2H) |
| 81 | 400,43 | 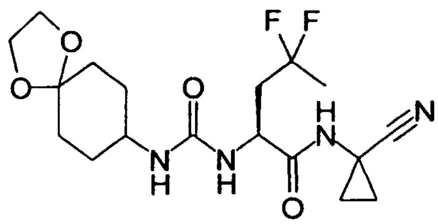 | 401,2 | 9,05 (s, 1 H); 6,69 (d, 1H); 6,60 (d, 1 H); 4,32 (m, 1H), 3,86 (s, 4H); 3,48 (m, 1H); 2,05-2,32 (m, 2H); 1,72 (m, 2H); 1,58-1,68 (m, 5H); 1,45-1,55 (m, 4H); 1,38 (m, 2H); 1,10 (dd, 2H) |
| 82 | 444,53 | 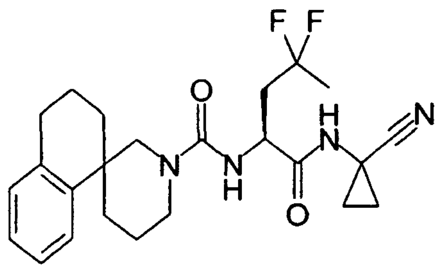 | 445,4 | 8,81 (s, 1H); 7,50 (t, 1H); 7,05-7,29 (m, 3H); 6,60 (t, 1 H); 4,35 (m,1H); 3,90-4,10 (m, 2H); 2,72-2,90 (m, 2H); 2,70 (s, 2H); 2,19-2,35 (m, 2H); 1,85-2,05 (m, 2H); 1,39-1,75 (m, 11H); 1,10 (d, 2H) |
| 83 | 409,48 | 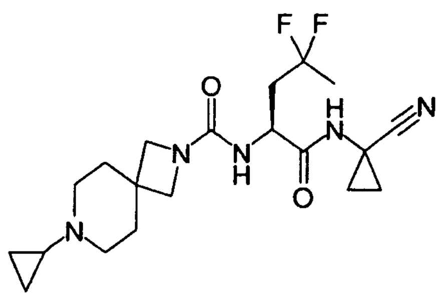 | 410,3 | Fumarat : 8,90 (s, 1H); 6,60 (s, 2H); 6,50 (d, 1H); 4,25 (m, 1H); 3,58 (d, 2H); 3,48 (d, 2H); 2,25-2,55 (m, 6H); 2,15 (m, 1H); 1,50-1,68 (m, 7 H); 1,45 (d, 2H); 1,10 (d, 2H); 0,40 (d, 2H); 0,28 (d, 2H) |
| 84 | 409,48 | 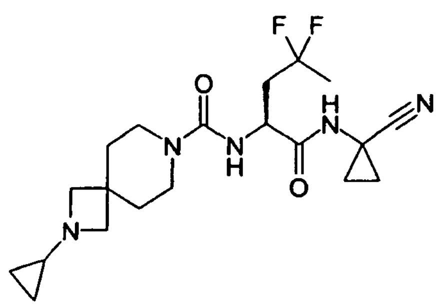 | 410,3 | Fumarat: 8,85 (s, 1 H); 6,68 (d, 1 H); 6,60 (s, 2H); 4,30 (m, 1 H); 3,15-3,50 (m, 4H); 3,05 (s, 4H); 2,25 (m, 2H); 1,95 (m, 1 H); 1,52-1,65 (m, 7H); 1,48 (m, 2H); 1,10 (m, 2H); 0,34 (d, 2H); 0,25 (d, 2H) |

(fortgesetzt)

| Beispiel | Molmasse ( Stammverbindung) | Struktur | MS (ESI+) | 1H-NMR |
|---|---|---|---|---|
| 85 | 411,50 | 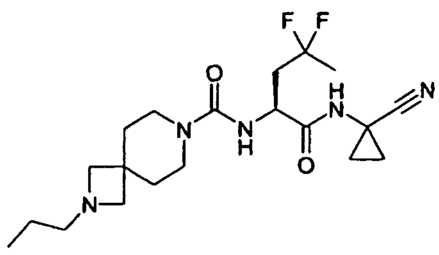 | 412,4 | Fumarat: 8,90 (s, 1 H); 6,72 (d, 1H), 6,50 (s, 2H); 4,28 (m, 1H); 3,10-3,50 (m, 8H); 2,65 (m, 2H); 2,25 (m, 2H); 1,53-1,65 (m, 7H); 1,45 (d, 2H); 1,38 (q, 2H); 1,10 (m, 2H); 0,87 (t, 3H) |
| 86 | 439,51 | 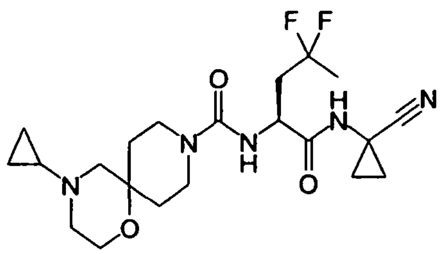 | 440,3 | TFA-Salz: 9,02 ($S_b$, 1 H); 8,88 (s, 1 H); 6,75 (d, 1 H); 4,30 (m, 1 H); 3,40-3,90 (m, 6H); 2,88-3,25 (m, 6H); 2,15-2,35 (m, 3H); 1,62 (t, 3H); 1,35-1,48 (m, 4H); 1,10 (d, 2H); 0,90-1,05 (m, 2H); 0,78 (m, 2H) |
| 87 | 439,51 | 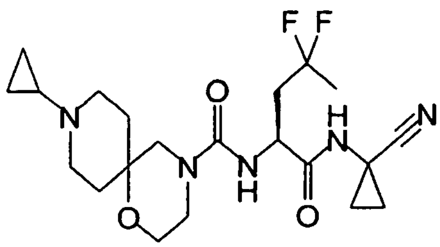 | 440,3 | TFA-Salz: 8,90 (s, 1 H); 8,70 ($S_b$, 1 H); 6,82 (d, 1 H); 4,32 (m, 1 H); 3,35-3,70 (m, 8H); 3,15-3,33 (m, 4H); 2,95 (m, 1 H); 2,25 (m, 2H); 2,03 (t, 2H); 1,62 (t, 3H); 1,50 (d, 2H); 1,10 (d, 2H); 0,90 (d, 2H); 0,80 (d, 2H) |
| 88 | 451,52 | 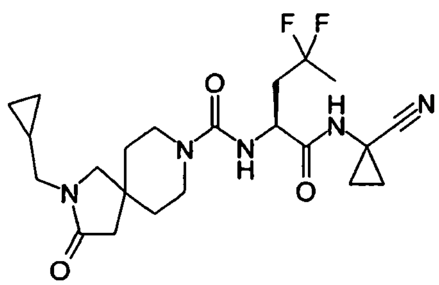 | 452,3 | 8,85 (s, 1 H); 6,72 (d, 1 H); 4,30 (m, 1 H); 3,40 (m, 4H); 3,25 (m, 4H); 3,05 (d, 2H); 2,25 (m, 2H); 2,15 (s, 2H); 1,62 (t, 3 H); 1,45 (m, 5H); 1,10 (m, 2H); 0,45 (d, 2H); 0,20 (d, 2H) |

(fortgesetzt)

| Beispiel | Molmasse ( Stammverbindung) | Struktur | MS (ESI+) | $^1$H-NMR |
|---|---|---|---|---|
| 89 | 444,53 | 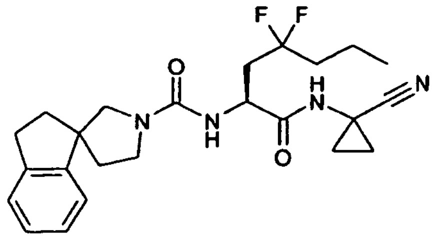 | 445,1 | 8,45 (s, 1 H); 7,10-7,28 (m, 4H); 5,90 (d, 1 H); 4,38 (m, 1 H); 3,30-3,60 (m, 4H); 2,90 (t, 2H); 2,15-2,40 (m, 2H); 1,75-2,10 (m, 6H); 1,38-1,52 (m, 4H); 1,15 (m, 2H); 0,90 (t, 3H) |
| 90 | 531,61 | 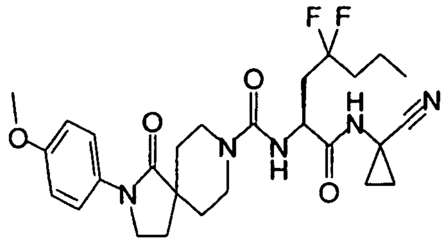 | 532,2 | 8,88 (s, 1H); 7,58 (d, 2H); 6,95 (d, 2H); 6,77 (d, 1H); 4,30 (m, 1H); 3,90 (m, 2H); 3,77 (m, 2H); 3,73 (s, 3H); 2,90 (m, 2H); 2,25 (m, 2H); 2,10 (t, 2H); 1,85 (m, 2H); 1,65 (q, 2H); 1,40-1,50 (m, 6H); 1,10 (m, 2H); 0,90 (t, 3H) |
| 91 | 517,62 | 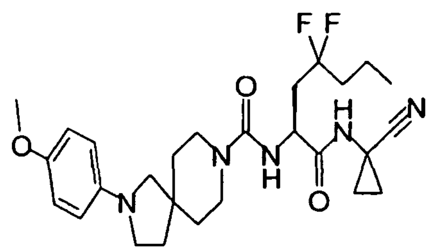 | 518,2 | 8,85 (s, 1H); 6,82 (d, 2H); 6,70 (d, 1H); 6,55 (d, 2H), 4,30 (m, 1H); 3,68 (s, 3H); 3,25-3,55 (m, 6H); 3,10 (s, 2H); 2,25 (m, 2H); 1,80 (m, 4H); 1,35-1,55 (m, 8H), 1,10 (d, 2H); 0,90 (t, 3H) |
| 92 | 437, 54 | 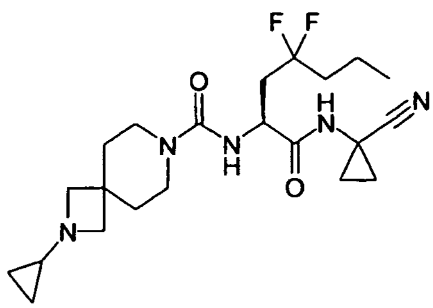 | 436,3 (ES-) | TFA-Salz: 9,80 ($S_b$, 1 H); 8,90 (s, 1 H); 6,75 (d, 1 H); 4,30 (m, 1 H); 3,97 (m, 2H); 3,90 (m, 2H); 3,15-3,30 (m, 4H); 3,10 (m, 1 H); 2,15-2,30 (m, 2H); 1,75-1,86 (m, 2H); 1,70 (s, 4H); 1,45 (d, 2H); 1,40 (m, 2H); 1,10 (d, 2H); 0,89 (t, 3H); 0,80 (d, 4H) |

(fortgesetzt)

| Beispiel | Molmasse ( Stammverbindung) | Struktur | MS (ESI+) | 1H-NMR |
|---|---|---|---|---|
| 93 | 451,56 | 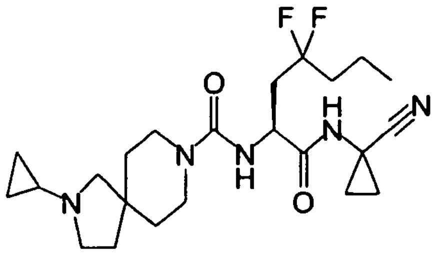 | 452,3 | TFA-Salz: 9,45 ($S_b$, 1 H); 8,90 (s, 1H); 6,72 (d, 1H); 4,31 (m, 1 H); 3,52-3,70 (m, 2H); 3,20-3,40 (m, 4H); 3,05 (m, 1 H); 2,95 (m, 1 H); 2,25 (m, 2H); 2,00 (m, 1 H); 1,72-1,88 (m, 4H); 1,58 (m, 1H); 1,38-1,55 (m, 7H); 1,10 (d, 2H); 0,90 (m, 5H); 0,82 (d, 2H) |
| 94 | 465,59 | 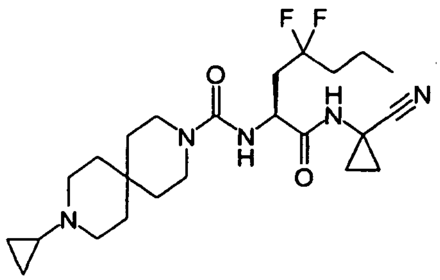 | 464,3 (ES) | TFA-Salz: 8,89 (s, 1 H); 8,52 ($S_b$, 1 H); 6,70 (d, 1 H); 4,30 (m, 1 H); 3,20-3,40 (m, 8H); 2,90 (m, 1 H); 2,20-2,30 (m, 2H); 1,78-1,88 (m, 4H); 1,57 ($S_b$, 2H); 1,35-1,49 (m, 6H); 1,29 ($S_b$, 2H); 1,10 (d, 2 H); 0,90 (m, 5H); 0,82 (d, 2H) |
| 95 | 437,54 | 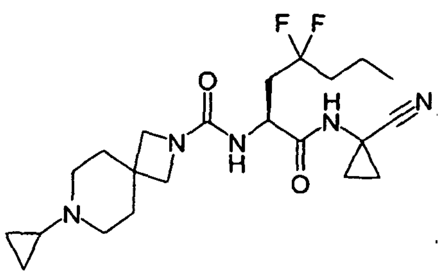 | 438,2 | TFA-Salz: 8,90 (s, 1 H); 8,68 ($S_b$, 1 H); 6,65 (d, 1 H); 4,29 (m, 1 H); 3,70 (d, 1 H); 3,65 (dd, 2H); 3,55 (d, 1 H); 3,30-3,45 (m, 2H); 3,12 (m, 2H); 2,87 ($S_b$, 1 H); 2,12-2,40 (m, 2H); 2,05 (m, 2H); 1,73-1,89 (m, 4H); 1,50 (d, 2H); 1,40 (m, 2H); 1,10 (dd, 2H); 0,90 (m, 5H); 0,85 (d, 2H) |

(fortgesetzt)

| Beispiel | Molmasse ( Stammverbindung) | Struktur | MS (ESI+) | 1H-NMR |
|---|---|---|---|---|
| 96 | 439,55 | 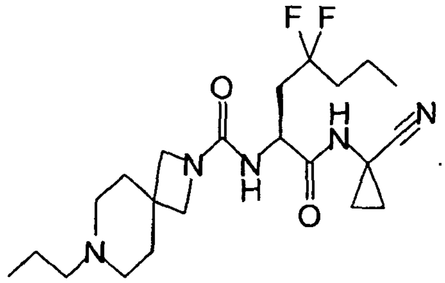 | 438,3 (ES-) | TFA-Salz: 9,01 ($S_b$, 1H); 8,90 (s, 1 H); 6,62 (d, 1H); 4,29 (m, 1 H); 3,50-3,68 (m, 4H); 3,25-. 3,40 (m, 2H); 3,00 (m, 2H); 2,87 (q, 2H); 2,10-2,38 (m, 2H); 2,02 (m, 2H); 1,80 (m, 4H); 1,65 (m, 2H); 1,50 (d, 2H); 1,40 (m, 2H); 1,10 (d, 2H); 0,90 (t, 6H) |
| 97 | 513,64 | 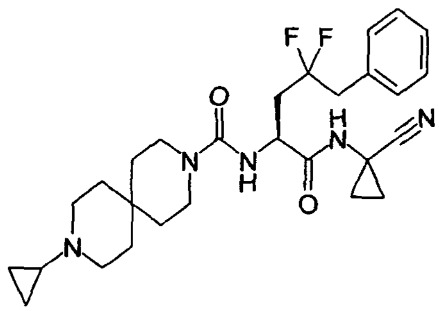 | 514,2 | TFA-Salz: 8,89 (s, 1H); 8,51 ($S_b$, 1 H); 7,22-7,39 (m, 5H); 6,72 (d, 1H); 4,39 (m, 1H); 3,20-3,40 (m, 10H); 2,90 (m, 1H); 2,35 (m, 1H); 2,18 (m, 1 H); 1,80-1,90 (m, 2H); 1,57 ($S_b$, 2H); 1,38-1,48 (m, 4H); 1,28 (t, 2 H); 1,10 (dd, 2H); 0,90 (d, 2H); 0,80 (d, 2H) |
| 98 | 485,58 | 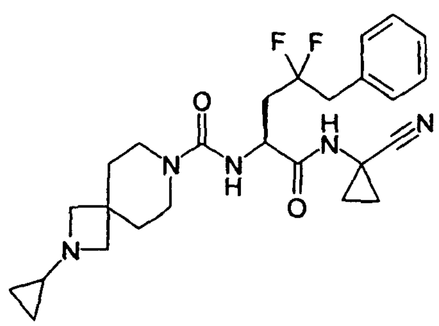 | 486,2 | TFA-Salz: 9,80 ($S_b$, 1 H); 8,90 (s, 1 H); 7,23-7,40 (m, 5H); 6,75 (d, 1H); 4,38 (m, 1 H); 3,95 (m, 2H); 3,89 (m, 2H); 3,15-3,42 (m, 6H); 3,09 (m, 1 H); 2,38 (m, 1 H); 2,20 (m, 1 H); 1,59-1,69 (m, 4H); 1,50 (d, 2H); 1,10 (dd, 2H); 0,80 (d, 4H) |

(fortgesetzt)

| Beispiel | Molmasse ( Stammverbindung) | Struktur | MS ($ESI^+$) | $^1$H-NMR |
|---|---|---|---|---|
| 99 | 487,60 | 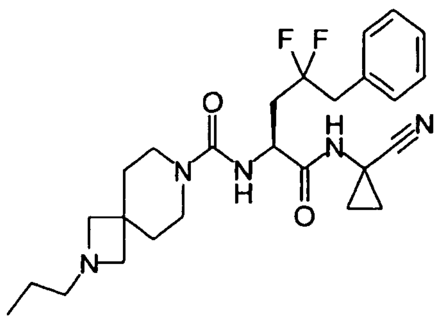 | 488,2 | TFA-Salz: 9,80 ($S_b$, 1 H); 8,90 (s, 1H); 7,23-7,42 (m, 5H); 6,75 (d, 1 H); 4,38 (m, 1 H); 3,92 (m, 2H); 3,82 (m, 2H); 3,15-3,40 (m, 6H); 3,11 (t, 2H); 2,35 (m, 1 H); 2,15 (m, 1H); 1,60-1,75 (m, 4H); 1,39-1,46 (m, 4 H); 1,10 (dd, 2H); 0,90 (t, 3H) |
| 100 | 485,58 | 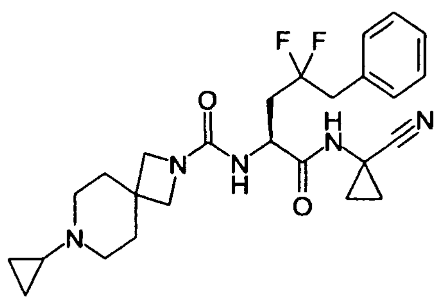 | 486,2 | TFA-Salz: 8,90 (s, 1 H); 8,60 ($d_b$, 1 H); 7,20-7,40 (m, 5H); 6,60 (d, 1 H); 4,40 (m, 1 H); 3,40-3,70 (m, 6H); 3,25 (t, 2H); 3,10 (q, 2H); 2,85 (m, 1 H); 2,35 (m, 1 H), 2,15 (m, 1 H); 2,0 (m, 2H); 1,72 (t, 2H); 1,50 (d, 2H); 1,10 (dd, 2H); 0,90 (d, 2H); 0,80 (d, 2H) |
| 101 | 328,36 | 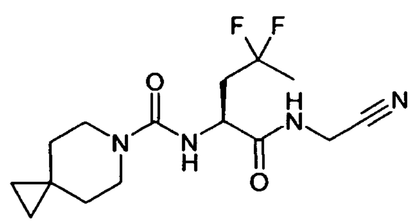 | 329,2 | 8,58 (t, 1 H); 6,75 (d, 1 H); 4,40 (m, 1H); 4,10 (d, 2 H); 3,30-3,40 (m, 4 H); 2,25 (m, 2H); 1,62 (t, 3H); 1,25(m, 4H); 0,30 (s, 4 H) |
| 102 | 382,46 | 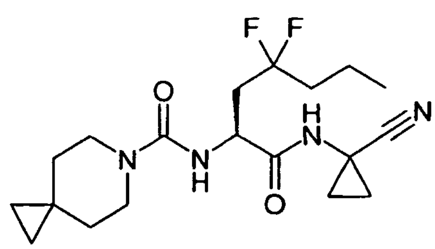 | 383,3 | 8,85 (s, 1 H); 6,70 (d, 1 H); 4,30 (m, 1 H); 3,30-3,40 (m, 4H); 2,25 (m, 2H); 1,82 (m, 2 H); 1,48 (d, 2H); 1,42 (m, 2H); 1,25 (m, 4H), 1,10 (m, 2H), 0,90 (t, 3H), 0,30 (s, 4 H) |
| 103 | 411,50 | 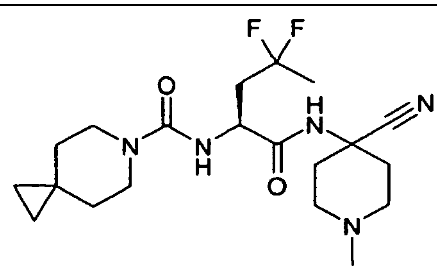 | 412,2 | 8,41 (s, 1 H); 6,70 (d, 2H); 4,42 (m, 1 H); 3,30-3,40 (m, 4H); 2,55 (m, 2H); 2,10-2,40 (m, 9H); 1,88 (m, 2H); 1.25 (m, 6H); 0.30 (s, 4H) |

(fortgesetzt)

| Beispiel | Molmasse ( Stammverbindung) | Struktur | MS (ESI+) | 1H-NMR |
|---|---|---|---|---|
| 104 | 439,55 | 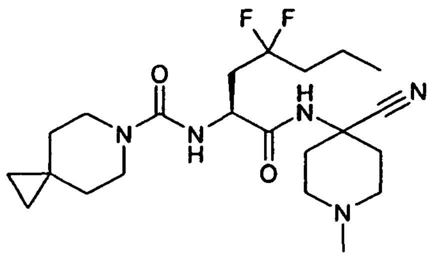 | 440,5 | 8.40 (s, 1 H); 6,70 (d, 1 H); 4,40 (q, 1 H); 4,05 (m, 2H); 3,40 (m, 4H); 2,18-2,35 (m, 4H); 2,15 (s, 3H); 1,78-1,94 (m, 4H); 1,42 (m, 2H); 1,26 (t, 4H); 1,19 (m, 2H); 0,90 (t, 3H); 0,30 (s, 4H) |
| 105 | 368,43 | 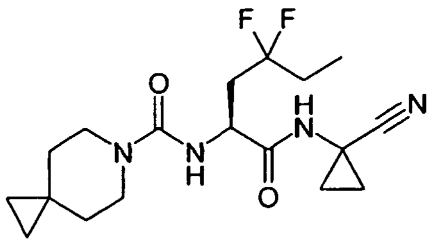 | 369,4 | 8,82 (s, 1 H); 6,68 (d, 1 H); 4,30 (m, 1 H); 3,29-3,40 (m, 4H); 2,30 (m, 2H); 1,88 (m, 2H); 1,48 (m, 2H); 1,25 (t, 4H); 1,10 (m, 2H); 0,90 (t, 3H); 0,30 (s, 4H) |

Die nachfolgenden Beispiele können in analoger Weise hergestellt werden:

6-Aza-spiro[2.5]octan-6-carbonsäure [(S)-1-(1-cyano-cyclopropylcarbamoyl)-3,3-difluor-5-methyl-hexyl]-amid

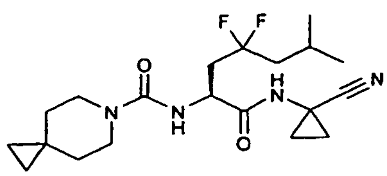

6-Aza-spiro[2.5]octan-6-carbonsäure [(S)-1-(1-cyano-cyclopropylcarbamoyl)-4-cyclopropyl-3,3-difluor-butyl]-amid

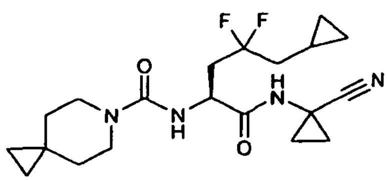

6-Aza-spiro[2.5]octan-6-carbonsäure [(S)-1-(1-cyano-cyclopropylcarbamoyl)-3,3-difluor-4-(2-methoxy-phenyl)-butyl]-amid

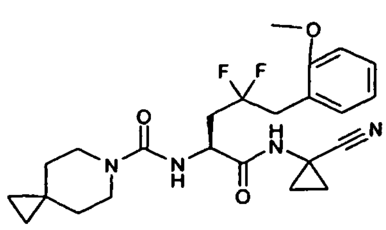

6-Aza-spiro[2.5]octan-6-carbonsäure [(S)-1-(1-cyano-cyclopropylcarbamoyl)-3,3-difluor-4-(2-trifluormethoxy-phe-

nyl)-butyl]-amid

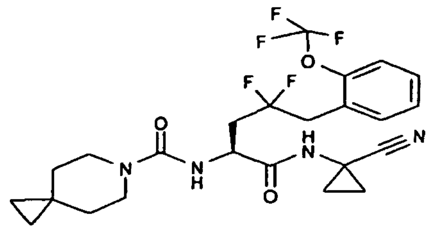

6-Aza-spiro[2.5]octan-6-carbonsäure [(S)-1-(1-cyano-cyclopropylcarbamoyl)-3,3-difluor-4-(2-fiuor-phenyl)-butyl]-amid

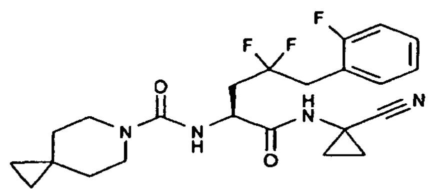

6-Aza-spiro[2.5]octan-6-carbonsäure [(S)-1-(1-cyano-cyclopropylcarbamoyl)-3,3-difluor-4-(4-fluor-phenyl)-butyl]-amid

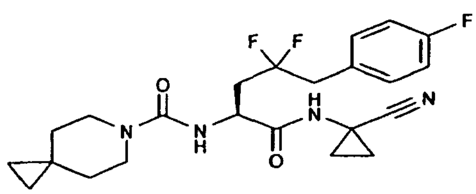

6-Aza-spiro[2.5]octan-6-carbonsäure [(S)-1-(1-cyano-cyclopropylcarbamoyl)-3,3-difluor-4-pyridin-2-yl-butyl]-amid

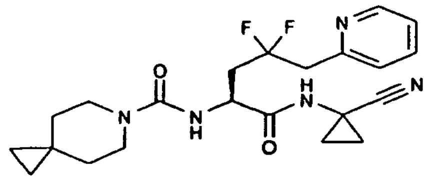

6-Aza-spiro[2.5]octan-6-carbonsäure [(S)-1-(1-cyano-cyclopropylcarbamoyl)-3,3-difluor-4-pyridin-3-yl-butyl]-amid

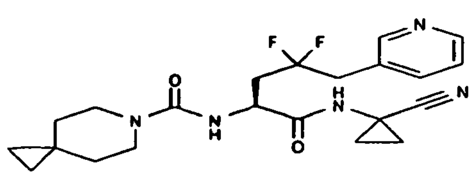

6-Aza-spiro[2.5]octan-6-carbonsäure [(S)-1-(1-cyano-cyclopropylcarbamoyl)-3,3-difluor-4-pyridin-4-yl-butyl]-amid

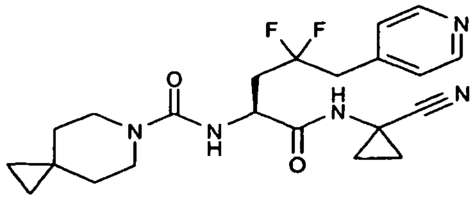

6-Aza-spiro[2.5]octan-6-carbonsäure [(S)-1-(cyanomethyl-carbamoyl)-3,3-difluor-4-phenyl-butyl]-amid

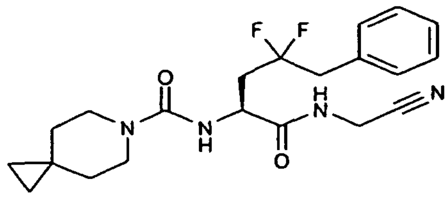

6-Aza-spiro[2.5]octan-6-carbonsäure [(S)-1-(cyanomethyl-carbamoyl)-4-cyclopropyl-3,3-difluor-butyl]-amid

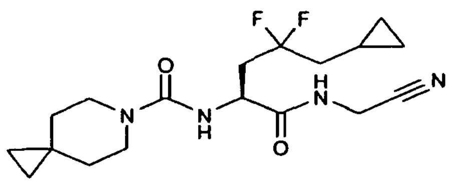

6-Aza-spiro[2.5]octan-6-carbonsäure [(S)-1-(cyanomethyl-carbamoyl)-3,3-difluor-4-pyridin-3-yl-butyl]-amid

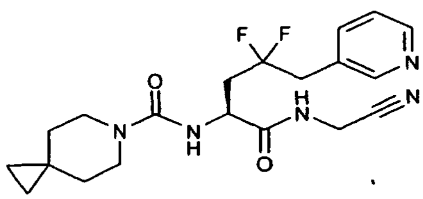

6-Aza-spiro[2.5]octan-6-carbonsäure [(S)-1-(cyanomethyl-carbamoyl)-4-(2-difluormethoxy-phenyl)-3,3-difluor-butyl]-amid

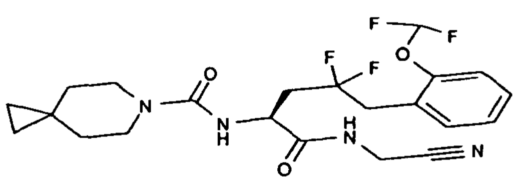

7-Aza-spiro[3.5]nonan-7-carbonsäure [(S)-1-(1-cyano-cyclopropylcarbamoyl)-3,3-difluor-butyl]-amid

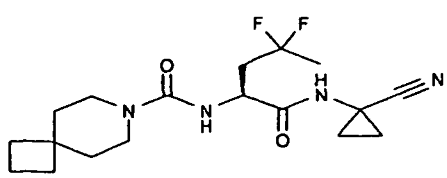

7-Aza-spiro[3.5]nonan-7-carbonsäure [(S)-1-(1-cyano-cyclopropylcarbamoyl)-4-cyclopropyl-3,3-difluor-butyl]-amid

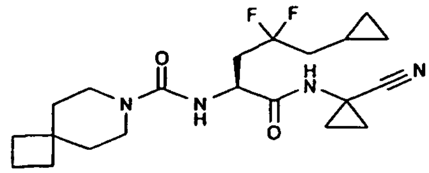

7-Aza-spiro[3.5]nonan-7-carbonsäure [(S)-1-(1-cyano-cyclopropylcarbamoyl)-3,3-difluor-4-phenyl-butyl]-amid

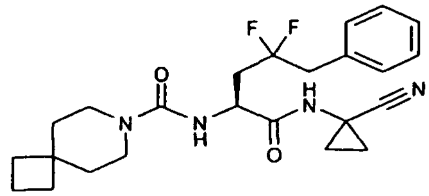

7-Aza-spiro[3.5]nonan-7-carbonsäure[(S)-1-(1-cyano-cyclopropylcarbamoyl)-3,3-difluor-4-pyridin-2-yl-butyl]-amid

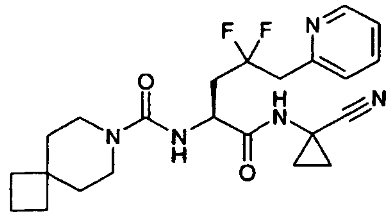

7-Aza-spiro[3.5]nonan-7-carbonsäure [(S)-1-(1-cyano-cyclopropylcarbamoyl)-3,3-difluor-4-pyridin-3-yl-butyl]-amid

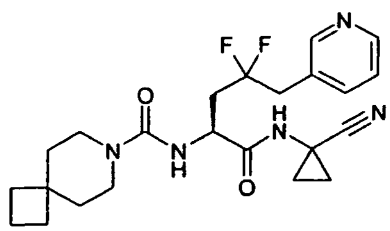

7-Aza-spiro[3.5]nonan-7-carbonsäure [(S)-1-(1-cyano-cyclopropylcarbamoyl)-3,3-difluor-4-pyridin-4-yl-butyl]-amid

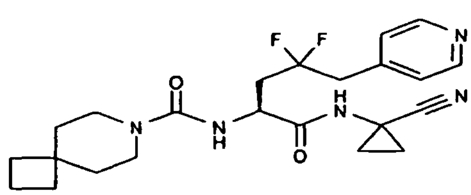

7-Cyclopropyl-2,7-diaza-spiro[3.5]nonan-2-carbonsäure [(S)-1-(1-cyano-cyclopropyl-carbamoyl)-3,3-difluor-4-(2-methoxy-phenyl)-butyl]-amid

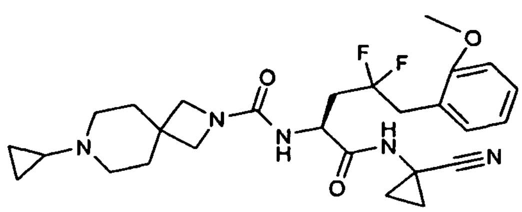

7-Cyclopropyl-2,7-diaza-spiro[3.5]nonan-2-carbonsäure [(S)-1-(1-cyano-cyclopropylcarbamoyl)-3,3-difluor-4-(2-fluor-phenyl)-butyl]-amid

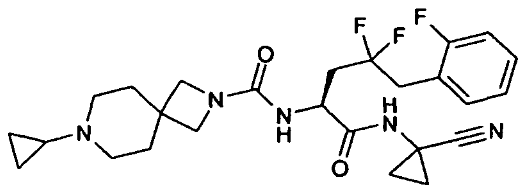

7-Cyclopropyl-2,7-diaza-spiro[3.5]nonan-2-carbonsäure [(S)-1-(1-cyano-cyclopropylcarbamoyl)-3,3-difluor-4-(4-fluor-phenyl)-butyl]-amid

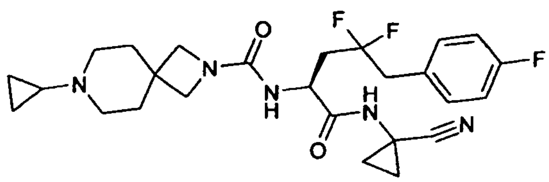

7-Cyclopropyl-2,7-diaza-spiro[3.5]nonan-2-carbonsäure [(S)-1-(1-cyano-cyclopropylcarbamoyl)-3,3-difluor-4-pyridin-2-yl-butyl]-amid

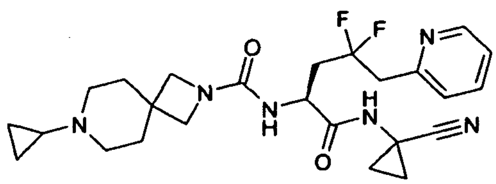

7-Cyclopropyl-2,7-diaza-spiro[3.5]nonan-2-carbonsäure [(S)-1-(1-cyano-cyclopropylcarbamoyl)-3,3-difluor-4-pyridin-3-yl-butyl]-amid

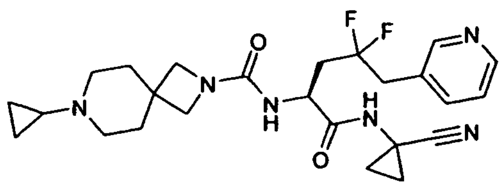

7-Cyclopropyl-2,7-diaza-spiro[3.5]nonan-2-carbonsäure [(S)-1-(1-cyano-cyclopropylcarbamoyl)-3,3-difluor-4-pyridin-4-yl-butyl]-amid

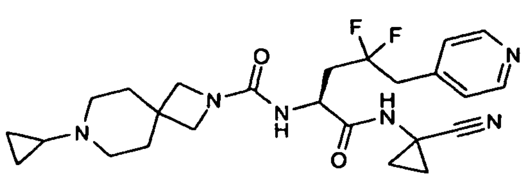

7-Cyclopropyl-2,7-diaza-spiro[3.5]nonan-2-carbonsäure [(S)-1-(1-cyano-cyclopropylcarbamoyl)-3,3-difluor-5-methyl-hexyl]-amid

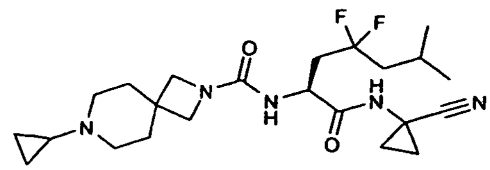

7-Cyclopropyl-2,7-diaza-spiro[3.5]nonan-2-carbonsäure [(S)-1-(1-cyano-cyclopropylcarbamoyl)-4-cyclopropyl-3,3-difluor-butyl]-amid

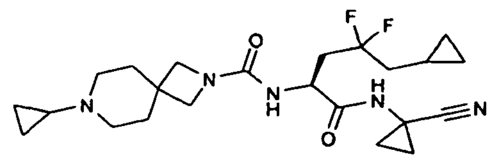

7-Cyclopropyl-2,7-diaza-spiro[3.5]nonan-2-carbonsäure [(S)-1-(cyanomethyl-carbamoyl)-3,3-difluor-4-phenyl-butyl]-amid

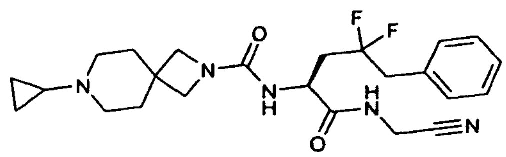

7-Cyclopropyl-2,7-diaza-spiro[3.5]nonan-2-carbonsäure [(S)-1-(cyanomethyl-carbamoyl)-3,3-difluor-4-pyridin-3-yl-butyl]-amid

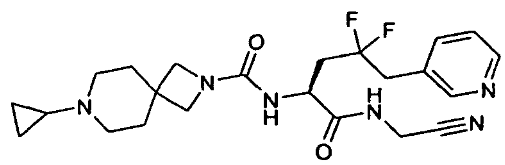

7-Cyclopropyl-2,7-diaza-spiro[3.5]nonan-2-carbonsäure [(S)-1-(cyanomethyl-carbamoyl)-4-cyclopropyl-3,3-difluor-butyl]-amid

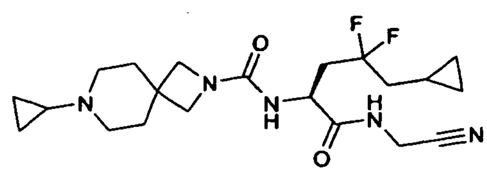

7-Cyclopropyl-2,7-diaza-spiro[3.5]nonan-2-carbonsäure [(S)-1-(cyanomethyl-carbamoyl)-3,3-difluor-4-(2-fluor-phenyl)-butyl]-amid

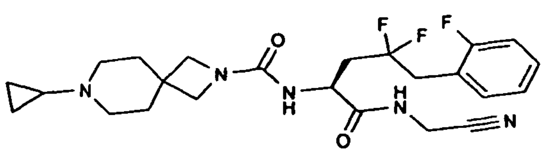

1,1-Difluor-6-aza-spiro[2.5]octan-6-carbonsäure [(S)-1-(1-cyano-cyclopropyl-carbamoyl)-3,3-difluor-butyl]-amid

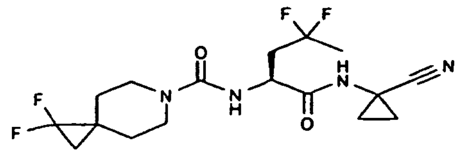

1,1-Difluor-6-aza-spiro[2.5]octan-6-carbonsäure [(S)-1-(1-cyano-cyclopropyl-carbamoyl)-3,3-difluor-pentyl]-amid

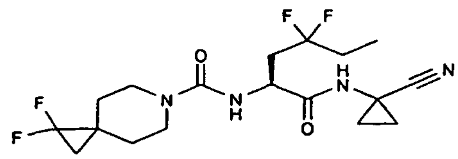

1,1-Difluor-6-aza-spiro[2.5]octan-6-carbonsäure [(S)-1-(1-cyano-cyclopropyl-carbamoyl)-4-cyclopropyl-3,3-difluor-butyl]-amid

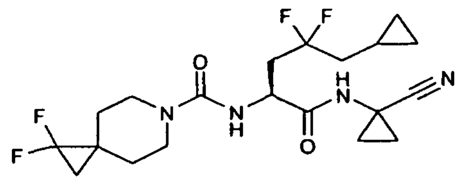

1,1-Difluor-6-aza-spiro[2.5]octan-6-carbonsäure [(S)-1-(1-cyano-cyclopropyl-carbamoyl)-3,3-difluor-4-pyridin-3-yl-butyl]-amid

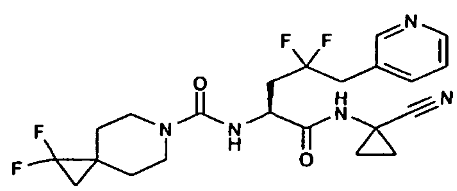

6-Aza-spiro[2.5]octan-6-carbonsäure [(S)-1-(1-cyano-cyclopropylcarbamoyl)-4,4-difluor-pentyl]-amid

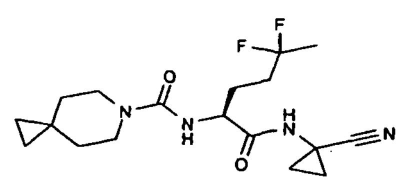

6-Aza-spiro[2.5]octan-6-carbonsäure [(S)-1-(1-cyano-cyclopropylcarbamoyl)-4,4-difluor-4-phenyl-butyl]-amid

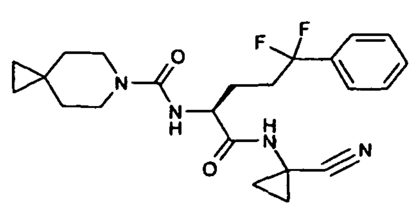

6-Aza-spiro[2.5]octan-6-carbonsäure [(S)-1-(1-cyano-cyclopropylcarbamoyl)-4,4-difluor-4-pyridin-2-yl-butyl]-amid

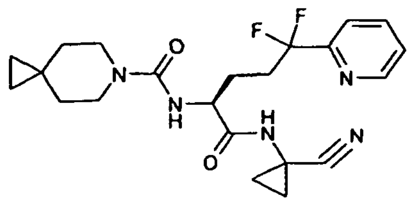

6-Aza-spiro[2.5]octan-6-carbonsäure [(S)-1-(1-cyano-cyclopropylcarbamoyl)-4,4-difluor-4-pyridin-3-yl-butyl]-amid

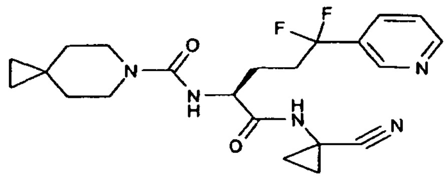

6-Aza-spiro[2.5]octan-6-carbonsäure [(S)-1-(1-cyano-cyclopropylcarbamoyl)-4,4-difluor-4-pyridin-4-yl-butyl]-amid

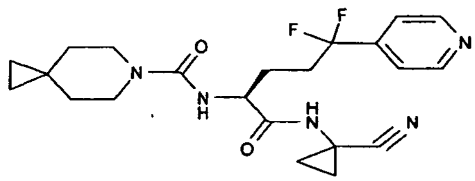

6-Aza-spiro[2.5]octan-6-carbonsäure [(S)-1-(1-cyano-cyclopropylcarbamoyl)-4-(2-difluormethoxy-phenyl)-4,4-difluor-butyl]-amid

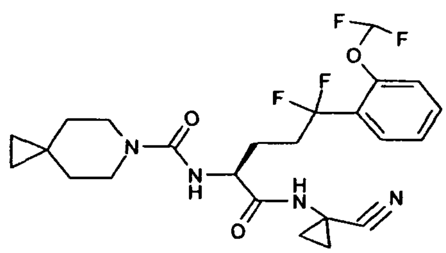

6-Aza-spiro[2.5]octan-6-carbonsäure [(S)-1-(1-cyano-cyclopropylcarbamoyl)-4,4-difluor-4-(4-fluor-phenyl)-butyl]-amid

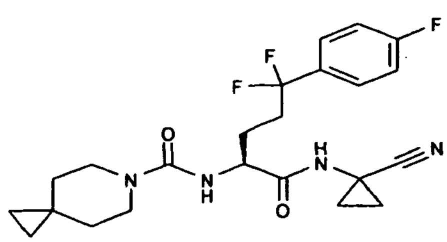

6-Aza-spiro[2.5]octan-6-carbonsäure [(S)-1-(1-cyano-cyclopropylcarbamoyl)-4,4-difluor-4-(2-fluor-phenyl)-butyl]-amid

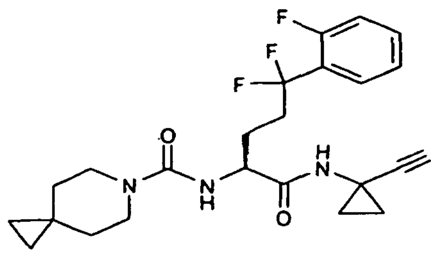

6-Aza-spiro[2.5]octan-6-carbonsäure [(S)-1-(4-cyano-1-methyl-piperidin-4-ylcarbamoyl)-4,4-difluor-4-phenyl-butyl]-amid

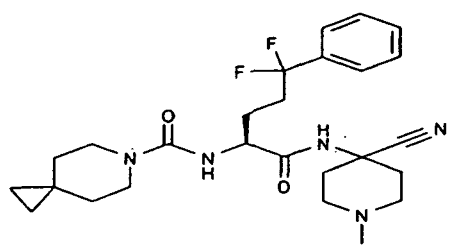

7-Cyclopropyl-2,7-diaza-spiro[3.5]nonan-2-carbonsäure [(S)-1-(1-cyano-cyclo-propylcarbamoyl)-4,4-difluor-4-phenyl-butyl]-amid

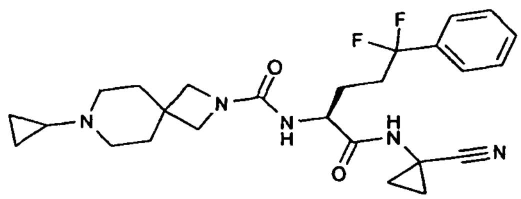

7-Cyclopropyl-2,7-diaza-spiro[3.5]nonan-2-carbonsäure [(S)-1-(1-cyano-cyclo-propylcarbamoyl)-4,4-difluor-4-(4-fluor-phenyl)-butyl]-amid

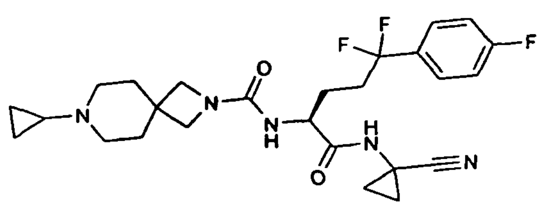

7-Cyclopropyl-2,7-diaza-spiro[3.5]nonan-2-carbonsäure [(S)-1-(1-cyano-cyclo-propylcarbamoyl)-4,4-difluor-4-(2-fluor-phenyl)-butyl]-amid

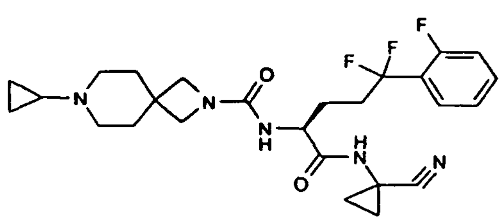

7-Cyclopropyl-2,7-diaza-spiro[3.5]nonan-2-carbonsäure [(S)-1-(1-cyano-cyclo-propylcarbamoyl)-4-(2-difluormethoxy-phenyl)-4,4-difluor-butyl]-amid

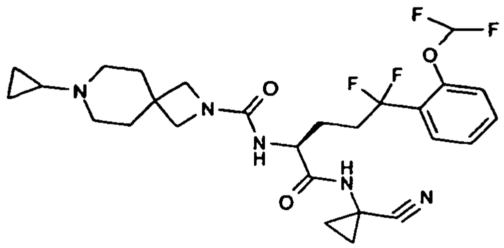

7-Cyclopropyl-2,7-diaza-spiro[3.5]nonan-2-carbonsäure [(S)-1-(1-cyano-cyclopropylcarbamoyl)-4,4-difluor-4-pyridin-2-yl-butyl]-amid

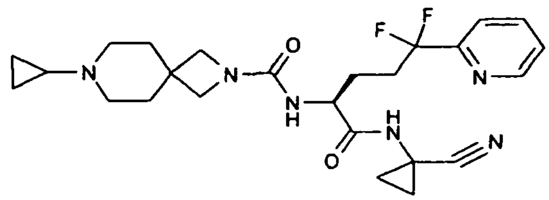

7-Cyclopropyl-2,7-diaza-spiro[3.5]nonan-2-carbonsäure [(S)-1-(1-cyano-cyclo-propylcarbamoyl)-4,4-difluor-4-pyridin-3-yl-butyl]-amid

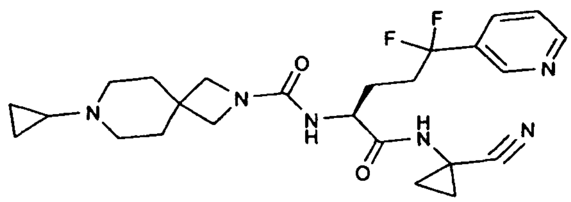

7-Cyclopropyl-2,7-diaza-spiro[3.5]nonan-2-carbonsäure [(S)-1-(1-cyano-cyclo-propylcarbamoyl)-4,4-difluor-4-pyridin-4-yl-butyl]-amid

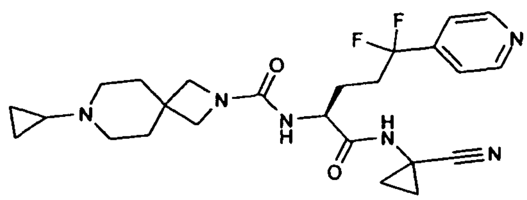

7-Methyl-2,7-diaza-spiro[3.5]nonan-2-carbonsäure [(S)-1-(1-cyano-cyclo-propylcarbamoyl)-3,3-difluor-4-phenyl-butyl]-amid

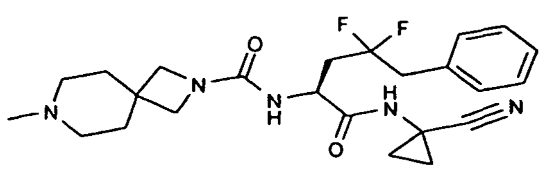

7-(2-Methoxy-ethyl)-2,7-diaza-spiro[3.5]nonan-2-carbonsäure [(S)-1-(1-cyano-cyclopropylcarbamoyl)-3,3-difluor-4-phenyl-butyl]-amid

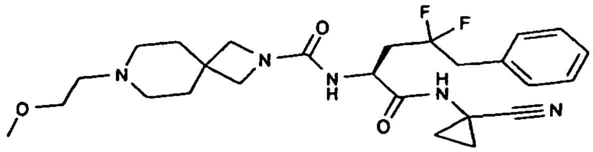

9-(2-Methoxy-ethyl)-3,9-diaza-spiro[5.5]undecan-3-carbonsäure [(S)-1-(1-cyano-cyclopropylcarbamoyl)-3,3-difluor-4-phenyl-butyl]-amid

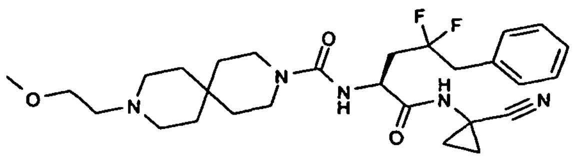

7-Amino-5-aza-spiro[2.4]heptan-5-carbonsäure [(S)-1-(1-cyano-cyclopropyl-carbamoyl)-3,3-difluor-4-phenyl-butyl]-amid

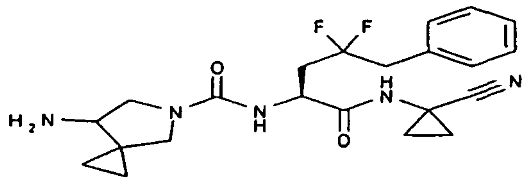

7-Dimethylamino-5-aza-spiro[2.4]heptan-5-carbonsäure [(S)-1-(1-cyano-cyclopropylcarbamoyl)-3,3-difluor-4-phenyl-butyl]-amid

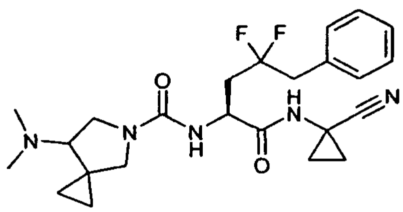

7-(Cyclopropancarbonyl-amino)-5-aza-spiro[2.4]heptan-5-carbonsäure [(S)-1-(1-cyano-cyclopropylcarbamoyl)-3,3-difluor-butyl]-amid

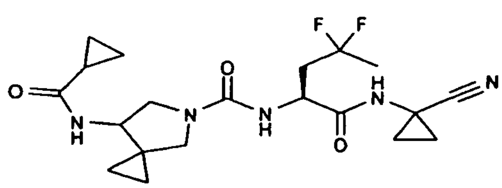

6-[(S)-1-(1-Cyano-cyclopropylcarbamoyl)-3,3-difluor-butylcarbamoyl]-6-aza-spiro[2.5]octan-1-carbonsäure

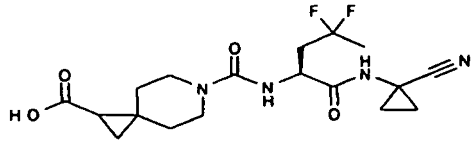

6-[(S)-1-(1-Cyano-cyclopropylcarbamoyl)-3,3-difluor-butylcarbamoyl]-6-aza-spiro[2.5]octan-1-carbonsäure-ethylester

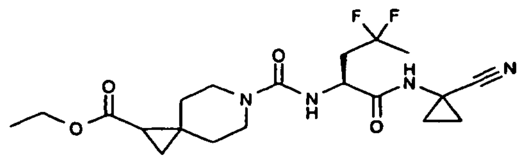

1-Hydroxymethyl-6-aza-spiro[2.5]octan-6-carbonsäure [(S)-1-(1-cyano-cyclopropyl-carbamoyl)-3,3-difluor-butyl]-amid

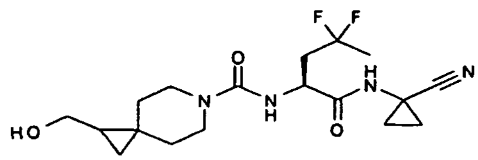

8-[(S)-1-(1-Cyano-cyclopropylcarbamoyl)-3,3-difluor-butylcarbamoyl]-1-oxa-2,8-diaza-spiro[4.5]dec-2-en-3-carbonsäure

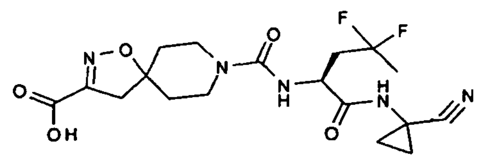

8-[(S)-1-(1-Cyano-cyclopropylcarbamoyl)-3,3-difluor-4-phenyl-butylcarbamoyl]-1-oxa-2,8-diaza-spiro[4.5]dec-2-en-3-carbonsäure

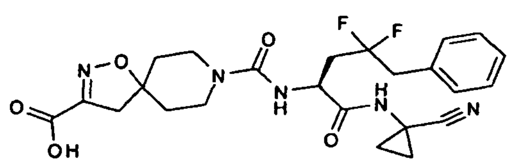

8-[(S)-1-(1-Cyano-cyclopropylcarbamoyl)-3,3-difluor-pentylcarbamoyl]-1-oxa-2,8-diaza-spiro[4.5]dec-2-en-3-carbonsäure

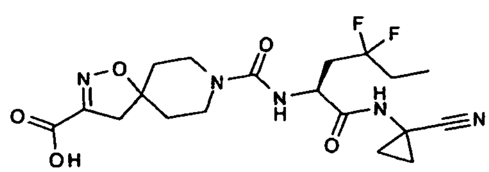

7-[(S)-1-(1-Cyano-cyclopropylcarbamoyl)-3,3-difluor-4-phenyl-butylcarbamoyl]-1-oxa-2,7-diaza-spiro[4.5]dec-2-en-3-carbonsäure

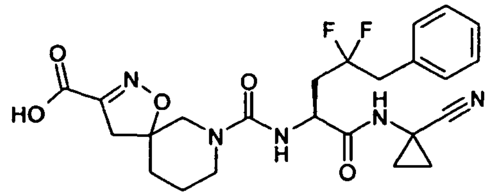

7-[(S)-1-(1-Cyano-cyclopropylcarbamoyl)-3,3-difluor-4-phenyl-butylcarbamoyl]-1-oxa-2,7-diaza-spiro[4.4]non-2-en-3-carbonsäure

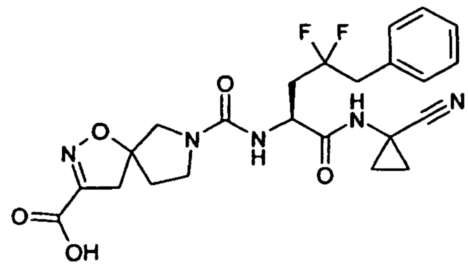

{3-[(S)-1-(1-Cyano-cyclopropylcarbamoyl)-3,3-difluor-butylcarbamoyl]-3-aza-spiro[5.5]undec-9-yl}-essigsäure

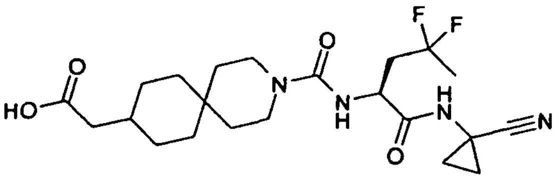

{3-[(S)-1-(1-Cyano-cyclopropylcarbamoyl)-3,3-difluor-pentylcarbamoyl]-3-aza-spiro[5.5]undec-9-yl}-essigsäure

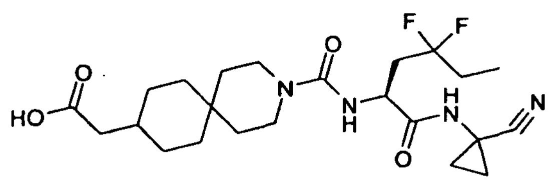

1,3-Dioxo-2,8-diaza-spiro[4.5]decan-8-carbonsäure [(S)-1-(1-cyano-cyclopropyl-carbamoyl)-3,3-difluor-pentyl]-amid

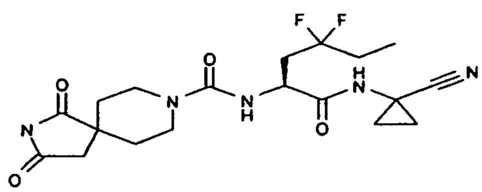

1,3-Dioxo-2,8-diaza-spiro[4.5]decan-8-carbonsäure [(S)-1-(1-cyano-cyclopropyl-carbamoyl)-3,3-difluor-butyl]-amid

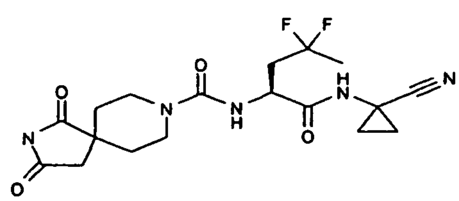

1,3-Dioxo-2,8-diaza-spiro[4.5]decan-8-carbonsäure [(S)-1-(1-cyano-cyclopropyl-carbamoyl)-3,3-difluor-4-phenylbutyl]-amid

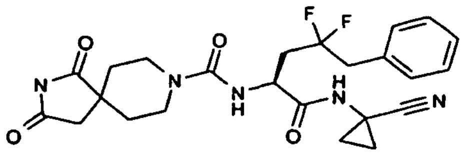

5-Aza-spiro[2.5]octan-5-carbonsäure [(S)-1-(1-cyano-cyclopropylcarbamoyl)-3,3-difluor-5-methyl-hexyl]-amid

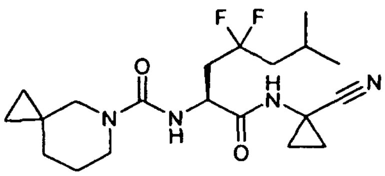

5-Aza-spiro[2.5]octan-5-carbonsäure [(S)-1-(1-cyano-cyclopropylcarbamoyl)-3,3-difluor-butyl]-amid

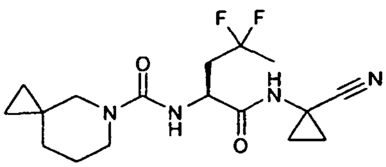

5-Aza-spiro[2.5]octan-5-carbonsäure [(S)-1-(1-cyano-cyclopropylcarbamoyl)-3,3-difluor-4-phenyl-butyl]-amid

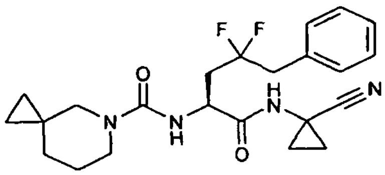

5-Aza-spiro[2.5]octan-5-carbonsäure [(S)-1-(cyanomethyl-carbamoyl)-3,3-difluor-butyl]-amid

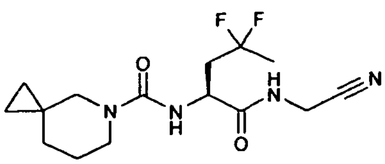

5-Aza-spiro[2.5]octan-5-carbonsäure [(S)-1-(cyanomethyl-carbamoyl)-3,3-difluor-4-phenyl-butyl]-amid

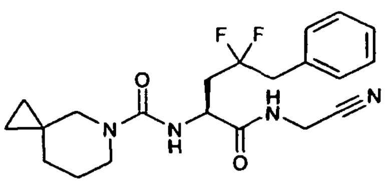

5-Aza-spiro[2.5]octan-5-carbonsäure [(S)-1-(1-cyano-cyclopropylcarbamoyl)-3,3-difluor-4-(4-fluor-phenyl)-butyl]-amid

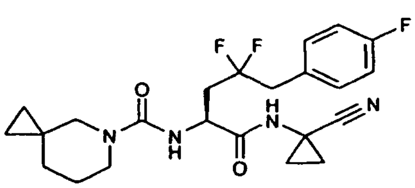

8-Cyclopropyl-1-oxa-2,8-diaza-spiro[4.5]dec-2-en-3-carbonsäure [(S)-1-(1-cyano-cyclopropylcarbamoyl)-3,3-difluor-butyl]-amid

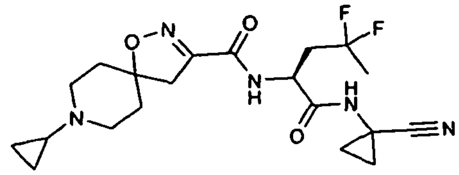

8-Propyl-1-oxa-2,8-diaza-spiro[4.5]dec-2-en-3-carbonsäure [(S)-1-(1-cyano-cyclopropylcarbamoyl)-3,3-difluor-butyl]-amid

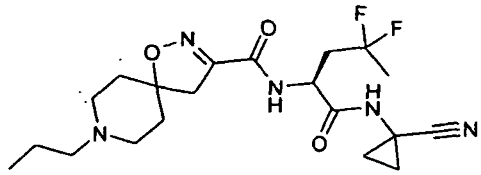

7-Cyclopropyl-1-oxa-2,7-diaza-spiro[4.5]dec-2-en-3-carbonsäure [(S)-1-(1-cyano-cyclopropylcarbamoyl)-3,3-difluor-butyl]-amid

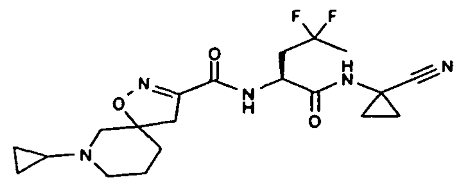

7-Propyl-1-oxa-2,7-diaza-spiro[4.5]dec-2-en-3-carbonsäure [(S)-1-(1-cyano-cyclopropylcarbamoyl)-3,3-difluor-butyl]-amid

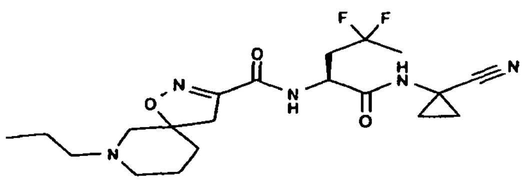

7-Cyclopropyl-1-oxa-2,7-diaza-spiro[4.4]non-2-en-3-carbonsäure [(S)-1-(1-cyano-cyclopropylcarbamoyl)-3,3-difluor-butyl]-amid

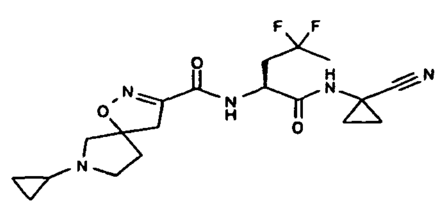

7-Methyl-1-oxa-2,7-diaza-spiro[4.4]non-2-en-3-carbonsäure [(S)-1-(1-cyano-cyclopropylcarbamoyl)-3,3-difluor-butyl]-amid

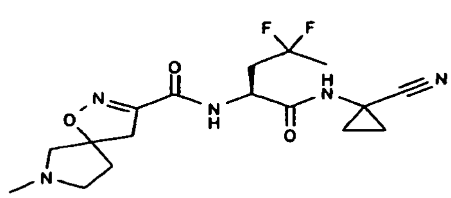

7-Methyl-1-oxa-2,7-diaza-spiro[4.4]non-2-en-3-carbonsäure [(S)-1-(1-cyano-cyclopropylcarbamoyl)-3,3-difluor-4-phenyl-butyl]-amid

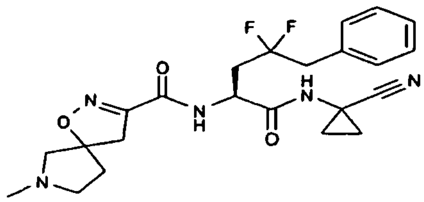

Pharmakologische Beispiele :

Bestimmung der enzymatischen Aktivität der katalytischen Domäne des humanen Cathepsin-B.

Dieses Protein wird als inaktives Enzym von der Fa. Sigma, Wiesbaden, Deutschland, erhalten (Katalog Nr. C8571). Das Enzym wird wie folgt aktiviert:

25 µg Enzym werden mit Acetat-Puffer auf eine Konzentration von 12,5 µg/mL verdünnt. 1 Volumenanteil Enzym wird mit 20 Volumenanteilen Cystein-Lösung versetzt und mit dem Acetat/Hepes-Puffer auf eine Konzentration von 0,11 µg/mL verdünnt und bei 37 °C für 5 Minuten inkubiert.

Zur Messung der Enzymaktivität werden 10 µL Enzymlösung mit 10 µL einer 3%igen (v/v) wässrigen Dimethylsulfoxid-Lösung (Reaktion 1) für 10 Minuten inkubiert. Zur Messung der Enzyminhibitoraktivität werden 10 µL Enzymlösung mit 10 µL einer 3%igen (v/v) wässrigen Dimethylsulfoxid-Lösung, die den Enzyminhibitor enthält, inkubiert (Reaktion 2).

Sowohl bei Reaktion 1 als auch bei Reaktion 2 wird nach Zugabe von 10 µL einer 3 %igen (v/v) wässrigen Dimethylsulfoxid-Lösung, die 0,3 mmol/L des Substrates enthält, die Enzymreaktion fluoreszenzspektroskopisch verfolgt (360nm (Anregung) /465nm (Emission)).

Die Enzymaktivität wird dargestellt als Fluoreszenzzunahme/Minute.

Die Inhibitorwirkung wird als prozentuale Hemmung nach folgender Formel berechnet:

$$\text{\% Hemmung} = 100 - [(\text{Fluoreszenzzunahme/Minute in Reaktion 2}) / (\text{Fluoreszenzzunahme/Minute in Reaktion 1}) \times 100].$$

Der $IC_{50}$, dies ist die für eine 50%ige Hemmung der Enzymaktivität erforderliche Inhibitorkonzentration wird grafisch durch Auftragen der prozentualen Hemmungen bei verschiedenen Inhibitorkonzentrationen ermittelt.

Der Acetat-Puffer enthält 0,1 mol/L Natriumacetat, 0,1 mol/L Natriumchlorid sowie 0,001 % Pluronic (Sigma, Deisenhofen, Deutschland) pH 4,5.

Die Cystein-Lösung enthält 0,3 mol/L Cystein in Wasser. Der Acetat/Hepes-Puffer enthält 0,15 mol/L Natriumacetat, 0,15 mol/L Hepes, 0,3 mol/L Natriumchlorid sowie 0,001 % Pluronic (Sigma, Deisenhofen, Deutschland) pH 6,5.

Die Enzymlösung enthält 0,11 µg/mL der Enzymdomäne.

Die Substratlösung enthält 0,3 mmol/L des fluorogenen Substrates Z-Arg-Arg-AMC (Bachem, Heidelberg, Deutschland).

Bestimmung der enzymatischen Aktivität der katalytischen Domäne des humanen Cathepsin-K.

Dieses Protein wird als inaktives Enzym von der Fa. Sanofi-Aventis, Frankfurt, Deutschland, erhalten. Das Enzym wird wie folgt aktiviert:

1 Volumenanteil Enzym wird mit 4 Volumenanteilen Cystein-Lösung versetzt. Anschließend wird dieses mit dem Acetat/Hepes Puffer auf eine Konzentration von 0,22 µg/mL verdünnt.

Zur Messung der Enzymaktivität werden 10 µL Enzymlösung mit 10 µL einer 3%igen (v/v) wässrigen Dimethylsulfoxid-Lösung (Reaktion 1) für 10 Minuten inkubiert. Zur Messung der Enzyminhibitoraktivität werden 10 µL Enzymlösung mit 10 µL einer 3%igen (v/v) wässrigen Dimethylsulfoxid-Lösung, die den Enzyminhibitor enthält, inkubiert (Reaktion 2).

Sowohl bei Reaktion 1 als auch bei Reaktion 2 wird nach Zugabe von 10 µL einer 3%igen (v/v) wässrigen Dimethylsulfoxid-Lösung, die 0,3 mmol/L des Substrates enthält, die Enzymreaktion fluoreszenzspektroskopisch verfolgt (360nm (Anregung) /465nm (Emission)).

Die Enzymaktivität wird dargestellt als Fluoreszenzzunahme/Minute.

Die Inhibitorwirkung wird als prozentuale Hemmung nach folgender Formel berechnet:

$$\% \text{ Hemmung} = 100 - [(\text{Fluoreszenzzunahme/Minute in Reaktion 2}) / (\text{Fluoreszenzzunahme/Minute in Reaktion 1}) \times 100].$$

Der $IC_{50}$, die für eine 50%ige Hemmung der Enzymaktivität erforderliche Inhibitorkonzentration wird grafisch durch Auftragen der prozentualen Hemmungen bei verschiedenen Inhibitorkonzentrationen ermittelt.

Die Cystein-Lösung enthält 0,3 mol/L Cystein in Wasser. Der Acetat/Hepes-Puffer enthält 0,15 mol/L Natriumacetat, 0,15 mol/L Hepes, 0,3 mol/L Natriumchlorid sowie 0,001% Pluronic (Sigma, Deisenhofen, Deutschland) pH 6,5.

Die Enzymlösung enthält 0,22 µg/mL der Enzymdomäne.

Die Substratlösung enthält 0,3 mmol/L des fluorogenen Substrates Boc-Ala-Gly-Pro-Arg-AMC (Bachem, Heidelberg, Deutschland).

Bestimmung der enzymatischen Aktivität der katalytischen Domäne des humanen Cathepsin-S.

Dieses Protein wird als inaktives Enzym von der Fa. R&D Systems, Wiesbaden, Deutschland, erhalten (Katalog Nr. 1183-CY). Das Enzym wird wie folgt aktiviert:

5 Volumenanteile Enzym werden mit 20 Volumenanteilen Acetat-Puffer und 50 Volumenanteilen Cystein-Lösung bei 37 °C für 5 Minuten inkubiert. Nach der Aktivierung des Enzyms wird dieses mit dem Tris/HCl Puffer auf eine Konzentration von 0,65 µg/mL verdünnt. Zur Messung der Enzymaktivität werden 10 µL Enzymlösung mit 10 µL einer 3%igen (v/v) wässrigen Dimethylsulfoxid-Lösung (Reaktion 1) für 10 Minuten inkubiert. Zur Messung der Enzyminhibitoraktivität werden 10 µL Enzymlösung mit 10 µL einer 3%igen (v/v) wässrigen Dimethylsulfoxid-Lösung, die den Enzyminhibitor enthält, inkubiert (Reaktion 2).

Sowohl bei Reaktion 1 als auch bei Reaktion 2 wird nach Zugabe von 10 µL einer 1,5 %igen (v/v) wässrigen Dimethylsulfoxid-Lösung, die 0,15 mmol/L des Substrates enthält, die Enzymreaktion fluoreszenzspektroskopisch verfolgt (360nm (Anregung) /465nm (Emission)). Die Enzymaktivität wird dargestellt als

Fluoreszenzzunahme/Minute.

Die Inhibitorwirkung wird als prozentuale Hemmung nach folgender Formel berechnet:

$$\% \text{ Hemmung} = 100 - [(\text{Fluoreszenzzunahme/Minute in Reaktion 2}) / (\text{Fluoreszenzzunahme/Minute in Reaktion 1}) \times 100].$$

Der $IC_{50}$, dies ist die für eine 50%ige Hemmung der Enzymaktivität erforderliche Inhibitorkonzentration wird grafisch durch Auftragen der prozentualen Hemmungen bei verschiedenen Inhibitorkonzentrationen ermittelt.

Der Acetat-Puffer enthält 0,05 mol/L Natriumacetat, 0,1 mol/L Natriumchlorid sowie 0,001 % Pluronic (Sigma, Deisenhofen, Deutschland) pH 5,5.

Die Cystein-Lösung enthält 0,3 mol/L Cystein in Wasser. Der Tris/HCl-Puffer enthält 0,1 mol/L Tris/HCl, 0,04 mol/L EDTA, sowie 0,001 % Pluronic pH=7,5.

Die Enzymlösung enthält 0,65 µg/mL der Enzymdomäne.

Die Substratlösung enthält 0,15 mmol/L des fluorogenen Substrates Z-Val-Val-Arg-AMC (Bachem, Heidelberg, Deutschland).

Entsprechende Ki-Werte werden durch Anwendung der Cheng-Prusoff-Gleichung erhalten:

$$K_i = K_{i,app}/(1+[S]/K_M)\ ; \quad \text{wobei } K_{i,app} = IC_{50}$$

($K_{i.\,app}$ ist die Konzentration der kompetitierenden Substanz, die zu einer 50 %igen Inhibition der enzymatischen Aktivität bei einer Substrat-Konzentration [S] führt)

In Tabelle 2 sind für einige repräsentative Beispiele entsprechende Inhibitionswerte in Form von Ki-Werten angegeben:

Tabelle 2:

| Beispiel | Ki [nM] Cathepsin K | Ki [nM] Cathepsin B | Ki [nM] Cathepsin S |
|---|---|---|---|
| **9** | 1,8 | 46 | 3,2 |
| **35** | 31 | >7100 | 150 |
| **36** | 3 | 44 | 2 |

(fortgesetzt)

| Beispiel | Ki [nM] Cathepsin K | Ki [nM] Cathepsin B | Ki [nM] Cathepsin S |
|---|---|---|---|
| **37** | 21 | 560 | 13 |
| **42** | 32 | 4500 | 11 |
| **45** | 21 | 1200 | 24 |
| **49** | 52 | 2200 | 16 |
| **53** | 4,5 | 1500 | 53 |
| **56** | 7,5 | 210 | 7,2 |
| **63** | 22 | 260 | 5 |
| **70** | 910 | 110 | 0,3 |
| **74** | 150 | >7100 | 16 |
| **76** | 47 | 930 | 22 |
| **77** | 20 | 220 | 7 |
| **78** | 38 | 530 | 19 |
| **79** | 16 | 620 | 9 |
| **80** | 36 | 3900 | 9 |
| **83** | 28 | 2300 | 8 |
| **88** | 15 | 310 | 9 |
| **91** | 43 | 140 | 2 |
| **94** | 24 | 84 | 2 |
| **97** | 400 | 100 | 1 |
| **102** | 33 | 62 | 0,5 |
| **103** | 84 | 99 | 27 |

Caco-2/TC7-Permeabilitätsbestimmungen zur Vorhersage der Absorption der erfindungsgemäßen Verbindungen: (Caco-2/TC7-Permeabilitätstest)

Es werden Caco-2/TC-7-Zellen der Firma Sanofi verwendet. Desweiteren werden HTS Multiwell-Platten (24-well, non-coated, Becton Dickinson, Oberfläche der Becton Dickinson Filter 0.3 $cm^2$) verwendet. Die Zelldichte auf den Filtern war 2 x $10^5$ /$cm^2$ und 1.3 x $10^4$ /$cm^2$ auf den T-75 flasks (Costar mit vent-cap) für das Zelllinien-Wachstum. Die Inkubationsbedingungen sind 37 ˚C, 95 % Luftfeuchtigkeit, 10 % $CO_2$. Das Medium wird dreimal pro Woche gewechselt (DMEM, Glutamax I, nicht-essentielle AA, Penicillin/Streptomycin, FBS 20 %).

Permeabilitäts-Assay-Konditionen: Asymmetrische Konditionen für das Screening mit apikalem Puffer HBSS (mit 10 mM HEPES und 0.5 % BSA bei pH 6.5) und basalem Puffer HBSS (mit 10 μM HEPES und 5% BSA bei pH 7.4). Die Permeabilitäts-Experimente werden 2 h lang bei 37 ˚C unter Agitation durchgeführt. Die Proben werden durch LC-MS analysiert. Die Ergebnisse werden als mittlerer Papp-Wert (single point, cm/sec) (Permeabilitätskoeffizient) angegeben:

$$P = \frac{B_2}{[A_0] \times S \times t}$$

B2 : basolaterale Menge der zu untersuchenden Verbindung nach 2 h
[$A_0$] : apikale Konzentration der Test-Lösung
S : "insert area" : 0.3 $cm^2$
t : Zeit : 2 h

## Patentansprüche

1. Verbindung der Formel Ia

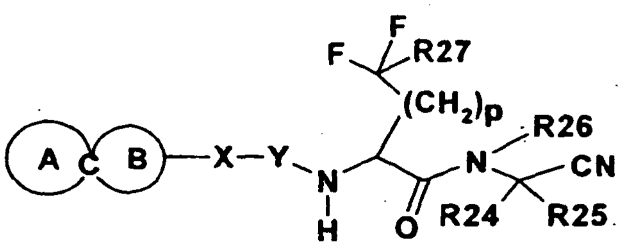

(Ia)

und/oder alle stereoisomeren Formen der Verbindung der Formel Ia und/oder Gemische dieser Formen in jedem Verhältnis; und/oder ein physiologisch verträgliches Salz der Verbindung der Formel Ia, und/oder Solvate oder Hydrate der Verbindung der Formel Ia, wobei.
der Rest

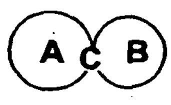

für eine Spiroverbindung steht,
worin die Teilringe

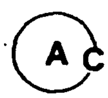

und

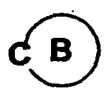

jeweils gleich oder verschieden sind und unabhängig voneinander für

a) ein gesättigtes oder teilweise gesättigtes -($C_3$-$C_{11}$)-Cycloalkyl stehen, worin Cycloalkyl unverbrückt, verbrückt oder annelliert ist und unsubstituiert oder unabhängig voneinander je nach Ringgröße ein-, zwei-, drei-, vier oder fünffach durch R4 substituiert ist, oder
b) einen gesättigten oder teilweise gesättigten drei- bis elfgliedrigen Heterocyclus stehen, die je nach Ringgröße ein, zwei, drei oder vier gleiche oder verschiedene Heteroatome aus der Reihe Sauerstoff, Stickstoff oder Schwefel enthalten können und worin der Heterocyclus unverbrückt, verbrückt oder annelliert ist und unsubstituiert oder unabhängig voneinander je nach Ringgröße ein-, zwei-, drei-, vier oder fünffach durch R4 substituiert ist, wobei R4 für -$NO_2$, -CN, =O, =S, -OH, -$CF_3$, -$SF_5$, -($C_0$-$C_3$)-Alkylen-S-R10, -O-$CF_3$, -Si-$(CH_3)_3$, -($C_0$-$C_5$)-Alkylen-O-C(O)-R21, -($C_0$-$C_5$)-Alkylen-C(O)-O-R10, -($C_0$-$C_3$)-Alkylen-O-R10, -($C_0$-$C_3$)-Alkylen-N (R21)-R22, -($C_0$-$C_3$)-Alkylen-N($R_{10}$)-S($O_2$)-R10, -($C_0$-$C_5$)-Alkylen-($C_3$-$C_8$)-Cycloalkyl-R23, -S-$CF_3$, -($C_0$-$C_5$)-Alkylen-($C_1$-$C_3$)-Fluoralkyl, -($C_0$-$C_5$)-Alkylen-N(R10)-C(O)-R21, -($C_0$-$C_3$)-Alkylen-C(O)-N(R21)-R22, -($C_0$-$C_4$)-Alkyl, wobei Alkyl unsubstituiert oder ein-, zwei- oder dreifach unabhängig voneinander durch R9 substituiert ist, -($C_0$-$C_4$)-Alkylen-Aryl, wobei Aryl ausgewählt ist aus der Gruppe Phenyl, Indanyl, Indenyl, Naphthyl, wobei Aryl unsubstituiert oder ein-, zwei- oder dreifach unabhängig voneinander durch R8 substituiert ist, oder -($C_0$-$C_4$)-Alkylen-Het, wobei Het ausgewählt ist aus der Gruppe Azetidinyl, Benzimidazolinyl, Benzimidazolyl, Benzofuranyl, Benzothiophenyl, Benzoxazolyl, Benzthiazolyl, Benzisoxazolyl, Benzisothiazolyl, Chinolinyl, Dioxolyl, Dioxanyl, Furanyl, Imidazolidinyl, Imidazolinyl, Imidazolyl, Indolinyl, Indolyl, 3H-Indolyl, Isoindolinyl, Isoindolyl, Isochinolinyl, Isothiazolidinyl, 2-Isothiazolinyl, Isothiazolyl, Isoxazolyl, Isoxazolidinyl, 2-Isoxazolinyl, Morpholinyl, Octahydroisochinolinyl, Oxazolyl, Oxazolidinyl, Pyrimidinyl, Piperazinyl, Piperidinyl, Pyranyl, Pyrazinyl, Pyrazolinyl, Pyrazolyl, Pyridazinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, Thiazolyl, Thienyl, Thienopyridinyl, Thiomorpholinyl, Thiophenyl, ist und dieser Het-Rest unsubstituiert oder unabhängig voneinander ein-, zwei- oder dreifach durch R8 substituiert ist, steht, R8 für Halogen, Carbamimidoyl, $-NO_2$, =O, $-CF_3$, $-SF_5$, -C(O)-O-R10, -CN, $-C(O)-NH_2$, -OH, $-NH_2$, $-O-CF_3$, -C(O)-N(R10)-R20, -N(R10)-R20, $-(C_3-C_8)$-Cycloalkyl, $-O-(C_1-C_8)$-Alkyl, $-O-(C_0-C_4)$-Alkylen-$(C_3-C_6)$-Cycloalkyl, $-(C_1-C_8)$-Alkyl, $-(C_0-C_4)$-Alkylen-$(C_3-C_8)$-Cycloalkyl, wobei die genannten Alkylreste jeweils unsubstituiert oder ein-, zwei- oder dreifach unabhängig voneinander durch Halogen, $NH_2$, -OH, $-O-CH_3$, $-SO_2-CH_3$ oder $-SO_2-CF_3$ substituiert sind, steht,

R9 für Halogen, $-NO_2$, -CN, =O, -OH, $-CF_3$, -C(O)-O-R10, -C(O)-N(R21)-R22, -N(R21)-R22, $-(C_3-C_8)$-Cycloalkyl, $-(C_0-C_3)$-Alkylen-O-R10, $-Si-(CH_3)_3$, $-N(R10)-S(O)_u-R10$, wobei u die ganze Zahl 1 oder 2 bedeutet, -S-R10, $-SO_r-R10$, wobei r die ganze Zahl 1 oder 2 bedeutet, $-S(O)_v-N(R10)-R20$, wobei v die ganze Zahl 1 oder 2 bedeutet, -C(O)-R10, $-(C_1-C_8)$-Alkyl, $-(C_1-C_8)$-Alkoxy, Phenyl, Phenyloxy-, $-(C_1-C_3)$-Fluoralkyl, -O-R19, -NH-C (O)-NH-R10, $-(C_0-C_4)$-Alkyl-C(O)-O-C(R11, R19)-O-C(O)-R12, -NH-C(O)-NH-R21, -N(R21)-C(O)-R22, $-(C_0-C_4)$-Alkyl-C(O)-O-C(R11, R19)-O-C(O)-O-R12, -NH-C(O)-O-R10, $-O-CF_3$ oder Het, wobei Het wie oben definiert ist und unsubstituiert oder ein-, zwei- oder dreifach unabhängig voneinander durch R8 substituiert ist, steht,

R10 und R20 sind gleich oder verschieden und stehen unabhängig voneinander für Wasserstoffatom, $-(C_1-C_6)$-Alkyl, $-(C_0-C_4)$-Alkyl-OH, $-(C_1-C_3)$-Fluoralkyl, $-(C_0-C_4)$-Alkyl-O-$(C_1-C_4)$-Alkyl, $-(C_0-C_5)$-Alkyl-$(C_3-C_8)$-Cycloalkyl, $-(C_0-C_2)$-Alkylen-Aryl, wobei Aryl wie oben definiert ist und unsubstituiert oder ein-, zwei- oder dreifach unabhängig voneinander durch $-(C_1-C_6)$-Alkyl, $-O-(C_1-C_6)$-Alkyl, Halogen oder $-(C_3-C_8)$-Cycloalkyl substituiert ist, oder $-(C_0-C_2)$-Alkylen-Het, wobei Het wie oben definiert ist und unsubstituiert oder ein-, zwei- oder dreifach unabhängig voneinander durch $-(C_1-C_6)$-Alkyl, $-O-(C_1-C_6)$-Alkyl, Halogen oder $-(C_3-C_8)$-Cycloalkyl substituiert ist,

R11 und R19 gleich oder verschieden sind und unabhängig voneinander für Wasserstoffatom oder $-(C_1-C_6)$-Alkyl stehen,

R12 für $-(C_1-C_6)$-Alkyl, $-(C_1-C_6)$-Alkyl-OH, $-(C_1-C_6)$-Alkyl-O-$(C_1-C_6)$-Alkyl, $-(C_3-C_8)$-Cycloalkyl, $-(C_1-C_6)$-Alkyl-O-$(C_1-C_8)$-Alkyl-$(C_3-C_8)$-Cycloalkyl, $-(C_1-C_6)$-Alkyl-$(C_3-C_8)$-Cycloalkyl, wobei der Cycloalkylrest unsubstituiert oder ein-, zwei- oder dreifach unabhängig voneinander durch -OH, $-O-(C_1-C_4)$-Alkyl oder R10 substituiert ist, steht,

R21 und R22 sind gleich oder verschieden und stehen unabhängig voneinander für Wasserstoffatom, $-(C_1-C_6)$-Alkyl, wobei Alkyl unsubstituiert oder ein-, zwei- oder dreifach unabhängig voneinander durch R8 substituiert ist, $-(C_0-C_6)$Alkylen-$C_3-C_8$)-Cycloalkyl, $-SO_t-R10$, wobei t die ganze Zahl 1 oder 2 bedeutet, $-(C_1-C_3)$-Fluoralkyl, -O-R12, $-(C_0-C_6)$-Alkylen-Aryl, wobei Aryl wie oben definiert ist und Alkylen und Aryl unsubstituiert oder ein-, zwei- oder dreifach unabhängig voneinander durch R8 substituiert sind oder $-(C_0-C_6)$-Alkylen-Het, wobei Het wie oben definiert ist und Alkylen und Het unsubstituiert oder ein-, zwei- oder dreifach unabhängig voneinander durch R8 substituiert sind,

R21 und R22 zusammen mit dem Stickstoffatom an das sie gebunden sind einen vier- bis achtgliedrigen monocyclischen heterocyclischen Ring bilden, der neben dem Stickstoffatom zusätzlich noch je nach Ringgröße ein oder zwei gleiche oder verschiedene Heteroatome aus der Reihe Sauerstoff, Stickstoff oder Schwefel enthalten kann und worin der Heterocyclus unsubstituiert oder ein-, zwei- oder dreifach unabhängig voneinander durch R8 substituiert ist,

R23 für Wasserstoffatom, -OH oder $-O-(C_1-C_4)$-Alkyl steht,

X für eine kovalente Bindung, -N(R7)- oder -O- steht, wobei R7 für Wasserstoffatom, $-(C_0-C_4)$-Alkylen-$(C_3-C_6)$-Cycloalkyl oder $-(C_1-C_4)$-Alkyl steht,

Y für -C(O)-, -C(S)- oder $-S(O_2)$- steht,

p für die ganze Zahl 1 oder 2 steht,

R27 für Wasserstoffatom, $-(C_1-C_6)$-Alkyl, Halogen, $-(C_0-C_4)$-Alkylen-$(C_3-C_6)$-Cycloalkyl, $-(C_0-C_4)$-Alkylen-Het, wobei Het wie oben definiert ist und unsubstituiert oder durch Halogen, $-(C_1-C_6)$-Alkyl, $-O-(C_1-C_3)$-Fluoralkyl oder $-O-(C_1-C_6)$-Alkyl substitutiert ist, oder $-(C_2-C_2)$-Alkylen-Phenyl steht, wobei Phenyl unsubstituiert oder durch Halogen, $-(C_1-C_6)$-Alkyl, $-O-(C_1-C_3)$-Fluoralkyl oder $-O-(C_1-C_6)$-Alkyl substitutiert ist,

R26 für Wasserstoffatom, $-(C_1-C_4)$-Alkyl oder $-(C_0-C_4)$-Alkylen-$(C_3-C_6)$-Cycloalkyl steht,

R24 und R25 sind gleich oder verschieden und stehen unabhängig voneinander für Wasserstoffatom, $-(C_1-C_6)$-Alkyl, $-(C_1-C_3)$-Fluoralkyl, $-(C_0-C_4)$-Alkylen-$(C_3-C_6)$-Cycloalkyl, $-(C_0-C_4)$-Alkylen-Aryl, wobei Aryl wie oben definiert ist und unsubstituiert oder ein-, zwei- oder dreifach unabhängig voneinander durch R8 substituiert ist, oder $-(C_0-C_4)$-Alkylen-Het, wobei Het wie oben definiert ist und unsubstituiert oder ein-, zwei- oder dreifach unabhängig voneinander durch R8 substituiert ist,

R24 und R25 bilden zusammen mit dem Kohlenstoffatom an das sie gebunden sind einen drei- bis sechsgliedrigen Cycloalkylring, der unsubstituiert oder ein-, zwei- oder dreifach unabhängig voneinander durch R10 oder Fluor substituiert ist,
R24 und R25 bilden zusammen mit dem Kohlenstoffatom an das sie gebunden sind einen drei- bis sechsgliedrigen Hetero-Cycloalkylrest, der unsubstituiert oder ein-, zwei- oder dreifach unabhängig voneinander durch R10 oder Fluor substituiert ist.

**2.** Verbindung der Formel I gemäß Anspruch 1, wobei der Teilring

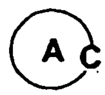

aus der folgenden Gruppe ausgewählt wurde

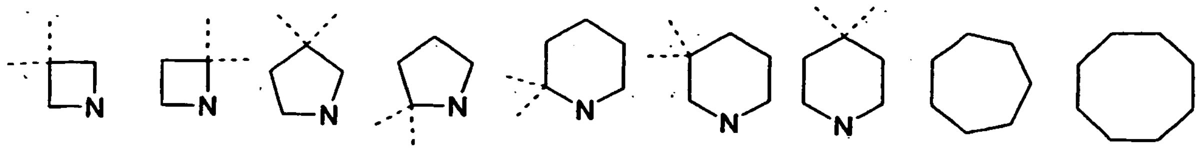

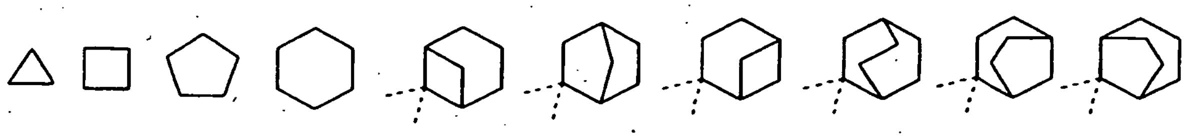

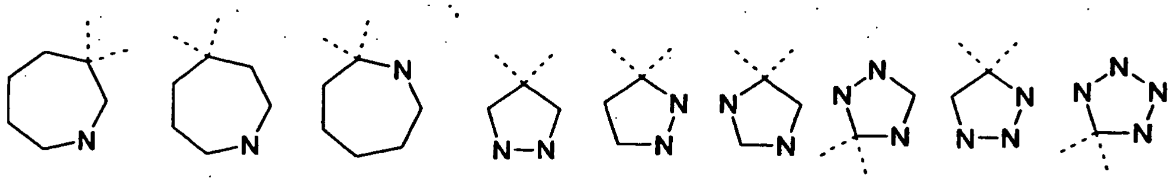

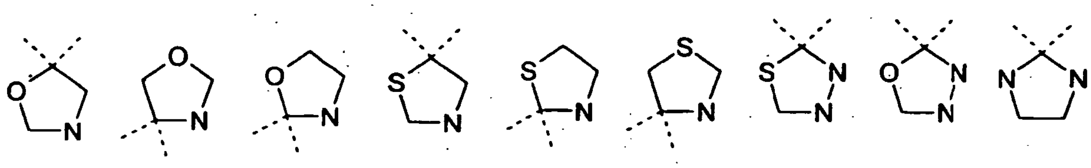

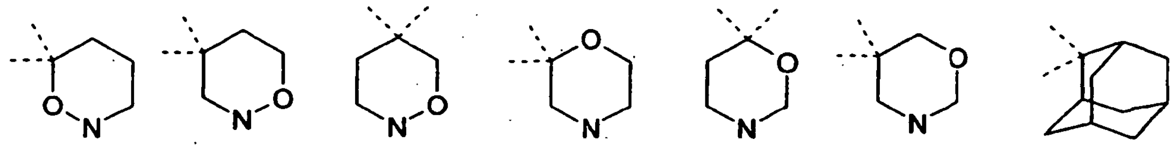

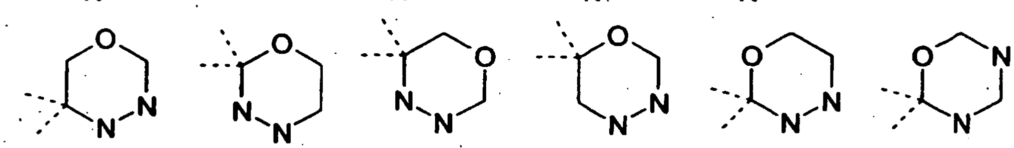

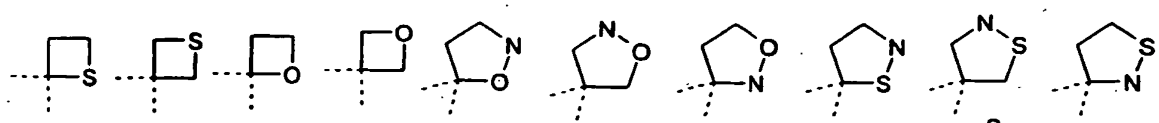

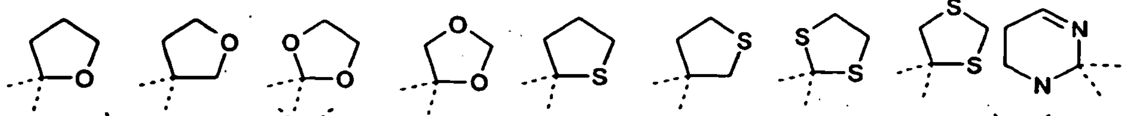

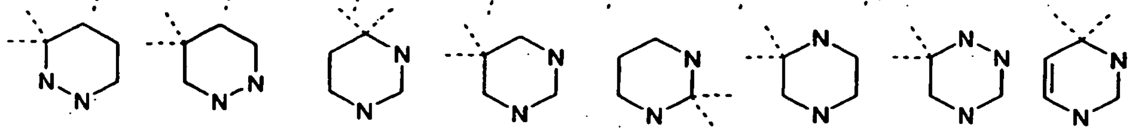

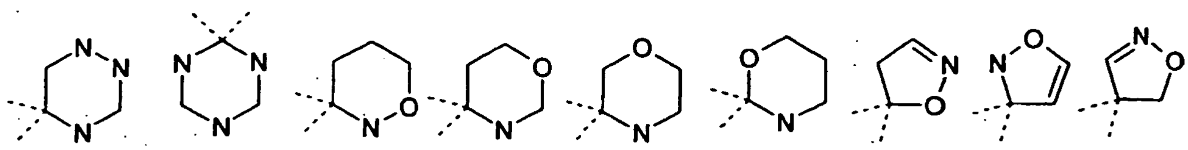

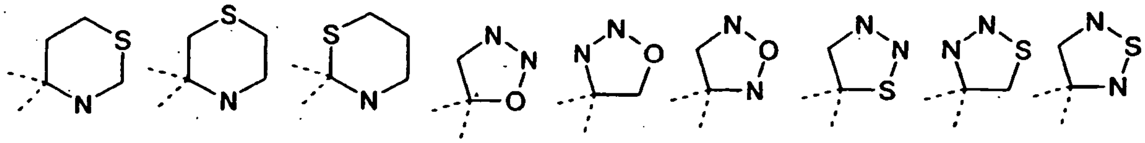

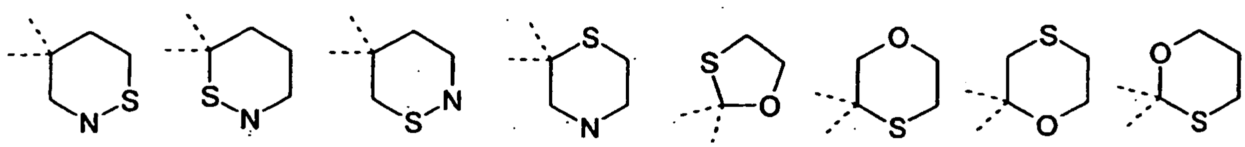

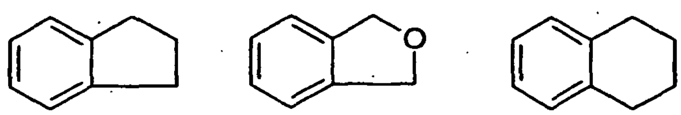

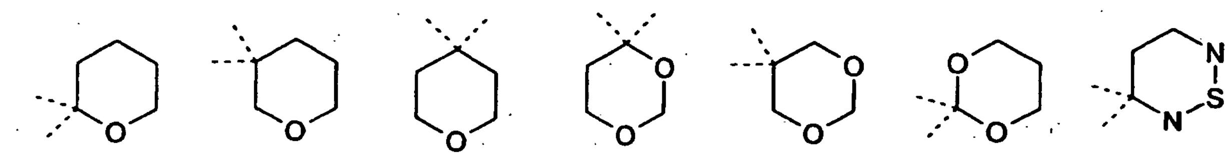

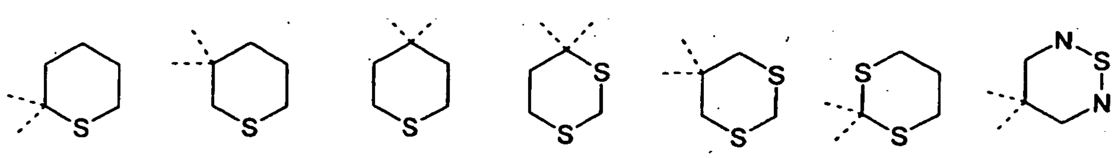

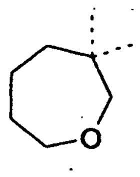 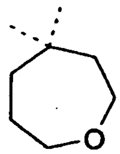 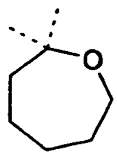 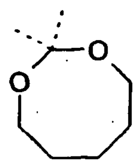 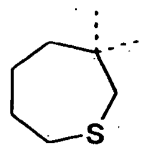 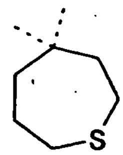 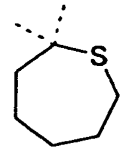 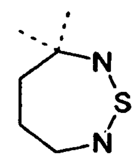

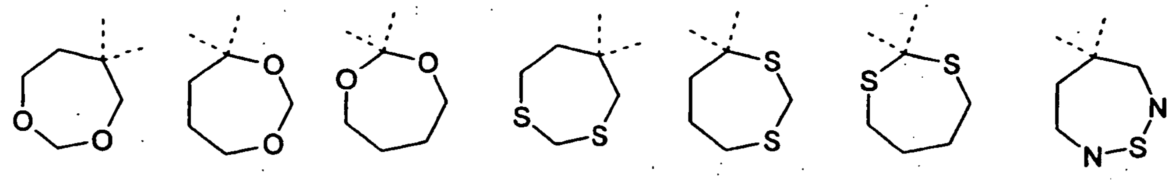

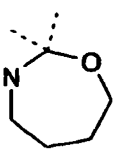 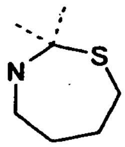 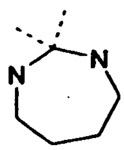 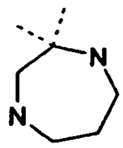 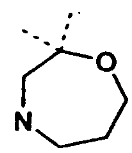 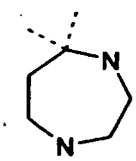 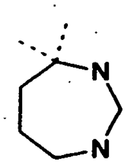

wobei die gestrichelten Linien den jeweiligen Anknüpfungspunkt zum zweiten Teilring darstellen, Einfachbindungen in den aufgeführten Strukturen zum Teil durch Doppelbindungen ersetzt sein können oder weitere Ringsysteme ankondensiert sein können, und worin der Teilring

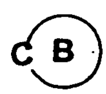

aus der folgenden Gruppe ausgewählt wurde

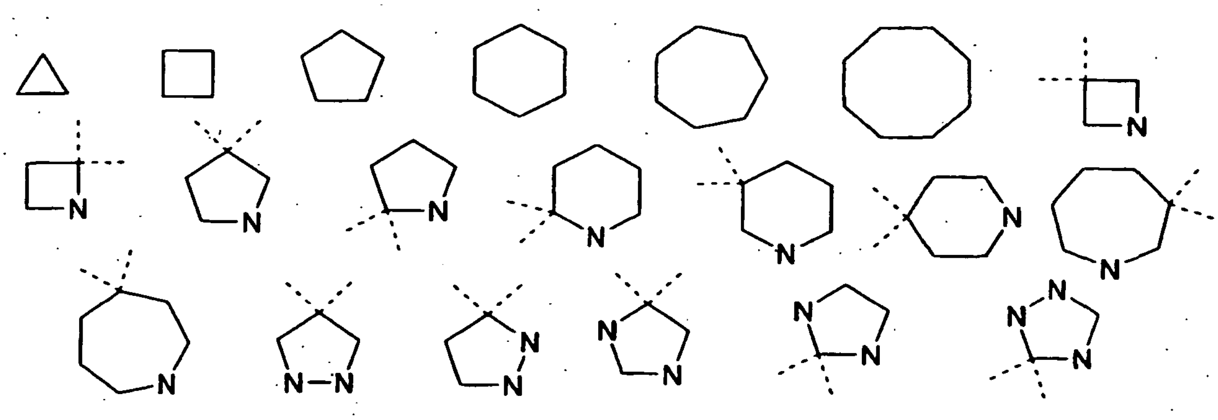

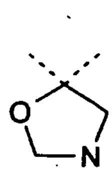 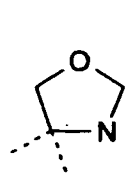 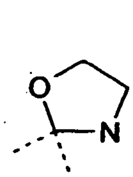 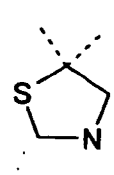 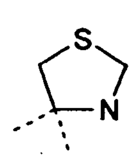 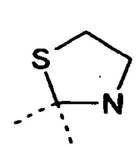

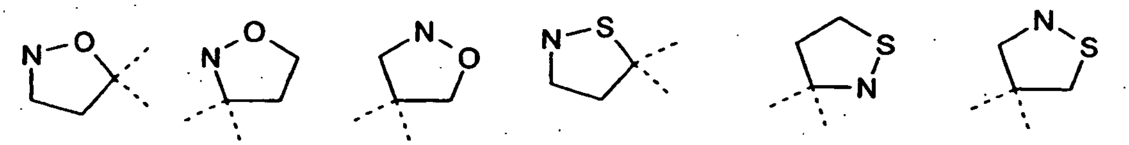

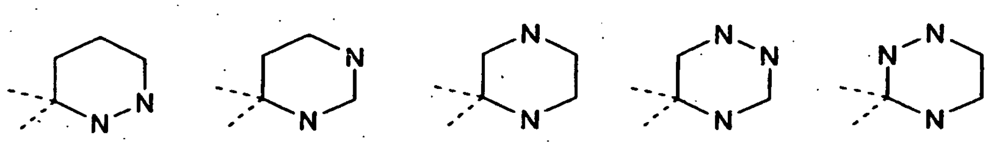

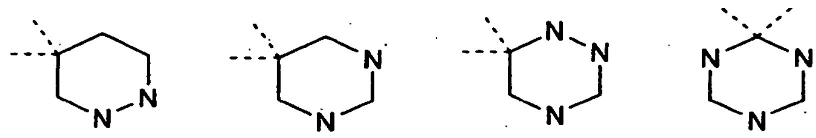

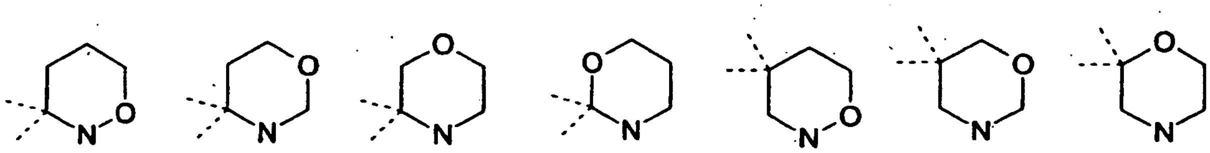

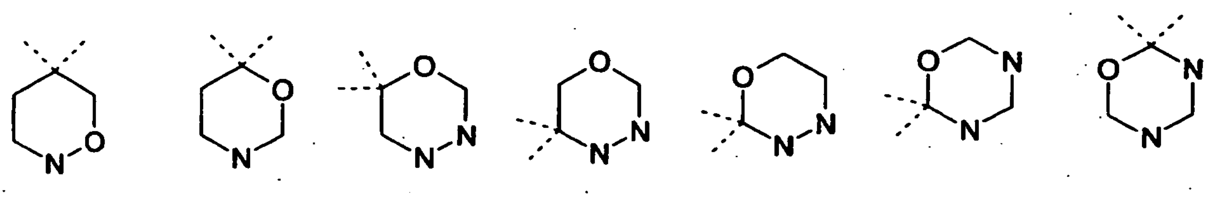

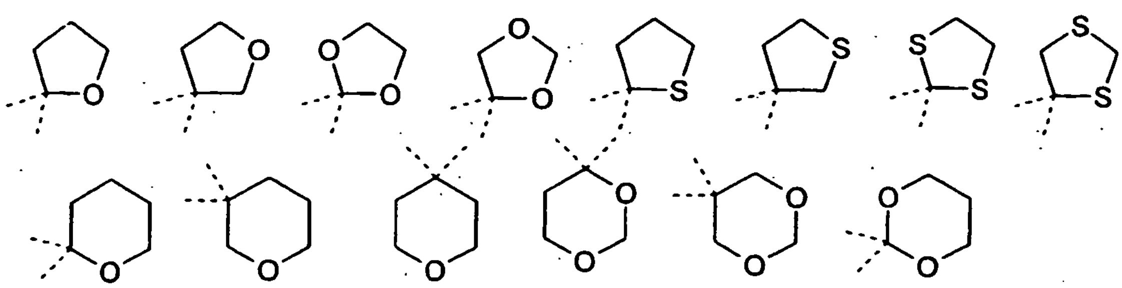

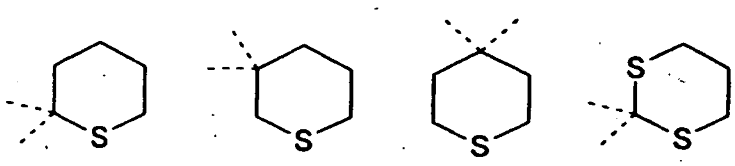

wobei die gestrichelten Linien den jeweiligen Anknüpfungspunkt zum zweiten Teilring darstellen, Einfachbindungen in den aufgeführten Strukturen zum Teil durch Doppelbindungen ersetzt sein können und die beiden Teilringe A

und B unsubstituiert oder unabhängig voneinander einfach bis vierfach durch R4 substituiert sind, und die Reste X, Y, R27, p, R26, R24, R25 und R4 wie in Anspruch 1 definiert sind.

**3.** Verbindung der Formel Ia gemäß Anspruch 1, wobei die Teilringe

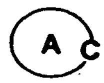

und

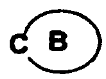

jeweils ausgewählt wurden aus Cyclopropan, Cyclobutan, Cyclopentan, Cyclohexan, Cycloheptan, Cyclooctan, Bicyclo[4.2.0]octan, Octahydro-inden, Decalin, Decahydro-benzocyclohepten, Dodecahydro-heptalen, Bicyclo[3.1.1]heptan, Bicycto[2.2.1]heptan, Bicyclo[3.3.0]octan, Bicyclo[2.2.2]octan, Spiro[2.5]octan, Spiro[3.4]octan, Azepan, Azepin, Azetidin, Aziridin, Azirin, Azocan, Benzimidazolin, 2,3-Dihydro-benzo[b]thiophen, 1,3-Dihydro-benzo[c]thiophen, 2,3-Dihydro-benzofuran, 2,3-Dihydro-benzooxazol, 2,3-Dihydro-benzothiazol, 1,3-Dihydro-isobenzofuran, 4,5-Dihydro-isothiazol, 2,3-Dihydro-isoxazol, 2,5-Dihydro-isoxazol, 4,5-Dihydro-isoxazol, 5,6-Dihydro-4H-[1,2]oxazin, Benzo[1,3]dioxol, 1,4-Diazepan, 1,2-Diazepin, 1,3-Diazepin, 1,4-Diazepin, Diaziridin, Diazirin, 1,4-Diazocan, Dioxan, 1,3-Dioxan, Dioxazin, [1,3]Dioxepan, 1,4-Diozocan, Dioxol, Dioxolan, 1,3-Dioxolan, 1,3-Dioxolen, [1,3]Dithian, [1,3]Dithiolan, Hexahydro-pyridazin, Hexahydro-pyrimidin, Imidazolin, Imidazolidin, Indan, Indolin, Isoindolin, Isothiazolidin, Isothiazolin, Isoxazolin, Isoxazolidin, 2-Isoxazolin, Morpholin, [1,3,4]Oxadiazinan, [1,3,5]Oxadiazinan, [1,2,3]Oxadiazolidin, [1,3,4]Oxadiazolidin, 1,2-Oxa-thiepan, 1,2-Oxathiolan, [1,3]Oxathiolan, 1,4-Oxazepan, 1,2-Oxazin, 1,3-Oxazin, 1,4-Oxazin, Oxazinan, 1,3-Oxazinan, Oxazocane, Oxaziridin, Oxazolidin, Oxepan, Oxetan, Oxiran, Oxocane, Piperazin, Piperidin, Pyran, Pyrazolin, Pyrazolidin, Pyrrolidin, Pyrrolidinon, Pyrrolin, Tetrahydrochinolin, Tetrahydrofuran, Tetrahydroisochinolin, 1,2,3,4-Tetrahydro-naphthalen, Tetrahydropyran, Tetrahydropyridin, 1,2,3,4-Tetrahydro-pyrimidin, 1,2,5,6-Tetrahydro-pyrimidin, Tetrahydro-thiophen, Tetrazin, Thiadiazin, [1,2,6]Thiadiazinan, [1,3,4]Thiadiazolidin, 1,2-Thiazin, 1,3-Thiazin, 1,4-Thiazin, [1,2]Thiazinan, [1,3]Thiazinan, Thiazolidin, Thiazolin, Thiepan, Thietan, Thiomorpholin, Thiopyran, 1,2,3-Triazin, 1,2,4-Triazin, 1,3,5-Triazin, [1,2,4]Triazinan oder [1,2,4]Triazolidin, und worin die beiden Teilringe jeweils unsubstituiert oder unabhängig voneinander je nach Ringgröße ein-, zwei-, drei-, vier oder fünffach durch R4 substituiert sind, und die Reste X, Y, R27, p, R26, R24, R25 und R4 wie in Anspruch 1 definiert sind.

**4.** Verbindung der Formel Ia gemäß Anspruch 1, wobei die Teilring

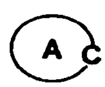

und

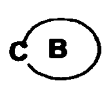

unabhängig voneinander jeweils ausgewählt werden aus der Gruppe Azetidin, Cyclopropyl, Cyclobutyl, Cyclopentyl, Cyclohexyl, 1,3-Dihydro-isobenzofuran, 2,3-Dihydro-isoxazol, 2,5-Dihydro-isoxazol, 4,5-Dihydro-isoxazol, 1,3-Dioxan, Dioxolan, 1,3-Dioxolan, Imidazolidin, Indan, Morpholin, 1,3-Oxazinan, Oxazolidin, Piperazin, Piperidin, Pyrrolidin, Tetrahydrofuran, und 1,2,3,4-Tetrahydro-naphthalen,
und worin die beiden Teilringe unsubstituiert oder unabhängig voneinander je nach Ringgröße ein-, zwei- oder dreifach durch R4 substituiert sind,
R4 für =O, =S, -($C_0$-$C_3$)-Alkylen-C(O)-O-R10, -($C_0$-$C_3$)-Alkylen-N(R21)-R22, -($C_0$-$C_3$)-Alkylen-NH-C(O)-R21, -($C_0$-$C_4$)-Alkylen-($C_3$-$C_6$)-Cycloalkyl-R23, -($C_0$-$C_3$)-Alkylen-O-R10, -($C_0$-$C_4$)-Alkylen-Phenyl, wobei Phenyl unsubstituiert oder ein-, zwei-oder dreifach unabhängig voneinander durch R8 substituiert ist, oder -($C_0$-$C_4$)-Alkyl, wobei Alkyl unsubstituiert oder ein-, zwei- oder dreifach unabhängig voneinander durch R9 substituiert ist, steht,

R8 für Fluor, Chlor, Brom, -O-($C_1$-$C_3$)-Fluoralkyl oder -O-($C_1$-$C_4$)-Alkyl steht,
R9 für Halogen, -$NO_2$, -CN, =O, -OH, -$CF_3$, -C(O)-O-R10, -C(O)-N(R21)-R22, -N(R21)-R22, -($C_3$-$C_8$)-Cycloalkyl, -($C_0$-$C_3$)-Alkylen-O-R10, -Si-$(CH_3)_3$, -N(R10)-$S(O)_u$-$R_{10}$, wobei u die ganze Zahl 1 oder 2 bedeutet, -S-R10, -$SO_r$-R10, wobei r die ganze Zahl 1 oder 2 bedeutet, -$S(O)_v$-N(R10)-R20, wobei v die ganze Zahl 1 oder 2 bedeutet, -C(O)-R10, -($C_1$-$C_8$)-Alkyl, -($C_1$-$C_8$)-Alkoxy,
Phenyl, Phenyloxy-, -($C_1$-$C_3$)-Fluoralkyl, -O-R19, -NH-C(O)-NH-R10, -($C_0$-$C_4$)-Alkyl-C(O)-O-C(R11,R19)-O-C(O)-R12, -NH-C(O)-NH-R21, -N(R21)-C(O)-R22, -($C_0$-$C_4$)-Alkyl-C(O)-O-C(R11, R19)-O-C(O)-O-R12, -NH-C(O)-O-R10 oder -O-$CF_3$, steht,
R10 und R20 gleich oder verschieden sich und unabhängig voneinander für Wasserstoffatom oder-($C_1$-$C_6$)-Alkyl stehen,
R11 und R19 sind gleich oder verschieden und stehen unabhängig voneinander für Wasserstoffatom, oder -(C $_1$-$C_6$)-Alkyl,
R12 für -(C $_1$-$C_6$)-Alkyl, -($C_1$-$C_6$)-Alkyl-OH, -($C_1$-$C_6$)-Alkyl-O-($C_1$-$C_6$)-Alkyl, -($C_3$-$C_8$)-Cycloalkyl, -($C_1$-$C_6$)-Alkyl-O-($C_1$-$C_8$)-Alkyl-($C_3$-$C_8$)-Cycloalkyl, -($C_1$-$C_6$)-Alkyl-($C_3$-$C_8$)-Cycloalkyl, wobei der Cycloalkylrest unsubstituiert oder ein-, zwei- oder dreifach unabhängig voneinander durch-OH, -O-($C_1$-$C_4$)-Alkyl oder R10 substituiert ist, steht,
R21 und R22 sind gleich oder verschieden und stehen unabhängig voneinander für Wasserstoffatom, -($C_1$-$C_6$)-Alkyl, -O-R12, -($C_0$-$C_6$)-Alkylen-($C_3$-$C_8$)-Cycloalkyl, -$SO_t$-R10, wobei t die ganze Zahl 1 oder 2 bedeutet, oder -($C_1$-$C_3$)-Fluoralkyl,
R23 für Wasserstoffatom, -OH oder-O-($C_1$-$C_4$)-Alkyl steht,
X für eine kovalente Bindung oder -N(R7)- steht, wobei
R7 für Wasserstoffatom oder -($C_1$-$C_4$)-Alkyl steht,
Y für -C(O)-oder-S($O_2$)-steht,
p für die ganze Zahl 1 oder 2 steht,
R26 für Wasserstoffatom steht,
R27 für Wasserstoffatom, ($C_0$-$C_4$)-Alkylen-($C_3$-$C_6$)-Cycloalkyl, -($C_1$-$C_6$)-Alkyl, -($C_0$-$C_2$)-Alkylen-Phenyl, wobei Phenyl unsubstituiert oder durch Halogen, -($C_1$-$C_6$)-Alkyl, -O-($C_1$-$C_3$)-Fluoralkyl oder-O-($C_1$-$C_6$)-Alkyl substitutiert ist, oder -($C_0$-$C_2$)-Alkylen-Pyridyl steht,
R24 und R25 sind gleich oder verschieden und stehen unabhängig voneinander für Wasserstoffatom, -($C_1$-$C_4$)-Alkyl oder -($C_0$-$C_4$)-Alkylen-($C_3$-$C_6$)-Cycloalkyl,
R24 und R25 bilden zusammen mit dem Kohlenstoffatom an das sie gebunden sind einen Cycloalkylring ausgewählt aus der Gruppe Cyclopropyl, Cyclobutyl, Cyclopentyl oder Cyclohexyl, der unsubstituiert oder ein-, zwei- oder dreifach unabhangig voneinander durch R10 oder Fluor substituiert ist,
R24 und R25 bilden zusammen mit dem Kohlenstoffatom an das sie gebunden sind einen drei- bis sechsgliedrigen Hetero-Cycloalkylrest, ausgewählt aus der Gruppe Aziridin, Azetidin, Diazetidin, Diaziridin, Hexohydropyridazin, Hexohydropyrimidin, Imidazolidin, Morpholin, Oxadiazinan, Oxadiazolidin, Oxathianan, Oxathiolan, Oxazetidin, Oxazolidin, Oxetan, Oxirane, Piperazin, Piperidin, Pyrazolidin, Pyrrolidin, Tetrahydrofuran, Tetrahydropyran, Tetrahydrothiophen, Tetrahydrothiopyran, Tetrazinan, Thiadiazolidin, Thiazetidin, Thiaziridin, Thiazolidine, Thietan, Thiiran, Thiomorpholin, Triazetidin, Triazinan oder Triazolidin, der unsubstituiert oder ein-, zwei- oder dreifach unabhängig voneinander durch R10 oder Fluor substituiert ist.

**5.** Verbindung der Formel la gemäß einem oder mehreren der Ansprüche 1 bis 4, wobei der Teilring

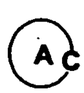

ausgewählt wird aus der Gruppe Azetidin, Cyclopropyl, Cyclobutyl, Cyclopentyl, Cyclohexyl, 1,3-Dihydro-isobenzofuran, 1,3-Dioxan, 1,3-Dioxolan, Imidazolidin, Indan, Morpholin, 1,3-Oxazinan, Piperazin, Piperidin, Pyrrolidin, Tetrahydrofuran, und 1,2,3,4-Tetrahydro-naphthaten, der Teilring

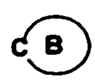

ausgewählt wird aus der Gruppe Azetidin, Cyclopropyl, Cyclopentyl, Cyclohexyl, Morpholin, Oxazolidin, Piperidin und Pyrrolidin, und worin die beiden Teilringe unsubstituiert oder unabhängig voneinander je nach Ringgröße ein-, zwei-, oder dreifach durch R4 substituiert sind, wobei R4 für -O-($C_1$-$C_4$)-Alkyl, =O, -($C_0$-$C_4$)-Alkylen-($C_3$-$C_6$)-Cycloalkyl, -($C_1$-$C_4$)-Alkyl oder -($C_0$-$C_4$)-Alkylen-Phenyl, wobei Phenyl unsubstituiert oder durch F; Cl. Br oder -O-($C_1$-$C_4$)-Al-

kyl substituiert ist, steht,
X für eine kovalente Bindung oder -NH- steht,
Y für -C(O)- oder -S($O_2$)- steht,
p für die ganze Zahl 1 steht,
R27 für Wasserstoffatom, -($C_1$-$C_6$)-Alkyl, 4-F-Benzyl oder Benzyl steht, R26 für Wasserstoffatom steht,
R24 und R25 sind gleich oder verschieden und stehen unabhängig voneinander für Wasserstoffatom, Methyl oder Ethyl,
R24 und R25 bilden zusammen mit dem Kohlenstoffatom, an das sie gebunden sind, einen Cyclopropyl oder Cyclobutyl-Rest, oder
R24 und R25 bilden zusammen mit dem Kohlenstoffatom an das sie gebunden sind einen Piperidin-Ring, der unsubstituiert oder durch -($C_1$-$C_4$)-Alkyl substitutiert ist.

**6.** Verbindung der Formel Ia gemäß einem oder mehreren der Ansprüche 1 bis 5, **dadurch gekennzeichnet, dass** es die Verbindung 3-Azaspiro[5.5]undecan-3-carbonsäure [(S)-1-(1-cyanocyclopropylcarbamoyl)-3,3-difluorbutyl]-amid, 8-Azaspiro[4.5]decan-8-carbonsäure [(S)-1-(1-cyanocyclopropylcarbamoyl)-3,3-difluor-butyl]-amid, 1,4-Dioxa-8-azaspiro[4.5]decan-8-carbonsäure [(S)-1-(1-cyanocyclopropylcarbamoyl)-3,3-difluorbutyl]-amid, 2-Azaspiro[5.5]undecan-2-carbonsäure [(S)-1-(1-cyanocyclopropylcarbamoyl)-3,3-difluor-butyl]-amid, 8-Azaspiro[4.5]decan-8-carbonsäure [(S)-1-(1-cyanocyclopropylcarbamoyl)-3,3-difluorohexyl]-amid, 3-Azaspiro[5.5]undecan-3-carbonsäure [(S)-1-1-cyanocyclopropylcarbamoyl)-3,3-difluorhexyl]-amid, 2-(4-Methoxyphenyl)-1-oxo-2,8-diazaspiro[4.5]decan-8-carbonsäure [(S)-1-(1-cyano-cyclopropylcarbamoyl)-3,3-difluorbutyl]-amid, 4-Oxo-1-phenyl-1,3,8-triazaspiro[4.5]decan-8-carbonsäure [(S)-1-(1-cyano-cyclopropylcarbamoyl)-3,3-difluorbutyl]-amid, 1,5-Dioxa-9-azaspiro[5.5]undecan-9-carbonsäure [(S)-1-(1-cyano-cyclopropylcarbamoyl)-3,3-difluorbutyl]-amid, 1-Oxo-2,8-diazaspiro[4.5]decan-8-carbonsäure [(S)-1-(1-cyanocyclopropy-Icarbamoyl)-3,3-difluorbutyl]-amid, 2-Methyl-1-oxo-2,8-diazaspiro[4.5]decan-8-carbonsäure [(S)-1-(1-cyano-cyclopropylcarbamoyl)-3,3-difluorbutyl]-amid, 3,3-Dimethyl-1-oxa-5,9-diazaspiro[5.5]undecan-9-carbonsäure [(S)-1-(1-cyano-cyclopropylcarbamoyl)-3,3-difluorbutyl]-amid, 8-Azaspiro[4.5]decan-8-carbonsäure [(S)-1-(1-cyanocyclopropylcarbamoyl)-3,3-difluor-4-phenylbutyl]-amid, 2,4-Dioxo-1,3,8-triazaspiro[4.5]decan-8-carbonsäure [(S)-1 -(l -cyano-cyclopropylcarbamoyl)-3,3-difluorbutyl]-amid, 2-Azaspiro[4.4]nonan-2-carbonsäure[(S)-1-(1-cyanocyclopropylcarbamoyl)-3,3-difluorbutyl]-amid, 2-Benzyl- 1- oxo- 2,8- diazaspiro [4.5] decan- 8- carbonsäure[(S)- 1-(1- cyano- cyclopropylcarbamoyl)- 3,3- difluorbutyl]-amid, 2-(4-Fluorphenyl)-1-oxo-2,8-diazaspiro[4.5]decan-8-carbonsäure[(S)-1-(1-cyano-cyclopropylcarbamoyl)-3,3-difluorbutyl]-amid, 3-Phenyl-1,5-dioxa-9-azaspiro[5.5]undecan-9-carbonsäure [(S)-1-(1-cyano-cyclopropylcarbamoyl)-3,3-difluorbutyl]-amid, 9-Butyl-3,9-diazaspiro[5.5]undecan-3-carbonsäure[(S)-1-(1-cyano-cyclopropylcarbamoyl)-3,3-difluorbutyl]-amid, 9-Cyclopropyl-3,9-diazaspiro[5.5]undecan-3-carbonsäure-[(S)-1-(1-cyano-cyclopropylcarbamoyl)-3,3-difluorbutyl]-amid, Spiro[2.3]hexan-1-carbonsäure [(S)-1-(1-cyano-cyclopropylcarbamoyl)-3,3-difluor-butyl]-amid, 2,2-Dimethyl-1-oxa-8-aza-spiro[4.5]decan-8-carbonsäure [(S)-1-(1-cyano-cyclopropylcarbamoyl)-3,3-difluor-butyl]-amid, 2-Aza-spiro[4.5]decan-2-carbonsäure[(S)-1-(1-cyano-cyclopropylcarbamoyl)-3,3-difluor-butyl]-amid,

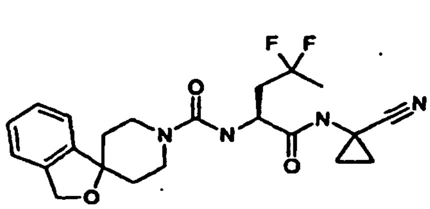

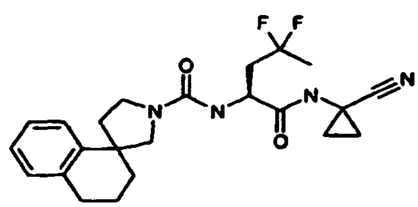

1-Oxa-4-aza-spiro[4.5]decan-4-carbonsäure [(S)-1-(1-cyano-cyclopropyl-carbamoyl)-3,3-difluor-butyl]-amid, (S)-2-[3,-(1,4-Dioxa-spiro[4.5]dec-8-yl)-ureido]-4,4-difluor-pentansäure (1-cyano-cyclopropyl)-amid,

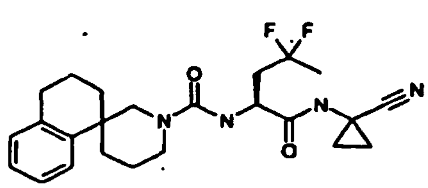

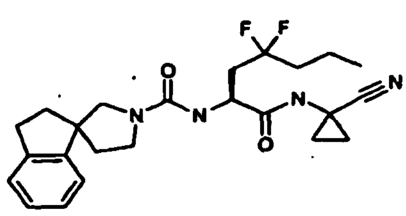

7-Cyclopropyl-2,7-diaza-spiro[3.5]nonan-2-carbonsäure [(S)-1-(1-cyano-cyclopropylcarbamoyl)-3,3-difluor-butyl]-amid, 2-Cyclopropyl-2,7-diaza-spiro[3.5]nonane-7-carbonsäure [(S)-1-(1-cyano-cyclopropylcarbamoyl)-3,3-difluor-butyl]-amid, 2-Propyl-2,7-diaza-spiro[3.5]nonan-7-carbonsäure [(S)-1-(1-cyano-cyclopropylcarbamoyl)-3,3-difluor-butyl]-amid, (S)-2-(8-Aza-spiro[4.5]decan-8-sulfonylamino)-4,4-difluor-pentansäure (1-cyano-cyclopropyl)-amid, 4-Cyclopropyl-1-oxa-4,9-diaza-spiro[5.5]undecan-9-carbonsäure [(S)-1-(1-cyano-cyclopropylcarbamoyl)-3,3-difluor-butyl]-amid, 9-Cyclopropyl-1-oxa-4,9-diaza-spiro[5.5]undecan-4-carbonsäure [(S)-1-(1-cyano-cyclopropylcarbamoyl)-3,3-difluor-butyl]-amid, 2-Cyclopropylmethyl-3-oxo-2,8-diaza-spiro[4.5]decan-8-carbonsäure[(S)-1-(1-cyano-cyclopropylcarbamoyl)-3,3-difluor-butyl]-amid, 2-(4-Methoxy-phenyl)-1-oxo-2,8-diaza-spiro[4.5]decan-8-carbonsäure [(S)-1-(1-cyano-cyclopropylcarbamoyl)-3,3-difluor-hexyl]-amid, 2-(4-Methoxy-phenyl)-2,8-diaza-spiro[4.5]decan-8-carbonsäure [(S)-1-(1-cyano-cyclopropylcarbamoyl)-3,3-difluor-hexyl]-amid, 2-Cyclopropyl-2,7-diaza-spiro[3.5]nonan-7-carbonsäure [(S)-1-(1-cyano-cyclo-propylcarbamoyl)-3,3-difluor-hexyl]-amid, 2-Cyclopropyl-2,8-diaza-spiro[4.5]decan-8-carbonsäure [(S)-1-(1-cyano-cyclopropylcarbamoyl)-3,3-difluor-hexyl]-amid, 9-Cyclopropyl-3,9-diaza-spiro[5.5]undecan-3-carbonsäure [(S)-1-(1-cyano-cyclo-propylcarbamoyl)-3,3-difluor-hexyl]-amid, 7-Cyclopropyl-2,7-diaza-spiro[3.5]nonan-2-carbonsäure [(S)-1-(1-cyano-cyclo-propylcarbamoyl)-3,3-difluor-hexyl]-amid, 7-Propyl-2,7-diaza-spiro[3.5]nonan-2-carbonsäure [(S)-1-(1-cyano-cyclopropylcarbamoyl)-3,3-difluor-hexyl]-amid, 9-Cyclopropyl-3,9-diaza-spiro[5.5]undecan-3-carbonsäure [(S)-1-(1-cyano-cyclo-propylcarbamoyl)-3,3-difluor-4-phenyl-butyl]-amid, 2-Cyclopropyl-2,7-diaza-spiro[3.5]nonan-7-carbonsäure [(S)-1-(1-cyano-cyclopropyl-carbamoyl)-3,3-difluor-4-phenyl-butyl]-amid, 2-Propyl-2,7-diaza-spiro[3.5]nonan-7-carbonsäure [(S)-1-(1-cyano-cyclopropyl-carbamoyl)-3,3-difluor-4-phenyl-butyl]-amid, 7-Cyclopropyl-2,7-diaza-spiro[3.5]nonan-2-carbonsäure [(S)-1-(1-cyano-cyclopropyl-carbamoyl)-3,3-difluor-4-phenyl-butyl]-amid, 6-Aza-spiro[2.5]octan-6-carbonsäure [(S)-1-(1-cyano-cyclopropylcarbamoyl)-3,3-difluor-butyl]-amid, 6-Aza-spiro[2.5]octan-6-carbonsäure [(S)-1-(cyanomethyl-carbamoyl)-3,3-difluor-butyl]-amid, 6-Aza-spiro[2.5]octan-6-carbonsäure [(S)-1-(1-cyano-cyclopropylcarbamoyl)-3,3-difluor-hexyl]-amid, 6-Aza-spiro[2.5]octan-6-carbonsäure [(S)-1-(4-cyano-1-methyl-piperidin-4-ylcarbamoyl)-3,3-difluor-butyl]-amid, 6-Aza-spiro[2.5]octan-6-carbonsäure [(S)-1-(4-cyano-1-methyl-piperidin-4-ylcarbamoyl)-3,3-difluor-hexyl]-amid, 6-Aza-spiro[2.5]octan-6-carbonsäure [(S)-1-(1-cyano-cyclopropylcarbamoyl)-3,3-difluor-pentyl]-amid oder 6-Aza-spiro[2.5]octan-6-carbonsäure [(S)-1-(1-cyano-cyclopropylcarbamoyl)-3,3-difluor-4-phenyl-butyl]-amid ist.

7. Verfahren zur Herstellung der Verbindung der Formel Ia und/oder einer stereoisomeren Form der Verbindung der Formel Ia und/oder eines physiologisch verträglichen Salzes der Verbindung der Formel Ia und/oder eines Solvats oder Hydrats der Verbindung der Formel Ia, und/oder eines N-Oxids der Verbindungen der Formel Ia gemäß einem oder mehreren der Ansprüche 1 bis 6, **dadurch gekennzeichnet, dass** man

a) eine Verbindung der Formel II,

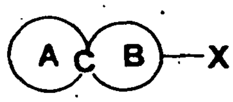

**(II)**

wobei A und B wie in der Verbindung der Formel Ia definiert ist, mit einer Verbindung der Formel IIIa oder IIIb oder IIIc,

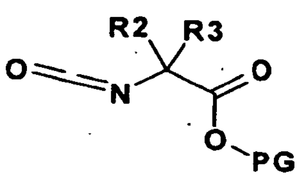

**(IIIa)**

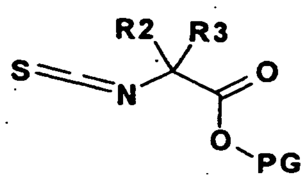

**(IIIb)**

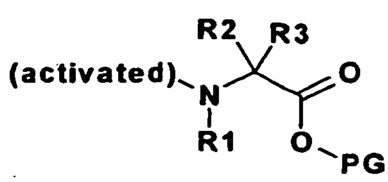

**(IIIc)**

wobei X, R1, R2 und R3 die Bedeutung wie in der Verbindung der Formel Ia haben, PG eine Ester-Schutzgruppe bedeutet, und "activated" bedeutet, dass das Amin in einer aktivierten Form vorliegt, beispielsweise als Chlorcarbonyl-Verbindung, zu einer Verbindung der Formel IVa oder IVb umsetzt,

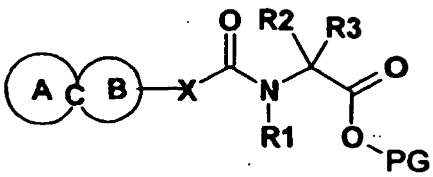
(IVa)

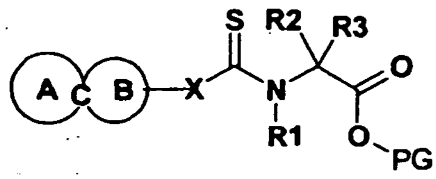
(IVb)

und die erhaltenen Verbindungen der Formel IVa oder IVb nach Umwandlung des Esters in die Carbonsäure mit Z zur Verbindung der Formel Ia umsetzt, oder

b) eine Verbindung der Formel Va oder Vb, wobei A, B, X und Y die Bedeutung wie in der Verbindung der Formel Ia haben,

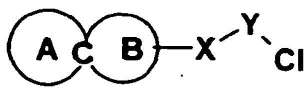
(Va)

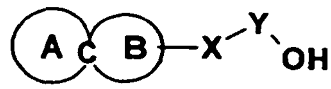
(Vb)

mit einer Verbindung der Formel VI, wobei R1, R2 und R3 die Bedeutung wie in der Verbindung der Formel Ia haben und PG eine Ester-Schutzgruppe darstellt,

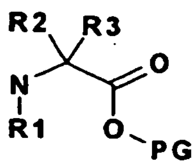
(VI)

zu einer Verbindung der Formel IVa oder IVb umsetzt und die erhaltene Verbindung der Formel IVa oder IVb nach Umwandlung der Ester-Schutzgruppe in die Carbonsäure mit Z zur Verbindung der Formel Ia umsetzt, oder

c) eine Verbindung der Formel VIIa oder VIIb, wobei A, B und X die Bedeutung wie in der Verbindung der Formel Ia haben,

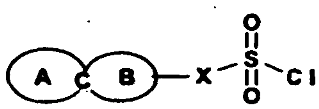
(VIIa)

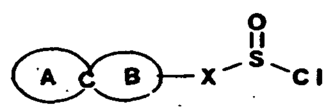
(VIIb)

mit einer Verbindung der Formel VI,

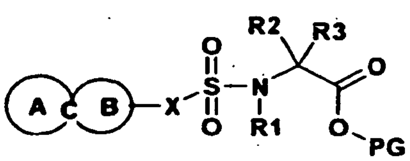
(VIIIa)

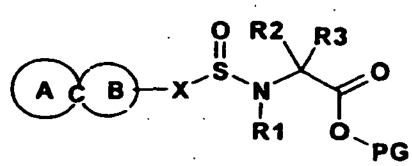
(VIIIb)

zu einer Verbindung der Formel VIIIa oder VIIIb umsetzt und die erhaltene Verbindung der Formel VIIIa oder VIIIb nach Umwandlung des Esters in die entsprechende Carbonsäure mit Z zur Verbindung der Formel Ia umsetzt, oder

d) eine Verbindung der Formel IX

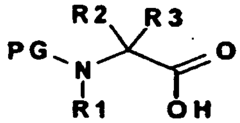

(IX)

mit einem Amin Z, wobei Z die Bedeutung wie in der Verbindung der Formel Ia hat, zu einer Verbindung der Formel X umsetzt,

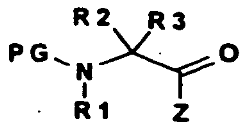

(X)

und die so erhaltene Verbindung X anschließend im Sinne einer Schutzgruppenabspaltung zu einer Verbindung der Formel XI umsetzt,

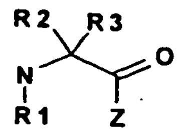

(XI)

und diese Verbindung XI anschließend mit einer Verbindung Va oder Vb, wie unter b) aufgeführt, zur Verbindung der Formel Ia umsetzt, oder
e) eine nach den Verfahren a), b), c) oder d) hergestellte Verbindung der Formel Ia, oder eine geeignete Vorstufe der Formel Ia, die aufgrund ihrer chemischen Struktur in enantiomeren oder diastereomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren oder Diastereomeren auftrennt, oder
f) die nach den Verfahren a), b), c) oder d) hergestellte Verbindung der Formel Ia entweder in freier Form isoliert oder aus physiologisch unverträglichen Salzen freisetzt oder im Falle des Vorliegens von sauren oder basischen Gruppen in physiologisch verträgliche Salze umwandelt, oder
g) die nach den Verfahren a), b), c) oder d) hergestellte Verbindung der Formel Ia, oder eine geeignete Vorstufe der Formel Ia, die aufgrund ihrer chemischen Struktur dazu befähigt ist, ein N-Oxid zu bilden, in dieses zu überführen oder im Falle des Vorliegens eines N-Oxids, dieses in das freie Amin oder das Salz eines Amins umwandelt.

8. Arzneimittel, **gekennzeichnet durch** eine Verbindung der Formel Ia gemäß einem oder mehreren der Ansprüche 1 bis 6 zusammen mit einem pharmazeutisch geeigneten und physiologisch verträglichen Trägerstoff, Zusatzstoff und/oder anderen Wirk- und Hilfsstoffen.

9. Verwendung der Verbindung der Formel Ia gemäß einem oder mehreren der Ansprüche 1 bis 6 zur Herstellung eines Arzneimittels zur Prophylaxe, Sekundärprevention und Therapie von abnormal erhöhtem Knochenabbau, Allergien, der Alzheimer'schen Krankheit, Amyloidosis, ARDS, Arterieller Thrombose, Asthma, Atheromen, Atherosklerose, autoimmun-Erkrankungen, bakteriellen Infektionen, Bronchiolitis, Cerebrater Hämorrhagie, Cerebrovaskulärer Ischämie, Chorea-Huntington, chronischen Entzündungen, CIPD (chronischer entzündlicher demyelinisierende Polyradiculoneuropathie), Creutzfeldt-Jakob-Krankheit, der Crohn'schen Krankheit, Diabetes, besonders der juvenilen Form, Emphysem, Encephalomyelitis, Endometriose, entzündlichen Atemwegserkrankungen, entzündlicher Pankreatitis, Epilepsie, Erkrankungen, die durch verstärkte Angiogenese gekennzeichnet sind, exzessiver Atemwegs-Elastolyse, Gewebsverpflanzungen, Gingivitis, Glomerulonephritis, glucocorticoid induzierter Osteoporose, Graves' Krankheit, Guillain-Barre-Syndrom, Hashimotos Thyroiditis, Hepatitis, HIV-Infektion, Huntington-Krankheit, Hypercalcämie, IBD, Immunschwäche, Interstitieller Cystitis, Knochenbruch, Knochenverlust, Krebserkrankungen, Lupus Erythematosus, Malaria, metachromer Leukodystrophie, metastatisierender Osteogenese, Metastatisierung, Multipler Sklerose, multiplem Myelom, Muskel-Dystrophie, Myasthenia gravis, Neurodegenerativen

Erkrankungen, neuropathischem Schmerz, chronische oder diabetische Neuropathie, postherpetische Neuralgie, trigeminale Neuralgie, schmerzhafte diabetische Polyneuropathie, Schmerz nach Schlaganfall, Postamputationsschmerz, myelopathischer oder radiculopathischer Schmerz, atypischer Gesichtsschmerz und Causalgie-ähnliche Syndrome, Organabstoßung bei Transplantationen, Osteoarthritis, Osteogenesis imperfecta, Osteoporose, Paget's Krankheit, Pankreatitis, Parkinson-Krankheit, Pemphigus vulgaris, Periodontitis, Plaque-Ruptur, Pneumocystisis Carinii, Pneumonitis, Psoriasis, Restenose, Rheumatoider Arthritis, Skleroderma, systemischem Lupus erythematosus, Hirn- Traumata, Rückenmarks-Traumata, Tumorzell-Invasion, viralen Infektionen, Zahnverlust, Brustkrebs, Darmkrebs, Eierstockkrebs, Gebärmutterhalskrebs, Hautkrebs, Hirntumor, Kaposi-Sarkom, B- und T-Zell-Leukämie, Lungenkrebs, Lymphknotenkrebs, Pankreas-Krebs, Prostata-Krebs und Sarkomen.

## Claims

**1.** A compound of the formula Ia

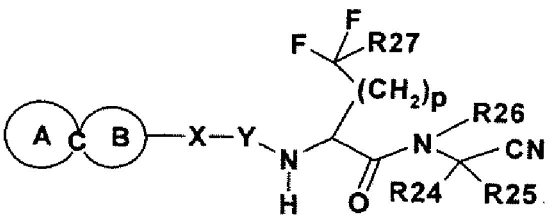

(Ia)

and/or all stereoisomeric forms of the compound of the formula Ia and/or mixtures of these forms in any ratio, and/or a physiologically acceptable salt of the compound of the formula Ia, and/or solvates or hydrates of the compound of the formula Ia, where
the

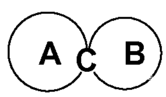

radical is a spiro compound,
in which the sub-rings

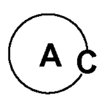

and

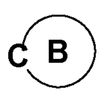

are in each case the same or different and are each independently

a) a saturated or partly saturated -($C_3$-$C_{11}$)-cycloalkyl, in which cycloalkyl is unbridged, bridged or fused and is unsubstituted or independently, according to the ring size, mono-, di-, tri, tetra- or pentasubstituted by R4, or
b) a saturated or partly saturated, three- to eleven-membered heterocycle which, according to the ring size, may contain one, two, three or four identical or different heteroatoms from the group of oxygen, nitrogen or sulfur, and in which the heterocycle is unbridged, bridged or fused and is unsubstituted or independently, according to the ring size, mono-, di-, tri-, tetra- or pentasubstituted by R4, where
R4 is -$NO_2$, -CN, =O, =S, -OH, -$CF_3$, -$SF_5$, - ($C_0$-$C_3$)-alkylene-S-R10, -O-$CF_3$, -Si-$(CH_3)_3$, -($C_0$-$C_5$)-alkylene-

O-C(O)-R21, $(C_0-C_5)$-alkylene-C(O)-O-R10, $-(C_0-C_3)$-alkylene-O-R10, $-(C_0-C_3)$-alkylene-N(R21)-R22, $-(C_0-C_3)$- alkylene-N(R10)-S$(O_2)$-R10, $-(C_0-C_5)$-alkylene-$(C_3-C_8)$-cycloalkyl-R23, -S-$CF_3$, $-(C_0-C_5)$-alkylene-$(C_1-C_3)$- fluoroalkyl, $-(C_0-C_5)$-alkylene-N(R10)-C(O)-R21, $-(C_0-C_3)$-alkylene-C(O)-N(R21) -R22, $-(C_0-C_4)$-alkyl where alkyl is unsubstituted or mono-, di- or trisubstituted independently by R9, $-(C_0-C_4)$-alkylene-aryl where aryl is selected from the group of phenyl, indanyl, indenyl, naphthyl, where aryl is unsubstituted or mono-, di- or trisubstituted independently by R8, or $-(C_0-C_4)$-alkylene-Het where Het is selected from the group of azetidinyl, benzimidazolinyl, benzimidazolyl, benzofuranyl, benzothiophenyl, benzoxazolyl, benzthiazolyl, benzisoxazolyl, benzisothiazolyl, quinolinyl, dioxolyl, dioxanyl, furanyl, imidazolidinyl, imidazolinyl, imidazolyl, indolinyl, indolyl, 3H-indolyl, isoindolinyl, isoindolyl, isoquinolinyl, isothiazolidinyl, 2-isothiazolinyl, isothiazolyl, isoxazolyl, isoxazolidinyl, 2-isoxazolinyl, morpholinyl, octahydroisoquinolinyl, oxazolyl, oxazolidinyl, pyrimidinyl, piperazinyl, piperidinyl, pyranyl, pyrazinyl, pyrazolinyl, pyrazolyl, pyridazinyl, pyridyl, pyrimidinyl, pyrrolidinyl, pyrrolinyl, 2H-pyrrolyl, pyrrolyl, tetrahydrofuranyl, tetrahydroisoquinolinyl, tetrahydroquinolinyl, tetrahydropyridinyl, thiazolyl, thienyl, thienopyridinyl, thiomorpholinyl, thiophenyl, and this Het radical is unsubstituted or independently mono-, di- or trisubstituted by R8,

R8 is halogen, carbamimidoyl, $-NO_2$, =0, $-CF_3$, - $SF_5$, -C(O)-O-R10, -CN, -C(O)-$NH_2$, -OH, $-NH_2$, -O-$CF_3$, -C (O)-N(R10)-R20, -N(R10)-R20, $-(C_3-C_8)$ - cycloalkyl, -O-$(C_1-C_8)$-alkyl, -O-$(C_0-C_4)$-alkylene-$(C_3-C_6)$-cycloalkyl, $-(C_1-C_8)$-alkyl, $-(C_0-C_4)$-alkylene-$(C_3-C_8)$-cycloalkyl, where the alkyl radicals mentioned are each unsubstituted or mono- , di- or trisubstituted independently by halogen, $NH_2$, -OH, -O-$CH_3$, -$SO_2$-$CH_3$ or -$SO_2$-$CF_3$,

R9 is halogen, $-NO_2$, -CN, =O, -OH, $-CF_3$, -C(O)-OR10, -C(O)-N(R21)-R22, -N(R21)-R22, $-(C_3-C_8)$-cycloalkyl, $-(C_0-C_3)$-alkylene-O-R10, -Si-$(CH_3)_3$, -N(R10)-S$(O)_u$-R10 where u is the integer 1 or 2, - S-R10, -$SO_r$-R10 where r is the integer 1 or 2, - S$(O)_v$-N(R10)-R20 where v is the integer 1 or 2, - C (O)-R10,-$(C_1-C_8)$-alkyl,-$(C_1-C_8)$-alkoxy, phenyl, phenyloxy-, $-(C_1-C_3)$-fluoroalkyl, -O-R19, -NH-C(O)-NH-R10, -$(C_0-C_4)$ -alkyl-C(O)-O-C(R11, R19) -O-C(O)-R12, -NH-C(O)-NH-R21, -N(R21)-C(O)-R22, - $(C_0-C_4)$-alkyl-C (O)-O-C(R11, R19)-O-C(O)-O-R12, -NH-C(O)-O-R10, -O-$CF_3$ or Het where Het is as defined above and is unsubstituted or mono-, di- or trisubstituted independently by R8,

R10 and R20 are the same or different and are each independently a hydrogen atom, $-(C_1-C_6)$-alkyl, -$(C_0-C_4)$-alkyl-OH, $-(C_1-C_3)$-fluoroalkyl,-$(C_0-C_4)$ - alkyl-O-$(C_1-C_4)$-alkyl, $-(C_0-C_5)$-alkyl-$(C_3-C_8)$-cycloalkyl, $-(C_0-C_2)$-alkylene-aryl where aryl is as defined above and is unsubstituted or mono-, di- or trisubstituted independently by $-(C_1-C_6)$-alkyl, -O-$(C_1-C_6)$-alkyl, halogen or $-(C_3-C_8)$-cycloalkyl, or $-(C_0-C_2)$-alkylene-Het where Het is as defined above and is unsubstituted or mono-, di- or trisubstituted independently by $-(C_1-C_6)$-alkyl, -O-$(C_1-C_6)$-alkyl, halogen or - $(C_3-C_8)$-cycloalkyl,

R11 and R19 are the same or different and are each independently a hydrogen atom or $-(C_1-C_6)$-alkyl, R12 is $-(C_1-C_6)$ -alkyl, $-(C_1-C_6)$-alkyl-OH, $-(C_1-C_6)$-alkyl-O-$(C_1-C_6)$-alkyl, $-(C_3-C_8)$-cycloalkyl, - $(C_1-C_6)$-alkyl-O-$(C_1-C_8)$-alkyl-$(C_3-C_8)$-cycloalkyl, $-(C_1-C_6)$-alkyl-$(C_3-C_8)$-cycloalkyl, where the cycloalkyl radical is unsubstituted or mono-, di- or trisubstituted independently by -OH, -O-$(C_1-C_4)$-alkyl or R10,

R21 and R22 are the same or different and are each independently a hydrogen atom, $-(C_1-C_6)$-alkyl where alkyl is unsubstituted or mono-, di- or trisubstituted independently by R8, $-(C_0-C_6)$-alkylene-$(C_3-C_8)$-cycloalkyl, -$SO_t$-$R_{10}$ where t is the integer 1 or 2, $-(C_1-C_3)$-fluoroalkyl, -O-R12, $-(C_0-C_6)$-alkylene-aryl where aryl is as defined above and alkylene and aryl are each unsubstituted or mono-, di- or trisubstituted independently by R8 or $-(C_0-C_6)$-alkylene-Het where Het is as defined above and alkylene and Het are each unsubstituted or mono-, di- or trisubstituted independently by R8,

R21 and R22, together with the nitrogen atom to which they are bonded, form a four- to eight-membered monocyclic heterocyclic ring which, as well as the nitrogen atom, additionally, according to the ring size, may contain one or two identical or different heteroatoms from the group of oxygen, nitrogen or sulfur and in which the heterocycle is unsubstituted or mono-, di- or trisubstituted independently by R8,

R23 is a hydrogen atom, -OH or -O-$(C_1-C_4)$-alkyl,

X is a covalent bond, -N(R7)- or -0-, where R7 is a hydrogen atom, $-(C_0-C_4)$-alkylene-$(C_3-C_6)$-cycloalkyl or-$(C_1-C_4)$-alkyl,

Y is -C(O)-, -C(S)- or -S$(O_2)$-,

p is the integer 1 or 2,

R27 is a hydrogen atom, $-(C_1-C_6)$-alkyl, halogen, - $(C_0-C_4)$-alkylene-$(C_3-C_6)$-cycloalkyl, $-(C_0-C_4)$-alkylene-Het where Het is as defined above and is unsubstituted or substituted by halogen, $-(C_1-C_6)$-alkyl, -O-$(C_1-C_3)$-fluoroalkyl or -O-$(C_1-C_6)$-alkyl, or $-(C_0-C_2)$-alkylene-phenyl where phenyl is unsubstituted or substituted by halogen, $-(C_1-C_6)$-alkyl, -O-$(C_1-C_3)$-fluoroalkyl or -O-$(C_1-C_6)$-alkyl, R26 is a hydrogen atom, $-(C_1-C_4)$-alkyl or $-(C_0-C_4)$-alkylene-$(C_3-C_6)$-cycloalkyl, R24 and R25 are the same or different and are each independently a hydrogen atom, $-(C_1-C_6)$-alkyl, - $(C_1-C_3)$-fluoroalkyl, $-(C_0-C_4)$-alkylene-$(C_3-C_6)$-cycloalkyl, $-(C_0-C_4)$-alkylene-aryl where aryl is as defined above and is unsubstituted or mono-, di- or trisubstituted independently by R8, or - $(C_0-C_4)$-alkylene-Het where Het is as defined above and is unsubstituted or mono-, di- or trisubstituted independently by R8,

R24 and R25, together with the carbon atom to which they are bonded, form a three- to six-membered cycloalkyl ring which is unsubstituted or mono-, di- or trisubstituted independently by R10 or fluorine,

R24 and R25, together with the carbon atom to which they are bonded, form a three- to six-membered heterocycloalkyl radical which is unsubstituted or mono-, di- or trisubstituted independently by R10 or fluorine.

**2.** The compound of the formula I as claimed in claim 1 where the sub-ring

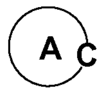

has been selected from the following group

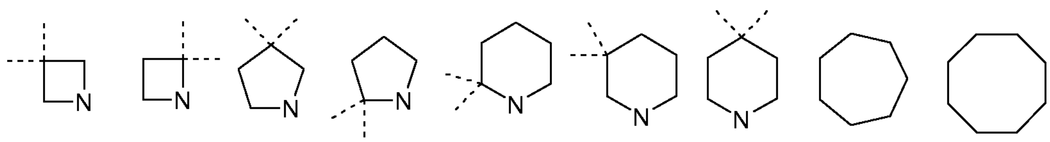

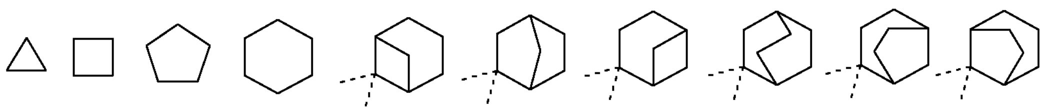

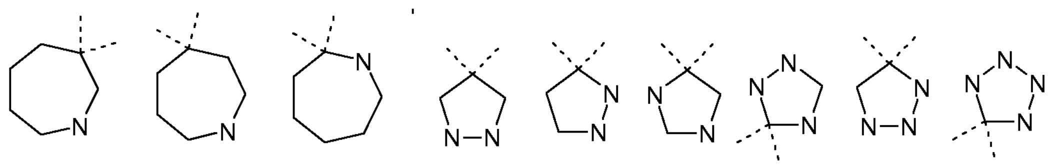

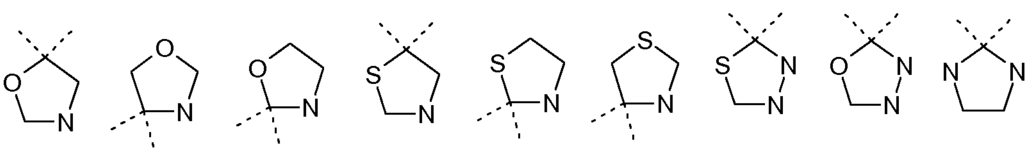

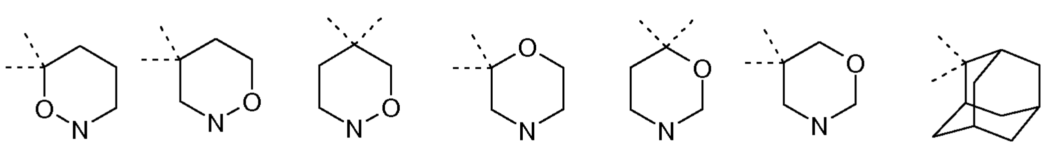

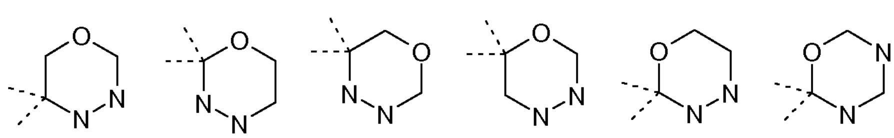

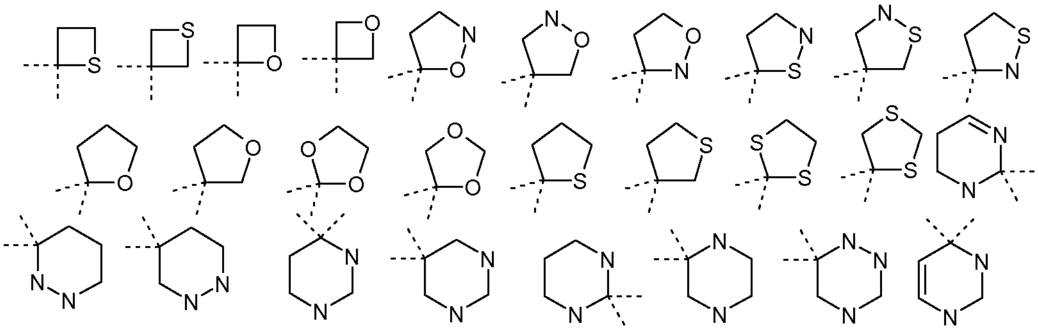

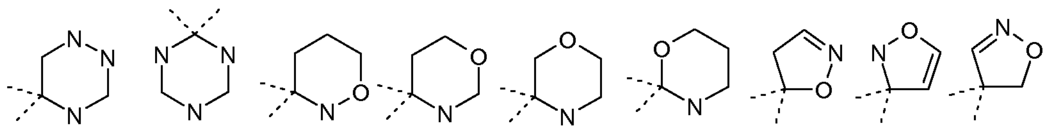

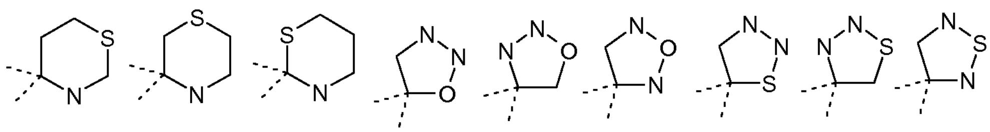

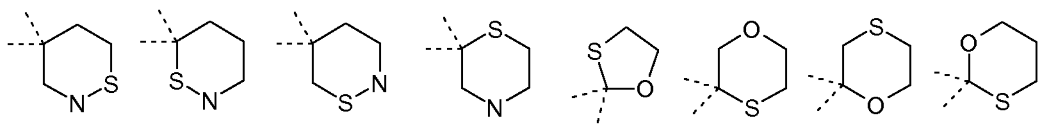

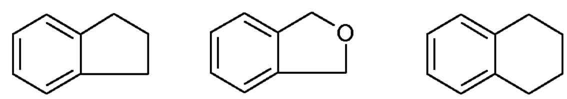

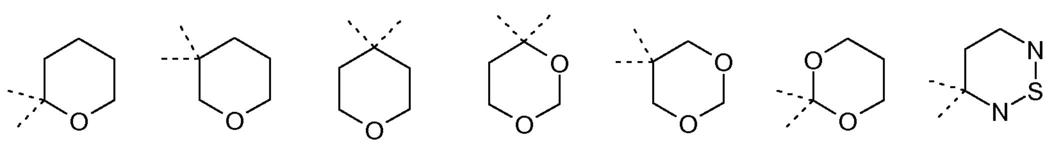

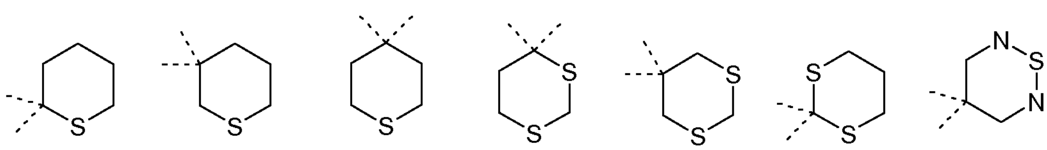

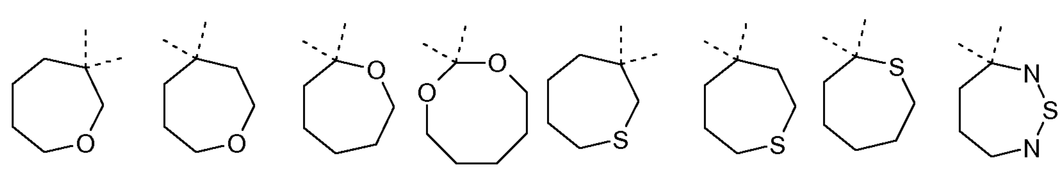

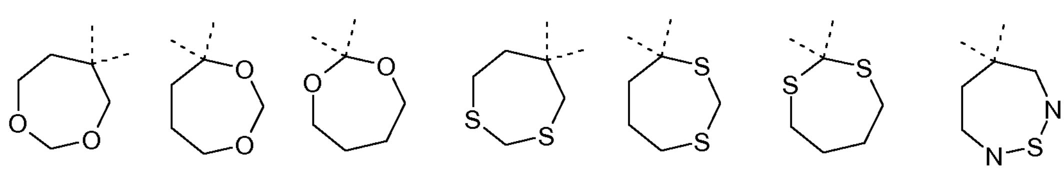

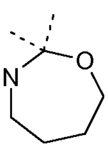 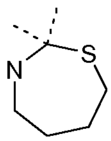 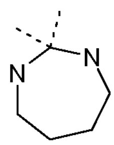 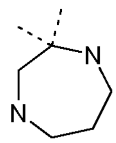 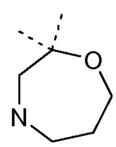 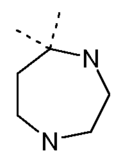 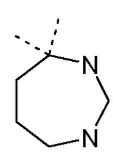

where the dotted lines indicate the particular point of attachment to the second sub-ring, single bonds in the structures listed may be replaced partly by double bonds, or further ring systems may be fused on, and in which the sub-ring

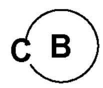

has been selected from the following group

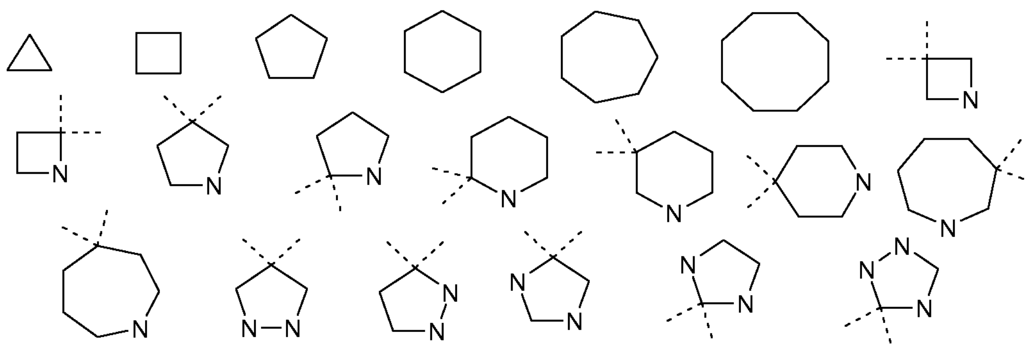

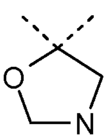 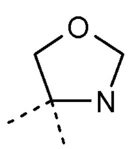 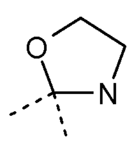 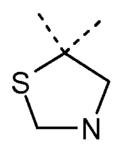 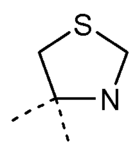 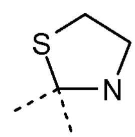

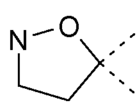 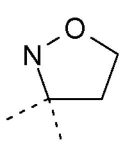 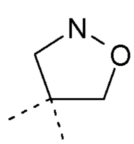 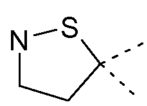 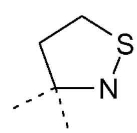 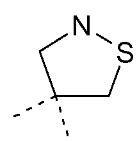

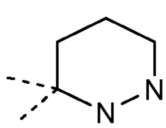 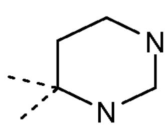 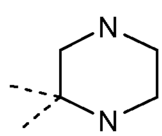 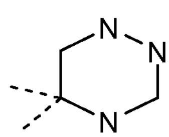 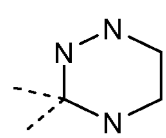

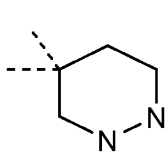 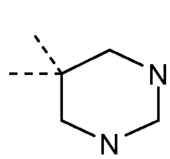 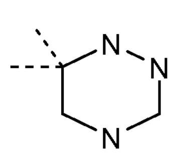 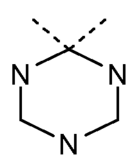

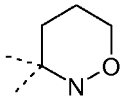 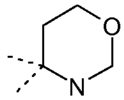 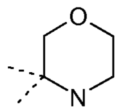 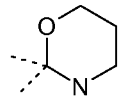 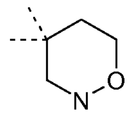 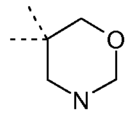 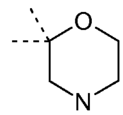

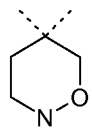 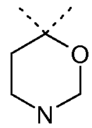 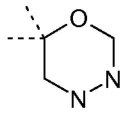 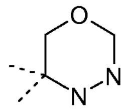 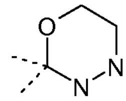 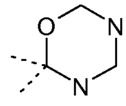 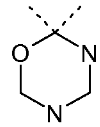

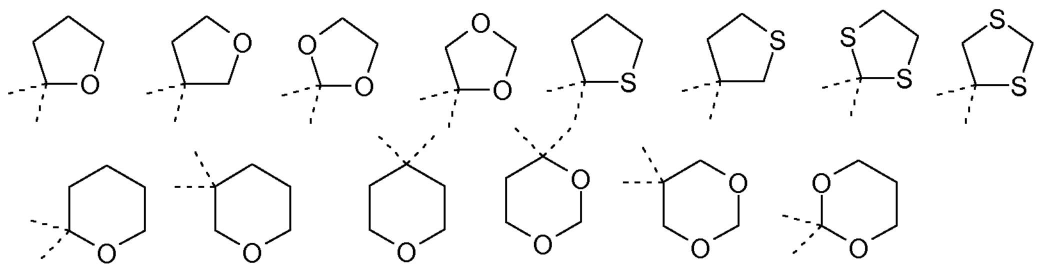

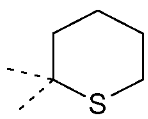 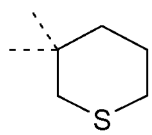 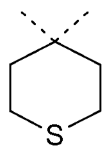 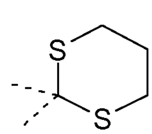

where the dotted lines indicate the particular point of attachment to the second sub-ring, single bonds in the structures listed may be replaced partly by double bonds, and the two sub-rings A and B are unsubstituted or independently mono- to tetrasubstituted by R4, and the X, Y, R27, p, R26, R24, R25 and R4 radicals are each as defined in claim 1.

**3.** A compound of the formula Ia as claimed in claim 1 where the sub-rings

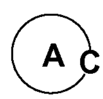

and

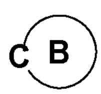

have in each case been selected from cyclopropane, cyclobutane, cyclopentane, cyclohexane, cycloheptane, cyclooctane, bicyclo[4.2.0]octane, octahydroindene, decalin, decahydrobenzocycloheptene, dodecahydroheptalene, bicyclo[3.1.1]heptane, bicyclo[2.2.1]heptane, bicyclo[3.3.0]octane, bicyclo[2.2.2]octane, spiro[2.5]octane, spiro[3.4]octane, azepane, azepine, azetidine, aziridine, azirine, azocane, benzimidazoline, 2,3-dihydrobenzo[b]thiophene, 1,3-dihydrobenzo[c]thiophene, 2,3-dihydrobenzofuran, 2,3-dihydrobenzooxazole, 2,3-dihydrobenzothiazole, 1,3-dihydroisobenzofuran, 4,5-dihydroisothiazole, 2,3-dihydroisoxazole, 2,5-dihydroisoxazole, 4,5-dihydroisoxazole, 5,6-dihydro-4H-[1,2]oxazine, benzo[1,3]dioxole, 1,4-diazepane, 1,2-diazepine, 1,3-diazepine, 1,4-diazepine, diaziridine, diazirine, 1,4-diazocane, dioxane, 1,3-dioxane, dioxazine, [1,3]dioxepane, 1,4-diozocane, dioxole, dioxolane, 1,3-dioxolane, 1,3-dioxolene, [1,3]dithiane, [1,3]dithiolane, hexahydropyridazine, hexahydropyrimidine, imidazoline, imidazolidine, indane, indoline, isoindoline, isothiazolidine, isothiazoline, isoxazoline, isoxazolidine, 2-isoxazoline, morpholine, [1,3,4]oxadiazinane, [1,3,5]oxadiazinane, [1,2,3]oxadiazolidine, [1,3,4]oxadiazolidine, 1,2-oxathiepane, 1,2-oxathiolane, [1,3]oxathiolane, 1,4-oxazepane, 1,2-oxazine, 1,3-oxazine, 1,4-oxazine, oxazinane, 1,3-oxazinane,

oxazocane, oxaziridine, oxazolidine, oxepane, oxetane, oxirane, oxocane, piperazine, piperidine, pyran, pyrazoline, pyrazolidine, pyrrolidine, pyrrolidinone, pyrroline, tetrahydroquinoline, tetrahydrofuran, tetrahydroisoquinoline, 1,2,3,4-tetrahydronaphthalene, tetrahydropyran, tetrahydropyridine, 1,2,3,4-tetrahydro-pyrimidine, 1,2,5,6-tetrahydropyrimidine, tetrahydrothiophene, tetrazine, thiadiazine, [1,2,6]thiadiazinane, [1,3,4]thiadiazolidine, 1,2-thiazine, 1,3-thiazine, 1,4-thiazine, [1,2]thiazinane, [1,3]thiazinane, thiazolidine, thiazoline, thiepane, thietane, thiomorpholine, thiopyran, 1,2,3-triazine, 1,2,4-triazine, 1,3,5-triazine, [1,2,4]triazinane or [1,2,4]triazolidine, and in which the two sub-rings are each unsubstituted or independently, according to the ring size, mono-, di-, tri-, tetra- or pentasubstituted by R4, and the radicals X, Y, R27, p, R26, R24, R25 and R4 are each as defined in claim 1.

**4.** A compound of the formula Ia as claimed in claim 1 where the sub-rings

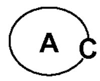

and

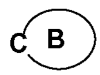

are each independently selected from the group of azetidine, cyclopropyl, cyclobutyl, cyclopentyl, cyclohexyl, 1,3-dihydroisobenzofuran, 2,3-dihydroisoxazole, 2,5-dihydroisoxazole, 4,5-dihydroisoxazole, 1,3-dioxane, dioxolane, 1,3-dioxolane, imidazolidine, indane, morpholine, 1,3-oxazinane, oxazolidine, piperazine, piperidine, pyrrolidine, tetrahydrofuran, and 1,2,3,4-tetrahydronaphthalene,
and in which the two sub-rings are each unsubstituted or independently, according to the ring size, mono-, di- or trisubstituted by R4,
R4 is =O, =S, -($C_0$-$C_3$)-alkylene-C(O)-O-R10, -($C_0$-$C_3$)-alkylene-N(R21)-R22, -($C_0$-$C_3$)-alkylene-NH-C(O)-R21, -($C_0$-$C_4$)-alkylene-($C_3$-$C_6$)-Cycloalkyl-R23, -($C_0$-$C_3$)-alkylene-O-R10, -($C_0$-$C_4$)-alkylene-phenyl, where phenyl is unsubstituted or mono-, di- or trisubstituted independently by R8, or - ($C_0$-$C_4$)-alkyl where alkyl is unsubstituted or mono-, di- or trisubstituted independently by R9, R8 is fluorine, chlorine, bromine, -O-($C_1$-$C_3$)-fluoroalkyl or -O-($C_1$-$C_4$)-alkyl,
R9 is halogen, $-NO_2$, -CN, =0, -OH, $-CF_3$, -C(0)-0-R10, -C(O)-N(R21)-R22, -N(R21)-R22, - ($C_3$-$C_8$) - cycloalkyl, -($C_0$-$C_3$)-alkylene-O-R10, -Si-$(CH_3)_3$, -N(R10)-$S(O)_u$-R10 where u is the integer 1 or 2, - S-R10, -$SO_r$-R10 where r is the integer 1 or 2, - $S(O)_v$-N(R10)-R20 where v is the integer 1 or 2, - C(O)-R10, -($C_1$-$C_8$)-alkyl, -($C_1$-$C_8$)-alkoxy, phenyl, phenyloxy-, -($C_1$-$C_3$)-fluoroalkyl, -O-R19, -NH-C(O)-NH-R10, -($C_0$-$C_4$)-alkyl-C(O)-O-C(R11,R19)-O-C(O)-R12, -NH-C(O)-NH-R21, -N(R21)-C(O)-R22, -($C_0$-$C_4$)-alkyl-C(O)-O-C(R11, R19)-O-C(O)-O-R12, -NH-C(O)-O-R10 or $-O-CF_3$,
R10 and R20 are the same or different and are each independently a hydrogen atom or -($C_1$-$C_6$)-alkyl, R11 and R19 are the same or different and are each independently a hydrogen atom or -($C_1$-$C_6$)-alkyl, R12 is -($C_1$-$C_6$)-alkyl, -($C_1$-$C_6$)-alkyl-OH, -($C_1$-$C_6$)-alkyl-O-($C_1$-$C_6$)-alkyl, -($C_3$-$C_8$)-cycloalkyl, - ($C_1$-$C_6$)-alkyl-O-($C_1$-$C_8$)-alkyl-($C_3$-$C_8$)-cycloalkyl, -($C_1$-$C_6$)-alkyl-($C_3$-$C_8$)-cycloalkyl, where the cycloalkyl radical is unsubstituted or mono-, di- or trisubstituted independently by -OH, -O-($C_1$-$C_4$)-alkyl or R10,
R21 and R22 are the same or different and are each independently a hydrogen atom, - ($C_1$-$C_6$)-alkyl, -0-R12, -($C_0$-$C_6$)-alkylene-($C_3$-$C_8$)-cycloalkyl, -$SO_t$-R10 where t is the integer 1 or 2, or -($C_1$-$C_3$)-fluoroalkyl,
R23 is a hydrogen atom, -OH or -O-($C_1$-$C_4$)-alkyl,
X is a covalent bond or -N(R7)- where R7 is a hydrogen atom or -($C_1$-$C_4$)-alkyl,
Y is -C(O)- or -$S(O_2)$-,
p is the integer 1 or 2,
R26 is a hydrogen atom,
R27 is a hydrogen atom,-($C_0$-$C_4$)-alkylene-($C_3$-$C_6$)-cycloalkyl, -($C_1$-$C_6$)-alkyl, -($C_0$-$C_2$)-alkylene-phenyl where phenyl is unsubstituted or substituted by halogen, -($C_1$-$C_6$)-alkyl, -O-($C_1$-$C_3$)-fluoroalkyl or -O-($C_1$-$C_6$)-alkyl, or -($C_0$-$C_2$) - alkylene-pyridyl,
R24 and R25 are the same or different and are each independently a hydrogen atom, -($C_1$-$C_4$)-alkyl or -($C_0$-$C_4$)-alkylene-($C_3$-$C_6$)-Cycloalkyl,
R24 and R25, together with the carbon atom to which they are bonded, form a cycloalkyl ring which is selected from the group of cyclopropyl, cyclobutyl, cyclopentyl and cyclohexyl, and is unsubstituted or mono-, di- or trisubstituted

independently by R10 or fluorine,
R24 and R25, together with the carbon atom to which they are bonded, form a three- to six-membered heterocycloalkyl radical selected from the group of aziridine, azetidine, diazetidine, diaziridine, hexohydropyridazine, hexohydropyrimidine, imidazolidine, morpholine, oxadiazinane, oxadiazolidine, oxathianane, oxathiolane, oxazetidine, oxazolidine, oxetane, oxirane, piperazine, piperidine, pyrazolidine, pyrrolidine, tetrahydrofuran, tetrahydropyran, tetrahydrothiophene, tetrahydrothiopyran, tetrazinane, thiadiazolidine, thiazetidine, thiaziridine, thiazolidine, thietane, thiirane, thiomorpholine, triazetidine, triazinane or triazolidine, which is unsubstituted or mono-, di- or trisubstituted independently by R10 or fluorine.

**5.** A compound of the formula Ia as claimed in one or more of claims 1 to 4 where
the sub-ring

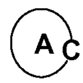

is selected from the group of azetidine, cyclopropyl, cyclobutyl, cyclopentyl, cyclohexyl, 1,3-dihydroisobenzofuran, 1,3-dioxane, 1,3-dioxolane, imidazolidine, indane, morpholine, 1,3-oxazinane, piperazine, piperidine, pyrrolidine, tetrahydrofuran, and 1,2,3,4-tetrahydronaphthalene,
the sub-ring

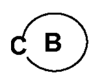

is selected from the group of azetidine, cyclopropyl, cyclopentyl, cyclohexyl, morpholine, oxazolidine, piperidine and pyrrolidine, and in which the two sub-rings are unsubstituted or independently, according to the ring size, mono-, di- or tri-substituted by R4 where
R4 is -O-($C_1$-$C_4$)-alkyl, =O, -($C_0$-$C_4$)-alkylene-($C_3$-$C_6$)-cycloalkyl, -($C_1$-$C_4$)-alkyl or -($C_0$-$C_4$)-alkylene-phenyl where phenyl is unsubstituted or substituted by F, Cl,
Br or -O-($C_1$-$C_4$)-alkyl,
X is a covalent bond or -NH-,
Y is -C(O) - or -S($O_2$)-,
p is the integer 1,
R27 is a hydrogen atom, -($C_1$-$C_6$)-alkyl, 4-F-benzyl or benzyl,
R26 is a hydrogen atom,
R24 and R25 are the same or different and are each independently a hydrogen atom, methyl or ethyl, R24 and R25, together with the carbon atom to which they are bonded, form a cyclopropyl or cyclobutyl radical, or
R24 and R25, together with the carbon atom to which they are bonded, form a piperidine ring which is unsubstituted or substituted by -($C_1$-$C_4$)-alkyl.

**6.** A compound of the formula Ia as claimed in one or more of claims 1 to 5, which is the compound N-[(S)-1-(1-cyanocyclopropylcarbamoyl)-3,3-difluorobutyl]-3-azaspiro[5.5]undecane-3-carboxamide, N-[(S)-1-(1-cyanocyclopropylcarbamoyl)-3,3-difluorobutyl]-8-azaspiro[4.5]decane-8-carboxamide, N-[(S)-1-(1-cyanocyclopropylcarbamoyl)-3,3-difluorobutyl]-1,4-dioxa-8-azaspiro[4.5]decane-8-carboxamide, N-[(S)-1-(1-cyanocyclopropylcarbamoyl)-3,3-difluorobutyl]-2-azaspiro[5.5]undecane-2-carboxamide, N-[(S)-1-(1-cyanocyclopropylcarbamoyl)-3,3-difluorohexyl]-8-azaspiro[4.5]decane-8-carboxamide, N-[(S)-1-(1-cyanocyclopropylcarbamoyl)-3,3-difluorohexyl]-3-azaspiro[5.5]undecane-3-carboxamide, N-[(S)-1-(1-cyanocyclopropylcarbamoyl)-3,3-difluorobutyl]-2-(4-methoxyphenyl)-1-oxo-2,8-diaza-spiro[4.5]decane-8-carboxamide, N-[(S)-1-(1-cyanocyclopropylcarbamoyl)-3,3-difluorobutyl]-4-oxo-1-phenyl-1,3,8-triazaspiro[4.5]decane-8-carboxamide, N-[(S)-1-(1-cyanocyclopropylcarbamoyl)-3,3-difluorobutyl]-1,5-dioxa-9-azaspiro[5.5]undecane-9-carboxamide, N-[(S)-1-(1-cyanocyclopropylcarbamoyl)-3,3-difluorobutyl]-1-oxo-2,8-diazaspiro[4.5]decane-8-carboxamide, N-[(S)-1-(1-cyanocyclopropyl-carbamoyl)-3,3-difluorobutyl]-2-methyl-1-oxo-2,8-diazaspiro[4.5]decane-8-carboxamide, N-[(S)-1-(1-cyanocyclopropylcarbamoyl)-3,3-difluorobutyl]-3,3-dimethyl-1-oxa-5,9-diazaspiro[5.5]undecane-9-carboxamide, N-[(S)-1-(1-cyanocyclo-propylcarbamoyl)-3,3-difluoro-4-phenylbutyl]-8-azaspiro[4.5]decane-8-carboxamide, N-[(S)-1-(1-cyanocyclopropylcarbamoyl)-3,3-difluorobutyl]-2,4-dioxo-1,3,8-triazaspiro[4.5]decane-8-carboxamide, N-[(S)-1-(1-cyanocyclopropylcarbamoyl)-3,3-difluorobutyl]-2-azaspiro[4.4]nonane-2-carboxamide, N-[(S)-1-(1-cyanocyclopropylcarbamoyl)-3,3-difluorobutyl]-2-benzyl-1-oxo-2,8-diazaspiro[4.5]decane-8-carboxamide, N-[(S)-1-(1-cyanocyclopropylcarbamoyl)-3,3-difluorobutyl]-2-(4-fluoro phenyl)-1-oxo-2,8-diazaspiro[4.5]decane-8-carboxamide, N-[(S)-1-(1-cyanocyclopropylcarbamoyl)-3,3-difluorobutyl]-3-phenyl-1,5-dioxa-9-azaspiro[5.5]undecane-9-carboxamide, N-[(S)-1-(1-cyanocyclopropylcarbamoyl)-3,3-difluorobutyl]-9-butyl-3,9-diazaspiro[5.5]undecane-3-carboxamide, N-[(S)-1-(1-cyanocyclopropylcarbamoyl)-3,3-difluorobutyl]-9-CyClopropyl-3,9-diazaspiro[5.5]undecane-3-carboxamide, N-[(S)-1-(1-cyanocyclopropylcarbamoyl)-3,3-difluoro-butyl]spiro[2.3]hexane-1-carboxamide, N-[(S)-1-(1-cyanocyclopropylcarbamoyl)-3,3-difluorobutyl]-2,2-dimethyl-1-oxa-8-azaspiro[4.5]decane-8-carboxamide, N-[(S)-1-cyanocyclopropylcarbamoyl)-3,3-difluorobutyl]-2-azaspiro[4.5]decane-2-carboxamide.

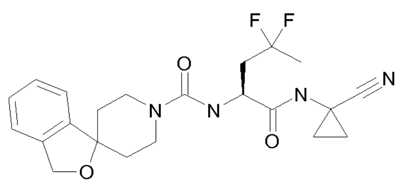
,
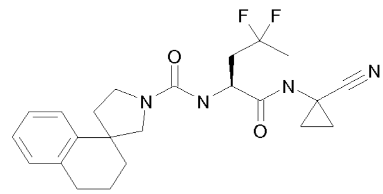

N-[(S)-1-(1-cyanocyclopropyl-carbamoyl)-3,3-difluorobutyl]-1-oxa-4-azaspiro[4.5]decane-4-carboxamide, N-(1-cyanocyclopropyl)-(S)-2-[3-(1,4-dioxaspiro[4.5]dec-8-yl)-ureido]-4,4-difluoropentoxide,

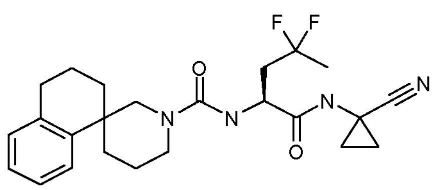
,
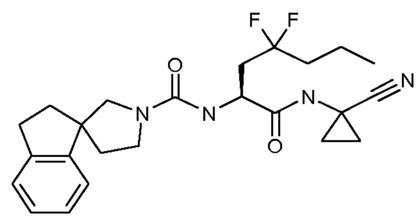

N-[(S)-1-(1-cyanocyclopropylcarbamoyl)-3,3-difluorobutyl]-7-CyClopropyl-2,7-diazaspiro[3.5]nonane-2-carboxamide, N-[(S)-1-(1-cyanocyclopropylcarbamoyl)-3,3-difluoro-butyl]-2-cyclopropyl-2,7-diazaspiro[3.5]nonane-7-carboxamide, N-[(S)-1-(1-cyanocyclopropylcarbamoyl)-3,3-difluorobutyl]-2-propyl-2,7-diazaspiro[3.5]nonane-7-carboxamide, N-(1-cyanocyclopropyl)-(S)-2-(8-azaspiro[4.5]decane-8-sulfonylamino)-4,4-difluoropentanamide, N-[(S)-1-(1-cyanocyclopropylcarbamoyl)-3,3-difluorobutyl]-4-cyclopropyl-1-oxa-4,9-diazaspiro[5.5]undecane-9-carboxamide, N-[(S)-1-(1-cyanocyclopropylcarbamoyl)-3,3-difluorobutyl]-9-cyclopropyl-1-oxa-4,9-diazaspiro[5.5]undecane-4-carboxamide, N-[(S)-1-(1-cyanocyclopropylcarbamoyl)-3,3-difluorobutyl]-2-cyclopropylmethyl-3-oxo-2,8-diazaspiro[4.5]decane-8-carboxamide, N-[(S)-1-(1-cyanocyclopropylcarbamoyl)-3,3-difluorohexyl]-2-(4-methoxyphenyl)-1-oxo-2,8-diazaspiro[4.5]decane-8-carboxamide, N-[(S)-1-(1-cyanocyclopropylcarbamoyl)-3,3-difluorohexyl]-2-(4-methoxyphenyl)-2,8-diazaspiro[4.5]decane-8-carboxamide, N-[(S)-1-(1-cyanocyclo-propylcarbamoyl)-3,3-difluorohexyl]-2-cyclopropyl-2,7-diazaspiro[3.5]nonane-7-carboxamide, N-[(S)-1-(1-cyanocyclopropylcarbamoyl)-3,3-difluorohexyl]-2-cyclopropyl-2,8-diazaspiro[4.5]decane-8-carboxamide, N-[(S)-1-(1-cyanocyclo-propylcarbamoyl)-3,3-difluorohexyl]-9-cyclopropyl-3,9-diazaspiro[5.5]undecane-3-carboxamide, N-[(S)-1-(1-cyanocyclopropylcarbamoyl)-3,3-difluorohexyl]-7-cyclopropyl-2,7-diazaspiro[3.5]nonane-2-carboxamide, N-[(S)-1-(1-cyanocyclopropyl-carbamoyl)-3,3-difluorohexyl]-7-propyl-2,7-diazaspiro[3.5]nonane-2-carboxamide, N-[(S)-1-(1-cyanocyclopropylcarbamoyl)-3,3-difluoro-4-phenylbutyl]-9-CyClopropyl-3,9-diazaspiro[5.5]undecane-3-carboxamide, N-[(S)-1-(1-cyanocyclopropylcarbamoyl)-3,3-difluoro-4-phenylbutyl]-2-cyclopropyl-2,7-diazaspiro[3.5]nonane-7-carboxamide, N-[(S)-1-(1-cyanocyclopropylcarbamoyl)-3,3-difluoro-4-phenylbutyl]-2-propyl-2,7-diazaspiro[3.5]nonane-7-carboxamide, N-[(S)-1-(1-cyanocyclopropylcarbamoyl)-3,3-difluoro-4-phenylbutyl]-7-cyclopropyl-2,7-diazaspiro[3.5]nonane-2-carboxamide, N-[(S)-1-(1-cyanocyclopropylcarbamoyl)-3,3-difluorobutyl]-6-azaspiro[2.5]octane-6-carboxamide, N-[(S)-1-(cyanomethylcarbamoyl)-3,3-difluoro-butyl]-6-azaspiro[2.5]octane-6-carboxamide, N-[(S)-1-(1-cyanocyclopropylcarbamoyl)-3,3-difluorohexyl]-6-azaspiro[2.5]octane-6-carboxamide, N-[(S)-1-(4-cyano-1-methylpiperidin-4-ylcarbamoyl)-3,3-difluorobutyl]-6-azaspiro[2.5]octane-6-carboxamide, N-[(S)-1-(4-cyano-1-methylpiperidin-4-ylcarbamoyl)-3,3-difluorohexyl]-6-azaspiro[2.5]octane-6-carboxamide, N-[(S)-1-(1-cyanocyclopropylcarbamoyl)-3,3-difluoropentyl]-6-azaspiro[2.5]octane-6-carboxamide or N-[(S)-1-(1-cyanocyclopropylcarbamoyl)-3,3-difluoro-4-phenylbutyl]-6-azaspiro[2.5]octane-6-carboxamide.

**7.** A process for preparing the compound of the formula Ia and/or a stereoisomeric form of the compound of the formula Ia and/or a physiologically acceptable salt of the compound of the formula Ia and/or a solvate or hydrate of the compound of the formula Ia and/or an N-oxide of the compounds of the formulae Ia as claimed in one or more of

claims 1 to 6, which comprises

a) reacting a compound of the formula II

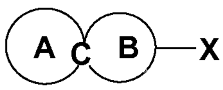

(II)

where A and B are each as defined in the compound of the formula Ia with a compound of the formula IIIa or IIIb or IIIc

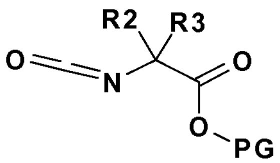

(IIIa)

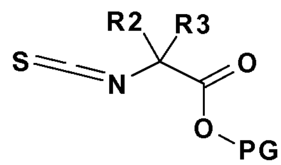

(IIIb)

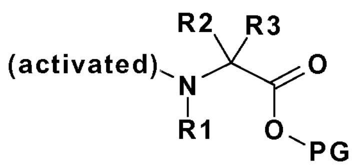

(IIIc)

where X, R1, R2 and R3 are each as defined in the compound of the formula Ia, PG is an ester protecting group and "activated" means that the amine is present in an activated form, for example as a chlorocarbonyl compound, to give a compound of the formula IVa or IVb

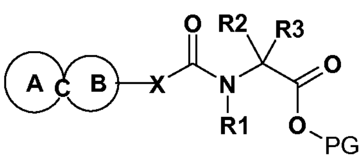

(IVa)

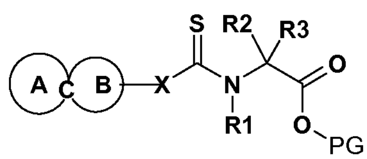

(IVb)

and reacting the resulting compounds of the formula IVa or IVb, after converting the ester to the carboxylic acid, with Z to give the compound of the formula Ia, or

b) reacting a compound of the formula Va or Vb where A, B, X and Y are each as defined in the compound of the formula Ia

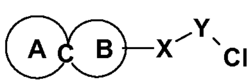

(Va)

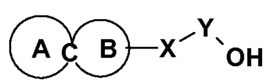

(Vb)

with a compound of the formula VI where R1, R2 and R3 are each as defined in the compound of the formula Ia and PG is an ester protecting group

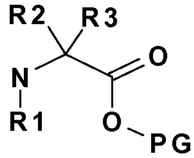

(VI)

to give a compound of the formula IVa or IVb, and reacting the resulting compound of the formula IVa or IVb, after converting the ester protecting group to the carboxylic acid, with Z to give the compound of the formula Ia, or

c) reacting a compound of the formula VIIa or VIIb where A, B and X are each as defined in the compound of the formula Ia

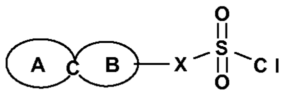 (VIIa) 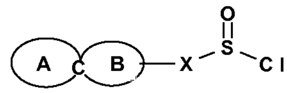 (VIIb)

with a compound of the formula VI

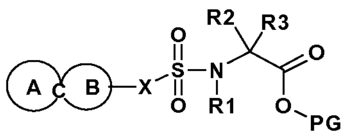 (VIIIa) 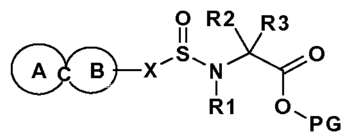 (VIIIb)

to give a compound of the formula VIIIa or VIIIb and reacting the resulting compound of the formula VIIIa or VIIIb, after converting the ester to the corresponding carboxylic acid, with Z to give the compound of the formula Ia, or

d) reacting a compound of the formula IX

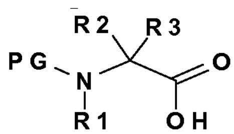 (IX)

with an amine Z where Z is as defined in the compound of the formula Ia to give a compound of the formula X

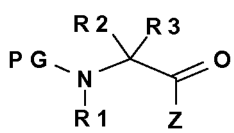 (X)

and then converting the compound X thus obtained in a protecting group elimination to give a compound of the formula XI

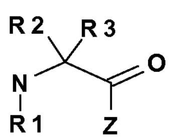 (XI)

and then reacting this compound XI with a compound Va or Vb, as detailed under b), to give the compound of the formula Ia, or

e) separating a compound of the formula Ia prepared by processes a), b), c) or d), or a suitable precursor of the formula Ia which, owing to its chemical structure, occurs in enantiomeric or diastereomeric forms, into the pure enantiomers or diastereomers by salt formation with enantiomerically pure acids or bases, chromatography on chiral stationary phases or derivatization by means of chiral enantiomerically pure compounds such as amino acids, separating the diastereomers thus obtained, and eliminating the chiral auxiliary groups, or

f) either isolating the compound of the formula Ia prepared by processes a), b), c) or d) in free form or releasing it from physiologically incompatible salts or, in the case of the presence or acidic or basic groups, converting it to physiologically acceptable salts, or

g) converting the compound of the formula Ia prepared by processes a), b), c) or d), or a suitable precursor of the formula Ia which, owing to its chemical structure, is capable of forming an N-oxide to an N-oxide or, in the case of the presence of an N-oxide, converting it to the free amine or the salt of an amine.

**8.** A medicament **characterized by** a compound of the formula Ia according to one or more of claims 1 to 6 together with a pharmaceutically acceptable and physiologically compatible carrier, additive and/or other active ingredients and/or excipients.

**9.** The use of the compound of the formula Ia as claimed in one or more of claims 1 to 6 for producing a medicament for the prophylaxis, secondary prevention and therapy of abnormally elevated bone degradation, allergies, Alzheimer's disease, amyloidosis, ARDS, arterial thrombosis, asthma, atheromas, atherosclerosis, autoimmune disorders, bacterial infections, bronchiolitis, cerebral hemorrhage, cerebrovascular ischemia, Huntington's chorea, chronic inflammations, CIPD (chronic inflammatory demyelinizing polyradiculoneuropathy), Creutzfeldt-Jakob disease, Crohn's disease, diabetes, particularly the juvenile form, emphysema, encephalomyelitis, endometriosis, inflammatory respiratory disorders, inflammatory pancreatitis, epilepsy, disorders **characterized by** enhanced angiogenesis, excessive respiratory pathway elastolysis, tissue grafts, gingivitis, glomerulonephritis, glucocorticoid-induced osteoporosis, Graves' disease, Guillain-Barre syndrome, Hashimoto's thyroiditis, hepatitis, HIV infection, Huntington's disease, hypercalcemia, IBD, immune impairment, interstitial cystitis, bone fracture, bone loss, cancers, lupus erythematosus, malaria, metachromic leukodystrophy, metastasizing osteogenesis, metastatis, multiple sclerosis, multiple myeloma, muscular dystrophy, myasthenia gravis, neurodegenerative disorders, neuropathic pain, chronic or diabetic neuropathy, post-herpetic neuralgia, trigeminal neuralgia, painful diabetic polyneuropathy, post-stroke pain, post-amputation pain, myelopathic or radiculopathic pain, atypical facial pain and causalgia-like syndromes, organ rejection in transplants, osteoarthritis, osteogenesis imperfecta, osteoporosis, Paget's disease, pancreatitis, Parkinson's disease, pemphigus vulgaris, periodontitis, plaque rupture, Pneumocystis carinii, pneumonitis, psoriasis, restenosis, rheumatoid arthritis, scleroderma, systemic lupus erythematosus, brain trauma, spinal cord trauma, tumor cell invasion, viral infections, tooth loss, breast cancer, intestinal cancer, ovarian cancer, cervical cancer, skin cancer, brain tumor, Kaposi's sarcoma, B- and T-cell leukemia, lung cancer, lymph node cancer, pancreatic cancer, prostrate cancer and sarcomas.

**Revendications**

**1.** Composé de formule Ia

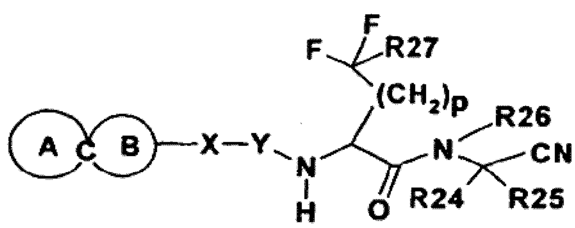

(Ia)

et/ou toutes les formes stéréo-isomères du composé de formule Ia et/ou les mélanges de ces formes, dans tous les rapports, et/ou un sel physiologiquement acceptable du composé de formule Ia, et/ou des solvates ou des hydrates du composé de formule Ia, où
le radical

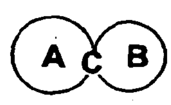

représente un composé spiro,
dans lequel les cycles partiels

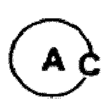

et

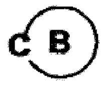

sont à chaque fois identiques ou différents et représentent, indépendamment l'un de l'autre

a) - ($C_3$-$C_{11}$) -cycloalkyle saturé ou partiellement saturé, cycloalkyle étant non ponté, ponté ou annelé et non substitué ou, indépendamment l'un de l'autre, en fonction de la taille du cycle, monosubstitué, disubstitué, trisubstitué, tétrasubstitué ou pentasubstitué par R4, ou
b) hétérocycle saturé ou partiellement saturé, à trois jusqu'à onze chaînons, qui, en fonction de la taille du cycle, peut contenir un, deux, trois ou quatre hétéroatomes identiques ou différents de la série oxygène, azote ou soufre et hétérocycle pouvant être non ponté, ponté ou annelé et non substitué ou, indépendamment l'un de l'autre, en fonction de la taille du cycle, monosubstitué, disubstitué, trisubstitué, tétrasubstitué ou pentasubstitué par R4

R4 représentant $-NO_2$, -CN, =O, =S, -OH, $-CF_3$, $-SF_5$, -($C_0$-$C_3$)-alkylène-S-R10, $-O-CF_3$, $-Si-(CH_3)_3$, -($C_0$-$C_5$)-alkylène-O-C(O)-R21, -($C_0$-$C_5$)-alkylène-C(O)-OR10, -($C_0$-$C_3$)-alkylène-O-R10, -($C_0$-$C_3$)-alkylène-N(R21)-R22, -($C_0$-$C_3$)-alkylène-N(R10)-S($O_2$)-R10, -($C_0$-$C_5$)-alkylène-($C_3$-$C_8$)-cycloalkyl-R23, $-S-CF_3$, -($C_0$-$C_5$)-alkylène-($C_1$-$C_3$)-fluoroalkyle, -($C_0$-$C_5$)-alkylène-N(R10)-C(O)-R21, -($C_0$-$C_3$)-alkylène-C(O)-N(R21)-R22, -($C_0$-$C_4$)-alkyle, alkyle étant non substitué ou monosubstitué, disubstitué ou trisubstitué, indépendamment l'un de l'autre, par R9, -($C_0$-$C_4$)-alkylène-aryle, aryle étant choisi dans le groupe phényle, indanyle, indényle, naphtyle, aryle étant non substitué ou monosubstitué, disubstitué ou trisubstitué, indépendamment l'un de l'autre, par R8, ou - ($C_0$-$C_4$)-alkylène-Het, Het étant choisi dans le groupe azétidinyle, benzimidazolinyle, benzimidazolyle, benzofurannyle, benzothiophényle, benzoxazolyle, benzothiazolyle, benzisoxazolyle, benzisothiazolyle, quinoléinyle, dioxolyle, dioxanyle, furannyle, imidazolidinyle, imidazolinyle, imidazolyle, indolinyle, indolyle, 3H-indolyle, iso-indolinyle, iso-indolyle, isoquinoléinyle, isothiazolidinyle, 2-isothiazolinyle, isothiazolyle, isoxazolyle, isoxazolidinyle, 2-isoxazolinyle, morpholinyle, octahydro-isoquinoléinyle, oxazolyle, oxazolidinyle, pyrimidinyle, pipérazinyle, pipéridinyle, pyrannyle, pyrazinyle, pyrazolinyle, pyrazolyle, pyridazinyle, pyridyle, pyrimidinyle, pyrrolidinyle, pyrrolinyle, 2H-pyrrolyle, pyrrolyle, tétrahydrofurannyle, tétrahydro-isoquinoléinyle, tétrahydroquinoléinyle, tétrahydropyridinyle, thiazolyle, thiényle, thiénopyridinyle, thiomorpholinyle, thiophényle, et ce radical Het étant non substitué ou monosubstitué, disubstitué ou trisubstitué, indépendamment l'un de l'autre, par R8,
R8 représentant halogène, carbamimidoyle, $-NO_2$, =0, $-CF_3$, $-SF_5$, -C(O)-O-R10, -CN, $-C(O)-NH_2$, -OH, $-NH_2$, $-O-CF_3$, -C(O)-N(R10)-R20, -N(R10)-R20, -($C_3$-$C_8$)-cycloalkyle, -O-($C_1$-$C_8$)-alkyle, -O-($C_0$-$C_4$)-alkylène-($C_3$-$C_6$)-cycloalkyle, -($C_1$-$C_8$)-alkyle, -($C_0$-$C_4$)-alkylène-($C_3$-$C_8$)-cycloalkyle, les radicaux alkyle mentionnés étant à chaque fois non substitués ou monosubstitués, disubstitués ou trisubstitués, indépendamment l'un de l' autre, par halogène, $NH_2$, -OH, $-O-CH_3$, $-SO_2-CH_3$ ou $-SO_2-CF_3$,
R9 représentant halogène, $-NO_2$, -CN, =O, -OH, $-CF_3$, -C(O)-O-R10, -C(O)-N(R21)-R22, -N(R21)-R22, -($C_3$-$C_8$)-cycloalkyle, -($C_0$-$C_3$)-alkylène-O-R10, $-Si-(CH_3)_3$, N(R10)-S$(O)_u$-R10, u signifiant le nombre entier 1 ou 2, -S-R10, $-SO_r$-R10, r signifiant le nombre entier 1 ou 2, -S$(O)_v$-N(R10)-R20, v représentant le nombre entier 1 ou 2, -C(O)-R10, -($C_1$-$C_8$)-alkyle, -($C_1$-$C_8$)-alcoxy, phényle, phényloxy-, -($C_1$-$C_3$)-fluoroalkyle, -O-R19, -NH-C(O)-NH-R10, -($C_0$-$C_4$)-alkyl-C(O)-O-C(R11,R19)-OC(O)-R12, -NH-C(O)-NH-R21, -N(R21)-C(O)-R22, -($C_0$-$C_4$)-alkyl-C(O)-O-C(R11, R19)-O-C(O)-O-R12, -NH-C(O)-O-R10, $-O-CF_3$ ou Het, Het étant tel que défini ci-dessus et non substitué ou monosubstitué, disubstitué ou trisubstitué, indépendamment l'un de l'autre, par R8,
R10 et R20 étant identiques ou différents et représentant, indépendamment l'un de l'autre, un atome d'hydrogène, -($C_1$-$C_6$)-alkyle, -($C_0$-$C_4$)-alkyl-OH, -($C_1$-$C_3$)-fluoroalkyle, -($C_0$-$C_4$)-alkyl-O-($C_1$-$C_4$) - alkyle, -($C_0$-$C_5$)-alkyl-($C_3$-$C_8$)-cycloalkyle, -($C_0$-$C_2$)-alkylène-aryle, aryle étant défini comme ci-dessus et non substitué ou monosubstitué, disubstitué ou trisubstitué, indépendamment l'un de l' autre, par -($C_1$-$C_6$)-alkyle, -O-($C_1$-$C_6$)-alkyle, halogène ou -($C_3$-$C_8$)-cycloalkyle, ou -($C_0$-$C_2$)-alkylène-Het, Het étant défini comme ci-dessus, et non substitué ou monosubstitué, disubstitué ou trisubstitué, indépendamment l'un de l'autre, par -($C_1$-$C_6$)-alkyle, -O-($C_1$-$C_6$)-alkyle, halogène ou -($C_3$-$C_8$))-cycloalkyle,
R11 et R19 étant identiques ou différents et représentant, indépendamment l'un de l'autre, un atome d'hydrogène ou - ($C_1$-$C_6$)-alkyle,
R12 représentant -($C_1$-$C_6$)-alkyle, -($C_1$-$C_6$)-alkyl-OH, - ($C_1$-$C_6$)-alkyl-O-($C_1$-$C_6$)-alkyle, -($C_3$-$C_8$)-cycloalkyle, -($C_1$-$C_6$)-alkyl-O-($C_1$-$C_8$)-alkyl-($C_3$-$C_8$)-cycloalkyle, -($C_1$-$C_6$)-alkyl-($C_3$-$C_8$)-cycloalkyle, le radical cycloalkyle étant non substitué ou monosubstitué, disubstitué ou trisubstitué, indépendamment l'un de l'autre, par

-OH, -O-($C_1$-$C_4$)-alkyle ou R10,

R21 et R22 étant identiques ou différents et représentant, indépendamment l'un de l'autre, un atome d'hydrogène, -($C_1$-$C_6$)-alkyle, alkyle étant non substitué ou monosubstitué, disubstitué ou trisubstitué, indépendamment l'un de l'autre, par R8, -($C_0$-$C_6$)-alkylène-($C_3$-$C_8$)-cycloalkyle, -$SO_t$-R10, t signifiant le nombre entier 1 ou 2, -($C_1$-$C_3$)-fluoroalkyle, -O-R12, -($C_0$-$C_6$)-alkylène-aryle, aryle étant défini comme ci-dessus et alkylène et aryle non substitués ou monosubstitués, disubstitués ou trisubstitués, indépendamment l'un de l' autre, par R8 ou -($C_0$-$C_6$)-alkylène-Het, Het étant défini comme ci-dessus et alkylène et Het étant non substitués ou monosubstitués, disubstitués ou trisubstitués, indépendamment l'un de l'autre, par R8,

R21 et R22 représentant, ensemble avec l'atome d'azote auquel ils sont liés, un hétérocycle monocyclique à quatre jusqu'à huit chaînons, qui peut contenir, en plus de l'atome d'azote, en outre encore, en fonction de la taille du cycle, un ou deux hétéroatomes identiques ou différents de la série oxygène, azote ou soufre et l'hétérocycle étant non substitué ou monosubstitué, disubstitué ou trisubstitué, indépendamment l'un de l'autre, par R8,

R23 représentant un atome d'hydrogène, -OH ou -O-($C_1$-$C_4$)-alkyle,

X représente une liaison covalente, -N(R7)- ou -O-,

R7 représentant un atome d'hydrogène, -($C_0$-$C_4$)-alkylène-($C_3$-$C_6$)-cycloalkyle ou -($C_1$-$C_4$)-alkyle,

Y représente -C(O)-, -C(S)- ou -S($O_2$)-,

p représente le nombre entier 1 ou 2,

R27 représente un atome d'hydrogène, ($C_1$-$C_6$)-alkyle, halogène, -($C_0$-$C_4$)-alkylène-($C_3$-$C_6$)-cycloalkyle, -($C_0$-$C_4$)-alkylène-Het, Het étant défini comme ci-dessus et non substitué ou substitué par halogène, -($C_1$-$C_6$)-alkyle, -O-($C_1$-$C_3$)-fluoroalkyle ou -O-($C_1$-$C_6$)-alkyle, ou -($C_0$-$C_2$)-alkylène-phényle, phényle étant non substitué ou substitué par halogène, -($C_1$-$C_6$)-alkyle, -O-($C_1$-$C_3$)-fluoroalkyle ou -O-($C_1$-$C_6$)-alkyle,

R26 représente un atome d'hydrogène, -($C_0$-$C_4$)-alkylène-($C_3$-$C_6$)-cycloalkyle ou - ($C_1$-$C_4$)-alkyle,

R24 et R25 sont identiques ou différents et représentent, indépendamment l'un de l'autre, un atome d'hydrogène, -($C_1$-$C_6$)-alkyle, -($C_1$-$C_3$)-fluoroalkyle, -($C_0$-$C_4$)-alkylène-($C_3$-$C_6$)-cycloalkyle, -($C_0$-$C_4$)-alkylène-aryle, aryle étant défini comme ci-dessus et non substitué ou monosubstitué, disubstitué ou trisubstitué, indépendamment l'un de l' autre, par R8, ou - ($C_0$-$C_4$)-alkylène-Het, Het étant défini comme ci-dessus et non substitué ou monosubstitué, disubstitué ou trisubstitué, indépendamment l'un de l'autre, par R8,

R24 et R25 formant ensemble avec l'atome de carbone auquel ils sont liés, cycloalkyle de trois à six chaînons, qui est non substitué ou monosubstitué, disubstitué ou trisubstitué, indépendamment l'un de l'autre, par R10 ou fluor,

R24 et R25 formant ensemble avec l'atome de carbone auquel ils sont liés, un radical hétérocycloalkyle de trois à six chaînons, qui est non substitué ou monosubstitué, disubstitué ou trisubstitué, indépendamment l'un de l'autre, par R10 ou fluor.

**2.** Composé de formule I selon la revendication 1, où le cycle partiel

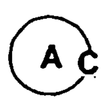

ayant été choisi dans le groupe suivant

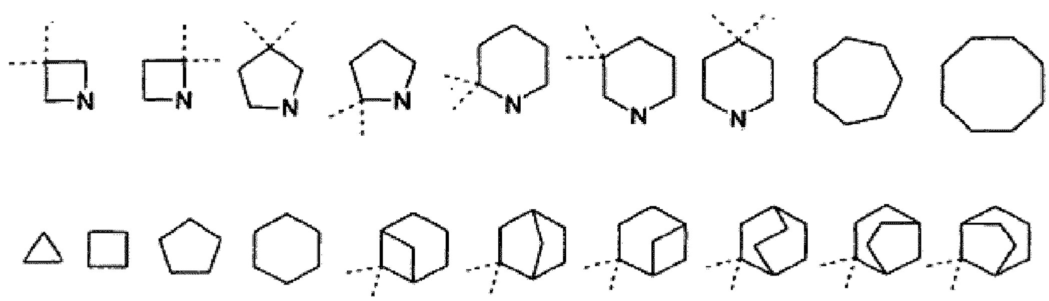

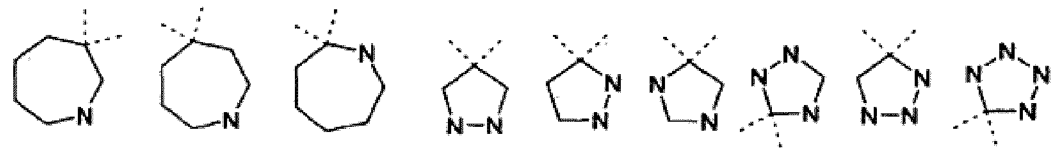

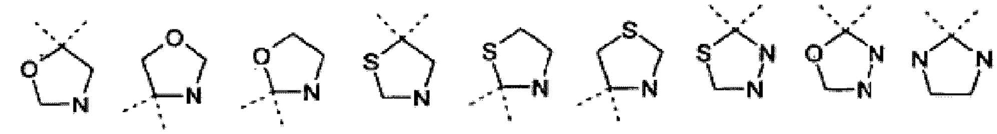

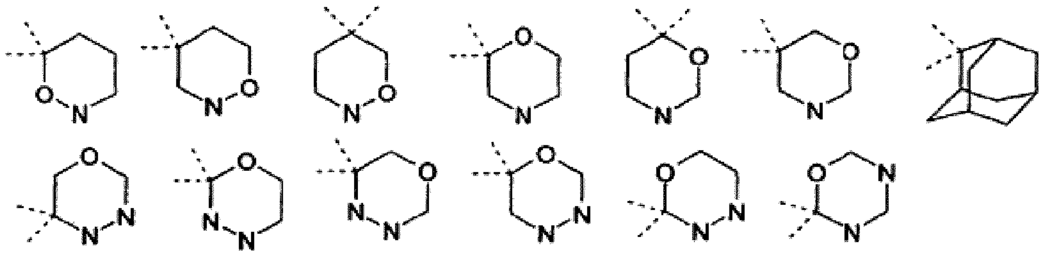

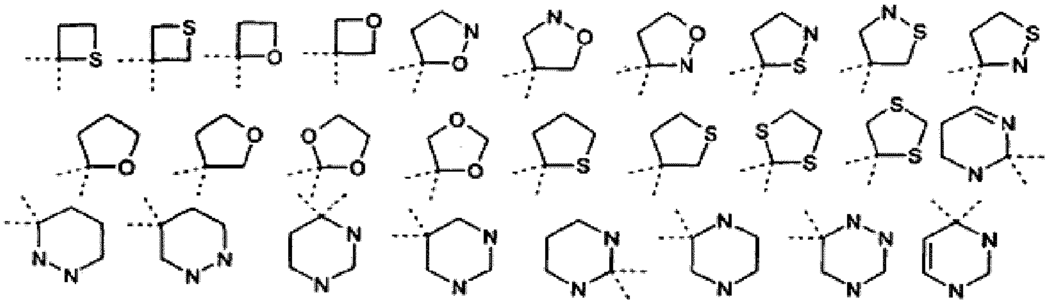

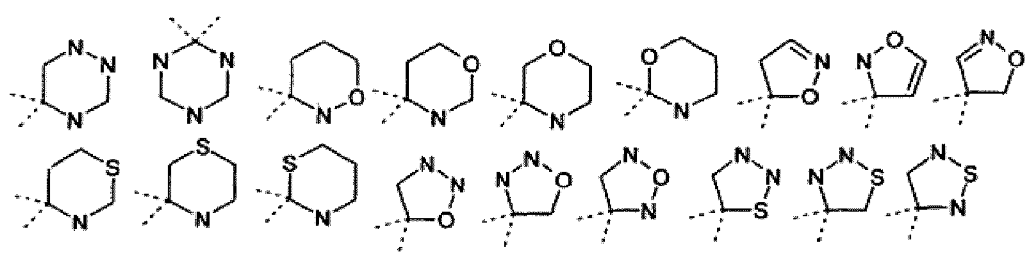

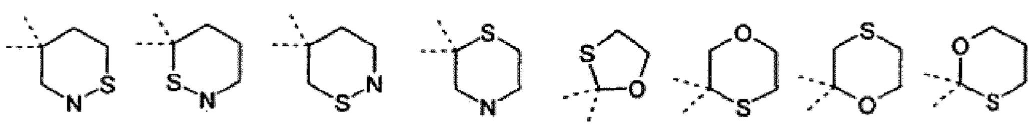

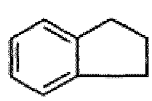

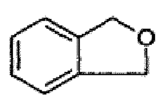

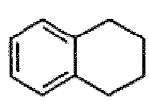

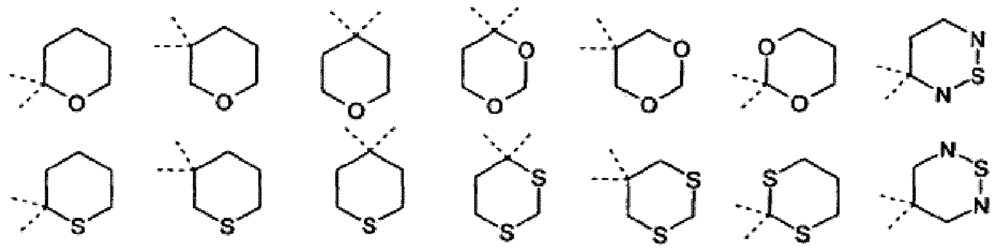

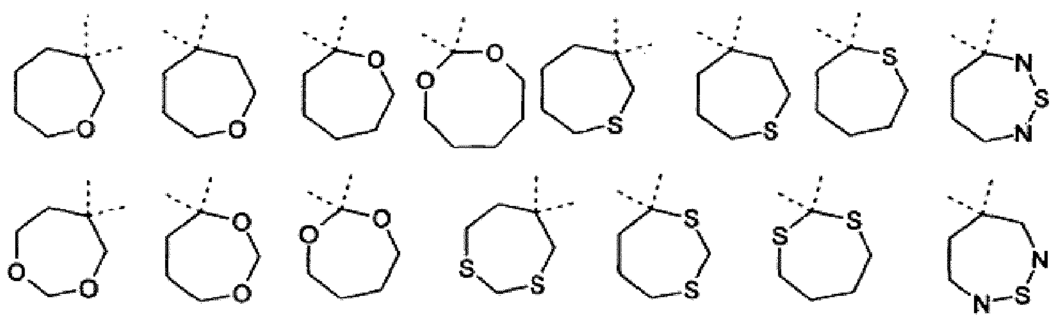

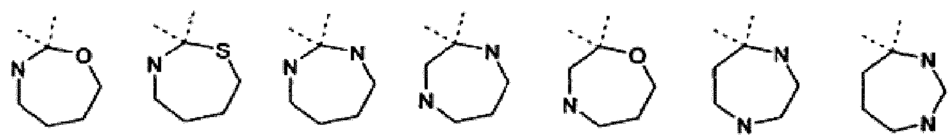

les lignes en pointillés représentant à chaque fois le point de liaison au deuxième cycle partiel, des liaisons simples dans les structures indiquées pouvant en partie être remplacées par les doubles liaisons ou d'autres systèmes cycliques pouvant être condensés, et
le cycle partiel

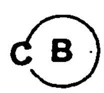

ayant été choisi dans le groupe suivant

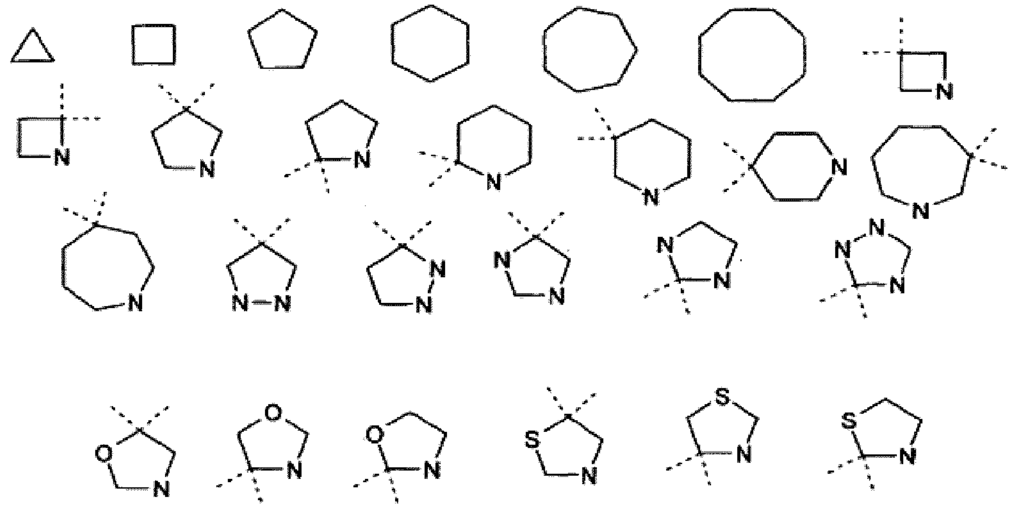

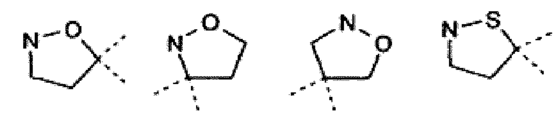

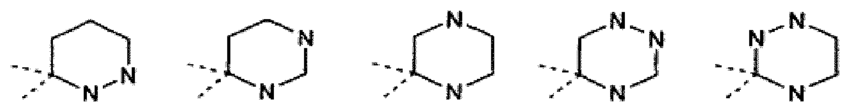

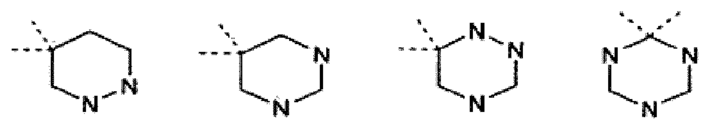

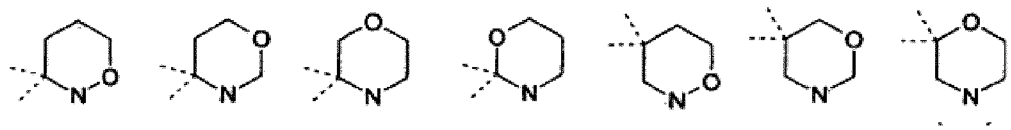

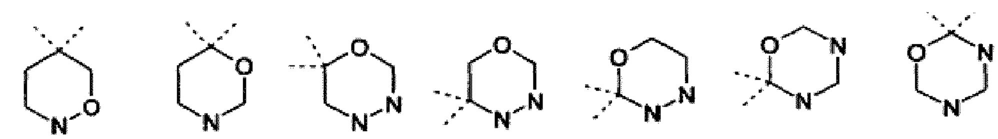

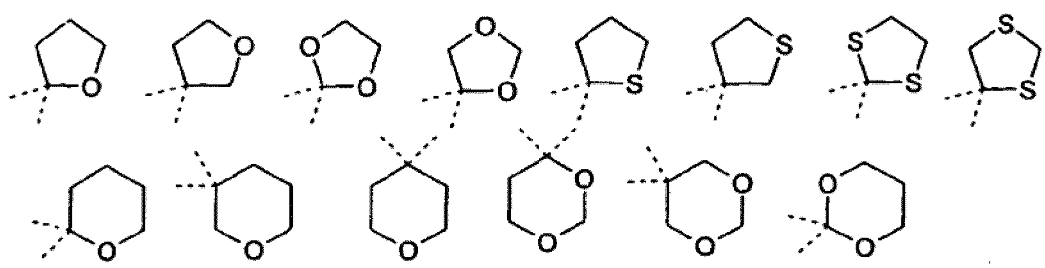

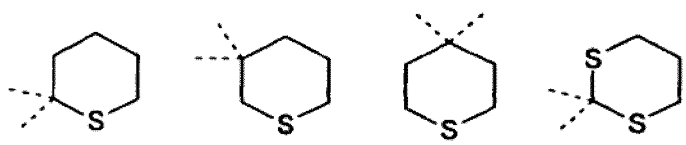

les lignes en pointillés représentant à chaque fois le point de liaison au deuxième cycle partiel, des liaisons simples dans les structures indiquées pouvant en partie être remplacées par des doubles liaisons et les deux cycles partiels A et B pouvant être non substitués ou monosubstitués à tétrasubstitués, indépendamment l'un de l'autre, par R4, et les radicaux X, Y, R27, p, R26, R24, R25 et R4 étant définis comme dans la revendication 1.

**3.** Composé de formule la selon la revendication 1, les cycles partiels

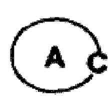

et

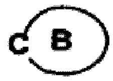

ayant à chaque fois été choisis parmi cyclopropane, cyclobutane, cyclopentane, cyclohexane, cycloheptane, cyclooctane, bicyclo[4,2,0]octane, octahydro-indène, décaline, décahydrobenzocycloheptène, dodécahydroheptalène, bicyclo[3,1,1]heptane, bicyclo[2,2,1]heptane, bicyclo[3,3,0]octane, bicyclo[2,2,2]octane, spiro[2,5]octane, spiro [3,4]octane, azépane, azépine, azétidine, aziridine, azirine, azocane, benzimidazoline, 2,3-dihydrobenzo[b]thiophène, 1,3-dihydrobenzo[c]thiophène, 2,3-dihydrobenzofuranne, 2,3-dihydrobenzooxazole, 2,3-dihydrobenzothiazole, 1,3-dihydro-isobenzofuranne, 4,5-dihydro-isothiazole, 2,3-dihydro-isoxazole, 2,5-dihydro-isoxazole, 4,5-dihydro-isoxazole, 5,6-dihydro-4H[1,2]oxazine, benzo[1,3]dioxole, 1,4-diazépane, 1,2-diazépine, 1,3-diazépine, 1,4-diazépine, diaziridine, diazirine, 1,4-diazocane, dioxane, 1,3-dioxane, dioxazine, [1,3]dioxépane, 1,4-diozocane, dioxole, dioxolane, 1,3-dioxolane, 1,3-dioxolène, [1,3]dithiane, [1,3]dithiolane, hexahydropyridazine, hexahydropyrimidine, imidazoline, imidazolidine, indane, indoline, iso-indoline, isothiazolidine, isothiazoline, isoxazoline, isoxazolidine, 2-isoxazoline, morpholine, [1,3,4]oxadiazinane, [1,3,5]oxadiazinane, [1,2,3]oxadiazolidine, [1,3,4]oxadiazolidine, 1,2-oxathiépane, 1,2-oxathiolane, [1,3]oxathiolane, 1,4-oxazépane, 1,2-oxazine, 1,3-oxazine, 1,4-oxazine, oxazinane, 1,3-oxazinane, oxazocane, oxaziridine, oxazolidine, oxépane, oxétane, oxirane, oxocane, pipérazine, pipéridine, pyranne, pyrazoline, pyrazolidine, pyrrolidine, pyrrolidinone, pyrroline, tétrahydroquinoléine, tétrahydrofuranne, tétrahydro-isoquinoléine, 1,2,3,4-tétrahydronaphtalène, tétrahydropyranne, tétrahydropyridine, 1,2,3,4-tétrahydropyrimidine, 1,2,5,6-tétrahydropyrimidine, tétrahydrothiophène, tétrazine, thiadiazine, [1,2,6]thiadiazinane, [1,3,4]thiadiazolidine, 1,2-thiazine, 1,3-thiazine, 1,4-thiazine, [1,2]thiazinane, [1,3]thiazinane, thiazolidine, thiazoline, thiépane, thiétane, thiomorpholine, thiopyranne, 1,2,3-triazine, 1,2,4-triazine, 1,3,5-triazine, [1,2,4]triazinane ou [1,2,4]triazolidine, et les deux cycles partiels étant à chaque fois non substitués ou, indépendamment l'un de l'autre, en fonction de la taille du cycle, monosubstitués, disubstitués, trisubstitués, tétrasubstitués ou pentasubstitués par R4, et les radicaux X, Y, R27, p, R26, R24, R25 et R4 étant définis comme dans la revendication 1.

**4.** Composé de formule (Ia) selon la revendication 1, les cycles partiels

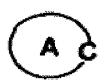

et

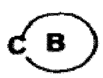

étant choisis, indépendamment l'un de l'autre, à chaque fois dans le groupe azétidine, cyclopropyle, cyclobutyle, cyclopentyle, cyclohexyle, 1,3-dihydro-isobenzofuranne, 2,3-dihydro-isoxazole, 2,5-dihydro-isoxazole, 4,5-dihydro-isoxazole, 1,3-dioxane, dioxolane, 1,3-dioxolane, imidazolidine, indane, morpholine, 1,3-oxazinane, oxazolidine, pipérazine, pipéridine, pyrrolidine, tétrahydrofuranne, et 1,2,3,4-tétrahydronaphtalène,
et les deux cycles partiels étant non substitués ou, indépendamment l'un de l'autre, en fonction de la taille du cycle, monosubstitués, disubstitués ou trisubstitués par R4,

R4 représentant =O, =S, -($C_0$-$C_3$)-alkylène-C(O)-O-R10, - ($C_0$-$C_3$) -alkylène-N (R21) -R22, - ($C_0$-$C_3$) -alkylène-NH-C(O)-R21, -($C_0$-$C_4$)-alkylène-($C_3$-$C_6$)-cycloalkyl-R23,- ($C_0$-$C_3$)-alkylène-O-R10, - ($C_0$-$C_4$)-alkylène-phényle, phényle étant non substitué ou monosubstitué, disubstitué ou trisubstitué indépendamment l'un de l' autre par R8, ou -($C_0$-$C_4$)-alkyle, alkyle étant non substitué ou monosubstitué, disubstitué ou trisubstitué, indépendamment l'un de l'autre, par R9,
R8 représentant fluor, chlore, brome, -O-($C_1$-$C_3$)-fluoroalkyle ou -O-($C_1$-$C_4$)-alkyle,
R9 représentant halogène, -$NO_2$, -CN, =0, -OH, -$CF_3$, -C(O)-O-R10, -C(O)-N(R21)-R22, -N(R21)-R22, -($C_3$-$C_8$)-cycloalkyle, -($C_0$-$C_3$)-alkylène-O-R10, -Si-$(CH_3)_3$, N(R10)-$S(O)_u$-R10, u signifiant le nombre entier 1 ou 2, -S-R10, -$SO_r$-R10, r signifiant le nombre entier 1 ou 2, -$S(O)_v$-N(R10)-R20, v représentant le nombre entier 1 ou 2, -C(O)-R10, - ($C_1$-$C_8$)-alkyle, -($C_1$-$C_8$)-alcoxy, phényle, phényloxy-, - ($C_1$-$C_3$) -fluoroalkyle, -O-R19, -NH-C(O)-NH-R10, - ($C_0$-$C_4$)-alkyl-C(O)-O-C(R11, R19)-OC(O)-R12, -NH-C(O)-NH-R21, -N(R21)-C (O)-R22, -($C_0$-$C_4$)-alkyl-C(O)-O-C(R11, R19)-O-C(O)-O-R12, -NH-C(O)-O-R10 ou -O-$CF_3$,
R10 et R20 étant identiques ou différents et représentant, indépendamment l'un de l'autre, un atome d'hydrogène

ou - $(C_1-C_6)$-alkyle,
R11 et R19 étant identiques ou différents et représentant, indépendamment l'un de l'autre, un atome d'hydrogène ou -$(C_1-C_6)$-alkyle,
R12 représentant -$(C_1-C_6)$-alkyle, -$(C_1-C_6)$-alkyl-OH, -$(C_1-C_6)$-alkyl-O-$(C_1-C_6)$ -alkyle, -$(C_3-C_8)$-cycloalkyle, -$(C_1-C_6)$-alkyl-O-$(C_1-C_8)$-alkyl-$(C_3-C_8)$-cycloalkyle, $(C_1-C_6)$-alkyl-$(C_3-C_8)$-cycloalkyle, le radical cycloalkyle étant non substitué ou monosubstitué, disubstitué ou trisubstitué, indépendamment l'un de l'autre, par -OH, -O-$(C_1-C_4)$-alkyle ou R10,
R21 et R22 étant identiques ou différents et représentant, indépendamment l'un de l'autre, un atome d' hydrogène, - $(C_1-C_6)$-alkyle, -O-R12, -$(C_0-C_6)$-alkylène-$(C_3-C_8)$-cycloalkyle,- $SO_t$-R10, t signifiant le nombre entier 1 ou 2, ou -$(C_1-C_3)$-fluoroalkyle,
R23 représentant un atome d'hydrogène, -OH ou -O-$(C_1-C_4)$-alkyle,
X représentant une liaison covalente ou -N(R7)-,
R7 représentant un atome d'hydrogène ou - $(C_1-C_4)$ - alkyle,
Y représentant -C(O)- ou -$S(O_2)$-,
p représentant le nombre entier 1 ou 2,
R26 représentant un atome d'hydrogène,
R27 représentant un atome d'hydrogène, -$(C_0-C_4)$-alkylène-$(C_3-C_6)$-cycloalkyle, -$(C_1-C_6)$-alkyle, -$(C_0-C_2)$-alkylène-phényle, phényle étant non substitué ou substitué par halogène, - $(C_1-C_6)$ -alkyle, -0- $(C_1-C_3)$-fluoroalkyle ou -O-$(C_1-C_6)$-alkyle, ou - $(C_0-C_2)$-alkylène-pyridyle,
R24 et R25 étant identiques ou différents et représentant, indépendamment l'un de l'autre, un atome d'hydrogène, -$(C_1-C_4)$-alkyle ou -$(C_0-C_4)$-alkylène-$(C_3-C_6)$-cycloalkyle,
R24 et R25 formant ensemble avec l'atome de carbone auquel ils sont liés, cycloalkyle, choisi dans le groupe cyclopropyle, cyclobutyle, cyclopentyle ou cyclohexyle, qui est non substitué ou monosubstitué, disubstitué ou trisubstitué, indépendamment l'un de l'autre, par R10 ou fluor,
R24 et R25 formant ensemble avec l'atome de carbone auquel ils sont liés un radical hétérocycloalkyle de trois à six chaînons, choisi dans le groupe aziridine, azétidine, diazétidine, diaziridine, hexahydropyridazine, hexahydropyrimidine, imidazolidine, morpholine, oxadiazinane, oxadiazolidine, oxathianane, oxathiolane, oxazétidine, oxazolidine, oxétane, oxirane, pipérazine, pipéridine, pyrazolidine, pyrrolidine, tétrahydrofuranne, tétrahydropyranne, tétrahydrothiophène, tétrahydrothiopyranne, tétrazinane, thiadiazolidine, thiazétidine, thiaziridine, thiazolidine, thiétane, thiirane, thiomorpholine, triazétidine, triazinane ou triazolidine, qui est non substitué ou monosubstitué, disubstitué ou trisubstitué indépendamment l'un de l'autre par R10 ou fluor.

**5.** Composés de formule Ia selon l'une ou plusieurs des revendications 1 à 4, le cycle partiel

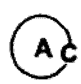

étant choisi dans le groupe azétidine, cyclopropyle, cyclobutyle, cyclopentyle, cyclohexyle, 1,3-dihydro-isobenzofuranne, 1,3-dioxane, 1,3-dioxolane, imidazolidine, indane, morpholine, 1,3-oxazinane, pipérazine, pipéridine, pyrrolidine, tétrahydrofuranne, et 1,2,3,4-tétrahydronaphtalène,
le cycle partiel

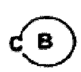

étant choisi dans le groupe azétidine, cyclopropyle, cyclopentyle, cyclohexyle, morpholine, oxazolidine, pipéridine et pyrrolidine, et les deux cycles partiels étant non substitués ou, indépendamment l'un de l'autre, en fonction de la taille du cycle, monosubstitués, disubstitués ou trisubstitués par R4,

R4 représentant -0- $(C_1-C_4)$ -alkyle, =O, -$(C_0-C_4)$-alkylène-$(C_3-C_6)$-cycloalkyle, -$(C_1-C_4)$-alkyle ou -$(C_0-C_4)$-alkylène-phényle, phényle étant non substitué ou substitué par F, Cl, Br ou -O-$(C_1-C_4)$-alkyle,
X représentant une liaison covalente ou -NH-,
Y représentant -C(O)- ou -$S(O_2)$-,
p représentant le nombre entier 1,
R27 représentant un atome d'hydrogène, -$(C_1-C_6)$-alkyle, 4-F-benzyle ou benzyle,
R26 représentant un atome d'hydrogène,

R24 et R25 étant identiques ou différents et représentant, indépendamment l'un de l'autre, un atome d'hydrogène, méthyle ou éthyle,

R24 et R25 formant ensemble avec l'atome de carbone auquel ils sont liés un radical cyclopropyle ou cyclobutyle, ou

R24 et R25 formant ensemble avec l'atome de carbone auquel ils sont liés un cycle pipéridine, qui est non substitué ou substitué par -($C_1$-$C_4$)-alkyle.

**6.** Composé de formule Ia selon l'une ou plusieurs des revendications 1 à 5, **caractérisé en ce qu'**il s'agit du composé [(S)-1-(1-cyanocyclopropylcarbamoyl)-3,3-difluorobutyl]-amide de l'acide 3-azaspiro[5,5]undécane-3-carboxylique, [(S)-1-(1-cyanocyclopropylcarbamoyl)-3,3-difluorobutyl]-amide de l'acide 8-azaspiro[4,5]décane-8-carboxylique, [(S)-1-(1-cyanocyclopropylcarbamoyl)-3,3-difluorobutyl]-amide de l'acide 1,4-dioxa-8-azaspiro[4,5]décane-8-carboxylique, [(S)-1-(1-cyanocyclopropylcarbamoyl)-3,3-difluorobutyl]-amide de l'acide 2-azaspiro[5,5]undécane-2-carboxylique, [(S)-1-(1-cyanocyclopropylcarbamoyl)-3,3-difluorohexyl]-amide de l'acide 8-azaspiro[4,5]décane-8-carboxylique, [(S)-1-(1-cyanocyclopropylcarbamoyl)-3,3-difluorohexyl]-amide de l'acide 3-azaspiro[5,5]undécane-3-carboxylique, [(S)-1-(1-cyanocyclopropylcarbamoyl)-3,3-difluorobutyl]-amide de l'acide 2-(4-méthoxyphényl)-1-oxo-2,8-diazaspiro[4,5]décane-8-carboxylique, [(S)-1-(1-cyanocyclopropylcarbamoyl)-3,3-difluorobutyl]-amide de l'acide 4-oxo-1-phényl-1,3,8-triazaspiro[4,5]décane-8-carboxylique, [(S)-1-(1-cyanocyclopropylcarbamoyl)-3,3-difluorobutyl]-amide de l'acide 1,5-dioxa-9-azaspiro[5,5]undécane-9-carboxylique, [(S)-1-(1-cyanocyclopropylcarbamoyl)-3,3-difluorobutyl]-amide de l'acide 1-oxo-2,8-diazaspiro[4,5]décane-8-carboxylique, [(S)-1-(1-cyanocyclopropylcarbamoyl)-3,3-difluorobutyl]-amide de l'acide 2-méthyl-1-oxo-2,8-diazaspiro[4,5]décane-8-carboxylique, [(S)-1-(1-cyanocyclopropylcarbamoyl)-3,3-difluorobutyl]-amide de l'acide 3,3-diméthyl-1-oxa-5,9-diazaspiro[5,5]undécane-9-carboxylique, [(S)-1-(1-cyanocyclopropylcarbamoyl)-3,3-difluoro-4-phénylbutyl]-amide de l'acide 8-azaspiro[4,5]décane-8-carboxylique, [(S)-1-(1-cyanocyclopropylcarbamoyl)-3,3-difluorobutyl]-amide de l'acide 2,4-dioxo-1,3,8-triazaspiro[4,5]décane-8-carboxylique, [(S)-1-(1-cyanocyclopropylcarbamoyl)-3,3-difluorobutyl]-amide de l'acide 2-azaspiro[4,4]nonane-2-carboxylique, [(S)-1-(1-cyanocyclopropylcarbamoyl)-3,3-difluorobutyl]-amide de l'acide 2-benzyl-1-oxo-2,8-diazaspiro[4,5]décane-8-carboxylique, [(S)-1-(1-cyanocyclopropylcarbamoyl)-3,3-difluorobutyl]-amide de l'acide 2-(4-fluorophényl)-1-oxo-2,8-diazaspiro[4,5]décane-8-carboxylique, [(S)-1-(1-cyanocyclopropylcarbamoyl)-3,3-difluorobutyl]-amide de l'acide 3-phényl-1,5-dioxa-9-azaspiro[5,5]undécane-9-carboxylique, [(S)-1-(1-cyanocyclopropylcarbamoyl)-3,3-difluorobutyl]-amide de l'acide 9-butyl-3,9-diazaspiro[5,5]undécane-3-carboxylique, [(S)-1-(1-cyanocyclopropylcarbamoyl)-3,3-difluorobutyl]-amide de l'acide 9-cyclopropyl-3,9-diazaspiro[5,5]undécane-3-carboxylique, [(S)-1-(1-cyanocyclopropylcarbamoyl)-3,3-difluorobutyl]-amide de l'acide spiro[2,3]hexane-1-carboxylique, [(S)-1-(1-cyanocyclopropylcarbamoyl)-3,3-difluorobutyl]-amide de l'acide 2,2-diméthyl-1-oxa-8-azaspiro[4,5]décane-8-carboxylique, [(S)-1-(1-cyanocyclopropylcarbamoyl)-3,3-difluorobutyl]-amide de l'acide 2-azaspiro[4,5]décane-2-carboxylique,

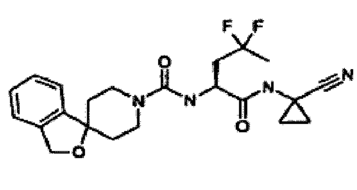 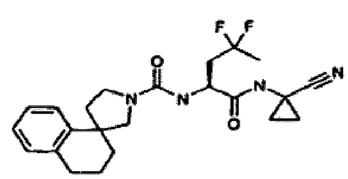

[(S)-1-(1-cyanocyclopropylcarbamoyl)-3,3-difluorobutyl]-amide de l'acide 1-oxa-4-azaspiro[4,5]décane-4-carboxylique, (1-cyanocyclopropyl)-amide de l'acide (S)-2-[3-(1,4-dioxaspiro[4,5]déc-8-yl)-uréido]-4,4-difluoropentanoïque,

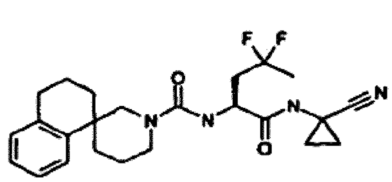 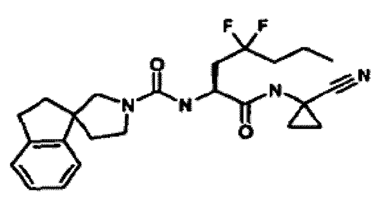

[(S)-1-(1-cyanocyclopropylcarbamoyl)-3,3-difluorobutyl]-amide de l'acide 7-cyclopropyl-2,7-diazaspiro[3,5]nonane-2-carboxylique, [(S)-1-(1-cyanocyclopropylcarbamoyl)-3,3-difluorobutyl]-amide de l'acide 2-cyclopropyl-2,7-diazaspiro[3,5]nonane-7-carboxylique, [(S)-1-(1-cyanocyclopropylcarbamoyl)-3,3-difluorobutyl]-amide de l'acide 2-propyl-2,7-diazaspiro[3,5]nonane-7-carboxylique, (1-cyanocyclopropyl)-amide de l'acide (S)-2-(8-azaspiro[4,5]décane-8-sulfonylamino)-4,4-difluoropentanoïque, [(S)-1-(1-cyanocyclopropylcarbamoyl)-3,3-difluorobutyl]-amide de l'acide 4-cyclopropyl-1-oxa-4,9-diazaspiro[5,5]undécane-9-carboxylique, [(S)-1-(1-cyanocyclopropylcarbamoyl)-3,3-difluorobutyl]-amide de l'acide 9-cyclopropyl-1-oxa-4,9-diazaspiro[5,5]undécane-4-carboxylique, [(S)-1-(1-cyanocyclopropylcarbamoyl)-3,3-difluorobutyl]-amide de l'acide 2-cyclopropylméthyl-3-oxo-2,8-diazaspiro[4,5]décane-8-carboxylique, [(S)-1-(1-cyanocyclopropylcarbamoyl)-3,3-difluorohexyl]-amide de l'acide 2-(4-méthoxyphényl)-1-oxo-2,8-diazaspiro[4,5]décane-8-carboxylique, [(S)-1-(1-cyanocyclopropylcarbamoyl)-3,3-difluorohexyl]-amide de l'acide 2-(4-méthoxyphényl)-2,8-diazaspiro[4,5]décane-8-carboxylique, [(S)-1-(1-cyanocyclopropylcarbamoyl)-3,3-difluorohexyl]-amide de l'acide 2-cyclopropyl-2,7-diazaspiro[3,5]nonane-7-carboxylique, [(S)-1-(1-cyanocyclopropylcarbamoyl)-3,3-difluorohexyl]-amide de l'acide 2-cyclopropyl-2,8-diazaspiro[4,5]décane-8-carboxylique, [(S)-1-(1-cyanocyclopropylcarbamoyl)-3,3-difluorohexyl]-amide de l'acide 9-cyclopropyl-3,9-diazaspiro[5,5]undécane-3-carboxylique, [(S)-1-(1-cyanocyclopropylcarbamoyl)-3,3-difluorohexyl]-amide de l'acide 7-cyclopropyl-2,7-diazaspiro[3,5]nonane-2-carboxylique, [(S)-1-(1-cyanocyclopropylcarbamoyl)-3,3-difluorohexyl]-amide de l'acide 7-propyl-2,7-diazaspiro[3,5]nonane-2-carboxylique, [(S)-1-(1-cyanocyclopropylcarbamoyl)-3,3-difluoro-4-phénylbutyl]-amide de l'acide 9-cyclopropyl-3,9-diazaspiro[5,5]undécane-3-carboxylique, [(S)-1-(1-cyanocyclopropylcarbamoyl)-3,3-difluoro-4-phénylbutyl]-amide de l'acide 2-cyclopropyl-2,7-diazaspiro[3,5]nonane-7-carboxylique, [(S)-1-(1-cyanocyclopropylcarbamoyl)-3,3-difluoro-4-phénylbutyl]-amide de l'acide 2-propyl-2,7-diazaspiro[3,5]nonane-7-carboxylique, [(S)-1-(1-cyanocyclopropylcarbamoyl)-3,3-difluoro-4-phénylbutyl]-amide de l'acide 7-cyclopropyl-2,7-diazaspiro[3,5]nonane-2-carboxylique, [(S)-1-(1-cyanocyclopropylcarbamoyl)-3,3-difluorobutyl]-amide de l'acide 6-azaspiro[2,5]octane-6-carboxylique, [(S)-1-(cyanométhylcarbamoyl)-3,3-difluorobutyl]-amide de l'acide 6-azaspiro[2,5]octane-6-carboxylique, [(S)-1-(1-cyanocyclopropylcarbamoyl)-3,3-difluorohexyl]-amide de l'acide 6-azaspiro[2,5]octane-6-carboxylique, [(S)-1-(4-cyano-1-méthylpipéridin-4-ylcarbamoyl)-3,3-difluorobutyl]-amide de l'acide 6-azaspiro[2,5]octane-6-carboxylique, [(S)-1-(4-cyano-1-méthylpipéridin-4-ylcarbamoyl)-3,3-difluorohexyl]-amide de l'acide 6-azaspiro[2,5]octane-6-carboxylique, [(S)-1-(1-cyanocyclopropylcarbamoyl)-3,3-difluoropentyl]-amide de l'acide 6-azaspiro[2,5]octane-6-carboxylique ou [(S)-1-(1-cyanocyclopropylcarbamoyl)-3,3-difluoro-4-phénylbutyl]-amide de l'acide 6-azaspiro[2,5]octane-6-carboxylique.

**7.** Procédé pour la préparation du composé de formule Ia et/ou d'une forme stéréo-isomère du composé de formule Ia et/ou d'un sel physiologiquement acceptable du composé de formule Ia et/ou d'un solvate ou d'un hydrate du composé de formule Ia et/ou d'un N-oxyde des composés de formule Ia selon l'une ou plusieurs des revendications 1 à 6, **caractérisé en ce qu'**on transforme

a) un composé de formule II

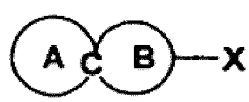

(II)

A et B étant définis comme dans le composé de formule Ia, avec un composé de formule IIIa ou IIIb ou IIIc,

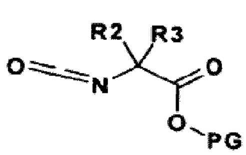

(IIIa)

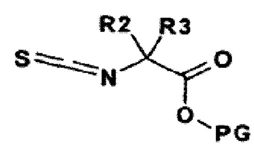

(IIIb)

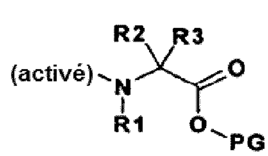

(IIIc)

X, R1, R2 et R3 ayant la signification comme dans le composé de formule Ia, PG signifiant un groupe de protection de la fonction ester et "activé" signifiant que l'amine se trouve sous une forme activée, par exemple sous forme de composé chlorocarbonyle, en un composé de formule IVa ou IVb,

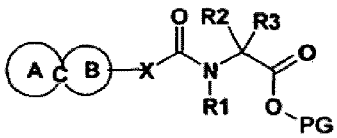 (IVa) 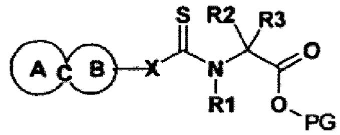 (IVb)

et on transforme les composés obtenus de formule IVa ou IVb, après conversion de l'ester en acide carboxylique, avec Z en composé de formule Ia, ou

b) un composé de formule Va ou Vb, A, B, X et Y ayant la signification comme dans le composé de formule Ia,

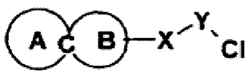 (Va) 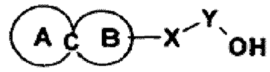 (Vb)

avec un composé de formule VI, R1, R2 et R3 ayant la signification comme dans le composé de formule Ia et PG représentant un groupe de protection de la fonction ester,

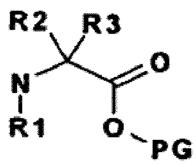 (VI)

en un composé de formule IVa ou IVb et on transforme le composé obtenu de formule IVa ou IVb, après conversion du groupe de protection de la fonction ester en acide carboxylique, avec Z en composé de formule Ia, ou

c) un composé de formule VIIa ou VIIb, A, B et X ayant la signification comme dans le composé de formule Ia,

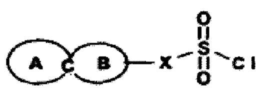 (VIIa) 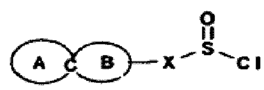 (VIIb)

avec un composé de formule VI

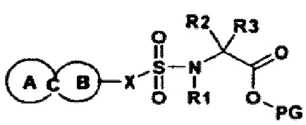 (VIIIa) 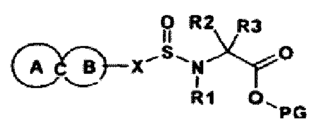 (VIIIb)

en un composé de formule VIIIa ou VIIIb et on transforme le composé obtenu de formule VIIIa ou VIIIb, après conversion de l'ester en acide carboxylique correspondant, avec Z en un composé de formule Ia, ou

d) un composé de formule IX

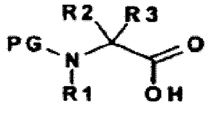 (IX)

avec une amine Z, Z ayant la signification comme dans le composé de formule Ia, en un composé de formule X,

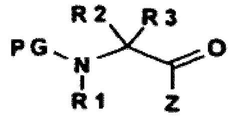 (X)

et on transforme ensuite le composé X ainsi obtenu, dans le sens d'une élimination d'un groupe de protection, en un composé de formule XI,

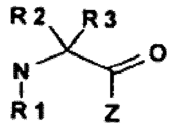

(XI)

et on transforme ce composé XI ensuite avec un composé Va ou Vb, comme indiqué sous b), en composé de formule Ia, ou

e) on sépare un composé de formule Ia, préparé selon les procédés a), b), c) ou d), ou un précurseur approprié de formule Ia, qui, en raison de sa structure chimique, apparaît sous des formes énantiomères ou diastéréo-isomères, par salification avec des acides ou des bases sous forme d'énantiomères purs, chromatographie sur des phases stationnaires chirales ou transformation en dérivés au moyen de composés sous forme d'énantiomères purs, tels que des aminoacides, séparation des diastéréo-isomères ainsi obtenus et élimination des groupes auxiliaires chiraux, en énantiomères purs, ou

f) on isole le composé de formule Ia, préparé selon les procédés a), b), c) ou d) sous forme libre ou on le libère à partir de sels physiologiquement non acceptables, ou, dans le cas de la présence de groupes acides ou basiques, on le convertit en sels physiologiquement acceptables, ou

g) on transforme le composé de formule Ia, préparé selon les procédés a), b), c) ou d) ou un précurseur approprié de formule Ia, qui est apte, en raison de sa structure chimique, à former un N-oxyde, en celui-ci ou, dans le cas de l'existence d'un N-oxyde, on transforme celui-ci en l'amine libre ou en sel d'une amine.

**8.** Médicament, **caractérisé par** un composé de formule Ia selon l'une ou plusieurs des revendications 1 à 6 avec un support, un additif et/ou d'autres substances actives et adjuvants pharmaceutiquement appropriéc(s) et physiologiquement acceptable(s).

**9.** Utilisation du composé de formule Ia selon l'une ou plusieurs des revendications 1 à 6 pour la préparation d'un médicament destiné à la prophylaxie, la prévention secondaire et la thérapie d'une dégradation anormalement augmentée des os, d'allergies, de la maladie d'Alzheimer, de l'amyloïdose, du syndrome de détresse respiratoire (ARDS), de la thrombose artérielle, de l'asthme, des athéromes, de l'athérosclérose, des maladies auto-immunes, des infections bactériennes, de la bronchiolite, d'une hémorragie cérébrale, d'une ischémie cérébrovasculaire, de la chorée de Huntington, d'inflammations chroniques, de la PDIA (polyradiculonévrite démyélinisante inflammatoire chronique), de la maladie de Creutzfeldt-Jakob, de la maladie de Crohn, du diabète, en particulier la forme juvénile, de l'emphysème, de l'encéphalomyélite, de l'endométriose, de maladies inflammatoires des voies respiratoires, de la pancréatite inflammatoire, de l'épilepsie, de maladies **caractérisées par** une angiogenèse renforcée, de l'élastolyse excessive des voies respiratoires, de transplantations de tissus, de la gingivite, de la glomérulonéphrite, de l'ostéoporose induite par les glucocorticoïdes, de la maladie de Graves, du syndrome de Guillain-Barré, de la thyroïdite d'Hashimoto, de l'hépatite, de l'infection par le VIH, de la maladie de Huntington, de l'hypercalcémie, de la maladie intestinale chronique inflammatoire, d'une immunodéficience, de la cystite interstitielle, d'une fracture osseuse, d'une perte osseuse, de maladies cancéreuses, du lupus érythémateux, de la malaria, de la leucodystrophie métachrone, de l'ostéogenèse avec métastases, de la formation de métastases, de la sclérose en plaques, du myélome multiple, de la dystrophie musculaire, de la myasthénie gravis, de maladies de neurodégénérescence, de la douleur neuropathique, de la neuropathie chronique ou diabétique, de la névralgie post-herpétique, de la névralgie du trijumeau, de la polyneuropathie diabétique douloureuse, de la douleur après une attaque, de la douleur post-amputation, de la douleur myélopathique ou radiculopathique, de la douleur atypique du visage et du syndrome analogue à une causalgie, d'un rejet d'organes lors de transplantation, de l'ostéoarthrite, de l'ostéogenèse imparfaite, de l'ostéoporose, de la maladie de Paget, de la pancréatite, de la maladie de Parkinson, du pemphigus vulgaire, de la parodontite, d'une rupture de plaque, d'une infection par Pneumocystisis Carinii, d'une pneumonie, du psoriasis, d'une resténose, de l'arthrite rhumatoïde, du scléroderme, du lupus érythémateux systémique, d'un traumatisme cérébral, d'un traumatisme de la moelle épinière, d'une invasion de cellules tumorales, d'infections virales, d'une perte de dents, du cancer du sein, du cancer de l'intestin, du cancer de l'ovaire, du cancer du col de l'utérus, du cancer de la peau, d'une tumeur cérébrale, du sarcome de Kaposi, d'une leucémie des cellules B et T, du cancer des poumons, du cancer des noeuds lymphatiques, du cancer du pancréas, du cancer et des sarcomes de la prostate.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9924460 A
- WO 200055125 A
- WO 2004052921 A
- WO 2005040142 A
- WO 0187838 A
- EP 0621267 A


### In der Beschreibung aufgeführte Nicht-Patentliteratur


- *Medicinal Chemistry,* 2005, vol. 1 (1), 71-104
- Handbook of Proteolytic Enzymes. Elsevier, 2004
- **A. BAICI et al.** *Seminars in Arthritis and Rheumatism,* 2005, vol. 34 (2), 24-28
- **D. BRÖMME et al.** *J. Biol. Chem.,* 1996, vol. 271, 2126-32
- **W.-S. HOU et al.** synoviale Fibroblastenvermittelter Collagen-Abbau durch Cathepsin K ist beschrieben. *Am. J. Pathol.,* 2001, vol. 159, 2167-2177
- **D. BRÖMME et al.** *Advanced Drug Delivery Reviews,* 2005, vol. 57, 973-993
- **W. KIM ; K. KANG.** *Expert Opin. Ther. Patents,* 2002, vol. 13 (3), 419-32
- **U. B. GRABOWSKA.** *Curr. Opin. Drug Disc Dev.,* 2005, vol. 8 (5), 619-30
- **R. L. THURMOND et al.** *Curr. Opin. Invest. Drugs,* 2005, vol. 6 (5), 473-482
- **M. BODANSZKY.** Principles of Peptide Synthesis. Springer, 1993
- **T. W. GREENE ; P. G. M. WUTS.** Protective Groups in Organic Synthesis. Wiley, 1999
- **G. SARTORI ; R. MAGGI.** Acyclic and cyclic ureas. *Science of Synthesis,* 2005, vol. 18, 665-758
- *J. Med. Chem.,* 2004, vol. 47 (8), 2037-61
- *Bull. Soc Chim. France,* 1964, vol. 10, 2572-81